# EUROPEAN PATENT APPLICATION

(11) **EP 4 813 991 A1**
(43) Date of publication of application: **30.09.2026**
(21) Application number: 24894706.1
(22) Date of filing: 22.11.2024
(51) Int. Cl.: C07D 487/04, A61K 31/5377, A61K 31/5386, A61K 31/5025, A61P 35/00, A61P 25/28, A61P 37/00, C07D 498/08, C07D 487/10

(54) **IMIDAZO [1,2-B] PYRIDAZINE-BASED NOVEL COMPOUND AS CDK INHIBITOR AND USE THEREOF**

(30) Priority: 24.11.2023 KR 20230165904
(71) Applicant: iLeadBMS Co., Ltd., Hwaseong-si, Gyeonggi-do 18469 (KR)
(72) Inventor: LEE, Yoon-Suk, Hwaseong-si, Gyeonggi-do 18469 (KR); KIM, Kyung-Sun, Hwaseong-si, Gyeonggi-do 18469 (KR); MOON, An-Na, Hwaseong-si, Gyeonggi-do 18469 (KR); SONG, Dong-Keun, Hwaseong-si, Gyeonggi-do 18469 (KR); KIM, Jeong-Ah, Hwaseong-si, Gyeonggi-do 18469 (KR)
(74) Representative: HGF
(86) International application number: PCT/KR2024/018695
(87) International publication number: WO 2025/110815

(57) **Abstract**

Provided are a novel compound, a use thereof for inhibiting the activity of CDK12 and/or CDK13 and degrading cyclin K, a pharmaceutical composition comprising the same, and a use thereof for the treatment of diseases associated with CDK12 and/or CDK13 and/or cyclin K, such as cancer. According to the present invention, the compounds exhibit excellent inhibitory activity and selectivity against CDK12 and/or CDK13, and induce degradation of cyclin K, and thus can be useful for the treatment of cancers such as breast cancer or gastric cancer.

## Description

### Technical Field

The present invention relates to a novel compound that inhibits the activity of cyclin dependent kinases (CDKs), such as CDK12 and/or CDK13, and degrades cyclin K. In addition, the present invention relates to a pharmaceutical composition comprising the compound of the present invention and a use thereof for the treatment of CDK-mediated diseases.

### Background Art

One of the key phenomena of cancer is uncontrolled cell growth, making targeted therapeutic agents that inhibit cell division effective in treating cancer. Cyclins and cyclin dependent kinases, a group of proteins, primarily control cell division (Mengna et al. CDK inhibitors in cancer therapy, an overview of recent development. 2021, Am J Cancer Res;11(5):1913-1935). Cyclin-dependent protein kinases (CDKs) are catalytic subunits of the serine/threonine protein kinase family. CDKs form complexes with cyclin proteins and activate them by phosphorylating serine or threonine residues in the matrix. Based on their cellular functions, CDKs can be classified into two categories: those that control the cell cycle and those that regulate cellular transcription.

The activity of CDK is regulated by specific associations with cyclin regulatory units, such as cyclin A, cyclin B, cyclin C, cyclin D, and cyclin E. For example, in mammalian cells, CDK1, in complex with cyclin A/B, regulates progression from G2 to M phase. CDK4 and CDK6, in complex with cyclin D, control progression from G1 to S phase, initiating DNA synthesis. CDK2, in complex with cyclin E/A, completes DNA synthesis in S phase. CDK7-9 form complexes with cyclins H, C, and T, respectively, to regulate gene transcription [Shigeaki et al. CDK1 inhibitor controls G2/M phase transition and reverses DNA damage sensitivity. 2021, Biochem Biophys Res Commun. Apr 23;550:56-61], [Manuel et al. A CDK4/6-dependent phosphorylation gradient regulates the early to late G1 phase transition. 2021, Sci Rep. Jul 19;11(1):14736], [Stefan et al. Structural basis for CDK7 activation by MAT1 and Cyclin H. 2020, Proc Natl Acad Sci USA. Oct 27;117(43):26739-26748].

CDK12 and its ortholog, CDK13, belong to the CDK family, which regulates transcriptional and post-transcriptional processes. Among CDKs, genetic mutations in CDK12 are reported to be relatively high in various carcinomas. Furthermore, CDK12 and CDK13 form a complex with their cyclin partner, cyclin K, and phosphorylate the C-terminal domain of RNA polymerase II, playing a crucial role in regulating kinase activity. In addition, CDK12 is known to regulate major signal transduction pathways related to cancer induction and DNA damage response, and inhibition of these pathways is expected to have an anticancer effect. SR-4835 (International Publication No. WO 2019/217421) is a representative CDK12/CDK13 inhibitor known to date, and numerous experiments have been reported to confirm its anticancer effects (Victor et al. Therapeutic Targeting of CDK12/CDK13 in Triple-Negative Breast Cancer. 2019, Cancer Cell. Nov 11;36(5):545-558.e7). However, there is a need in the art to develop CDK12/13 inhibitors with improved efficacy and selectivity compared to existing agents.

### Detailed Description of Invention

### Technical Problem

An object of the present invention is to provide a compound of Formula I below, a stereoisomer, hydrate, solvate, or pharmaceutically acceptable salt thereof that inhibits the activity of CDKs, such as CDK12 and/or CDK13, and degrades cyclin K: 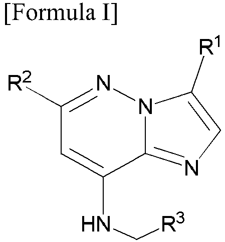 in Formula I above,
R¹ is halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-, wherein any C₁-C₆ alkoxy group, C₁-C₆ alkyl group, C₂-C₆ alkenyl group, C₂-C₆ alkynyl group, and C₃-C₆ cycloalkyl group in R¹ may be optionally substituted with halo, hydroxy, or cyano;
R² is C₆-C₁₀ aryl; a 5- or 6-membered heteroaryl containing one to two nitrogen atoms; or a monocyclic or bridged or spiro 4- to 12-membered heterocyclyl containing one nitrogen atom and linked to the imidazopyridazine ring of Formula I via said nitrogen atom, wherein the heterocyclyl may optionally contain one additional nitrogen atom or one oxygen atom, and
R² may be optionally substituted with one or more substituents selected from the group consisting of:
   (i) H, halo, hydroxy, cyano, C₁-C₆ alkyl, C₃-C₆ cycloalkyl,
   (ii) C₁-C₆ alkyl substituted with halo, hydroxy, or cyano,
   (iii) C₃-C₆ cycloalkyl substituted with halo, hydroxy, or cyano,
   (iv) amino, (C₁-C₆ alkyl)amino, di(C₁-C₆ alkyl)amino, (C₃-C₆ cycloalkyl)amino, (C₁-C₆ hydroxyalkyl)amino, (C₁-C₆ alkyl)carbonylamino, (C₁-C₆ hydroxyalkyl)carbonylamino, or (C₁-C₆ alkoxy)(C₁-C₆ alkyl)carbonylamino, and
   (v) (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, carboxy, or (C₁-C₆ alkoxy)carbonyl; and
R³ is 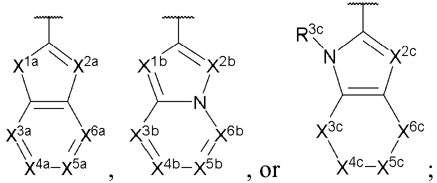
X^{1a} is NR^{3a}, O, or S, and X^{2a} is N or CR^{3a}, provided that if X^{2a} is CR^{3a}, then X^{1a} is NR^{3a};
one of X^{1b} and X^{2b} is N, and the other is CR^{3b};
X^{2c} is N or CR^{3c};
not more than one of X^{3a}, X^{4a}, X^{5a}, and X^{6a} is N, and the rest are CR^{3a};
not more than one of X^{3b}, X^{4b}, X^{5b}, and X^{6b} is N, and the rest are CR^{3e};
not more than one of X^{3c}, X^{4c}, X^{5c} and X^{6c} is NR^{3f}, and the rest are CR^{3f}R^{3g};
R^{3a}, R^{3b}, and R^{3c} are each independently H or C₁-C₆ alkyl; and
R^{3d}, R^{3e}, R^{3f}, and R^{3g} are each independently H, halo, cyano, hydroxy, amino, (C₁-C₆ alkyl)amino, di(C₁-C₆ alkyl)amino, C₁-C₆ alkoxy, or C₁-C₆ alkyl, wherein any C₁-C₆ alkoxy group and C₁-C₆ alkyl group in R^{3d}, R^{3e}, R^{3f}, and R^{3g} may be optionally substituted with halo, cyano, or hydroxy.

Another object of the present invention is to provide a pharmaceutical composition comprising a novel compound that inhibits the activity of CDK12 and/or CDK13 and degrades cyclin K, for example, a pharmaceutical composition for the treatment of cancer.

Another object of the present invention is to provide a method for inhibiting CDK12 and/or CDK13 and degrading cyclin K using a novel compound that inhibits the activity of CDK12 and/or CDK13 and degrades cyclin K; and a use of thereof for inhibiting CDK12 and/or CDK13 and degrading cyclin K.

Another object of the present invention is to provide a method for treating diseases associated with CDK12 and/or CDK13 and/or cyclin K, such as cancer, using a novel compound that inhibits the activity of CDK12 and/or CDK13 and degrades cyclin K.

### Solution to Problem

Each description and embodiment disclosed in the present application may also be applied to each other description and embodiment. That is, all combinations of said various elements disclosed in the present application fall within the scope of the present application. In addition, the scope of the present application cannot be considered limited by the specific description described below.

In one aspect of the present invention, there is provided a compound of Formula I below, a stereoisomer, hydrate, solvate, or pharmaceutically acceptable salt thereof: 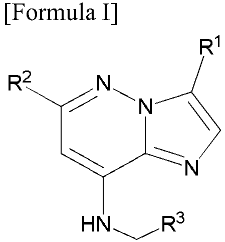 in Formula I herein, R¹ is halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-. In one embodiment, R¹, or any C₁-C₆ alkoxy group, C₁-C₆ alkyl group, C₂-C₆ alkenyl group, C₂-C₆ alkynyl group, and C₃-C₆ cycloalkyl group included in R¹ may be each independently optionally substituted with halo, hydroxy, or cyano.

In one embodiment, R¹ may be halo, C₁-C₆ alkyl optionally substituted with halo, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-. For example, R¹ may be ethynyl, methylethynyl, cyclopropylethynyl, methyl, ethyl, isopropyl, cyclopropyl, trifluoromethyl, trifluoroethyl, iodo, or chloro.

In one embodiment, R¹ may be C₁-C₆ haloalkyl, such as CF₃.

In Formula I herein, R² is C₆-C₁₀ aryl; a 5- or 6-membered heteroaryl containing one to two nitrogen atoms; or a monocyclic or bridged or spiro 4- to 12-membered heterocyclyl containing one nitrogen atom and linked to the imidazopyridazine ring of Formula I via said nitrogen atom.

In one embodiment, R² in Formula I may be a monocyclic or bridged or spiro 4- to 12-membered heterocyclyl. The 4- to 12-membered heterocyclyl may contain one nitrogen atom and may be linked to the imidazopyridazine ring of Formula I via said nitrogen atom. In one embodiment, the heterocyclyl may optionally contain one additional nitrogen atom or one oxygen atom.

In one embodiment, the 4- to 12-membered heterocyclyl may be azetidinyl, pyrrolidinyl, morpholinyl, piperidinyl, or piperazinyl. In one embodiment, any two non-adjacent carbon atoms of said morpholinyl, piperidinyl, or piperazinyl may be optionally linked to each other by a C₁-C₃ alkylene group to form a bridged ring. For example, the bridged heterocyclyl may be 3-oxa-8-azabicyclo[3.2.1]octan-8-yl, 8-oxa-3-azabicyclo[3.2.1]octan-3-yl, 3,8-diazabicyclo[3.2.1]octan-3-yl, 3,8-diazabicyclo[3.2.1]octan-8-yl, 2-oxa-5-azabicyclo[2.2.2]octan-5-yl, 8-azabicyclo[3.2.1]octan-8-yl, 3-azabicyclo[3.1.1]heptan-3-yl, 6-oxa-3-azabicyclo[3.1.1]heptan-3-yl, 3,6-diazabicyclo[3.1.1]heptan-3-yl, or 3,6-diazabicyclo[3.1.1]heptan-6-yl, but is not limited thereto.

Alternatively, the 4- to 12-membered heterocyclyl may be a 7- to 11-membered azaspiro ring which contains one nitrogen atom linked to the imidazopyridazine ring of Formula I and may optionally contain one additional nitrogen atom or one oxygen atom. For example, the 7- to 11-membered azaspiro ring may be 2-azaspiro[3.3]heptan-2-yl, 2,6-diazaspiro[3.3]heptan-2-yl, 6-oxa-2-azaspiro[3.3]heptan-2-yl, 2-azaspiro[3.4]octan-2-yl, 2,6-diazaspiro[3.4]octan-2-yl, 6-oxa-2-azaspiro[3.4]octan-2-yl, 6-azaspiro[3.4]octan-6-yl, 2,6-diazaspiro[3.4]octan-6-yl, 2-oxa-6-azaspiro[3.4]octan-6-yl, 2-azaspiro[4.4]nonan-2-yl, 2,7-diazaspiro[4.4]nonan-2-yl, or 2-oxa-7-azaspiro[4.4]nonan-7-yl. For example, the azaspiro ring may include 2-azaspiro[3.3]heptan-2-yl, 2,6-diazaspiro[3.3]heptan-2-yl, 6-oxa-2-azaspiro[3.3]heptan-2-yl, but is not limited thereto.

Alternatively, R² in Formula I herein may be a 5- or 6-membered heteroaryl containing one or two nitrogen atoms. In one embodiment, the 5- or 6-membered heteroaryl may be pyrimidinyl, pyridinyl, pyrrolyl, or imidazolyl. For example, the heteroaryl may be pyrimidinyl.

Alternatively, R² in Formula I herein may be C₆-C₁₀ aryl. For example, the C₆-C₁₀ aryl may be phenyl or naphthyl. For example, the C₆-C₁₀ aryl may be phenyl.

In Formula I above, R² may be optionally substituted with one or more substituents selected from the group consisting of:
(i) H, halo, hydroxy, cyano, C₁-C₆ alkyl, C₃-C₆ cycloalkyl,
(ii) C₁-C₆ alkyl substituted with halo, hydroxy, or cyano,
(iii) C₃-C₆ cycloalkyl substituted with halo, hydroxy, or cyano,
(iv) amino, (C₁-C₆ alkyl)amino, di(C₁-C₆ alkyl)amino, (C₃-C₆ cycloalkyl)amino, (C₁-C₆ hydroxyalkyl)amino, (C₁-C₆ alkyl)carbonylamino, (C₁-C₆ hydroxyalkyl)carbonylamino, or (C₁-C₆ alkoxy)(C₁-C₆ alkyl)carbonylamino, and
(v) (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, carboxy, or (C₁-C₆ alkoxy)carbonyl.

In one embodiment, R² in Formula I may not be substituted with the substituents listed in (i) to (v) above. Alternatively, R² in Formula I may be substituted with one or two substituents independently selected from the substituents listed in (i) to (iv) above.

In one embodiment, R² in Formula I above may be substituted with H, hydroxy, cyano, C₁-C₆ alkyl, C₃-C₆ cycloalkyl, hydroxy-(C₁-C₆ alkyl), hydroxy-(C₃-C₆)cycloalkyl, amino, (C₁-C₆ alkyl)amino, (C₃-C₆ cycloalkyl)amino, (C₁-C₆ hydroxyalkyl)carbonylamino, (C₁-C₆ alkyl)carbonyl, (C₁-C₆ hydroxyalkyl)carbonyl, aminocarbonyl, (C₁-C₆ alkyl)aminocarbonyl, or carboxy, but is not limited thereto. For example, R² in Formula I above may be substituted with H, hydroxy, cyano, methyl, ethyl, cyclopropyl, cyclobutyl, hydroxymethyl, hydroxyethyl, 2-hydroxyisopropyl, 1-hydroxycyclopropyl, 1-hydroxycyclobutyl, amino, methylamino, ethylamino, cyclopropylamino, hydroxyacetamido, methylcarbonyl(i.e., acetyl), hydroxyacetyl, aminocarbonyl, carbamoyl, or carboxy, but is not limited thereto.

In one embodiment, R² in Formula I herein is C₆-C₁₀ aryl, wherein the aryl may be optionally substituted with R^{2d} selected from the group consisting of H, hydroxy, amino, (C₁-C₆ alkyl)amino, di(C₁-C₆ alkyl)amino, and (C₃-C₆ cycloalkyl)amino. For example, R² may be phenyl, and R^{2d} may be hydroxy or amino.

In one embodiment, R² in Formula I herein is a 5- or 6-membered heteroaryl containing one to two nitrogen atoms, wherein the heteroaryl may be optionally substituted with R^{2d} selected from the group consisting of H, hydroxy, amino, (C₁-C₆ alkyl)amino, di(C₁-C₆ alkyl)amino, and (C₃-C₆ cycloalkyl)amino. For example, R² may be pyridinyl or pyrimidinyl, and R^{2d} may be hydroxy or amino.

In one embodiment, R² in Formula I herein may be selected from Formula A or Formula B below: 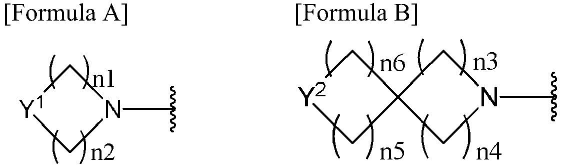 in Formula A above, n1 and n2 are each independently 1 or 2. In one embodiment, the carbon atoms constituting the ring of Formula A may be optionally substituted with C₁-C₆ alkyl.

In Formula A above, if both n1 and n2 are 2, then Y¹ may be CR^{2a}R^{2b}, NR^{2c}, or O. In this case, any two non-adjacent carbon atoms in the ring of Formula A may be optionally linked to each other by a C₁-C₃ alkylene group to form a bridged ring. Alternatively, if one or both of n1 and n2 are 1, then Y¹ is CR^{2a}R^{2b},

In Formula B above, n3, n4, n5 and n6 are each independently 1 or 2. For example, n3, n4, n5 and n6 may all be 1. In one embodiment, the carbon atoms constituting the ring of Formula B may be optionally substituted with C₁-C₆ alkyl. In Formula B above, Y² is CR^{2a}R^{2b}, NR^{2c}, or O.

In Formulae A and B above, R^{2a} and R^{2b} may be each independently selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy.

In one embodiment, in Formulae A and B above, one of R^{2a} and R^{2b} may be selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy. In this case, the other of R^{2a} and R^{2b} may be H, halo, hydroxy, cyano, or C₁-C₆ alkyl.

For example, in Formulae A and B above, R^{2a} and R^{2b} may be H, hydroxy, amino, cyclopropylamino, hydroxyacetamido, methyl, hydroxymethyl, 2-hydroxy-isopropyl, hydroxycyclopropyl, carboxy, carbamoyl, hydroxyacetyl, or cyano, but are not limited thereto.

In Formulae A and B above, R^{2c} may be H, C₁-C₆ alkyl, C₃-C₆ cycloalkyl, (C₁-C₆ alkyl)carbonyl, (C₁-C₆ hydroxyalkyl)carbonyl, aminocarbonyl, (C₁-C₆ alkyl)aminocarbonyl, or di(C₁-C₆ alkyl)aminocarbonyl. For example, R^{2c} may be H, methyl, cyclopropyl, acetyl, hydroxyacetyl, or carbamoyl, but is not limited thereto.

In one embodiment, R² in Formula I herein may be selected from the group consisting of:

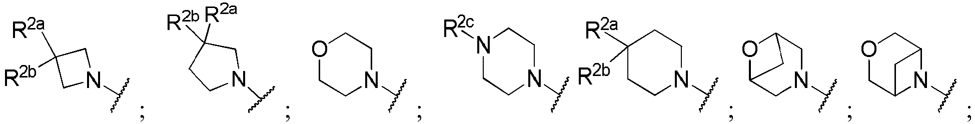

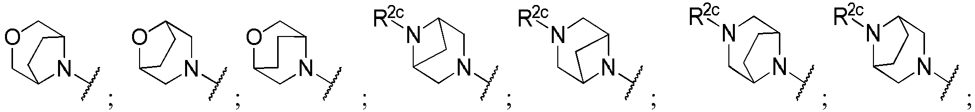

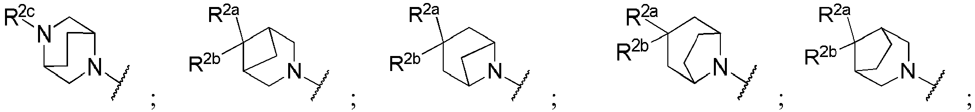

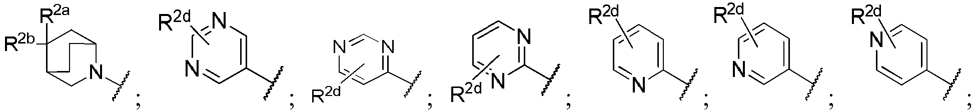

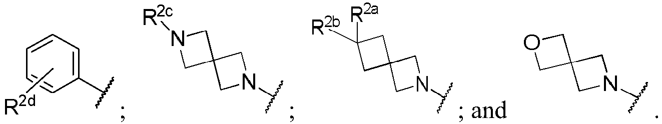

In the structure of R², R^{2a}, R^{2b}, R^{2c}, and R^{2d} are the same as described above for Formulae A and B.

For example, R² in Formula I herein may be selected from the group consisting of:

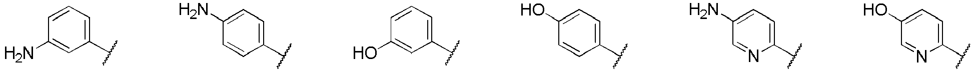

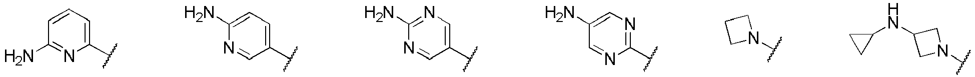

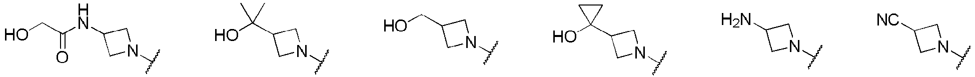

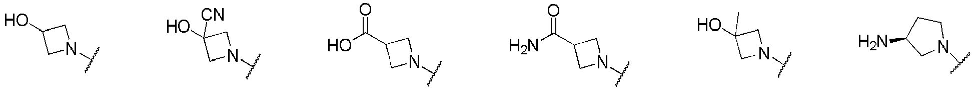

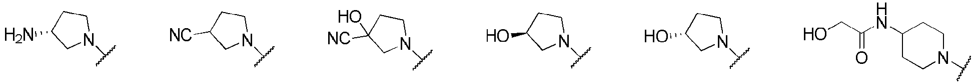

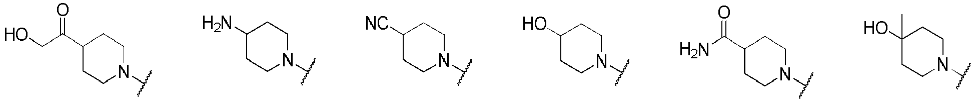

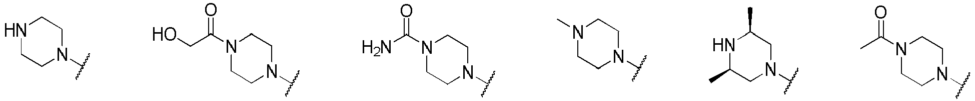

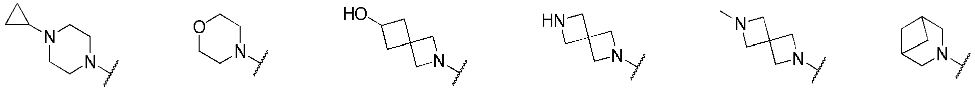

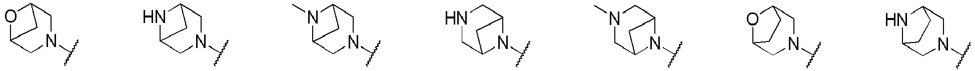

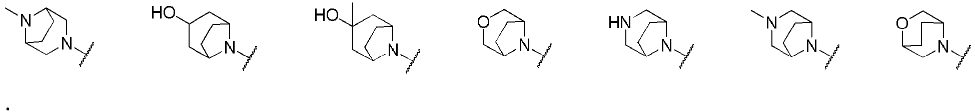

In Formula I herein, R³ is selected from the group consisting of:

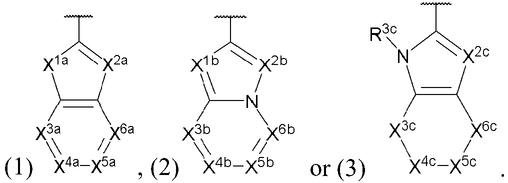

In (1), X^{1a} is NR^{3a}, O, or S, and X^{2a} is N or CR^{3a}, provided that if X^{2a} is CR^{3a}, then X^{1a} is NR^{3a}. In addition, not more than one of X^{3a}, X^{4a}, X^{5a}, and X^{6a} is N, and the rest are CR^{3d}.

In (2), one of X^{1b} and X^{2b} is N, and the other is CR^{3b}. In addition, not more than one of X^{3b}, X^{4b}, X^{5b}, and X^{6b} is N, and the rest are CR^{3e}.

In (3), X^{2c} is N or CR^{3c}. In addition, not more than one of X^{3c}, X^{4c}, X^{5c} and X^{6c} is NR^{3f}, and the rest are CR^{3f}R^{3g},

In (1) to (3), R^{3a}, R^{3b}, and R^{3c} are each independently H or C₁-C₆ alkyl. In addition, R^{3a}, R^{3e}, R^{3f}, and R^{3g} are each independently H, halo, cyano, hydroxy, amino, (C₁-C₆ alkyl)amino, di(C₁-C₆ alkyl)amino, C₁-C₆ alkoxy, or C₁-C₆ alkyl. In this case, any C₁-C₆ alkoxy group and C₁-C₆ alkyl group in R^{3d}, R^{3e}, R^{3f}, and R^{3g} may be optionally substituted with halo, cyano, or hydroxy.

In one embodiment, R³ in Formula I herein may be selected from the group consisting of:

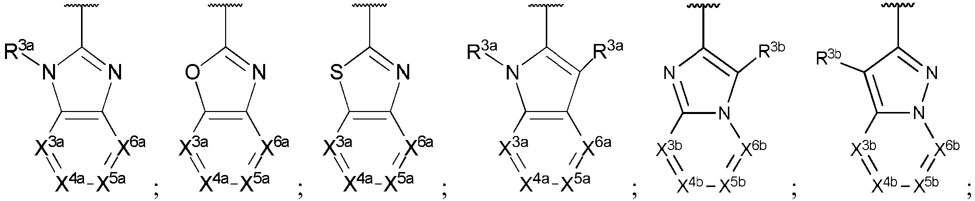

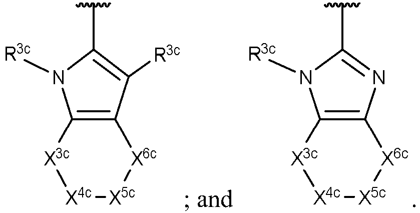

**In the structure** of R³, not more than one of X^{3a}, X^{4a}, X^{5a}, and X^{6a} is N, and the rest are CR^{3d}. Not more than one of X^{3b}, X^{4b}, X^{5b}, and X^{6b} is N, and the rest are CR^{3e}. Not more than one of X^{3c}, X^{4c}, X^{5c} and X^{6c} is NR^{3f}, and the rest are CR^{3f}R^{3g},

In this case, R^{3a}, R^{3b}, and R^{3c} are each independently H or C₁-C₆ alkyl. R^{3d}, R^{3e}, R^{3f}, and R^{3g} are each independently H, halo, cyano, hydroxy, amino, (C₁-C₆ alkyl)amino, di(C₁-C₆ alkyl)amino, C₁-C₆ alkoxy, or C₁-C₆ alkyl. Any C₁-C₆ alkoxy group and C₁-C₆ alkyl group in R^{3a}, R^{3e}, R^{3f}, and R^{3g} may be optionally substituted with halo, cyano, or hydroxy. In one embodiment, R^{3d}, R^{3e}, R^{3f}, and R^{3g} may be H, halo, hydroxy, amino, C₁-C₆ alkoxy, C₁-C₆ alkyl, or C₁-C₆ haloalkyl. For example, R^{3d}, R^{3e}, R^{3f}, and R^{3g} may be each independently H, F, Cl, hydroxy, amino, methoxy, methyl, and trifluoromethyl. For example, R^{3d}, R^{3e}, R^{3f}, and R^{3g} may be each independently halogen, such as F or Cl.

In one embodiment, R³ may be selected from the group consisting of:

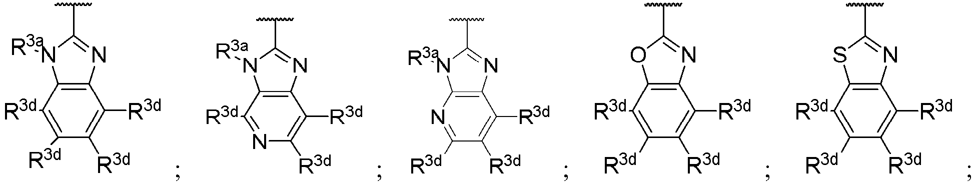

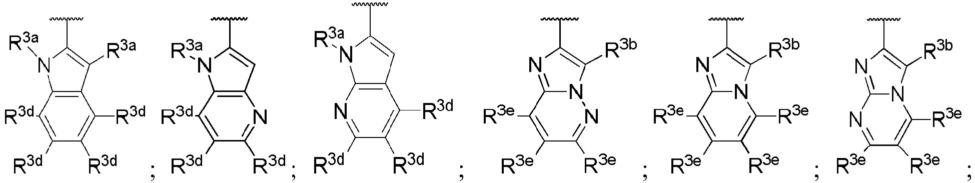

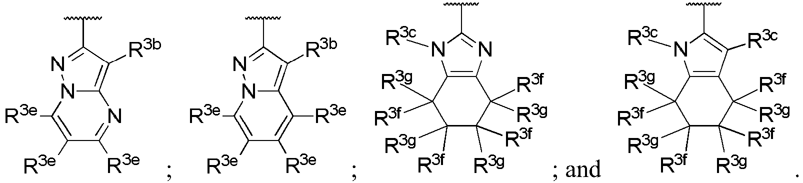

In the structure of R³, R^{3a}, R^{3b}, and R^{3c} are each independently H or C₁-C₆ alkyl.

In the structure of R³, R^{3d}, R^{3e}, R^{3f}, and R^{3g} are each independently H, halo, hydroxy, amino, C₁-C₆ alkoxy, C₁-C₆ alkyl, or C₁-C₆ haloalkyl. For example, R^{3d}, R^{3e}, R^{3f}, and R^{3g} may be each independently H, F, Cl, hydroxy, amino, methoxy, methyl, and trifluoromethyl.

For example, R³ in Formula I herein may be selected from the group consisting of:

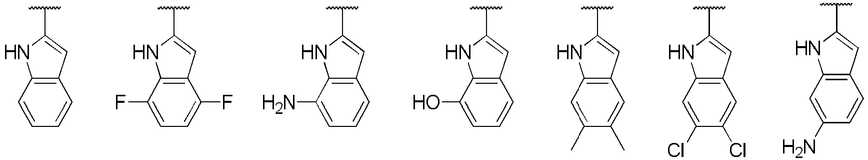

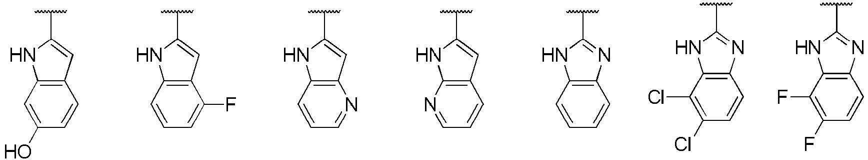

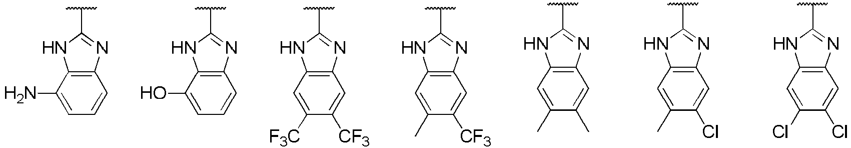

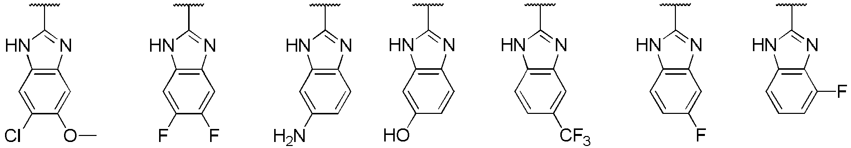

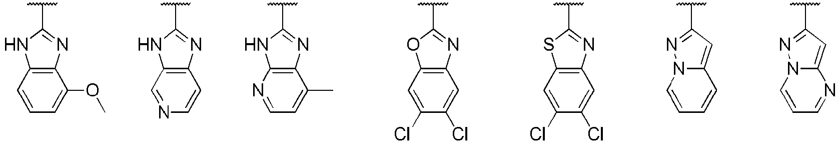

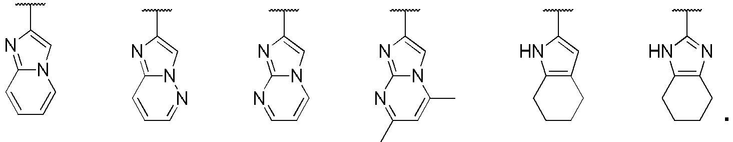

In one embodiment, the compound of Formula I of the present invention may be a compound of Formula IA below: 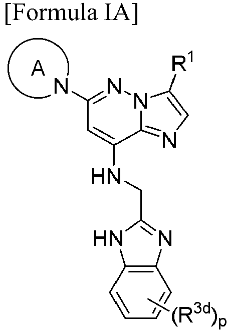 in Formula IA above, R¹ may be halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-. R¹, or any C₁-C₆ alkoxy group, C₁-C₆ alkyl group, C₂-C₆ alkenyl group, C₂-C₆ alkynyl group, and C₃-C₆ cycloalkyl group included in R¹ may be optionally substituted with halo, hydroxy, or cyano. The various examples of R¹ described above for Formula I may also be applied to Formula IA.

In Formula IA above, Ring A is a monocyclic or bridged or spiro 4- to 12-membered heterocyclyl which may optionally contain one additional nitrogen atom or one oxygen atom. The various examples of the monocyclic or bridged or spiro 4- to 12-membered heterocyclyl described above for R² of Formula I may also be applied to Formula IA.

In Formula IA above, Ring A may be optionally substituted with one or more substituents selected from the group consisting of:
(i) H, halo, hydroxy, cyano, C₁-C₆ alkyl, C₃-C₆ cycloalkyl,
(ii) C₁-C₆ alkyl substituted with halo, hydroxy, or cyano,
(iii) C₃-C₆ cycloalkyl substituted with halo, hydroxy, or cyano,
(iv) amino, (C₁-C₆ alkyl)amino, di(C₁-C₆ alkyl)amino, (C₃-C₆ cycloalkyl)amino, (C₁-C₆ hydroxyalkyl)amino, (C₁-C₆ alkyl)carbonylamino, (C₁-C₆ hydroxyalkyl)carbonylamino, or (C₁-C₆ alkoxy)(C₁-C₆ alkyl)carbonylamino, and
(v) (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, carboxy, or (C₁-C₆ alkoxy)carbonyl.

The various examples described above for the substituents of R² in Formula I and the corresponding substituents in Formulae A and B may also be applied to Formula IA.

In Formula IA above, R^{3d} is H, halo, cyano, hydroxy, amino, (C₁-C₆ alkyl)amino, di(C₁-C₆ alkyl)amino, C₁-C₆ alkoxy, or C₁-C₆ alkyl. In this case, any C₁-C₆ alkoxy group and C₁-C₆ alkyl group in R^{3d} may be optionally substituted with halo, cyano, or hydroxy. The various examples described above for R^{3d} in Formula I may also be applied to Formula IA.

In Formula IA above, p may be 0, 1, 2 or 3. In one embodiment, p may be 0, 1 or 2.

In one embodiment, the compound of Formula I of the present invention may be a compound of Formula IB below: 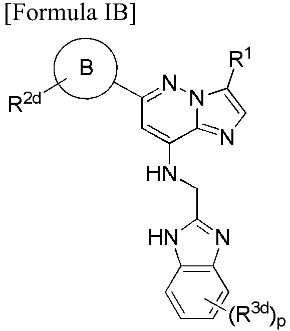 in Formula IB above, R¹ may be halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-. R¹, or any C₁-C₆ alkoxy group, C₁-C₆ alkyl group, C₂-C₆ alkenyl group, C₂-C₆ alkynyl group, and C₃-C₆ cycloalkyl group included in R¹ may be optionally substituted with halo, hydroxy, or cyano. The various examples of R¹ described above for Formula I may also be applied to Formula IB.

In Formula IB above, Ring B is selected from the group consisting of: 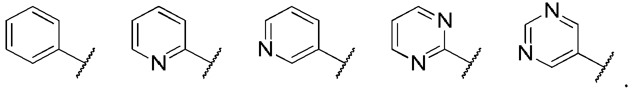 In one embodiment, Ring B is

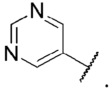

In Formula IB above, R^{2d} may be H, hydroxy, amino, (C₁-C₆ alkyl)amino, di(C₁-C₆ alkyl)amino, or (C₃-C₆ cycloalkyl)amino. For example, R^{2d} may include hydroxy or amino, but is not limited thereto.

In Formula IB above, R^{3d} is H, halo, cyano, hydroxy, C₁-C₆ alkoxy, or C₁-C₆ alkyl. In this case, any C₁-C₆ alkoxy group and C₁-C₆ alkyl group in R^{3d} may be optionally substituted with halo, cyano, or hydroxy. The various examples described above for R^{3d} of Formula I may also be applied to Formula IB.

In Formula IB above, p may be 0, 1, 2 or 3. In one embodiment, p may be 0, 1 or 2.

For example, in Formula IB above, 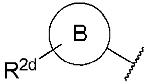 may be 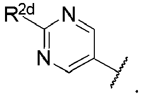 R^{2d} is the same as described above for Formula IB. For example, R^{2d} may be amino, (C₁-C₆ alkyl)amino, or di(C₁-C₆ alkyl)amino.

In one embodiment, the compound of Formula I of the present invention may be a compound of Formula IC-1 or Formula IC-2 below:

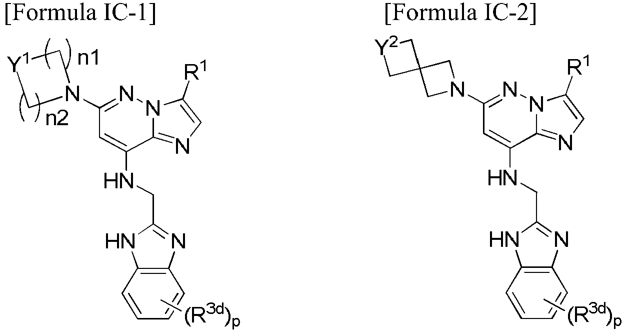

In Formula IC-1 above, n1 and n2 are each independently 1 or 2. A carbon atom of the ring containing Y¹ and N may be optionally substituted with C₁-C₆ alkyl. In one embodiment, if both n1 and n2 are 2, then Y¹ is CR^{2a}R^{2b}, NR^{2c}, or O. Alternatively, if one or both of n1 and n2 are 1, then Y¹ is CR^{2a}R^{2b},

In Formula IC-2 above, Y² is CR^{2a}R^{2b}, NR^{2c}, or O.

In Formulae IC-1 and IC-2 above, R¹ is halo, hydroxy, cyano, C₁-C₆ alkoxy, C₁-C₆ alkyl, or C₁-C₆ haloalkyl.

In Formulae IC-1 and IC-2 above, R^{3d} is each independently H, halo, hydroxy, cyano, C₁-C₆ alkoxy, C₁-C₆ alkyl, or C₁-C₆ haloalkyl.

In Formulae IC-1 and IC-2 above, p is an integer from 0 to 2. For example, p is 2.

In Formulae IC-1 and IC-2 above, R^{2a} and R^{2b} are each independently selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy.

In Formulae IC-1 and IC-2 above, R^{2c} is H, C₁-C₆ alkyl, C₃-C₆ cycloalkyl, (C₁-C₆ alkyl)carbonyl, (C₁-C₆ hydroxyalkyl)carbonyl, aminocarbonyl, (C₁-C₆ alkyl)aminocarbonyl, or di(C₁-C₆ alkyl)aminocarbonyl.

With respect to n1, n2, Y¹, Y², R¹, R^{2a}, R^{2b}, R^{2c}, R^{3d}, and p in Formula IC-1 or Formula IC-2, the specific examples described above for Formula 1, Formula A, Formula B, Formula IA, and Formula IB may also be applied to the corresponding variables and structures of Formula IC-1 or Formula IC-2.

The compound of Formula I may be a compound selected from the group consisting of:

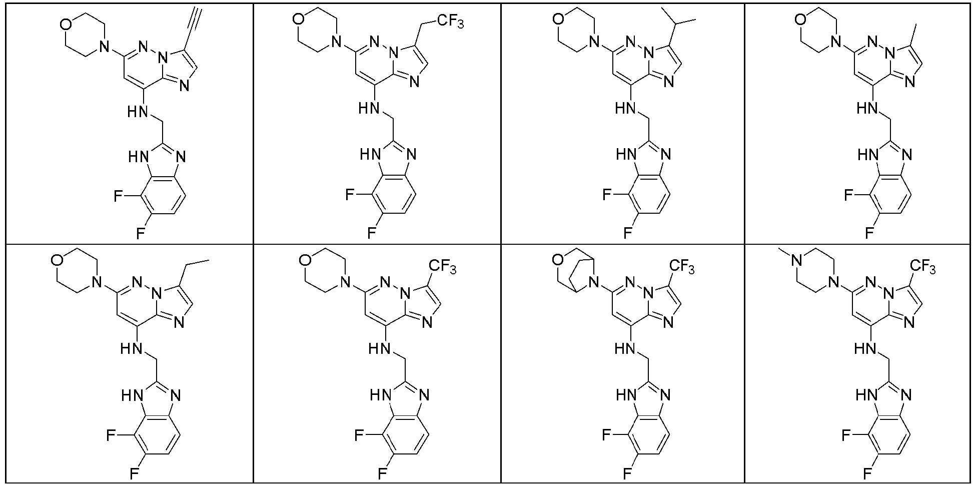

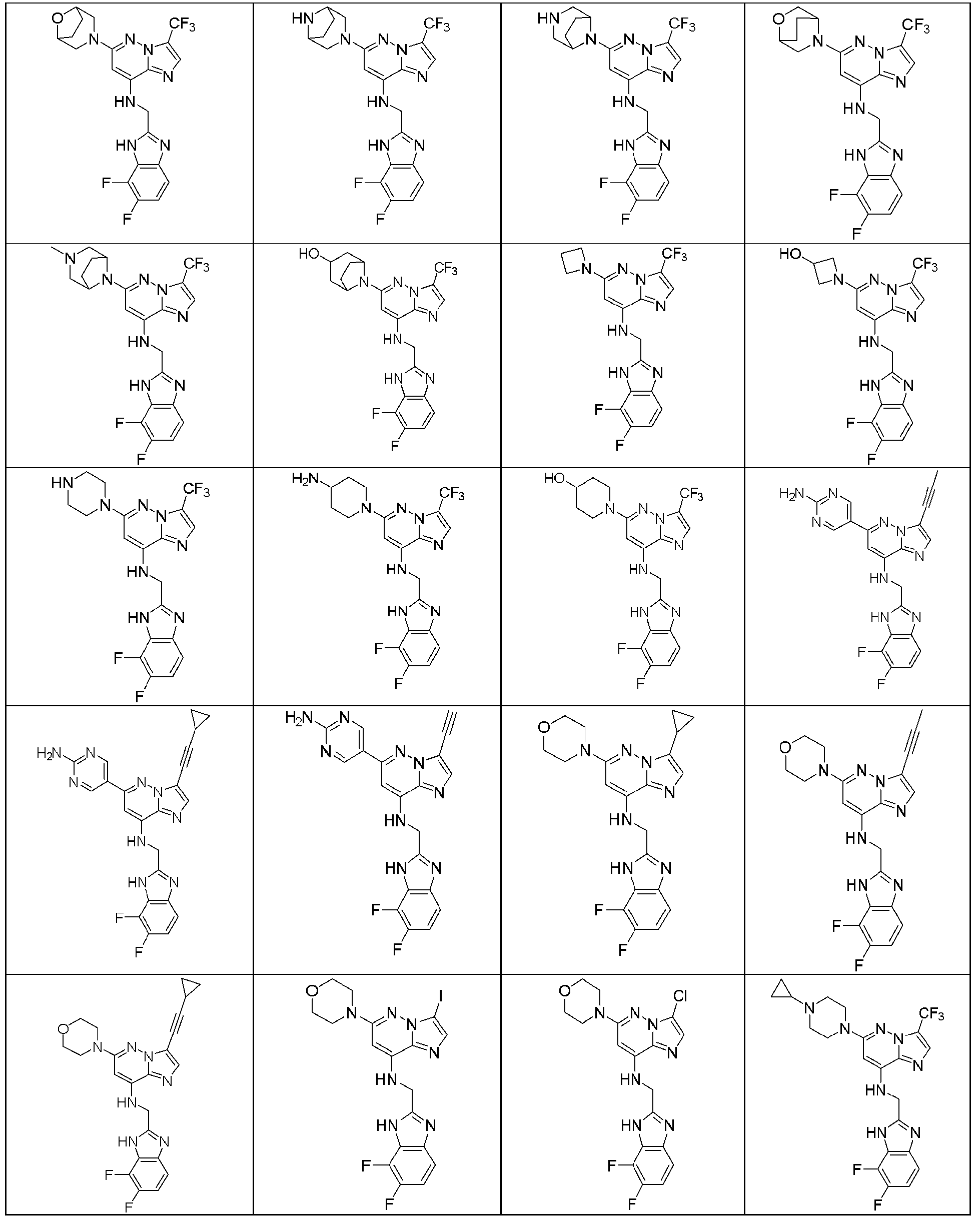

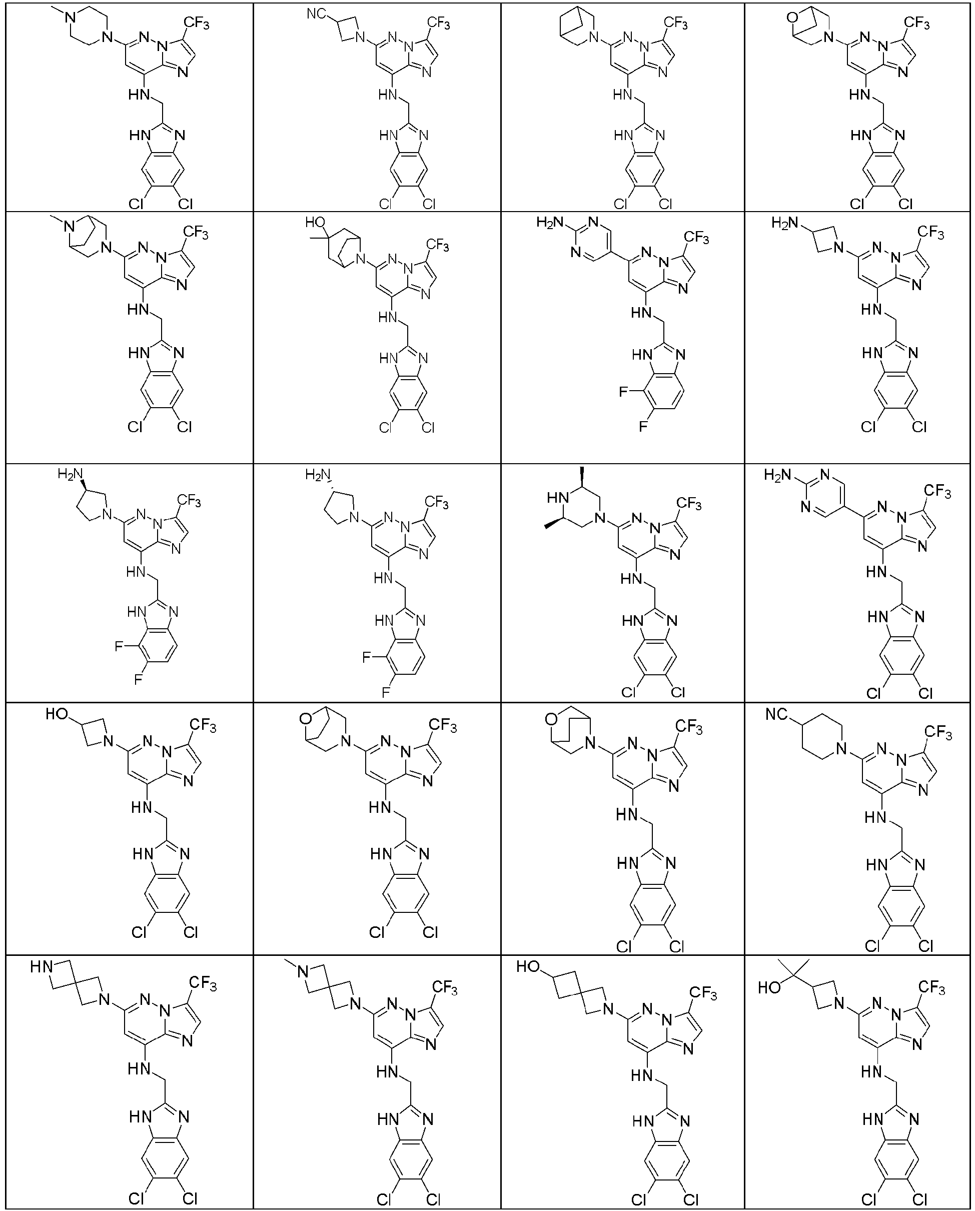

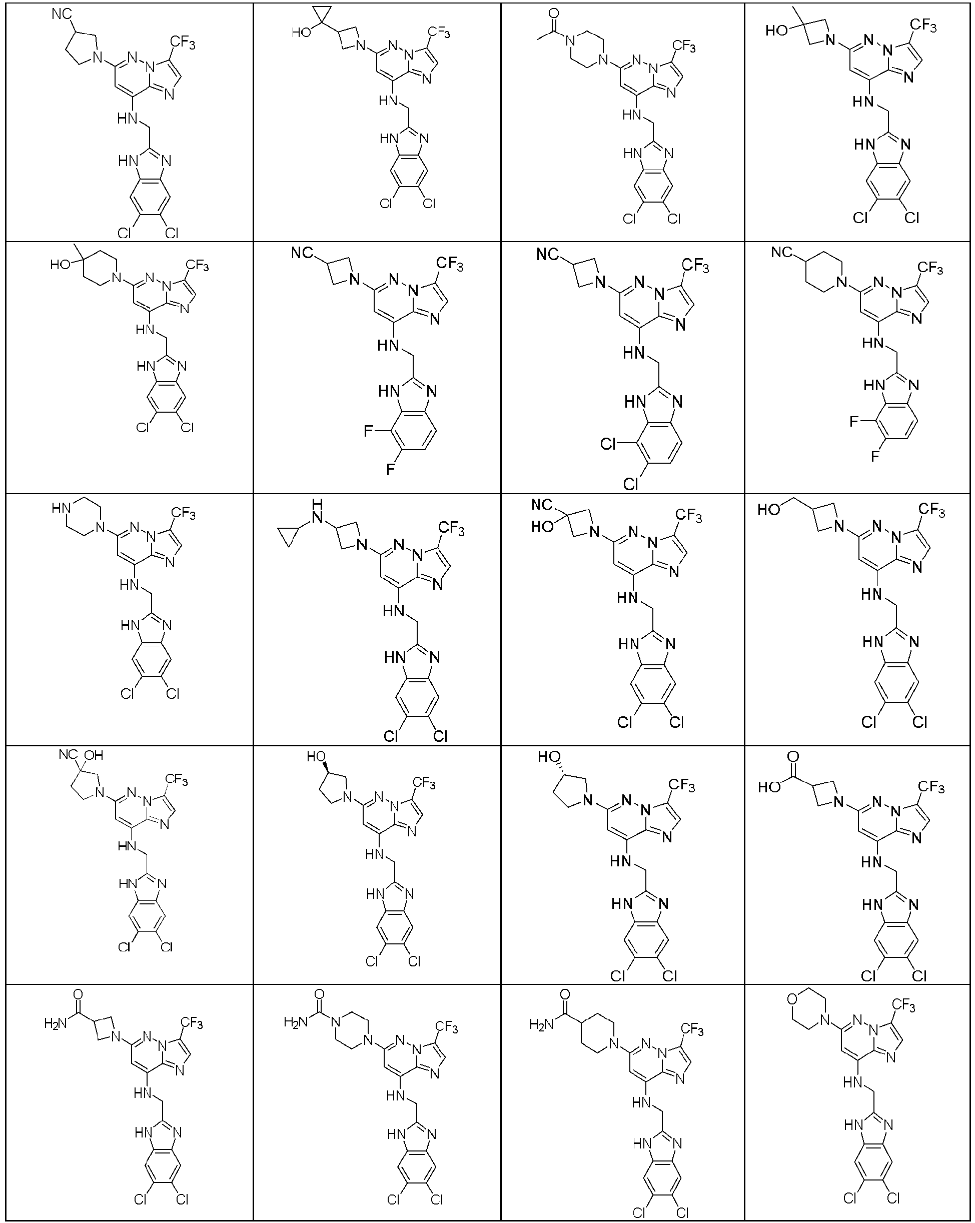

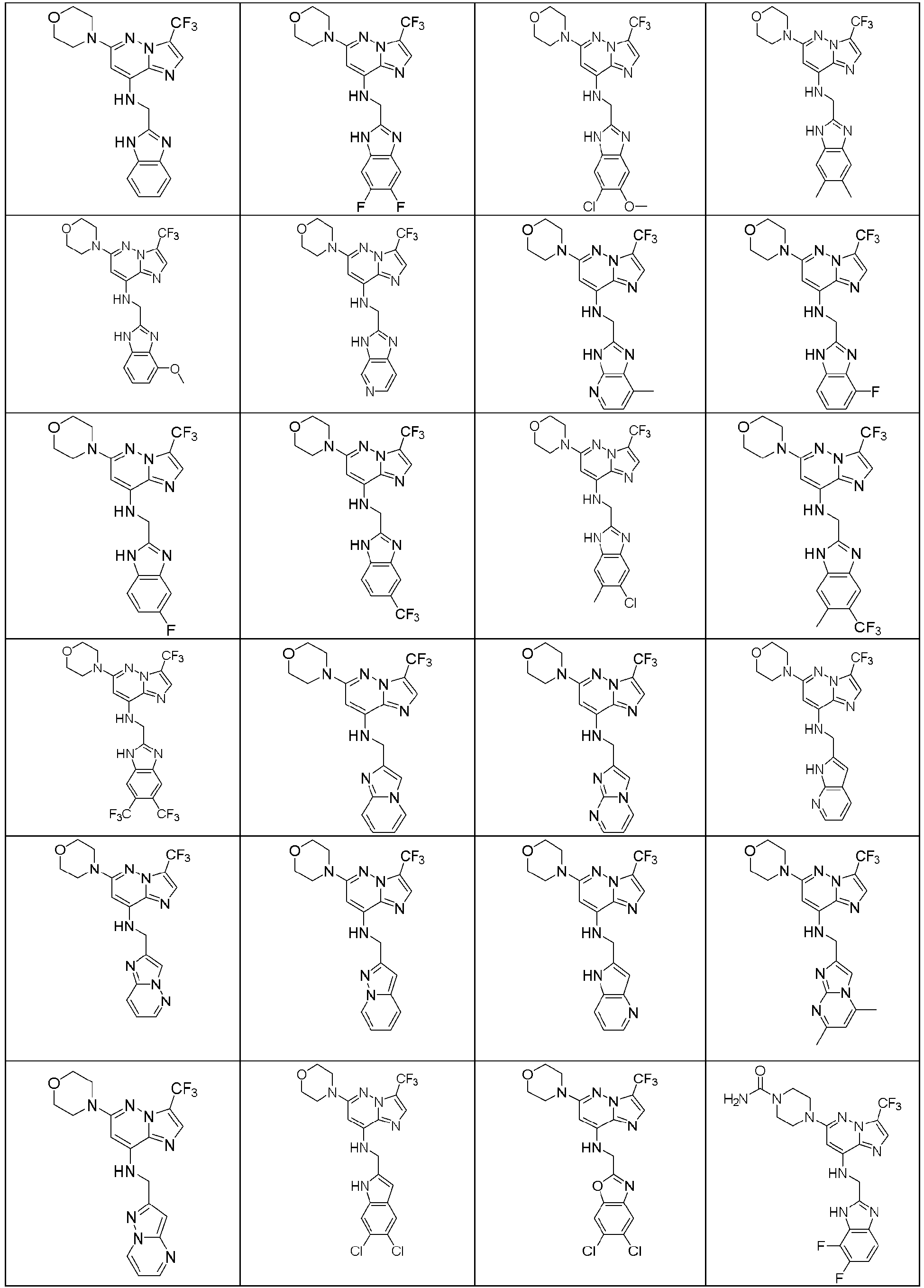

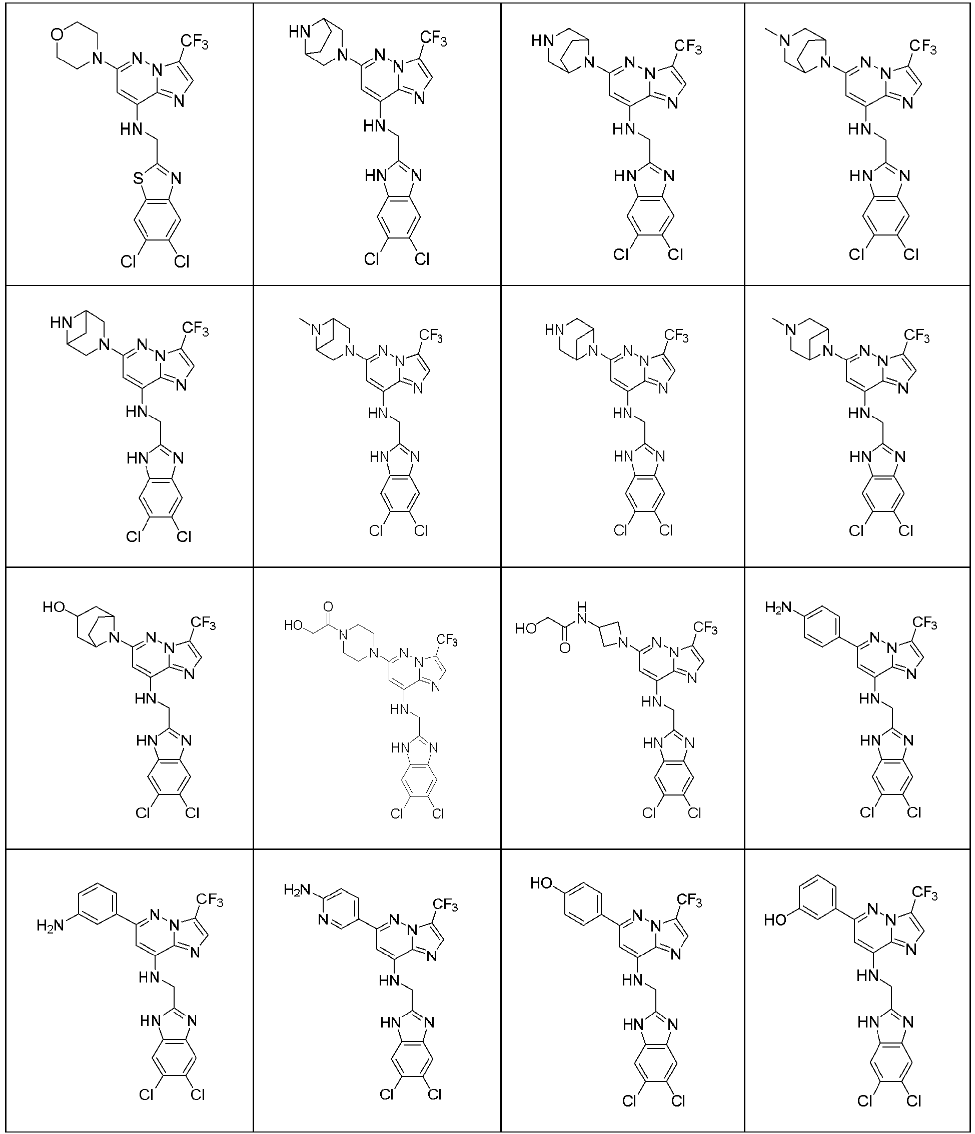

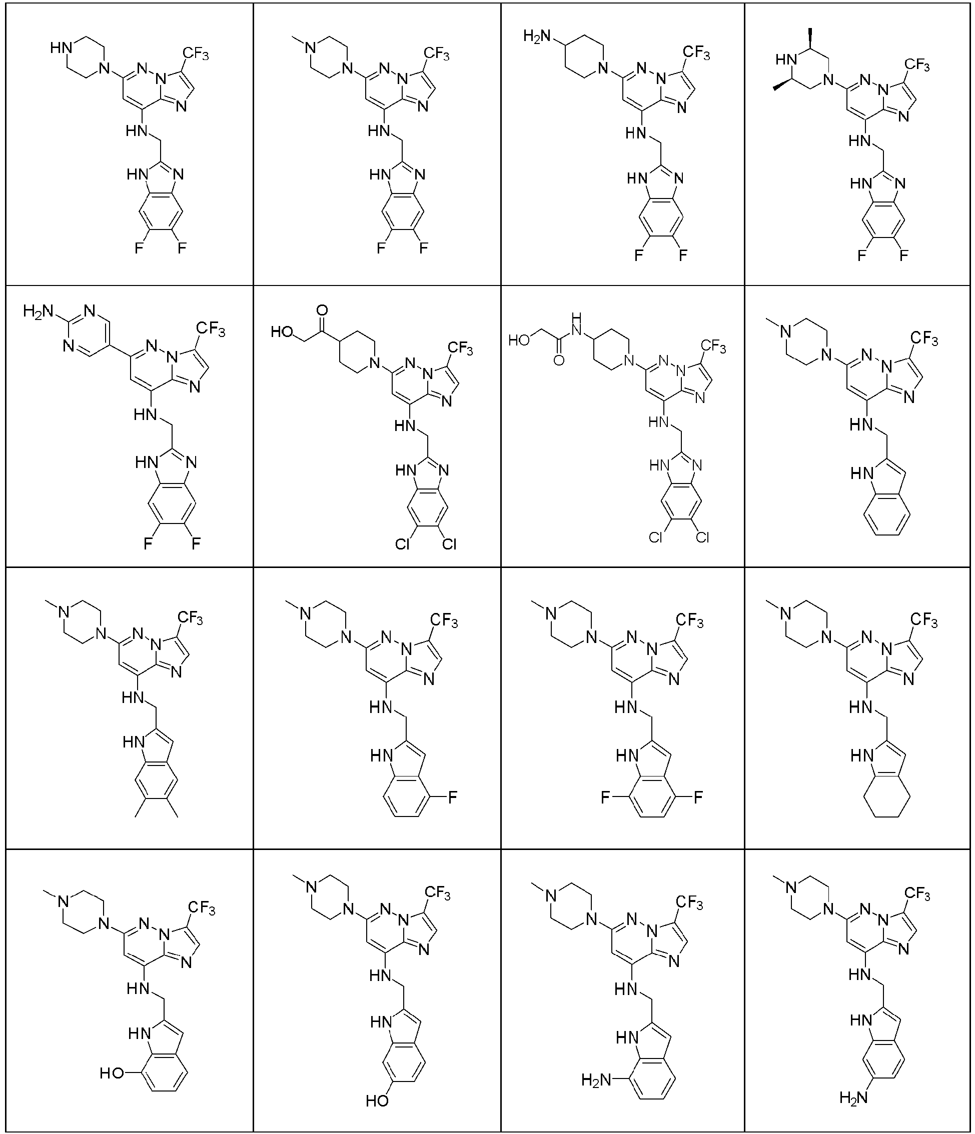

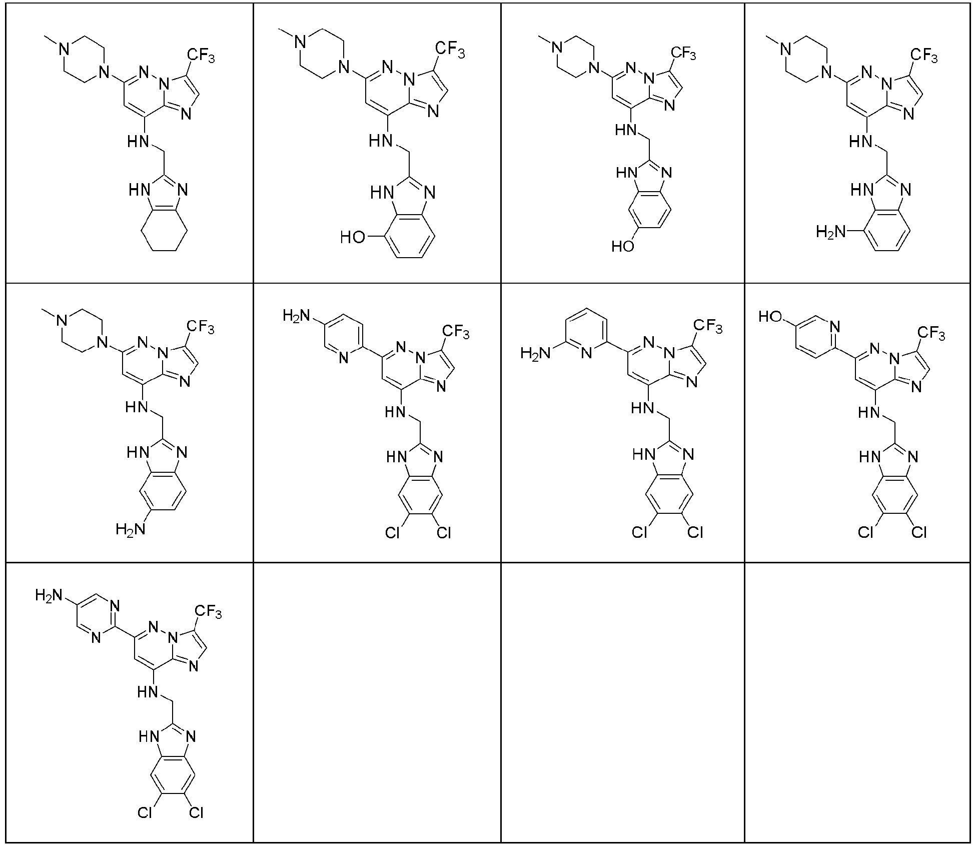

### Definition

All technical and scientific terms used herein have meanings commonly understood by those of ordinary skill in the art. Unless otherwise stated, conventional measurement methods, manufacturing methods, and conventional ingredients or materials are used based on conventional techniques such as pharmacology, pharmaceutical chemistry, mass spectrometry, NMR, HPLC, and biochemistry.

The individual features and components of each embodiment described and exemplified herein may be combined with the features and components of any other embodiment without departing from the scope or spirit of the present disclosure.

Unless otherwise specified, in the present specification and the appended claims, "and" and "or" mean "and/or." The terms "include" and "included" are open-ended and mean that a compound, composition, or method may include additional features or components in addition to the specific features or components listed.

As used herein, the numerical range indicated using the term "to" refers to a range that includes the numerical values described before and after the term "to" as the lower limit and the upper limit, respectively.

As used herein, the term "optional" or "optionally" means that a subsequently described event or circumstance may or may not occur, and that the description includes instances in which said event or circumstance occurs and instances in which it does not occur. For example, the term "optionally substituted" includes instances where the element is substituted or unsubstituted with the specified substituent.

### Compound

The term "halogen" refers to an atom belonging to Group 17 of the Periodic Table. Halogen atoms include fluorine (F), chlorine (Cl), bromine (Br), and iodine (I). The term "halogen" can be used interchangeably with the term "halo," which refers to a monovalent functional group composed of a halogen.

The term "hydroxy" refers to the -OH functional group (hydroxyl group).

The term "-CN" or "cyano" refers to a functional group composed of a triple bond between a carbon atom and a nitrogen atom.

The term "amino" refers to a functional group in which hydrogen is bonded to a nitrogen atom, i.e., -NH₂.

As used herein, the term "alkylamino" refers to a functional group in which one hydrogen atom of an amino group is substituted with an alkyl group. For example, C₁₋₆ alkylamino may include, but is not limited to, -NH(C₁₋C₆ alkyl) such as methylamino, ethylamino, propylamino, and butylamino.

As used herein, the term "dialkylamino" refers to a functional group in which two hydrogens of an amino group are each substituted with an alkyl group. In this case, the substituted alkyl groups may be the same or different. For example, di(C₁₋₆ alkyl)amino may include, but is not limited to, -N(C₁₋C₆ alkyl)₂ such as dimethylamino, diethylamino, dipropylamino, dibutylamino, ethylmethylamino, methylpropylamino, and ethylpropylamino.

The term "oxo" refers to =O, and "oxo-substituted" means that a carbon atom has an =O substituent in the form of -C(=O)-. The carbon atom substituted with an oxo group may be provided as a carbonyl group.

As used herein, the term "carbonyl" refers to a divalent functional group of -C(=O)-.

As used herein, the term "carboxy" refers to -COOH.

The term "alkyl" refers to a fully saturated, branched or unbranched (or straight-chain or linear) hydrocarbon. The alkyl group may be a substituted or unsubstituted alkyl group. The alkyl group may be a C₁ to C₈ alkyl group, a C₁ to C₇ alkyl group, a C₁ to C₆ alkyl group, a C₁ to C₅ alkyl group, a C₁ to C₄ alkyl group, a C₁ to C₃ alkyl group, or a C₁ to C₂ alkyl group. Non-limiting examples of the alkyl group may include methyl, ethyl, n-propyl, isopropyl, n-butyl, isobutyl, sec-butyl, n-pentyl, isopentyl, neopentyl, iso-amyl, or n-hexyl.

The term "haloalkyl" refers to a straight-chain or branched saturated aliphatic hydrocarbyl group having a specified number of carbon atoms, substituted with one or more halogen atoms. The haloalkyl group includes a perhaloalkyl group, in which all hydrogens of the alkyl group are substituted with halogens (e.g., -CF₃, -CF₂CF₃). The above halogens may be the same (e.g., CHF₂, -CF₃) or different (e.g., CF₂Cl). If specified, the haloalkyl group may be optionally substituted with one or more substituents other than the halogen. Examples of the haloalkyl group include, but are not limited to, fluoromethyl, dichloroethyl, trifluoromethyl, trichloromethyl, pentafluoroethyl, and pentachloroethyl groups.

As used herein, the term "alkylene" refers to a divalent, fully saturated, branched or unbranched (or straight-chain or linear) hydrocarbyl having a functional group represented by the formula -CₙH₂ₙ-. For example, C₁₋₆ alkylene may include ethylene, propylene, butylene, and hexylene.

The term "alkenyl" refers to a linear or branched hydrocarbyl group having 2 to 8 carbon atoms, and in some embodiments, 2 to 6 carbon atoms, or 2 to 4 carbon atoms, and having at least one vinyl unsaturation site (>C=C<). For example, (Cₓ-C_{y}) alkenyl refers to an alkenyl group having x to y carbon atoms, and may include, for example, ethenyl, propenyl, isopropylene, 1,3-butadienyl, and the like.

The term "alkynyl" refers to a linear or branched monovalent hydrocarbyl radical containing at least one triple bond. The term "alkynyl" may also include a hydrocarbyl group having one triple bond and one double bond. For example, (C₂-C₆) alkynyl may include ethynyl, propynyl, and the like.

The term "alkoxy" refers to a substituent in which a substituted or unsubstituted straight-chain or branched alkyl moiety is linked to another chemical structure via oxygen. The alkoxy may include, without limitation, all possible isomers thereof, such as methoxy, ethoxy, propoxy, and butoxy, or isopropoxy, isobutoxy, and t-butoxy.

The term "cycloalkyl" refers to a saturated or partially unsaturated hydrocarbon ring having a specified number of carbon atoms as ring elements. For example, C₃-C₆ cycloalkyl refers to a cycloalkyl group having 3, 4, 5, or 6 carbon atoms as ring elements. As used herein, the term "cycloalkyl" may refer to, for example, C₃-C₆ cycloalkyl, C₃-C₅ cycloalkyl, or C₃-C₄ cycloalkyl. Examples thereof may include cyclopropyl, cyclobutyl, cyclopentyl, cyclopentenyl, cyclohexyl, or cyclohexenyl.

The term "heterocyclyl" or "heterocyclic ring" refers to a saturated or partially unsaturated cyclic hydrocarbon group containing at least one heteroatom. A heterocyclyl group may be a monocyclic, bicyclic, or tricyclic group. The two ring groups described above may be spiro-rings, bridged-rings, or fused-rings. A spiro-ring group refers to a structure in which two rings share one common atom. A bridged-ring group refers to a structure in which two non-adjacent ring elements are linked by one or more bridging elements. The heterocyclyl group may contain 3 to 20, 3 to 12, 3 to 10, 3 to 7, 3 to 6, 4 to 6, or 5 to 6 ring elements. The heterocyclyl group may contain one or more heteroatoms selected from the group consisting of N, O, and S.

One or more N or S atoms in a heterocyclyl group may be oxidized, and the term "heterocyclyl" includes such oxidized forms (e.g., N → O-, S(O), SO₂). Non-limiting examples of the monocyclic heterocyclic ring group may include aziridinyl, azetidinyl, pyrrolidinyl, piperidinyl, piperazinyl, morpholinyl, and azepanyl. For example, the spiro heterocyclyl group may include 2-azaspiro[3.3]heptan-2-yl, 2,6-diazaspiro[3.3]heptan-2-yl, 6-oxa-2-azaspiro[3.3]heptan-2-yl, 2-azaspiro[3.4]octan-2-yl, 2,6-diazaspiro[3.4]octan-2-yl, 6-oxa-2-azaspiro[3.4]octan-2-yl, 6-azaspiro[3.4]octan-6-yl, 2,6-diazaspiro[3.4]octan-6-yl, 2-oxa-6-azaspiro[3.4]octan-6-yl, 2-azaspiro[4.4]nonan-2-yl, 2,7-diazaspiro[4.4]nonan-2-yl, or 2-oxa-7-azaspiro[4.4]nonan-7-yl, but is not limited thereto. For example, the bridged heterocyclyl group may include 3-oxa-8-azabicyclo[3.2.1]octan-8-yl, 8-oxa-3-azabicyclo[3.2.1]octan-3-yl, 3,8-diazabicyclo[3.2.1]octan-3-yl, 3,8-diazabicyclo[3.2.1]octan-8-yl, 2-oxa-5-azabicyclo[2,2,2]octan-5-yl, 8-azabicyclo[3.2.1]octan-8-yl, 3-azabicyclo[3.1.1]heptan-3-yl, or 6-oxa-3-azabicyclo[3.1.1]heptan-3-yl, 3,6-diazabicyclo[3.1.1]heptan-3-yl, or 3,6-diazabicyclo[3.1.1]heptan-6-yl, but is not limited thereto.

The term "aryl" also includes an aromatic ring or a group in which an aromatic ring is fused to one or more carbon rings. The C₆-C₁₂ aryl group may be, for example, a C₆ to C₁₀ aryl group or a C₆ to C₈ aryl group. Non-limiting examples of aryl may include phenyl or naphthyl.

The term "heteroaryl" refers to a monocyclic or bicyclic group containing one or more heteroatoms selected from the group consisting of N, O, and S, with the remaining ring atoms being carbon. The heteroaryl group may contain, for example, 1 to 3 heteroatoms and may contain 5 to 10 ring elements. For example, the heteroaryl may be a 5- or 6-membered monocyclic heteroaryl. The heteroaryl may include, for example, one or two nitrogen atoms. The S or N may be oxidized to have multiple oxidation states. Examples of the monocyclic heteroaryl group may include, but is not limited to, pyrimidinyl, pyridinyl, pyrrolyl, imidazolyl, pyrazolyl, thiazolyl, isothiazolyl, oxazolyl, isoxazolyl, triazolyl, oxadiazolyl, thiadiazolyl, tetrazolyl, pyridinyl, pyridazinyl, pyrimidinyl, pyrazinyl, triazinyl, and the like.

The term "substitution" in the above "optionally substituted" refers to introducing a substitute for the hydrogen atom in the case where a derivative is formed by substituting one or more hydrogen atoms in an organic compound with another atomic group, and "substituent" refers to the introduced atomic group. As used herein, the term "substituted" group refers to one in which one or more hydrogen atoms are replaced with one or more non-hydrogen atom groups, provided that valence requirements should be met and a chemically stable compound should occur from the substitution. In the present specification, unless explicitly stated as "unsubstituted," all substituents should be construed as being capable of being unsubstituted or substituted.

In the present specification, when a combination of substituents is mentioned such as one group, for example, haloalkyl, hydroxyalkyl, or the like, the last-mentioned group generally contains the atom attached to the remaining portion of the molecule.

As used herein, " 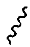 ", "*", or "-" is used to indicate a position at which a substituent is bonded to the moieties of the compound. For example, if "-" is indicated at the end of a substituent, it means that the end is attached to the remaining moieties of the compound. In addition, when two or more substituents are linked by "-," it means that the substituent immediately before "-" is bonded to a substitutable atom of the substituent immediately after "-."

As used herein, the term "solvate" may refer to a compound of the present invention or a salt thereof comprising a stoichiometric or non-stoichiometric amount of a solvent bound by noncovalent intermolecular forces. Preferred solvents therefor may be solvents that are volatile, nontoxic, and/or suitable for administration to humans. Those of ordinary skill in the art will be able to readily prepare a solvate, such as a hydrate, of the compound disclosed herein using appropriate techniques known in the art.

As used herein, the term "stereoisomer" may refer to a compound of the present invention or a salt thereof that has the same chemical formula or molecular formula but is optically or sterically different, and specifically, may be a diastereomer, an enantiomer, or a geometric isomer.

In some embodiments, the compound of the present invention contains one or more asymmetric centers and may be in the form of a racemate, a single enantiomer, a mixture of enantiomers, a single diastereomer, a mixture of diastereomers, etc. In one embodiment, due to the nature of the asymmetric center or limited rotation, the compound of the present invention may exist in the form of an enantiomer or a diastereomer.

When two or more asymmetric centers are present in the compound of the present invention, multiple diastereomers and enantiomers of the chemical structures disclosed herein may exist. Pure isomers, separated isomers, partially pure isomers, or racemic mixtures are all intended to be within the scope of the present invention.

Purification of the isomers and separation of isomer mixtures can be achieved by standard techniques known in the art. For example, a diastereomeric mixture can be separated into its respective diastereomers by a chromatographic process or crystallization, and a racemate can be separated into its respective enantiomers by a chromatographic process or resolution of the chiral phase.

The term "salt" refers to inorganic and organic acid addition salts of a compound. The compound of the present invention can be used in the form of a pharmaceutically acceptable salt derived from inorganic or organic acids. The pharmaceutically acceptable salt may be a salt that does not cause significant irritation to the organism to which the compound is administered and do not impair the biological activity and physical properties of the compound. The inorganic acid salts may be hydrochloride, bromate, phosphate, sulfate, or disulfate. The organic acid salt may be formate, acetate, propionate, lactate, oxalate, tartrate, malate, maleate, citrate, fumarate, besylate, camsylate, edisylate, trichloroacetic acid, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. In addition, the metal salt may be a calcium salt, sodium salt, magnesium salt, strontium salt, or potassium salt.

A pharmaceutically acceptable salt of the compound according to the present invention may be prepared by dissolving the compound of Formula I in a water-miscible organic solvent, such as acetone, methanol, ethanol, acetonitrile, or the like, and adding an excess of an organic acid or adding an aqueous acid solution of an inorganic acid, and then precipitating or crystallizing. Subsequently, after evaporating the solvent or an excess of acid from this mixture, it may be prepared by drying to obtain an addition salt or by suction filtration of the precipitated salt.

The compounds of the present invention, including stereoisomers, hydrates, solvates, and salts, can be prepared by known organic synthetic methods and can be synthesized via a number of synthetic routes.

The reaction for preparing the compound of the present invention can be performed in a suitable solvent that can be appropriately selected by those of ordinary skill in the art of organic synthesis. Suitable solvents are those that are substantially non-reactive with the starting materials (reactants), intermediates, or target products at the temperature at which the reaction occurs. Those of ordinary skill in the art will be able to appropriately select a suitable solvent for each reaction step.

Protection and deprotection of various functional groups can occur during the synthesis of the compound of the present invention. Those of ordinary skill in the art will readily be able to determine the necessity of protection and deprotection and select appropriate protecting groups.

Each reaction can be monitored by any suitable method known in the art. For example, the synthesis of the desired compound can be monitored by spectroscopic means, such as NMR (e.g., ¹H or ¹³C), mass spectroscopy, or chromatography (HPLC or TLC).

The compound of the present invention can be synthesized according to the synthetic procedures described in the examples below. Based on the above, the desired compound can be manufactured by appropriately changing the reactants and reaction conditions, etc., depending on the structure of the desired compound.

### Medicinal use, pharmaceutical composition, and administration method

In another aspect, there is provided a pharmaceutical composition comprising a compound, a stereoisomer, hydrate, solvate, or pharmaceutically acceptable salt thereof according to one aspect. The compound, the stereoisomer, the solvate, and the salt are the same as described above.

The compound according to one aspect of the present invention can inhibit overexpression, excessive activity, mutation of CDK12 and/or CDK13, and activation of a signaling pathway associated with cyclin K. Therefore, the compound can inhibit cell proliferation, inhibit cell cycle progression, and/or promote cell death.

CDK12 and its ortholog CDK13 belong to the CDK family, which regulate transcriptional and post-transcriptional processes. They are known to mediate gene transcription by forming a complex with cyclin K and phosphorylating the C-terminal domain of RNA polymerase II (e.g., Greifenberg et al. 2016, "Structural and Functional Analysis of the CDK13/Cyclin K Complex," Cell Rep. Vol. 14, No. 2, pp. 320-331). The CDK12/cyclin K and CDK13/cyclin K complexes phosphorylate Ser2 of the C-terminal domain of RNA polymerase II, a process believed to be a crucial step in transitioning from transcription initiation to elongation.

Recently, various compounds have been reported to directly degrade cyclin K by linking CDK12-cyclin K to the DDB1-CUL4-RBX1 E3 ligase (Zuzanna et al., 2023, "Design principles for cyclin K molecular glue degraders", Nat Chem Biol Sep 7). Thus, the cyclin K degradation ability of the compound is expected to exhibit characteristics completely different from those of conventional kinase inhibitors that regulate the phosphorylation of downstream signaling molecules.

The compound according to the present invention can exhibit the cyclin K degradation activity along with CDK12 and/or CDK13 inhibitory activity.

Cyclin K degradation can induce novel anticancer effects in addition to direct inhibition of CDK12 and/or CDK13 in cells. Cyclin K is an essential partner of both CDK12 and/or CDK13 and is required for their activity. Independent of CDK12 and/or CDK13, cyclin K expression is increased in various cancer cells compared to normal cells, suggesting that cyclin K itself is directly involved in the proliferation of various cancer cells. In addition, cyclin K has been shown to increase the expression of specific proteins associated with treatment resistance, potentially improving responsiveness to existing therapeutic agents (Yi Xiao and Jixin Dong, 2023, "Coming of Age: Targeting Cyclin K in Cancers", Cells. Aug 11;12(16):2044).

Therefore, the compound according to the present invention is expected to exhibit superior anticancer efficacy compared to existing therapeutic agents because it exhibits enhanced and sustained efficacy compared to CDK12/CDK13 inhibitors that only inhibit kinase activity.

Therefore, the pharmaceutical composition according to one aspect of the present invention may be used for the treatment of a disease caused by overexpression, excessive activity, mutation of CDK12 and/or CDK13, and/or activation of a signaling pathway associated with cyclin K. In addition, the pharmaceutical composition may be used for inhibiting cell proliferation, inhibiting cell cycle progression, and/or promoting cell death.

In one embodiment, the disease may be cancer, a proliferative disease, a neurodegenerative disease, an autoimmune disease, or a disease caused by an abnormality in intracellular protein translation function.

As used herein, the term "proliferative disease" refers to a disease characterized by excessive cell proliferation. The proliferative disease is associated with: (1) pathological proliferation of normally quiescent or proliferating cells; (2) pathological migration of cells from their normal location (e.g., metastasis of neoplastic cells); (3) pathological expression of proteolytic enzymes, such as matrix metalloproteinases (e.g., collagenase, gelatinase, and elastase), which may cause unwanted transformation of the cellular matrix; and/or (4) pathological angiogenesis, which occurs in proliferative retinopathy and tumor metastasis. Exemplary proliferative diseases include cancer, benign neoplasms, and angiogenesis (defined above as pathological angiogenesis) that accompanies and facilitates the disease state.

In one embodiment, the pharmaceutical composition may be a pharmaceutical composition for the treatment of cancer. In one embodiment, the cancer may be a cancer caused by overexpression, excessive activity, mutation of CDK12 and/or CDK13, and/or activation of a signaling pathway associated with cyclin K. For example, the cancer may be a cancer caused by overexpression, excessive activity, mutation of CDK12 and/or CDK13, and/or activation of a signaling pathway associated with cyclin K, such as a cancer characterized by amplification of the CDK12, CDK13, or cyclin K genes.

In one embodiment, the cancer may be selected from the group consisting of:
(1) carcinomas of the breast, liver, lung, colon, kidney, and bladder, including small cell lung cancer, non-small cell lung cancer, head and neck cancer, thyroid cancer, esophageal cancer, gastric cancer, pancreatic cancer, ovarian cancer, gallbladder cancer, cervical cancer, prostate cancer, and skin cancer, including squamous cell carcinoma;
(2) sarcomas, such as osteosarcoma and osteogenic sarcoma (bone), chondrosarcoma (cartilage), leiomyosarcoma (smooth muscle), rhabdomyosarcoma (skeletal muscle), mesothelioma and mesothelioma (membrane lining of body cavities), fibrosarcoma (fibrous tissue), angiosarcoma or hemangioendothelioma (blood vessels), liposarcoma (fat tissue), glioma and astrocytoma (neurogenic connective tissue found in the brain), myxosarcoma (primitive embryonic connective tissue), or mesenchymal and mixed mesodermal tumor (mixed connective tissue type);
(3) hematopoietic tumors of the lymphoid system, such as leukemia, acute lymphocytic leukemia, Hodgkin's lymphoma, non-Hodgkin's lymphoma, B-cell lymphoma, T-cell lymphoma, hairy cell lymphoma, myeloma, mantle cell lymphoma, and Burkett's lymphoma;
(4) hematopoietic tumors of the myeloid system, such as acute lymphoblastic leukemia (ALL), chronic lymphocytic leukemia (CLL), myelodysplastic syndrome (MDS), and promyelocytic leukemia;
(5) tumors of the central and peripheral nervous systems, such as astrocytoma, neuroblastoma, glioma, and schwannoma.

In one embodiment, the neurodegenerative disease may be Alzheimer's disease, Parkinson's disease, or Lou Gehrig's disease. For example, the autoimmune disease may be rheumatoid arthritis, systemic lupus erythematosus, psoriasis, or Sjögren's syndrome. For example, the disease caused by an abnormality in intracellular protein translation function may be muscular atrophy, myotonic dystrophy, amyotrophic lateral sclerosis, spinal muscular atrophy, or Fragile X syndrome.

As used herein, the term "treating" or "treatment" refers to inhibiting a disease, for example, inhibiting a disease, condition, or disorder in a subject who experiences or exhibits the pathology or signs of the disease, condition, or disorder, i.e., preventing further development of the pathology and/or signs, or ameliorating the disease, for example, ameliorating the disease, condition, or disorder in a subject who experiences or exhibits the pathology or signs of the disease, condition, or disorder, i.e., reversing the pathology and/or signs, for example, reducing the severity of the disease.

"Cancer," a disease to be prevented or treated by the pharmaceutical composition, refers to a general term for a disease caused by cells that have aggressive characteristics in which cells divide and grow while ignoring normal growth limits, invasive characteristics in which cells infiltrate surrounding tissues, and metastatic characteristics in which cells spread to other parts of the body. Cancers for which the compound of the present invention has therapeutic activity may include any cancer that can be treated, alleviated, delayed, inhibited or prevented, for example, due to overexpression, excessive activity, mutation of CDK12 and/or CDK13, and/or activation of a signaling pathway associated with cyclin K.

For example, the cancer may include breast cancer, ovarian cancer, colorectal cancer, lung cancer, prostate cancer, gastric cancer, liver cancer, kidney cancer, bladder cancer, pancreatic cancer, head and neck cancer, thyroid cancer, skin cancer, bile duct cancer, esophageal cancer, hematopoietic tumors of the lymphoid and myeloid systems, tumors of the central and peripheral nervous systems, and sarcoma, but is not limited thereto. In one embodiment, the cancer may include breast cancer, lung cancer, gastric cancer, pancreatic cancer, or colorectal cancer. In one embodiment, the cancer may be breast cancer, such as triple-negative breast cancer.

For example, the pharmaceutical composition described herein can be used for the treatment of "high-grade" cancers (e.g., high-grade serous ovarian cancer, metastatic non-small cell lung cancer, metastatic breast cancer (e.g., triple-negative breast cancer), metastatic gastric cancer, metastatic liver cancer), tumors exhibiting a particular phenotype (e.g., estrogen receptor-positive (ER+) breast cancer, human epidermal growth factor receptor 2 (HER2) positive breast or gastric cancer, tyrosine kinase-positive non-small cell lung cancer, or PD-L1 positive solid tumors), and/or cancers that have become resistant to treatment with previously administered therapeutic agents (e.g., chemotherapeutic agents (e.g., cisplatin or fluorouracil and the like), CDK4/6 inhibitors (e.g., palbociclib), estrogen receptor-degrading agents (e.g., fulvestrant), or PARP inhibitors (e.g., olaparib)).

The pharmaceutical composition may comprise an additional anticancer agent. The pharmaceutical composition may be a single composition or individual compositions. For example, the pharmaceutical composition according to one aspect may be an oral dosage form, and the anticancer agent may be a parenteral dosage form.

When used for cancer treatment, the compound of the present invention may be used alone or in combination with conventional surgery, radiotherapy, chemotherapy, or immunotherapy.

For example, the compound of the present invention may be administered in combination with other anticancer therapies, such as radiation therapy, taxane derivatives (e.g., paclitaxel, docetaxel, cabazitaxel), platinum compounds (e.g., cisplatin, carboplatin), antimetabolites (e.g., 5-FU, gemcitabine, cytarabine), anti-CTLA4 therapies (e.g., ipilimumab, tremelimumab), anti-PD1 therapies (e.g., nivolumab, pembrolizumab), anti-PD-L1 therapies (e.g., atezolizumab, durvalumab), anti-VEGF therapies (e.g., bevacizumab, ramucirumab, aflibercept), anti-EGFR therapies (e.g., cetuximab), topoisomerase inhibitors (e.g., irinotecan), anti-HER2 therapies (e.g., trastuzumab, pertuzumab), anti-hormone therapies (e.g., tamoxifen, exemestane, letrozole, anastrozole), estrogen receptor inhibitors (e.g., elacestrant, fulvestrant), ERK inhibitors (e.g., ulixertinib), PARP inhibitors (e.g., olaparib, niraparib, talazoparib), mTOR inhibitors (e.g., everolimus, temsirolimus), CDK4/6 inhibitors (e.g., abemaciclib, palbociclib), EGFR inhibitors (e.g., afatinib, erlotinib, osimertinib, gefitinib, dacomitinib), HER2 inhibitors (e.g., neratinib, lapatinib), ALK inhibitors (e.g., crizotinib, alectinib, brigatinib, ceritinib), tyrosine kinase inhibitors (e.g., avapritinib, ripretinib, sunitinib, sorafenib, pazopanib, regorafenib, cabozantinib, lenvatinib), MEK inhibitors (e.g., trametinib), BCR-ABL inhibitors (e.g., imatinib, nilotinib, dasatinib), PI3K inhibitors (e.g., alpelisib), FGFR inhibitors (e.g., futibatinib, pemigatinib), ROS1 inhibitors (e.g., crizotinib, entrectinib), androgen biosynthesis inhibitors (e.g., abiraterone acetate), androgen receptor inhibitors (e.g., enzalutamide, darolutamide, apalutamide), Hedgehog inhibitors (e.g., sonidegib, vismodegib), MET inhibitors (e.g., capmatinib, tepotinib), AXL inhibitors, NTRK1 inhibitors, RET inhibitors (e.g., pralsetinib, selpercatinib), KRAS inhibitors (e.g., adagrasib, sotorasib), or RAF inhibitors (e.g., encorafenib, vemurafenib).

The pharmaceutical composition may comprise a pharmaceutically acceptable carrier. The term "carrier" is used to mean to include an excipient, diluent, or adjuvant. For example, the carrier may be selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinyl pyrrolidone, water, physiological saline, a buffer such as PBS, methyl hydroxybenzoate, propyl hydroxybenzoate, talc, magnesium stearate, and mineral oil. The composition may comprise a filler, an anticoagulant, a lubricant, a wetting agent, a flavoring agent, an emulsifier, a preservative, or a combination thereof.

The pharmaceutical composition may be prepared in any dosage form using conventional methods. The composition may be formulated, for example, as an oral dosage form (e.g., a powder, tablet, capsule, syrup, pill, or granule) or a parenteral dosage form (e.g., an injection). In addition, the composition may be formulated as a systemic dosage form or a topical dosage form.

In the pharmaceutical composition, the solid dosage form for oral administration may be a tablet, pill, powder, granule, or capsule. The solid dosage form may further comprise an excipient. Examples of such excipients may include starch, calcium carbonate, sucrose, lactose, or gelatin. In addition, the solid dosage form may further comprise a lubricant such as magnesium stearate or talc. In the pharmaceutical composition, the liquid dosage form for oral administration may be a suspension, an oral solution, an emulsion, or a syrup. The liquid dosage form may comprise water or liquid paraffin. The liquid dosage form may comprise an excipient, such as a wetting agent, a sweetener, a flavoring agent, or a preservative. In the pharmaceutical composition, a dosage form for parenteral administration may be a sterilized aqueous solution, a non-aqueous solution, a suspension, an emulsion, a lyophilized formulation, or a suppository. The non-aqueous solution or suspension may comprise a vegetable oil or an ester. The vegetable oil may be, for example, propylene glycol, polyethylene glycol, or olive oil. The ester may be, for example, ethyl oleate. The suppository base may be witepsol, macrogol, Tween 61, cacao butter, laurin butter, or glycerogelatin.

The pharmaceutical composition comprises a compound, a stereoisomer, solvate, or pharmaceutically acceptable salt thereof according to one aspect as an active ingredient of the pharmaceutical composition. The term "active ingredient" refers to a physiologically active substance used to achieve pharmacological activity (e.g., cancer).

The pharmaceutical composition may comprise an effective amount of a compound, a stereoisomer, solvate, or pharmaceutically acceptable salt thereof according to one aspect. The term "effective amount" refers to an amount sufficient to exhibit the effect of preventing or treating a disease when administered to a subject in need of prevention or treatment. The effective amount can be appropriately selected by those of ordinary skill in the art depending on the cell or subject being selected. The preferred dosage of the pharmaceutical composition varies depending on the subject's condition and body weight, the severity of the disease, the drug form, the route of administration, and the duration of administration, but can be appropriately selected by those of ordinary skill in the art. However, the compound, a stereoisomer, solvate, or pharmaceutically acceptable salt thereof may be administered in divided doses, for example, from about 0.0001 mg/kg to about 100 mg/kg, or from about 0.001 mg/kg to about 100 mg/kg, once to 24 times daily, once to 7 times every 2 days to 1 week, or once to 24 times every 1 to 12 months. In the pharmaceutical composition, the compound, a stereoisomer, solvate, or pharmaceutically acceptable salt thereof may be included in an amount of from about 0.0001 wt% to about 10 wt%, or from about 0.001 wt% to about 1 wt%, based on the total weight of the entire composition.

The administration method may be oral or parenteral. Examples of administration routes may include oral, transdermal, subcutaneous, rectal, intravenous, intraarterial, intraperitoneal, intramuscular, intrasternal, topical, intranasal, intratracheal, or intradermal routes. The composition may be administered systemically or locally, and may be administered alone or in combination with other pharmaceutically active compounds.

In another aspect, there is provided a method for treating a disease caused by overexpression, excessive activity, mutation of CDK12 and CDK13, and/or activation of a signaling pathway associated therewith, comprising administering a compound, a stereoisomer, hydrate, solvate, or pharmaceutically acceptable salt thereof according to one aspect to a subject. The compound, the stereoisomer, the hydrate, the solvate, the pharmaceutically acceptable salt, and the treatable disease are the same as described above.

For example, the pharmaceutical composition described herein can be used for the treatment of "high-grade" cancers (e.g., high-grade serous ovarian cancer, metastatic non-small cell lung cancer, metastatic breast cancer (e.g., triple-negative breast cancer), metastatic gastric cancer, metastatic liver cancer), tumors exhibiting a particular phenotype (e.g., estrogen receptor-positive (ER+) breast cancer, human epidermal growth factor receptor 2 (HER2) positive breast or gastric cancer, tyrosine kinase-positive non-small cell lung cancer, or PD-L1 positive solid tumors), and/or cancers that have become resistant to treatment with previously administered therapeutic agents (e.g., chemotherapeutic agents (e.g., cisplatin or fluorouracil and the like), CDK4/6 inhibitors (e.g., palbociclib), estrogen receptor-degrading agents (e.g., fulvestrant), or PARP inhibitors (e.g., olaparib)).

In one embodiment, the method may comprise the steps of determining whether the subject has high-grade cancer or tumor cells of a specific phenotype, or has developed resistance to a previously administered therapeutic agent; and administering the compound of the present invention if the subject is determined to have high-grade cancer or tumor cells of a specific phenotype, or has developed resistance.

The subject may be a mammal, such as a human, mouse, rat, cow, horse, pig, dog, monkey, sheep, goat, ape, or cat. The subject may be a subject suffering from, or at high risk of suffering from, symptoms associated with cancer.

The method may further comprise administering to the subject a known active ingredient for treating cancer. The known active ingredient may be administered to a subject simultaneously, separately, or sequentially with a compound, a stereoisomer, hydrate, solvate, or pharmaceutically acceptable salt thereof according to one aspect.

The administration method may be oral or parenteral. Examples of administration routes may include oral, transdermal, subcutaneous, rectal, intravenous, intraarterial, intraperitoneal, intramuscular, intrasternal, topical, intranasal, intratracheal, or intradermal routes.

In another aspect, there is provided a method for inhibiting CDK12 and/or CDK13 and degrading cyclin K, comprising adding to a cell a compound, a stereoisomer, hydrate, solvate, or pharmaceutically acceptable salt thereof according to one aspect. In one embodiment, the method may be performed in vitro. The compound, the stereoisomer, the hydrate, the solvate, the pharmaceutically acceptable salt, and the treatable disease are the same as described above.

### Effects of Invention

The present invention provides a compound according to one aspect, a use thereof for inhibiting the activity of CDK12 and/or CDK13, a pharmaceutical composition comprising the same, and a use thereof for the treatment of diseases associated with CDK12 and/or CDK13, such as cancer. According to the present invention, the compounds exhibit excellent inhibitory activity and selectivity against CDK12 and/or CDK13, and thus can be useful for the treatment of cancers such as breast cancer, gastric cancer, lung cancer, pancreatic cancer, and colorectal cancer.

### Brief Description of Drawings

Figure 1 is a diagram showing the results of measuring the cyclin K degradation ability of the compound of Example 8 in HCC70 breast cancer cells.
Figure 2 is a diagram showing the results of measuring the cyclin K degradation ability of the compound of Example 30 in HCC70 breast cancer cells.
Figure 3 is a diagram showing the results of measuring the cyclin K degradation ability of the compound of Example 40 in HCC70 breast cancer cells.
Figure 4 is a diagram showing the results of measuring the cyclin K degradation ability of the compound of Example 8 in MKN45 gastric cancer cells.
Figure 5 is a diagram showing the results of measuring the cyclin K degradation ability of the compound of Example 40 in MKN45 gastric cancer cells.
Figure 6 is a diagram showing the relative tumor size reduction by the compound of Example 30 in the HCC70 zebrafish xenograft breast cancer model.
Figures 7 and 8 are diagrams showing the relative tumor size reduction and the reduction in the number of metastatic cancer cells by the compound of Example 8 in the AGS zebrafish xenograft gastric cancer model.

### Mode for Carrying out the Invention

The present invention will be described in more detail through the following examples. However, these examples are for illustrative purposes, and the scope of the present invention is not limited to these examples.

The meanings of abbreviations used in the following examples are as follows, and abbreviations not listed below have meanings commonly used in the relevant field.
NCS: *N*-chlorosuccinimide
NBS: *N*-bromosuccinimide
NIS: *N*-iodosuccinimide
MeCN: acetonitrile
THF: tetrahydrofuran
BnBr: benzyl bromide
PMB: *p*-methoxybenzyl
Cbz: benzyloxycarbonyl
NaBH(OAc)₃: sodium triacetoxyborohydride
CbzOSu: *N*-(benzyloxycarbonyloxy)succinimide
TBDMSCl: *tert*-butyldimethylsilyl chloride
TBDPSCl: *tert*-butyldiphenylsilyl chloride
DMAP: 4-dimethylaminopyridine
TBAF: tetra-*N*-butylammonium fluoride
HATU: 1-[bis(dimethylamino)methylene]-1*H*-1,2,3-triazolo[4,5-*b*]pyridinium 3-oxide hexafluorophosphate
HOBT: 1-hydroxybenzotriazole
EDCI: 1-ethyl-3-(3-dimethylaminopropyl)carbodiimide
DIEA: *N,N*-diisopropylethylamine
DMF: *N,N*-dimethylformamide
DCM: dichloromethane
DMSO: dimethylsulfoxide
TFA: trifluoroacetic acid
AcOH: acetic acid
EtOAc: ethyl acetate
NMP: *N*-methyl-2-pyrrolidone
HMPA: hexamethylphosphoramide
CDI: 1,1'-carbonyldiimidazole
SEMC1: 2-(trimethylsilyl)ethoxymethyl chloride
TsOH: *p*-toluenesulfonic acid
Na₂SO₄: sodium sulfate
CuI: copper(I) iodide
Pd/C: palladium on carbon
Pd(OH)₂/C: palladium hydroxide on carbon
RuPhos-Pd-G3: (2-dicyclohexylphosphino-2',6'-diisopropoxy-1,1'-biphenyl)[2-(2'-amino-1,1'-biphenyl)]palladium(II) methanesulfonate
NaH: sodium hydride
TEA: triethylamine
Pd(PPh₃)₄: tetrakis(triphenylphosphine)palladium(0)
Ti(OEt)₄: titanium(IV) ethoxide
Ti(*i*-PrO)₄: titanium(IV) isopropoxide
Pd(PPh₃)₂Cl₂: bis(triphenylphosphine)palladium(II) dichloride
NaBH₄: sodium borohydride
NaOH: sodium hydroxide
Pd(dppf)Cl₂: [1,1'-bis(diphenylphosphino)ferrocene]dichloropalladium(II)

### Preparation of Intermediate S1: tert-butyl N-benzyl-N-(6-morpholinoimidazo[1,2-b]pyridazin-8-yl)glycinate

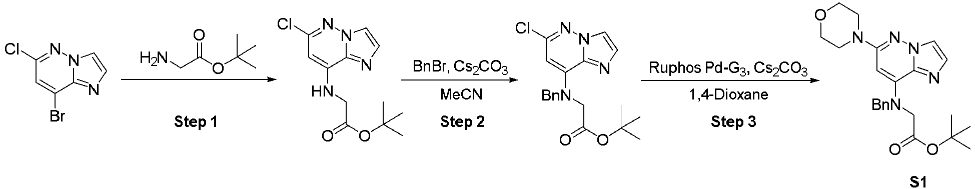

### Step 1: Preparation of tert-butyl (6-chloroimidazo[1,2-b]pyridazin-8-yl)glycinate

8-bromo-6-chloro-imidazo[1,2-b]pyridazine (24 g, 103.24 mmol) was dissolved in *tert-*butyl glycinate (240 mL), and then stirred at 120 °C for 16 hours. Water (2000 mL) was added to the reaction mixture, and the product was extracted three times with EtOAc (800 mL). The organic layer was washed three times with brine (2000 mL), dried over Na₂SO₄, filtered, and concentrated, and then the concentrate was purified by silica gel chromatography to obtain *tert*-butyl (6-chloroimidazo[1,2-*b*]pyridazin-8-yl)glycinate (26.6 g, 91.13% yield). MS: m/z = 283.1 (M+1, ESI+).

### Step 2: Preparation of tert-butyl N-benzyl-N-(6-chloroimidazo[1,2-b]pyridazin-8-yl)glycinate

The product obtained in Step 1 (26.3 g, 93.02 mmol) and benzyl bromide (25.2 mL, 212 mmol) were dissolved in MeCN (200 mL), and then cesium carbonate (92.2 g, 283 mmol) was added and stirred at 60 °C for 16 hours. Water (1200 mL) was added to the reaction mixture, and the product was extracted three times with EtOAc (300 mL). The organic layer was washed three times with brine (1200 mL), dried over Na₂SO₄, filtered, and concentrated, and then the concentrate was purified by silica gel chromatography to obtain *tert*-butyl *N*-benzyl-*N*-(6-chloroimidazo[1,2-*b*]pyridazin-8-yl)glycinate (24.22 g, 68.25% yield). MS: m/z = 373.1 (M+1, ESI+).

### Step 3: Preparation of tert-butyl N-benzyl-N-(6-morpholinoimidazo[1,2-b]pyridazin-8-yl)glycinate

The product obtained in Step 2 (14.2 g, 38.09 mmol) and morpholine (33.3 mL, 380.9 mmol) were dissolved in 1,4-dioxane (300 mL), and then cesium carbonate (37.23 g, 114.3 mmol) and RuPhos-Pd-G3 (3.19 g, 3.81 mmol) were added and stirred at 110 °C for 16 hours. Water (900 mL) was added to the reaction mixture, and the product was extracted three times with EtOAc (300 mL). The organic layer was washed three times with brine (150 mL), dried over Na₂SO₄, filtered, and concentrated, and then the concentrate was purified by silica gel chromatography to obtain **Intermediate S1** (11.8 g, 73.16% yield). MS: m/z = 424.2 (M+1, ESI+).

### Preparation of Intermediate S2: tert-butyl N-benzyl-N-(3-bromo-6-morpholinoimidazo[1,2-b]pyridazin-8-yl)glycinate

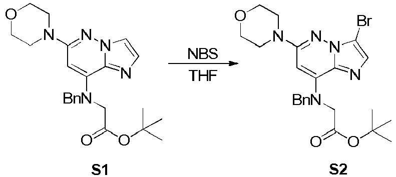

**Intermediate S1** (5.31 g, 12.54 mmol) was dissolved in THF (80 mL), and then NBS (2.23 g, 12.54 mmol) was added in portions at -60 °C and stirred for 6 hours under N₂ atmosphere at the same temperature. Water (200 mL) was added to the reaction mixture, and the product was extracted three times with EtOAc (60 mL). The organic layer was washed three times with a sodium sulfite solution (200 mL), dried over Na₂SO₄, filtered, and concentrated, and then the concentrate was purified by silica gel chromatography to obtain **Intermediate S2** (4.1 g, 65.09 % yield). MS: m/z = 502.1 (M+1, ESI+).

### Preparation of Intermediate S3: tert-butyl N-benzyl-N-(6-morpholino-3-(trifluoromethyl)imidazo[1,2-b]pyridazin-8-yl)glycinate

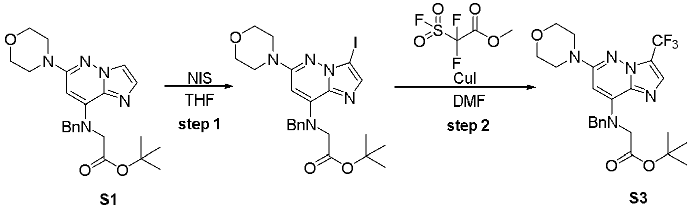

### Step 1: Preparation of tert-butyl N-benzyl-N-(3-iodo-6-morpholinoimidazo[1,2-b]pyridazin-8-yl)glycinate

**Intermediate S1** (5.1 g, 11.34 mmol) was dissolved in THF (80 mL), and then NIS (3.72 g, 16.53 mmol) was added at 0 °C and stirred for 2 hours. Water (300 mL) was added to the reaction mixture, and the product was extracted three times with EtOAc (80 mL). The organic layer was washed three times with brine (400 mL), dried over Na₂SO₄, filtered, and concentrated, and then the concentrate was purified by silica gel chromatography to obtain *tert*-butyl *N*-benzyl-*N*-(3-iodo-6-morpholinoimidazo[1,2-*b*]pyridazin-8-yl)glycinate (5.99 g, 72.13 % yield). MS: m/z = 550.1 (M+1, ESI+).

### Step 2: Preparation of tert-butyl N-benzyl-N-(6-moipholino-3-(trifluoromethyl)imidazo[1,2-b]pyridazin-8-yl)glycinate

The product obtained in Step 1 (5.95 g, 10.83 mmol) and methyl 2,2-difluoro-2-(fluorosulfonyl)acetate (20.81 g, 108.30 mmol) were dissolved in DMF (150 mL), and then CuI (10.31 g, 54.15 mmol) was added and stirred at 100 °C for 16 hours. Water (1000 mL) was added to the reaction mixture, and the product was extracted three times with EtOAc (300 mL). The organic layer was washed three times with brine (800 mL), dried over Na₂SO₄, filtered, and concentrated, and then the concentrate was purified by silica gel chromatography to obtain **Intermediate S3** (1.4 g, 26.30 % yield). MS: m/z = 492.2 (M+1, ESI+).

### Preparation of Intermediate S4: tert-butyl N-(6-chloroimidazo[1,2-b]pyridazin-8-yl)-N-(4-methoxybenzyl)glycinate

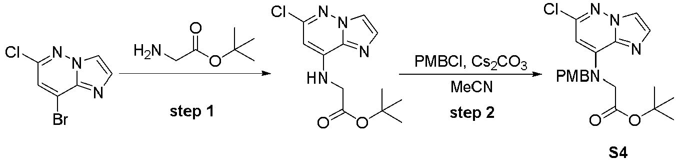

### Step 1: Preparation of tert-butyl (6-chloroimidazo[1,2-b]pyridazin-8-yl)glycinate

8-bromo-6-chloro-imidazo[1,2-*b*]pyridazine (100 g, 430.17 mmol) was dissolved in *tert-*butyl glycinate (1000 mL), and then stirred at 120 °C for 16 hours. Water (3000 mL) was added to the reaction mixture, and the product was extracted three times with EtOAc (1000 mL). The organic layer was washed three times with brine (3000 mL), dried over Na₂SO₄, filtered, and concentrated, and then the concentrate was purified by silica gel chromatography to obtain *tert-*butyl (6-chloroimidazo[1,2-*b*]pyridazin-8-yl)glycinate (102 g, 83.87 % yield). MS: m/z = 283.1 (M+1, ESI+).

### Step 2: Preparation of tert-butyl N-(6-chloroimidazo[1,2-b]pyridazin-8-yl)-N-(4-methoxybenzyl)glycinate

The product obtained in Step 1 (102 g, 361.7 mmol) and 4-methoxybenzyl chloride (62.1 g, 397.9 mmol) were dissolved in MeCN (1000 mL), and then cesium carbonate (178 g, 542.6 mmol) was added and stirred at 60 °C for 16 hours. Water (5000 mL) was added to the reaction mixture, and the product was extracted three times with EtOAc (1000 mL). The organic layer was washed three times with brine (5000 mL), dried over Na₂SO₄, filtered, and concentrated, and then the concentrate was purified by silica gel chromatography to obtain **Intermediate S4** (110.5 g, 76 % yield). MS: m/z = 403.1 (M+1, ESI+).

### Preparation of Intermediate S5: tert-butyl N-(3-bromo-6-morpholinoimidazo[1,2-b]pyridazin-8-yl)-N-(4-methoxybenzyl)glycinate

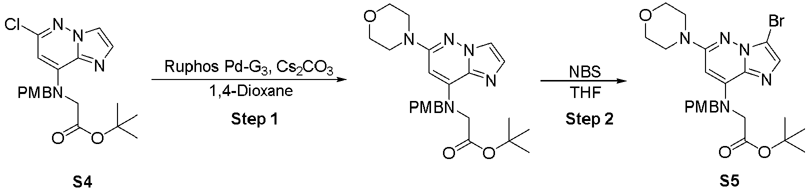

### Step 1: Preparation of tert-butyl N-(4-methoxybenzyl)-N-(6-morpholinoimidazo[1,2-b]pyridazin-8-yl)glycinate

**Intermediate S4** (15 g, 37.23 mmol) and morpholine (33.1 mL, 372.33 mmol) were dissolved in 1,4-dioxane (300 mL), and then cesium carbonate (37 g, 112 mmol) and RuPhos-Pd-G3 (3.12 g, 3.72 mmol) were added and stirred at 110 °C for 16 hours. Water (800 mL) was added to the reaction mixture, and the product was extracted three times with EtOAc (300 mL). The organic layer was washed three times with brine (800 mL), dried over Na₂SO₄, filtered, and concentrated, and then the concentrate was purified by silica gel chromatography to obtain *tert-*butyl *N*-(4-methoxybenzyl)-*N*-(6-morpholinoimidazo[1,2-*b*]pyridazin-8-yl)glycinate (15.2 g, 90.01 % yield). MS: m/z = 454.2 (M+1, ESI+).

### Step 2: Preparation of tert-butyl N-(3-bromo-6-moipholinoimidazo[1,2-b]pyridazin-8-yl)-N-(4-methoxybenzyl)glycinate

The product obtained in Step 1 (15.2 g, 33.51 mmol) was dissolved in THF (200 mL), and then NBS (5.96 g, 33.51 mmol) was added at -60 °C and stirred at this temperature for 1 hour under N₂ atmosphere. Water (800 mL) was added to the reaction mixture, and the product was extracted three times with EtOAc (200 mL). The organic layer was washed three times with sodium sulfite solution (800 mL), dried over Na₂SO₄, filtered, and concentrated, and then the concentrate was purified by silica gel chromatography to obtain **Intermediate S5** (12 g, 67.25 % yield). MS: m/z = 532.1 (M+1, ESI+).

### Preparation of Intermediate S6: 6-chloro-N,N-bis(4-methoxybenzyl)imidazo[1,2-b]pyridazin-8-amine

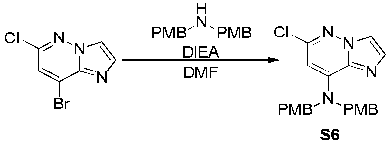

8-bromo-6-chloroimidazo[1,2-*b*]pyridazine (90 g, 390 mmol) and bis(4-methoxybenzyl)amine (120 g, 468 mmol) were dissolved in DMF (800 mL), and then DIEA (135.6 mL, 780 mmol) was added and stirred at 90 °C overnight. Water (4000 mL) was added to the reaction mixture, and the product was extracted three times with EtOAc (800 mL). The organic layer was washed three times with brine (4000 mL), dried over Na₂SO₄, filtered, and concentrated, and then the concentrate was purified by silica gel chromatography to obtain **Intermediate S6** (120 g, 75.95 % yield). MS: m/z = 409.1 (M+1, ESI+).

### Preparation of Intermediate S7: 6-chloro-N,N-bis(4-methoxybenzyl)-3-(trifluoromethyl)imidazo[1,2-b]pyridazin-8-amine

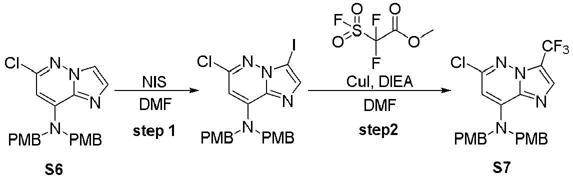

### Step 1: Preparation of 6-chloro-3-iodo-N,N-bis(4-methoxybenzyl)imidazo[1,2-b]pyridazin-8-amine

**Intermediate S6** (120 g, 294 mmol) was dissolved in DMF (800 mL), and then NIS (66 g, 294 mmol) was added and stirred at 60 °C for 16 hours. Sodium thiosulfate solution (8000 mL) was added to the reaction mixture, and the product was extracted three times with EtOAc (1000 mL). The organic layer was washed three times with brine (8000 mL), dried over Na₂SO₄, filtered, and concentrated, and then the concentrate was purified by silica gel chromatography to obtain 6-chloro-3-iodo-*N,N*-bis(4-methoxybenzyl)imidazo[1,2-*b*]pyridazin-8-amine (139 g, 88.53 % yield). MS: m/z = 535.1 (M+1, ESI+).

### Step 2: Preparation of 6-chloro-N,N-bis(4-methoxybenzyl)-3-(trifluoromethyl)imidazo[1,2-b]pyridazin-8-amine

The product obtained in Step 1 (139 g, 260.3 mmol) and methyl 2,2-difluoro-2-(fluorosulfonyl)acetate (250 g, 1.3 mol) were dissolved in DMF (1000 mL), and then CuI (123.6 g, 650.8 mmol) and DIEA (226 mL, 1.3 mol) were added and stirred at 85 °C for 16 hours. Water (8000 mL) was added to the reaction mixture, and the product was extracted three times with EtOAc (2000 mL). The organic layer was washed three times with brine (8000 mL), dried over Na₂SO₄, filtered, and concentrated, and then the concentrate was purified by silica gel chromatography to obtain **Intermediate S7** (106 g, 85.48 % yield). MS: m/z = 476.1 (M+1, ESI+).

### Preparation of Intermediate S8: ethyl N-(6-chloro-3-(trifluoromethyl)imidazo[1,2-b]pyridazin-8-yl)-N-(4-methoxybenzyl)glycinate

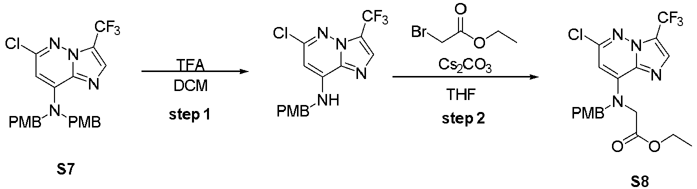

### Step 1: Preparation of 6-chloro-N-(4-methoxybenzyl)-3-(trifluoromethyl)imidazo[1,2-b]pyridazin-8-amine

**Intermediate S7** (106 g, 222.7 mmol) was dissolved in DCM (600 mL), and then TFA (200 mL) was added and stirred at 25 °C for 1 hour. Sodium bicarbonate solution (3000 mL) was added to the reaction mixture, and the product was extracted three times with DCM (500 mL). The organic layer was washed three times with brine (3000 mL), dried over Na₂SO₄, filtered, and concentrated, and then the concentrate was purified by silica gel chromatography to obtain 6-chloro-N-(4-methoxybenzyl)-3-(trifluoromethyl)imidazo[1,2-b]pyridazin-8-amine (72 g, 90.8 % yield). MS: m/z = 357.2 (M+1, ESI+).

### Step 2: Preparation of ethyl N-(6-chloro-3-(trifluoromethyl)imidazo[1,2-b]pyridazin-8-yl)-N-(4-methoxybenzyl)glycinate

The product obtained in Step 1 (29 g, 81.5 mmol) and ethyl 2-bromoacetate (20.4 g, 122.2 mmol) were dissolved in THF (200 mL), and then cesium carbonate (53.14 g, 163 mmol) was added and stirred at 70 °C for 16 hours. Water (800 mL) was added to the reaction mixture, and the product was extracted three times with EtOAc (200 mL). The organic layer was washed three times with brine (800 mL), dried over Na₂SO₄, filtered, and concentrated, and then the concentrate was purified by silica gel chromatography to obtain **Intermediate S8** (27 g, 75 % yield). MS: m/z = 443.1 (M+1, ESI+).

### Preparation of Intermediate S9: N-(4-methoxybenzyl)-N-(6-morpholino-3-(trifluoromethyl)imidazo[1,2-b]pyridazin-8-yl)glycine

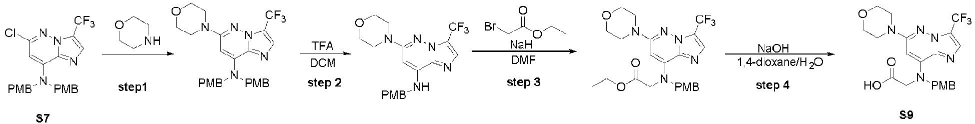

### Step 1 : Preparation of N,N-bis(4-methoxybenzyl)-6-morpholino-3-(trifluoromethyl)imidazo[1,2-b]pyridazin-8-amine

Morpholine (120 mL) was added to **Intermediate S7** (12 g, 25.2 mmol), and the mixture was stirred at 120 °C for 16 hours. After cooling and concentrating the reaction mixture, it was purified by silica gel chromatography to obtain *N,N*-bis(4-methoxybenzyl)-6-morpholino-3-(trifluoromethyl)imidazo[1,2-*b*]pyridazin-8-amine (5 g, 37.23 % yield). MS: m/z = 527.3 (M+1, ESI+).

### Step 2: Preparation of N-(4-methoxybenzyl)-6-morpholino-3-(trifluoromethyl)imidazo[1,2-b]pyridazin-8-amine

The product obtained in Step 1 (36 g, 68.31 mmol) was dissolved in DCM (300 mL), then TFA (100 mL) was added, and the mixture was stirred at 25 °C for 1 hour. Sodium bicarbonate solution (1000 mL) was added to the reaction mixture, and the product was extracted three times with DCM (100 mL). The organic layer was washed three times with brine (1000 mL), dried over Na₂SO₄, filtered, and concentrated, and then the concentrate was purified by silica gel chromatography to obtain *N*-(4-methoxybenzyl)-6-morpholino-3-(trifluoromethyl)imidazo[1,2-*b*]pyridazin-8-amine (27 g, 97.12 % yield). MS: m/z = 408.2 (M+1, ESI+).

### Step 3: Preparation of ethyl N-(4-methoxybenzyl)-N-(6-morpholino-3-(trifluoromethyl)imidazo[1,2-b]pyridazin-8-yl)glycinate

The product obtained in Step 2 (8.4 g, 20.62 mmol) was dissolved in DMF (100 mL), then NaH (1.24 g, 30.93 mmol, 60 % purity) was added in portions at 0 °C and stirred for 30 minutes. Ethyl 2-bromoacetate (5.17 g, 30.93 mmol) was added to this reaction mixture, and the mixture was stirred at 25 °C for 2 hours. Water (800 mL) was added to the reaction mixture, and the product was extracted three times with EtOAc (200 mL). The organic layer was washed three times with brine (800 mL), dried over Na₂SO₄, filtered, and concentrated, and then the concentrate was purified by silica gel chromatography to obtain ethyl *N*-(4-methoxybenzyl)-*N*-(6-morpholino-3-(trifluoromethyl)imidazo[1,2-*b*]pyridazin-8-yl)glycinate (8.2 g, 80.59 % yield). MS: m/z = 494.2 (M+1, ESI+).

### Step 4: Preparation of N-(4-methoxybenzyl)-N-(6-morpholino-3-(trifluoromethyl)imidazo[1,2-b]pyridazin-8-yl)glycine

The product obtained in Step 3 (8.2 g, 16.62 mmol) was dissolved in 1,4-dioxane (80 mL) and water (20 mL), then sodium hydroxide (1.6 g, 40 mmol) was added, and the mixture was stirred at 100 °C for 1 hour. 1 N hydrochloric acid (500 mL) was added to the reaction mixture, and the product was extracted three times with DCM (150 mL). The organic layer was washed three times with brine (500 mL), dried over Na₂SO₄, filtered, and concentrated, and then the concentrate was purified by silica gel chromatography to obtain **Intermediate S9** (7 g, 90.51 % yield). MS: m/z = 466.1 (M+1, ESI+).

### Preparation of Intermediate S10: N-(4-methoxybenzyl)-N-(6-morpholino-3-(trifluoromethyl)imidazo[1,2-b]pyridazin-8-yl)glycinonyl chloride

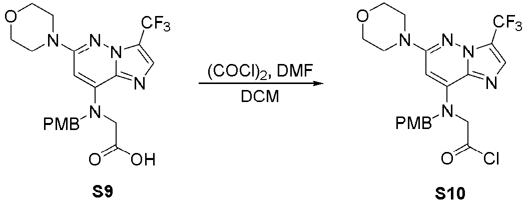

**Intermediate S9** (600 mg, 1.29 mmol) was dissolved in DMF (10 uL) and DCM (6 mL), then oxalyl chloride ((COCl)₂, 1.42 mmol, 0.12 mL) was added and stirred at 25 °C for 1 hour. The reaction mixture was concentrated to obtain **Intermediate S10** (600 mg, crude) as a yellow solid.

### Preparation of Intermediate S11: ethyl (6-(6-methyl-3,6-diazabicyclo[3.1.1]heptan-3-yl)-3-(trifluoromethyl)imidazo[1,2-b]pyridazin-8-yl)glycinate

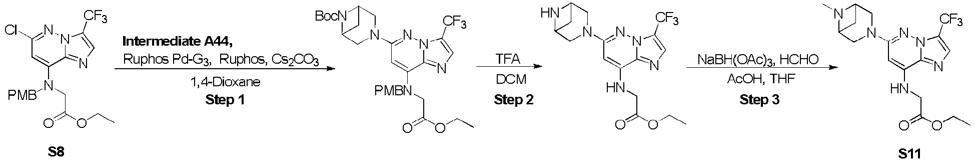

### Step 1: Preparation of tert-butyl 3-(8-((2-ethoxy-2-oxoethyl)(4-methoxybenzyl)amino)-3-(trifluoromethyl)imidazo[1,2-b]pyridazin-6-yl)-3,6-diazabicyclo[3.1.1]heptane-6-carboxylate

**Intermediate S8** (8.5 g, 19.23 mmol) and **Intermediate A44** (11.42 g, 57.69 mmol) were dissolved in 1,4-dioxane (100 mL), then RuPhos-Pd-G3 (1.6 g, 1.92 mmol), RuPhos (1.8 g, 3.84 mmol), and cesium carbonate (18.75 g, 57.69 mmol) were added, and the mixture was stirred at 110 °C for 48 hours under N₂ atmosphere. Water (500 mL) was added to the reaction mixture, and the product was extracted three times with EtOAc (100 mL). The organic layer was washed three times with brine (500 mL), dried over Na₂SO₄, filtered, and concentrated, and then the concentrate was purified by silica gel chromatography to obtain *tert*-butyl 3-(8-((2-ethoxy-2-oxoethyl)(4-methoxybenzyl)amino)-3-(trifluoromethyl)imidazo[1,2-*b*]pyridazin-6-yl)-3,6-diazabicyclo[3.1.1]heptane-6-carboxylate (1.2 g, 10.34 % yield) as a yellow solid. MS: m/z = 605.3 (M+1, ESI+).

### Step 2: Preparation of ethyl (6-(3,6-diazabicyclo[3.1. 1]heptan-3-yl)-3-(trifluoromethyl)imidazo[1,2-b]pyridazin-8-yl)glycinate

The product obtained in Step 1 (1.2 g, 1.98 mmol) was dissolved in DCM (18 mL), then TFA (6 mL) was added and stirred at 25 °C for 2 hours. Aqueous sodium bicarbonate solution (200 mL) was added to the reaction mixture, and the product was extracted three times with DCM (50 mL). The organic layer was washed three times with brine (200 mL), dried over Na₂SO₄, filtered, and concentrated, and then the concentrate was purified by silica gel chromatography to obtain ethyl (6-(3,6-diazabicyclo[3.1.1]heptan-3-yl)-3-(trifluoromethyl)imidazo[1,2-*b*]pyridazin-8-yl)glycinate (600 mg, 78.95 % yield) as a yellow solid. MS: m/z = 385.0 (M+1, ESI+).

### Step 3: Preparation of ethyl (6-(6-methyl-3,6-diazabicyclo[3.1.1]heptan-3-yl)-3-(trifluoromethyl)imidazo[1,2-b]pyridazin-8-yl)glycinate

The product obtained in Step 2 (600 mg, 1.56 mmol) was dissolved in THF (12 mL), then formaldehyde (163.8 mg, 1.638 mmol) and AcOH (2 drops) were added and stirred at 25 °C for 0.5 hours, after which NaBH(OAc)₃ (494 mg, 2.34 mmol) was added and stirred at 25 °C for 16 hours. Water (100 mL) was added to the reaction mixture, and the product was extracted three times with EtOAc (30 mL). The organic layer was washed three times with brine (100 mL), dried over Na₂SO₄, filtered, and concentrated, and the concentrate was purified by silica gel chromatography to obtain **Intermediate S11** (500 mg, 80.26 % yield) as a yellow solid. MS: m/z = 399.1 (M+1, ESI+).

### Preparation of Intermediate S12: ethyl (6-(3-methyl-3,6-diazabicyclo[3.1.1]heptan-6-yl)-3-(trifluoromethyl)imidazo [1,2-b] pyridazin-8-yl)glycinate

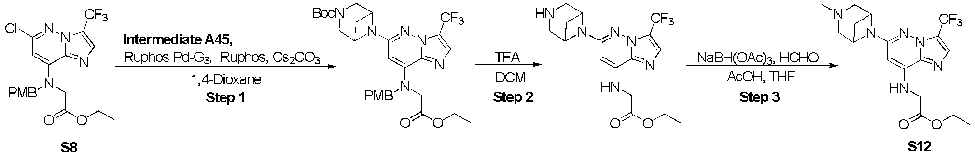

**Intermediate S12** (220 mg, 64.32% yield) was obtained as a yellow solid by following the same method as described in Steps 1 to 3 for the preparation of intermediate S11, except that **intermediate A45** (1.88 g, 9.48 mmol) was used instead of **intermediate A44** (11.42 g, 57.69 mmol) in Step 1. MS: m/z = 399.1 (M+1, ESI+).

### Preparation of Intermediate A7: 3-methyl-3,8-diazabicyclo[3.2.1]octan-8-ium trifluoroacetate salt

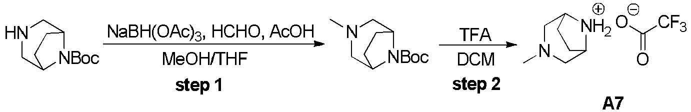

### Step 1: Preparation of tert-butyl 3-methyl-3,8-diazabicyclo[3.2.1]octane-8-carboxylate

*tert*-butyl 3,8-diazabicyclo[3.2.1]octane-8-carboxylate (7 g, 33.13 mmol) and formaldehyde (38.15 g, 508.23 mmol, 35.00 mL, 40 % purity) were dissolved in methanol (50 mL) and THF (50 mL), then AcOH (2.14 g, 16.56 mmol) was added and stirred at 25 °C for 1 hour. NaBH(OAc)₃ (10.53 g, 49.69 mmol) was added to the reaction mixture and stirred at 25 °C for 16 hours. The reaction mixture was concentrated, and the concentrate was purified by silica gel chromatography to obtain *tert*-butyl 3-methyl-3,8-diazabicyclo[3.2.1]octane-8-carboxylate (6.6 g, 88.42 % yield).

### Step 2: Preparation of 3-methyl-3,8-diazabicyclo[3.2.1]octan-8-ium trifluoroacetate salt

The product obtained in Step 1 (7.6 g, 33.58 mmol) was dissolved in DCM (30 mL), then TFA (12.9 mL, 167.91 mmol) was added and stirred at 25 °C for 16 hours. The reaction mixture was concentrated to obtain **Intermediate A7** (9.4 g, crude).

### Preparation of Intermediate A16: azetidine-3-carbonitrile trifluoroacetate salt

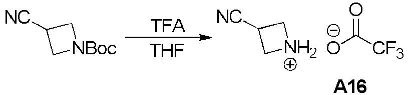

*tert*-butyl 3-cyanoazetidine-1-carboxylate (3 g, 16.46 mmol) was dissolved in THF (30 mL), then TFA (10 mL) was added and stirred at 25 °C for 16 hours. This reaction mixture was concentrated to obtain **Intermediate A16** (1.8 g, crude). MS: m/z = 83.2 (M+1, ESI+).

### Preparation of Intermediate A20: 3-methyl-8-azabicyclo[3.2.1]octan-3-ol

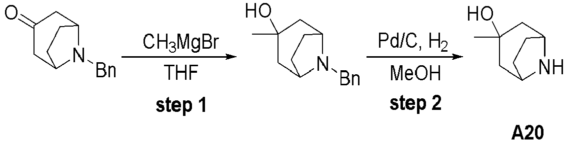

### Step 1: Preparation of 8-benzyl-3-methyl-8-azabicyclo[3.2.1]octan-3-ol

8-benzyl-8-azabicyclo[3.2.1]octan-3-one (10.00 g, 46.45 mmol) was dissolved in THF (100 mL), then 3 M methylmagnesium bromide solution (39 mL) was slowly added at 0 °C over 30 minutes, and the mixture was stirred at 25 °C for 3 hours. Ice water (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain 8-benzyl-3-methyl-8-azabicyclo[3.2.1]octan-3-ol (6 g, 55.84 % yield). MS: m/z = 232.2 (M+1, ESI+).

### Step 2: Preparation of 3-methyl-8-azabicyclo[3.2.1]octan-3-ol

The product obtained in Step 1 (6 g, 25.94 mmol) was dissolved in methanol (50 mL), then Pd/C (1 g) was added and stirred at 25 °C under H₂ atmosphere for 16 hours. This reaction mixture was filtered and concentrated to obtain **Intermediate A20** (3 g, 81.91 % yield). MS: m/z = 142.1 (M+1, ESI+).

### Preparation of Intermediate A26: 4-cyanopiperidin-1-ium trifluoroacetate salt

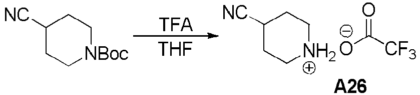

*tert*-butyl 4-cyanopiperidine-1-carboxylate (3 g, 14.27 mmol) was dissolved in THF (20 mL), then TFA (5 mL) was added and stirred at 25 °C for 16 hours. This reaction mixture was concentrated to obtain **Intermediate A26** (2.8 g, crude).

### Preparation of Intermediate A29: 2-azaspiro[3.3]heptan-6-ol trifluoroacetate salt

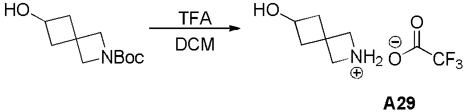

*tert*-butyl 6-hydroxy-2-azaspiro[3.3]heptane-2-carboxylate (4 g, 18.76 mmol) was dissolved in DCM (30 mL), then TFA (10 mL) was added and the mixture was stirred at 25 °C for 16 hours. This reaction mixture was concentrated to obtain **Intermediate A29** (2.5 g, crude). MS: m/z = 113.9 (M+1, ESI+).

### Preparation of Intermediate A31: pyrrolidine-3-carbonitrile trifluoroacetate salt

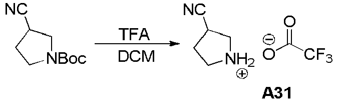

*tert*-butyl 3-cyanopyrrolidine-1-carboxylate (1.8 g, 9.17 mmol) was dissolved in DCM (15 mL), then TFA (3 mL) was added and the mixture was stirred at 25 °C for 1 hour. This reaction mixture was concentrated to obtain **Intermediate A31** (1.12 g, crude). MS: m/z = 97.1 (M+1, ESI+).

### Preparation of Intermediate A32: 1-(azetidin-3-yl)cyclopropan-1-ol

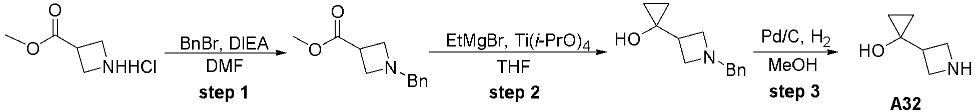

### Step 1: Preparation of methyl 1-benzylazetidine-3-carboxylate

Methyl azetidine-3-carboxylate hydrochloride (20 g, 132 mmol) and BnBr (17.22 mL, 145 mmol) were dissolved in DMF (300 mL), then DIEA (115 mL, 660 mmol) was added in several portions, and the mixture was stirred at 25 °C for 16 hours. Aqueous ammonium chloride solution (2000 mL) was added to the reaction mixture, and the mixture was extracted three times with EtOAc (400 mL). The organic layer was washed three times with brine (1000 mL), dried over Na₂SO₄, filtered, and concentrated, and then the concentrate was purified by silica gel chromatography to obtain methyl 1-benzylazetidine-3-carboxylate (22 g, 81.24 % yield). MS: m/z = 205.9 (M+1, ESI+).

### Step 2: Preparation of 1-(1-benzylazetidin-3-yl)cyclopropan-1-ol

The product obtained in Step 1 (16 g, 77.95 mmol) was dissolved in THF (200 mL), then Ti(*i*-PrO)₄ (26.59 g, 93.54 mmol) was added, and 1 M ethylmagnesium bromide solution (311.81 mL) was slowly added over 30 minutes at -60 °C under Ar atmosphere. This reaction mixture was warmed to room temperature and stirred for 16 hours. Aqueous ammonium chloride solution (1000 mL) was added to the reaction mixture, and the mixture was extracted three times with EtOAc (300 mL). The organic layer was washed with brine (100 mL), dried over Na₂SO₄, filtered, and concentrated, and then the concentrate was purified by silica gel chromatography to obtain 1-(1-benzylazetidin-3-yl)cyclopropan-1-ol (9.5 g, 59.95 % yield). MS: m/z = 204.2 (M+1, ESI+).

### Step 3: Preparation of 1-(azetidin-3-yl)cyclopropan-1-ol

The product obtained in Step 2 (9.5 g, 46.73 mmol) was dissolved in methanol (120 mL), then Pd/C (4.5 g, 10 % purity) was added and the mixture was stirred at 60 °C under H₂ atmosphere for 16 hours. This reaction mixture was filtered and concentrated to obtain **Intermediate A32** (4.1 g, 77.53 % yield). ¹H NMR (400 MHz, MeOD) δ 4.13 (dd, 4H), 2.76-2.62 (m, 1H), 0.79-0.76 (m, 2H), 0.54-0.47 (m, 2H); MS: m/z = 113.9 (M+1, ESI+).

### Preparation of Intermediate A36: benzyl azetidin-3-yl(cyclopropyl)carbamate

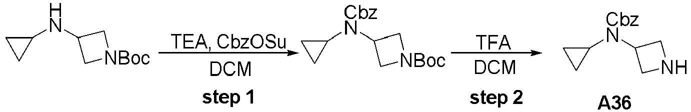

### Step 1: Preparation of tert-butyl 3-(((benzyloxy)carbonyl)(cyclopropyl)amino)azetidine-1-carboxylate

*tert*-butyl 3-(cyclopropylamino)azetidine-1-carboxylate (5 g, 23.55 mmol) and CbzOSu (8.80 g, 35.33 mmol) were dissolved in DCM (80 mL), then TEA (9.85 mL, 70.66 mmol) was added and stirred at 25 °C for 16 hours. Water (200 mL) was added to the reaction mixture, and the product was extracted three times with DCM (60 mL). The organic layer was washed three times with brine (200 mL), dried over Na₂SO₄, filtered, and concentrated, and then the concentrate was purified by silica gel chromatography to obtain *tert-butyl* 3-(((benzyloxy)carbonyl)(cyclopropyl)amino)azetidine-1-carboxylate (7 g, 85.79 % yield). MS: m/z = 347.1 (M+1, ESI+).

### Step 2: Preparation of benzyl azetidin-3-yl(cyclopropyl)carbamate

The product obtained in Step 1 (7 g, 20.21 mmol) was dissolved in DCM (60 mL), then TFA (20 mL) was added and stirred at 25 °C for 16 hours. After concentrating this reaction mixture, it was adjusted to pH 8-9 and extracted three times with DCM (80 mL). The organic layer was washed three times with brine (200 mL), dried over Na₂SO₄, filtered, and concentrated, and then the concentrate was purified by silica gel chromatography to obtain **Intermediate A36** (4 g, 80.37 % yield). MS: m/z = 247.2 (M+1, ESI+).

### Preparation of Intermediate A37: 3-((tert-butyldimethylsilyl)oxy)azetidine

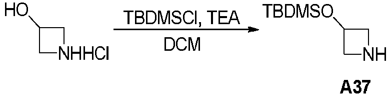

Azetidin-3-ol hydrochloride (5 g, 46 mmol) and TBDMSCl (13.8 g, 92 mmol) were dissolved in DCM (50 mL), then TEA (19.10 mL, 137 mmol) was added and stirred at 25 °C for 16 hours. Water (300 mL) was added to the reaction mixture, and the product was extracted three times with DCM (80 mL). The organic layer was washed three times with brine (800 mL), washed three times with brine (300 mL), dried over Na₂SO₄, filtered, and concentrated, and then the concentrate was purified by silica gel chromatography to obtain **Intermediate A37** (5.8 g, 67.83 % yield). MS: m/z = 188.2 (M+1, ESI+).

### Preparation of Intermediate A38: 3-(((tert-butyldiphenylsilyl)oxy)methyl)azetidine trifluoroacetate

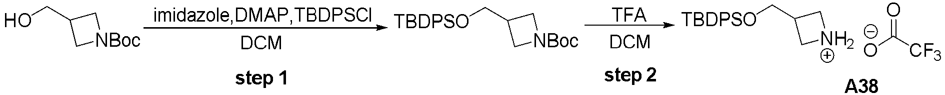

### Step 1: Preparation of tert-butyl 3-(((tert-butyldiphenylsilyl)oxy)methyl)azetidine-1-carboxylate

3-(hydroxymethyl)azetidine-1-carboxylate (5 g, 26.70 mmol) and TBDPSC1 (4.66 g, 40.06 mmol) were dissolved in DCM (80 mL), then DMAP (326 mg, 2.67 mmol) and imidazole (2.73 g, 40.06 mmol) were added and stirred at 25 °C for 2 hours. Water (300 mL) was added to the reaction mixture, and the product was extracted three times with DCM (80 mL). The organic layer was washed three times with brine (300 mL), dried over Na₂SO₄, filtered, and concentrated, and then the concentrate was purified by silica gel chromatography to obtain *tert-*butyl 3-(((*tert-*butyldiphenylsilyl)oxy)methyl)azetidine-1-carboxylate (8 g, 70.38% yield). MS: m/z = 426.0 (M+1, ESI+).

### Step 2: Preparation of 3-(((tert-butyldiphenylsilyl)oxy)methyl)azetidine trifluoroacetate

The product obtained in Step 1 (8 g, 18.80 mmol) was dissolved in DCM (60 mL), then TFA (20 mL) was added and stirred at 25 °C for 16 hours. After concentrating the reaction mixture, **Intermediate A38** (6 g, crude) was obtained. MS: m/z = 325.0 (M+1, ESI+).

### Preparation of Intermediate A40: (R)-3-((tert-butyldimethylsilyl)oxy)pyrrolidine

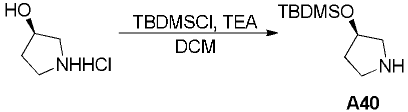

Using *(R)*-pyrrolidin-3-ol hydrochloride (5 g, 40.46 mmol) as a starting material, **Intermediate** A40 (6.2 g, 76.10% yield) was prepared according to the method described for Intermediate A37. MS: m/z = 202.0 (M+1, ESI+).

### Preparation of Intermediate A41: (S)-3-((tert-butyldimethylsilyl)oxy)pyrrolidine

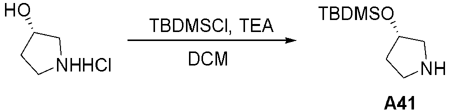

Using *(S)*-pyrrolidin-3-ol hydrochloride (11 g, 126 mmol) as a starting material, **Intermediate A41** (15 g, 58.99% yield) was prepared according to the method described for Intermediate A37. MS: m/z = 202.2 (M+1, ESI+).

### Preparation of Intermediate A47: 8-azaspiro[bicyclo[3.2.1]octane-3,2'-[1,3]dioxolane]

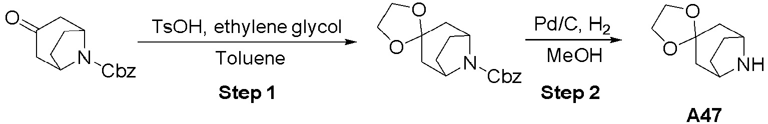

### Step 1: Preparation of benzyl 8-azaspiro[bicyclo[3.2.1]octane-3,2'-[1,3]dioxolane]-8-carboxylate

Benzyl 3-oxo-8-azabicyclo[3.2.1]octane-8-carboxylate (10 g, 38.61 mmol) was dissolved in toluene (60 mL), and then TsOH (664 mg, 3.86 mmol) and ethylene glycol (11.97 g, 193.05 mmol) were added and stirred at 120 °C for 48 hours. Water (500 mL) was added to the reaction mixture, and the product was extracted three times with EtOAc (100 mL). The organic layer was washed three times with brine (500 mL), dried over Na₂SO₄, filtered, and concentrated, and then the concentrate was purified by silica gel chromatography to obtain benzyl 8-azaspiro[bicyclo[3.2.1]octane-3,2'-[1,3]dioxolane]-8-carboxylate (6.8 g, 58.27 % yield) as a yellow solid. MS: m/z = 304.1 (M+1, ESI+).

### Step 2: Preparation of 8-azaspiro[bicyclo[3.2.1]octane-3,2'-[1,3]dioxolane]

The product obtained in Step 1 (6.8 g, 22.44 mmol) was dissolved in methanol (50 mL), then Pd/C (1 g) was added and stirred at 25 °C under H₂ atmosphere for 16 hours. The reaction mixture was concentrated and filtered, and the concentrate was purified by silica gel chromatography to obtain **Intermediate A47** (3.7 g, 97.63 % yield) as a yellow solid. MS: m/z = 170.1 (M+1, ESI+).

### Preparation of Intermediate B6: 4-chloro-5-methoxybenzene-1,2-diamine

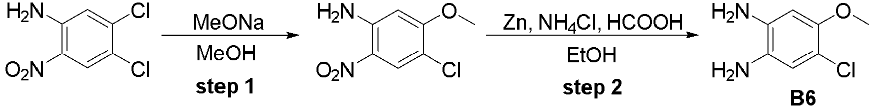

### Step 1: Preparation of 4-chloro-5-methoxy-2-nitroaniline

4,5-dichloro-2-nitro-aniline (4 g, 19.32 mmol) was dissolved in methanol (25 mL), then sodium methoxide (1.05 g, 19.32 mmol) was added and stirred at 70 °C for 4 hours. Ice water (200 mL) was added to the reaction mixture, and the product was extracted three times with EtOAc (80 mL). The organic layer was washed three times with brine (200 mL), dried over Na₂SO₄, filtered, and concentrated, and then the concentrate was purified by silica gel chromatography to obtain 4-chloro-5-methoxy-2-nitroaniline (3.4 g, 86.85 % yield). MS: m/z = 203.1 (M+1, ESI+).

### Step 2: Preparation of 4-chloro-5-methoxybenzene-1,2-diamine

The product obtained in Step 1 (3.4 g, 16.78 mmol) was dissolved in ethanol (50 mL), then ammonium chloride (13.5 g, 252 mmol), zinc (16.4 g, 252 mmol), and formic acid (1.98 mL, 50.4 mmol) were added at 0 °C and stirred at 25 °C for 2 hours. The reaction mixture was filtered and concentrated, then ice water (200 mL) was added, and the product was extracted three times with EtOAc (80 mL). The organic layer was washed three times with brine (200 mL), dried over Na₂SO₄, filtered, and concentrated. The concentrate was purified by silica gel chromatography to obtain **Intermediate B6** (2.3 g, 79.40 % yield). MS: m/z = 173.1 (M+1, ESI+).

### Preparation of Intermediate B15: 4-methyl-5-(trifluoromethyl)benzene-1,2-diamine

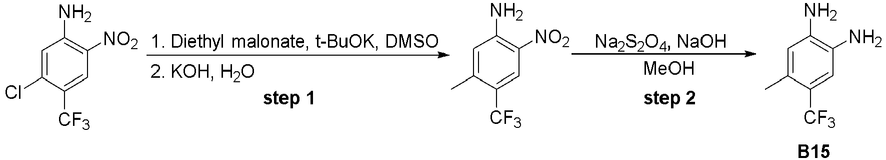

### Step 1: Preparation of 5-methyl-2-nitro-4-(trifluoromethyl)aniline

5-chloro-2-nitro-4-(trifluoromethyl)aniline (10 g, 41.57 mmol) was dissolved in dimethyl sulfoxide (30 mL), then diethyl malonate (6.65 g, 41.57 mmol) and potassium tert-butoxide (11.66 g, 103.92 mmol) were added and stirred at 60 °C for 16 hours. After cooling the reaction solution to room temperature, potassium hydroxide (10.96 g, 195 mmol) and water (30 mL) were added, and the mixture was stirred again at 60 °C for 16 hours. Water (400 mL) was added to the reaction mixture, and the product was extracted three times with EtOAc (80 mL). The organic layer was washed three times with brine (400 mL), dried over Na₂SO₄, filtered, and concentrated, and then the concentrate was purified by silica gel chromatography to obtain 5-methyl-2-nitro-4-(trifluoromethyl)aniline (5.97 g, 65.24 % yield). MS: m/z = 220.2 (M+1, ESI+).

### Step 2: Preparation of 4-methyl-5-(trifluoromethyl)benzene-1,2-diamine

The product obtained in Step 1 (5.97 g, 27.12 mmol) was dissolved in methanol (20 mL), then sodium hydrosulfite (23.61 g, 135.59 mmol) and sodium hydroxide (3.25 g, 81.35 mmol) were added and stirred at 25 °C for 16 hours. Water (200 mL) was added to the reaction mixture, and the product was extracted three times with EtOAc (50 mL). The organic layer was washed three times with brine (200 mL), dried over Na₂SO₄, filtered, and concentrated, and then the concentrate was purified by silica gel chromatography to obtain **Intermediate B15** (2.13 g, 41.30 % yield). MS: m/z = 191.1 (M+1, ESI+).

### Preparation of Intermediate B16: 4,5-bis(trifluoromethyl)benzene-1,2-diamine

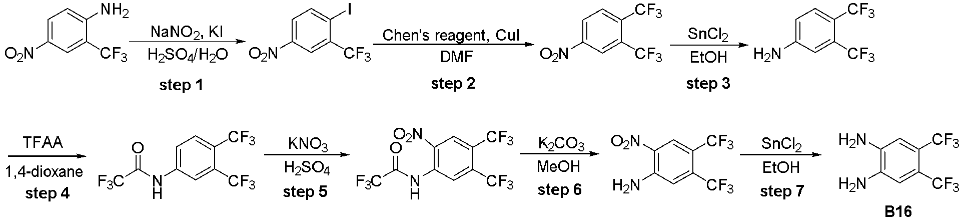

### Step 1: Preparation of 1-iodo-4-nitro-2-(trifluoromethyl)benzene

4-nitro-2-(trifluoromethyl)aniline (50 g, 242 mmol) was dissolved in water (600 mL) and sulfuric acid (400 mL) at 0 °C, and then a solution of sodium nitrite (33.48 g, 484 mmol) dissolved in water (160 mL) was slowly added. After stirring at the same temperature for 1 hour, a solution of potassium iodide (KI, 181.8 g, 1212 mmol) dissolved in water (1000 mL) was added, and the mixture was stirred at 25 °C for 16 hours. The reaction mixture was filtered, washed, and concentrated to obtain 1-iodo-4-nitro-2-(trifluoromethyl)benzene (74 g, 96.17 % yield). ¹H NMR (400 MHz, CDCl₃) δ 8.48 (d, 1H), 8.28 (d, 1H), 8.05 (dd, 1H).

### Step 2: Preparation of 4-nitro-1,2-bis(trifluoromethyl)benzene

The product obtained in Step 1 (74 g, 233.4 mmol) and methyl 2,2-difluoro-2-(fluorosulfonyl)acetate (Chen's reagent, 224.2 g, 1167.2 mmol) were dissolved in DMF (800 mL), then CuI (44.4 g, 233.4 mmol) was added and stirred at 80 °C for 16 hours under N₂ atmosphere. Water (8000 mL) was added to the reaction mixture, and the product was extracted three times with EtOAc (2000 mL). The organic layer was washed three times with brine (8000 mL), dried over Na₂SO₄, filtered, and concentrated, and then the concentrate was purified by silica gel chromatography to obtain 4-nitro-1,2-bis(trifluoromethyl)benzene (26 g, 42.97 % yield).

### Step 3: Preparation of 3,4-bis(trifluoromethyl)aniline

The product obtained in Step 2 (26 g, 100.4 mmol) and tin(II) chloride (SnCl₂, 114.2 g, 602.1 mmol) were dissolved in ethanol (2000 mL), and then stirred at 70 °C for 16 hours under N₂ atmosphere. Water (800 mL) was added to the reaction mixture, and the product was extracted three times with EtOAc (200 mL). The organic layer was washed three times with brine (800 mL), dried over Na₂SO₄, filtered, and concentrated, and then the concentrate was purified by silica gel chromatography to obtain 3,4-bis(trifluoromethyl)aniline (21 g, 91.3 % yield).

### Step 4: Preparation of N-(3,4-bis(trifluoromethyl)phenyl)-2,2,2-trifluoroacetamide

The product obtained in Step 3 (21.2 g, 92.54 mmol) was dissolved in 1,4-dioxane (150 mL), then trifluoroacetic anhydride (TFAA, 38.88 g, 185.05 mmol, 25.73 mL) was added and stirred at 25 °C for 16 hours. Water (1000 mL) was added to the reaction mixture, and the product was extracted three times with EtOAc (300 mL). The organic layer was washed three times with brine (1000 mL), dried over Na₂SO₄, filtered, and concentrated, and then the concentrate was purified by silica gel chromatography to obtain *N*-(3,4-bis(trifluoromethyl)phenyl)-2,2,2-trifluoroacetamide (21.7 g, 72.14 % yield). MS: m/z = 324.1 (M -1, ESI-).

### Step 5: Preparation of 2,2,2-trifluoro-N-(2-nitro-4,5-bis(trifluoromethyl)phenyl)acetamide

The product obtained in Step 4 (21.6 g, 66.31 mmol) was dissolved in sulfuric acid (200 mL), and then potassium nitrate (13.42 g, 132.62 mmol) was added at 0 °C and stirred at 25 °C for 16 hours. Ice water (2000 mL) was added to the reaction mixture, and the product was extracted three times with EtOAc (400 mL). The organic layer was washed three times with brine (2000 mL), dried over Na₂SO₄, filtered, and concentrated, and then the concentrate was purified by silica gel chromatography to obtain 2,2,2-trifluoro-N-(2-nitro-4,5-bis(trifluoromethyl)phenyl)acetamide (12.5 g, 50.83 % yield). MS: m/z = 369.0 (M -1, ESI-).

### Step 6: Preparation of 2-nitro-4,5-bis(trifluoromethyl)aniline

The product obtained in Step 5 (12.5 g, 33.77 mmol) was dissolved in methanol (150 mL), and then potassium carbonate (23.3 g, 168.85 mmol) was added and stirred at 25 °C for 16 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain 2-nitro-4,5-bis(trifluoromethyl)aniline (10.4 g, crude). MS: m/z = 273.0 (M-1, ESI-).

### Step 7: Preparation of 4,5-bis(trifluoromethyl)benzene-1,2-diamine

The product obtained in Step 6 (10.4 g, 37.96 mmol) and tin(II) chloride (SnCl₂, 43.18 g, 227.74 mmol) were dissolved in ethanol (100 mL), and then stirred at 70 °C for 16 hours under N₂ atmosphere. Water (800 mL) was added to the reaction mixture, and the product was extracted three times with EtOAc (200 mL). The organic layer was washed three times with brine (800 mL), dried over Na₂SO₄, filtered, and concentrated, and then the concentrate was purified by silica gel chromatography to obtain **Intermediate B16** (8.5 g, 94.55 % yield). MS: m/z = 243.0 (M-1, ESI-).

### Preparation of Intermediate B20: 5,6-dichloro-1-(phenylsulfonyl)-1H-indole-2-carbaldehyde

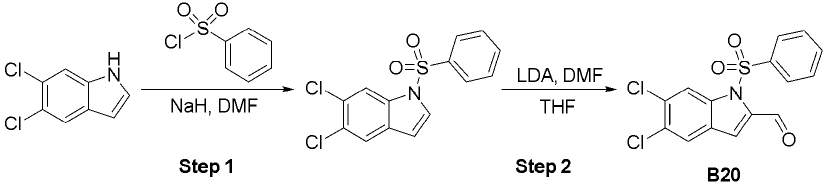

### Step 1: Preparation of 5,6-dichloro-1-(phenylsulfonyl)-1H-indole

5,6-dichloro-1*H*-indole (3 g, 16.13 mmol) was dissolved in DMF (30 mL), and then NaH (774 mg, 19.35 mmol, 60 % purity) was added in portions at 0 °C and stirred at the same temperature for 1 hour. After this, benzenesulfonyl chloride (4.27 g, 24.19 mmol) was added and stirred at room temperature for 2 hours. Water (300 mL) was added to the reaction mixture, and the product was extracted three times with EtOAc (60 mL). The organic layer was washed three times with brine (300 mL), dried over Na₂SO₄, filtered, and concentrated, and then the concentrate was purified by silica gel chromatography to obtain 5,6-dichloro-1-(phenylsulfonyl)-1*H*-indole (4.5 g, 85.56 % yield) as a white solid. ¹H NMR (400 MHz, DMSO-d₆) δ 8.13 (s, 1H), 8.09-8.03 (m, 2H), 7.97 (d, 1H), 7.93 (s, 1H), 7.74 (t, 1H), 7.64 (t, 2H), 6.87 (d, 1H).

### Step 2: Preparation of 5,6-dichloro-1-(phenylsulfonyl)-1H-indole-2-carbaldehyde

The product obtained in Step 1 (4.5 g, 13.80 mmol) was dissolved in THF (50 mL), and then 2 M lithium diisopropylamide solution (2 M LDA, 13.8 mL, 27.6 mmol) was slowly added at -60 °C and stirred for 1 hour at the same temperature. Thereafter, DMF (2.02 g, 27.59 mmol, 1.42 mL) was added and stirred at -60 °C for 2 hours. Water (200 mL) was added to the reaction mixture, and the mixture was extracted three times with EtOAc (40 mL). The organic layer was washed three times with brine (200 mL), dried over Na₂SO₄, filtered, and concentrated, and then the concentrate was purified by silica gel chromatography to obtain **Intermediate B20** (3 g, 61.39 % yield) as a yellow solid. ¹H NMR (400 MHz, DMSO-d₆) δ 8.12 (s, 1H), 8.05 (d, 2H), 7.98-7.93 (m, 2H), 7.74 (t, 1H), 7.63 (t, 2H), 6.86 (d, 1H); MS: m/z = 354.0 (M+1, ESI+).

### Preparation of Intermediate B22: 5,6-dimethyl-1-(phenylsulfonyl)-1H-indole-2-carbaldehyde

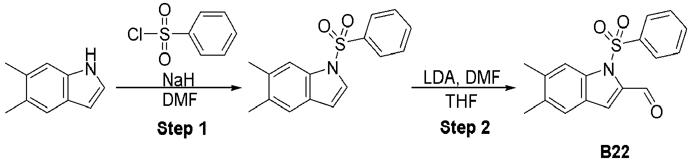

**Intermediate B22** (1.3 g, 33.82% yield) was obtained as a yellow solid by following the same method as described in Steps 1 and 2 for the preparation of intermediate B20, except that 5,6-dimethyl-1*H*-indole (2 g, 13.77 mmol) was used instead of 5,6-dichloro-1*H*-indole (3 g, 16.13 mmol) in Step 1.

¹H NMR (400 MHz, DMSO-d₆) δ 10.32 (s, 1H), 7.94 (s, 1H), 7.92-7.87 (m, 2H), 7.70 (t, 1H), 7.61-7.55 (m, 3H), 7.52 (s, 1H), 2.41 (s, 3H), 2.28 (s, 3H); MS: m/z = 314.1 (M+1, ESI+).

### Preparation of Intermediate B23: 4-fluoro-1-(phenylsulfonyl)-1H-indole-2-carbaldehyde

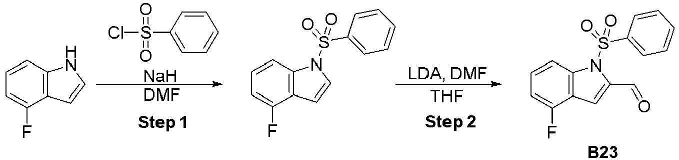

**Intermediate B23** (3.9 g, 64.36 % yield) was obtained as a yellow oil by following the same method as described in Steps 1 and 2 for the preparation of intermediate B20, except that 4-fluoro-1*H*-indole (5.1 g, 37.74 mmol) was used instead of 5,6-dichloro-1*H*-indole (3 g, 16.13 mmol) in Step 1.

### Preparation of Intermediate B24: 4,7-difluoro-1-(phenylsulfonyl)-1H-indole-2-carbaldehyde

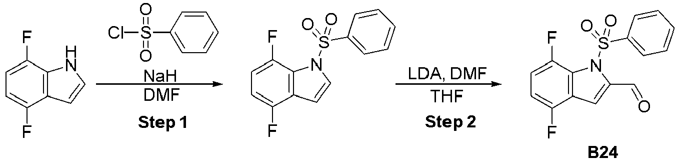

**Intermediate B24** (3 g, 78.24 % yield) was obtained as a yellow oil by following the same method as described in Steps 1 and 2 for the preparation of intermediate B20, except that 4,7-difluoro-1*H*-indole (2 g, 13.06 mmol) was used instead of 5,6-dichloro-1*H*-indole (3 g, 16.13 mmol) in Step 1. MS: m/z = 322.1 (M+1, ESI+).

### Preparation of Intermediate B25: 4,5,6,7-tetrahydro-1H-indole-2-carbaldehyde

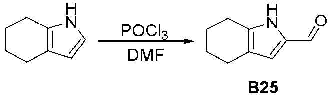

4,5,6,7-tetrahydro-1*H*-indole (2 g, 16.50 mmol) was dissolved in DMF (20 mL), then phosphoryl chloride (POCl₃, 2.46 mL, 26.41 mmol) was added in portions at -20 °C and stirred at room temperature for 2 hours. Water (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain **Intermediate B25** (1.9 g, 77.16 % yield). ¹H NMR (400 MHz, DMSO-d₆) δ 11.61 (s, 1H), 9.27 (s, 1H), 6.68 (d, 1H), 2.56 (t, 2H), 2.45 (t, 2H), 1.75-1.65 (m, 4H); MS: m/z = 150.1 (M+1, ESI+).

### Preparation of Intermediate B26: 7-hydroxy-1H-indole-2-carbaldehyde

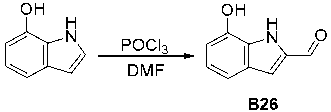

Following the same method as for the preparation of intermediate B25, **intermediate B26** (3.1 g, 85.37% yield) was obtained as a red solid, using 1*H*-indol-7-ol (3 g, 22.53 mmol) instead of 4,5,6,7-tetrahydro-1*H*-indole (2 g, 16.50 mmol). MS: m/z = 162.2 (M+1, ESI+).

### Preparation of Intermediate B27: 6-hydroxy-1H-indole-2-carbaldehyde

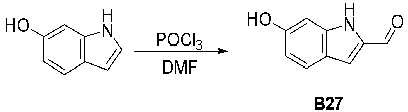

Following the same method as for the preparation of intermediate B25, **intermediate B27** (3 g, 82.62 % yield) was obtained as a yellow solid, using 1*H*-indol-6-ol (3 g, 22.53 mmol) instead of 4,5,6,7-tetrahydro-1*H*-indole (2 g, 16.50 mmol). MS: m/z = 162.1 (M+1, ESI+).

### Preparation of Intermediate B28: 7-nitro-1H-indole-2-carbaldehyde

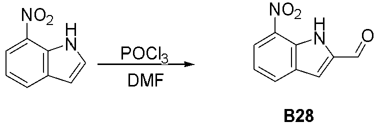

Following the same method as for the preparation of intermediate B25, **intermediate B28** (5 g, 96.90 % yield) was obtained as a yellow solid, using 7-nitro-1*H*-indole (4.4 g, 27.14 mmol) instead of 4,5,6,7-tetrahydro-1*H*-indole (2 g, 16.50 mmol). ¹H NMR (400 MHz, DMSO-d₆) δ 12.71 (s, 1H), 10.07 (s, 1H), 8.57 (d, 1H), 8.48 (s, 1H), 8.24 (d, 1H), 7.47 (t, 1H); MS: m/z = 191.1 (M+1, ESI+).

### Preparation of Intermediate B29: 6-nitro-1H-indole-2-carbaldehyde

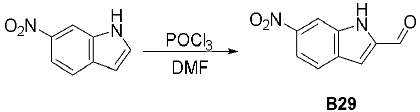

Following the same method as for the preparation of intermediate B25, **intermediate B29** (4 g, 50.76 % yield) was obtained as a yellow solid, using 6-nitro-1*H*-indole (4.2 g, 25.90 mmol) instead of 4,5,6,7-tetrahydro-1*H*-indole (2 g, 16.50 mmol). MS: m/z = 191.1 (M+1, ESI+).

### Preparation of Intermediate B30: 4,5,6,7-tetrahydro-1H-benzo[d]imidazole-2-carbaldehyde

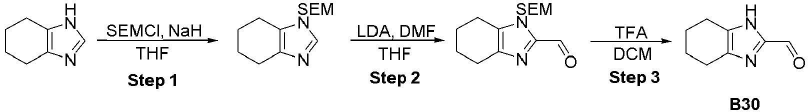

### Step 1: Preparation of 1-((2-(trimethylsilyl)ethoxy)methyl)-4,5,6,7-tetrahydro-1H-benzo[d]imidazole

4,5,6,7-tetrahydro-1*H*-benzo[*d*]imidazole (2.2 g, 18.01 mmol) was dissolved in THF (20 mL), then NaH (792 mg, 19.81 mmol, 60 % purity) was added in several portions at 0 °C and stirred at the same temperature for 1 hour. Thereafter, SEMCl (3.82 mL, 21.61 mmol) was added to the reaction mixture, and the mixture was stirred at room temperature for 16 hours. Water (300 mL) was added to the reaction mixture, and the product was extracted three times with EtOAc (60 mL). The organic layer was washed three times with brine (300 mL), dried over Na₂SO₄, filtered, and concentrated, and then the concentrate was purified by silica gel chromatography to obtain 1-((2-(trimethylsilyl)ethoxy)methyl)-4,5,6,7-tetrahydro-1*H*-benzo[*d*]imidazole (1.5 g, 33.0 % yield) as a yellow oil. MS: m/z = 253.3 (M+1, ESI+).

### Step 2: Preparation of 1-((2-(trimethylsilyl)ethoxy)methyl)-4,5,6,7-tetrahydro-1H-benzo[d]imidazole-2-carbaldehyde

1-((2-(trimethylsilyl)ethoxy)methyl)-4,5,6,7-tetrahydro-1*H*-benzo[*d*]imidazole-2-carbaldehyde (1.4 g, 84.01 % yield) was obtained as a yellow oil in the same method as Step 2 for the preparation of Intermediate B20, except that the product obtained in Step 1 (1.5 g, 5.94 mmol) was used. MS: m/z = 281.2 (M+1, ESI+).

### Step 3: Preparation of 4,5,6,7-tetrahydro-1H-benzo[d]imidazole-2-carbaldehyde

The product obtained in Step 2 (1.4 g, 4.99 mmol) was dissolved in DCM (12 mL), then TFA (4 mL) was added and stirred at 25 °C for 16 hours. Sodium bicarbonate solution (100 mL) was added to the reaction mixture, and the product was extracted three times with DCM (30 mL). The organic layer was washed three times with brine (200 mL), dried over Na₂SO₄, filtered, and concentrated to obtain **Intermediate B30** (700 mg, 93.37 % yield) as a yellow solid. ¹H NMR (400 MHz, DMSO-d₆) δ 13.11 (s, 1H), 9.46 (s, 1H), 2.56 (s, 4H), 1.76 (s, 4H); MS: m/z = 151.1 (M+1, ESI+).

### Example 1: Preparation of N-((6,7-difluoro-1H-benzo[d]imidazol-2-yl)methyl)-3-ethynyl-6-morpholinoimidazo[1,2-b]pyridazin-8-amine

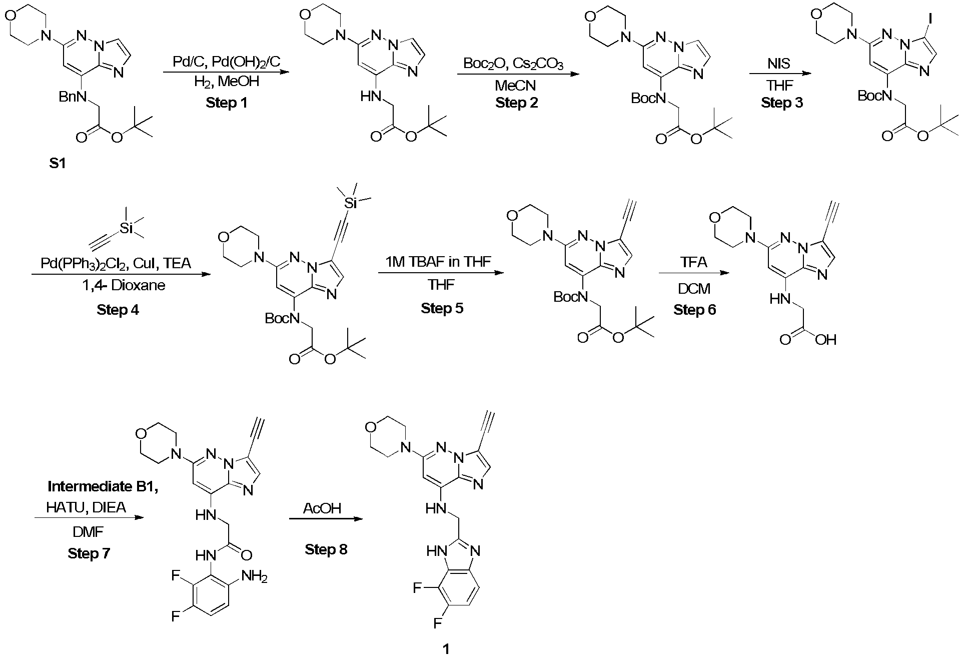

### Step 1: Preparation of tert-butyl (6-morpholinoimidazo[1,2-b]pyridazin-8-yl)glycinate

**Intermediate S1** (4.9 g, 11.57 mmol) was dissolved in methanol (70 mL), then Pd/C (1 g) and Pd(OH)₂/C (1 g) were added and stirred at 50 °C for 48 hours under H₂ atmosphere. After filtering and concentrating the reaction mixture, it was purified by silica gel chromatography to obtain *tert-*butyl (6-morpholinoimidazo[1,2-b]pyridazin-8-yl)glycinate (3 g, 77.78 % yield). MS: m/z = 334.1 (M+1, ESI+).

### Step 2: Preparation of tert-butyl-N-(tert-butoxycarbonyl)-N-(6-moipholinoimidazo[1,2-b]pyridazin-8-yl)glycinate

The product obtained in Step 1 (2.9 g, 10.26 mmol) and di-*tert*-butyl dicarbonate (8.95 g, 41.03 mmol) were dissolved in MeCN (60 mL), then cesium carbonate (13.37 g, 41.03 mmol) was added and stirred at 70 °C for 16 hours. Water (400 mL) was added to the reaction mixture, and the product was extracted three times with EtOAc (100 mL). The organic layer was washed three times with brine (400 mL), dried over Na₂SO₄, filtered, and concentrated, and then the concentrate was purified by silica gel chromatography to obtain *tert*-butyl-*N*-(*tert*-butoxycarbonyl)-*N*-(6-morpholinoimidazo[1,2-*b*]pyridazin-8-yl)glycinate (3.15 g, 80.22 % yield). MS: m/z = 434.2 (M+1, ESI+).

### Step 3: Preparation of tert-butyl N-(tert-butoxycarbonyl)-N-(3-iodo-6-morpholinoimidazo[1,2-b]pyridazin-8-yl)glycinate

The product obtained in Step 2 (3.15 g, 8.23 mmol) was dissolved in THF (50 mL), then NIS (1.63 g, 7.27 mmol) was added at 0 °C and stirred at 25 °C for 16 hours. Water (300 mL) was added to the reaction mixture, and the product was extracted three times with EtOAc (80 mL). This organic layer was washed three times with a sodium sulfite solution (400 mL), dried over Na₂SO₄, filtered, and concentrated, and then the concentrate was purified by silica gel chromatography to obtain *tert*-butyl *N*-(*tert*-butoxycarbonyl)-*N*-(3-iodo-6-morpholinoimidazo[1,2-*b*]pyridazin-8-yl)glycinate (3.83 g, 94.22 % yield). MS: m/z = 560.1 (M+1, ESI+).

### Step 4: Preparation of tert-butyl N-(tert-butoxycarbonyl)-N-(6-morpholino-3-((trimethylsilyl)ethynyl)imidazo[1,2-b]pyridazin-8-yl)glycinate

The product obtained in Step 3 (3.83 g, 6.85 mmol) and ethynyl(trimethyl)silane (1.01 g, 10.27 mmol) were dissolved in 1,4-dioxane (50 mL), then CuI (391 mg, 2.05 mmol), Pd(PPh₃)₂Cl₂ (481 mg, 685 umol), and TEA (2.86 mL, 20.54 mmol) were added and stirred at 50 °C for 4 hours under N₂ atmosphere. Water (400 mL) was added to the reaction mixture, and the product was extracted three times with EtOAc (80 mL). The organic layer was washed three times with brine (400 mL), dried over Na₂SO₄, filtered, and concentrated, and then the concentrate was purified by silica gel chromatography to obtain *tert*-butyl *N*-(*tert*-butoxycarbonyl)-*N*-(6-morpholino-3-((trimethylsilyl)ethynyl)imidazo[1,2-*b*]pyridazin-8-yl)glycinate (2.86 g, 78.86 % yield). MS: m/z = 530.2 (M+1, ESI+).

### Step 5: Preparation of tert-butyl N-(tert-butoxycarbonyl)-N-(3-ethynyl-6-morpholinoimidazo[1,2-b]pyridazin-8-yl)glycinate

The product obtained in Step 4 (2.86 g, 5.40 mmol) was dissolved in THF (30 mL), then 1 M TBAF solution (21.6 mL, 21.60 mmol) was added and stirred at 25 °C for 16 hours. Aqueous ammonium chloride solution (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain *tert*-butyl *N*-(*tert*-butoxycarbonyl)-*N*-(3-ethynyl-6-morpholinoimidazo[1,2-*b*]pyridazin-8-yl)glycinate (2 g, 80.96 % yield). MS: m/z = 458.2 (M+1, ESI+).

### Step 6: Preparation of (3-ethynyl-6-morpholinoimidazo[1,2-b]pyridazin-8-yl)glycine

The product obtained in Step 5 (2 g, 4.37 mmol) was dissolved in DCM (20 mL), then TFA (3.34 mL, 43.71 mmol) was added thereto, and the mixture was stirred at 25 °C for 16 hours. After concentrating the reaction mixture, the concentrate was purified by silica gel chromatography to obtain (3-ethynyl-6-morpholinoimidazo[1,2-*b*]pyridazin-8-yl)glycine (900 mg, 68.33 % yield). MS: m/z = 302.1 (M+1, ESI+).

### Step 7: Preparation of N-(6-amino-2,3-difluorophenyl)-2-((3-ethynyl-6-morpholinoimidazo[1,2-b]pyridazin-8-yl)amino)acetamide

The product obtained Step 6 (900 mg, 2.99 mmol) and **Intermediate B1** (517 mg, 3.58 mmol) were dissolved in DMF (20 mL), then HATU (1.70 g, 4.48 mmol) and DIEA (1.56 mL, 8.96 mmol) were added thereto, and the mixture was stirred at 25 °C for 16 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain N-(6-amino-2,3-difluorophenyl)-2-((3-ethynyl-6-morpholinoimidazo[1,2-b]pyridazin-8-yl)amino)acetamide (1 g, 78.33 % yield). MS: m/z = 428.1 (M+1, ESI+).

### Step 8: Preparation of N-((6,7-difluoro-1H-benzo[d]imidazol-2-yl)methyl)-3-ethynyl-6-morpholinoimidazo[1,2-b]pyridazin-8-amine

The product obtained in Step 7 (750 mg, 1.75 mmol) was dissolved in AcOH (10 mL), and then the mixture was stirred at 70 °C for 16 hours. Sodium bicarbonate solution (80 mL) was added to the reaction mixture, and the product was extracted three times with DCM (30 mL). The organic layer was washed three times with brine (80 mL), dried over Na₂SO₄, filtered, and concentrated, and then the concentrate was purified by Prep-HPLC to obtain **the compound of Example** 1 (28 mg, 3.90 % yield) as a white solid. ¹H NMR (400 MHz, DMSO-d₆) δ 12.69 (s, 1H), 7.71-7.67 (m, 2H), 7.23-7.18 (m, 2H), 6.09 (s, 1H), 4.82 (d, 2H), 4.72 (s, 1H), 3.67 (s, 4H), 3.32 (s, 4H); MS: m/z = 410.0 (M+1, ESI+).

### Example 2: Preparation of N-((6,7-difluoro-1H-benzo[d]imidazol-2-yl)methyl)-6-morpholino-3-(2,2,2-trifluoroethyl)imidazo[1,2-b]pyridazin-8-amine

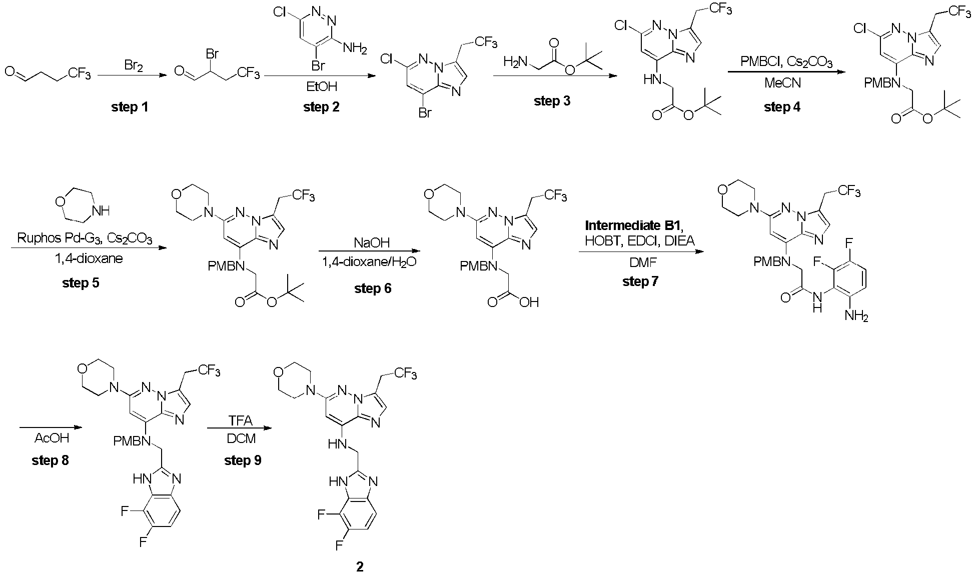

### Step 1: Preparation of 2-bromo-4,4,4-trifluorobutanal

4,4,4-trifluorobutanal (5 g, 39.66 mmol) was dissolved in 1,4-dioxane (50 mL), then bromine (2.62 mL, 47.59 mmol) was added at 0 °C and stirred at 25 °C for 1 hour under N₂ atmosphere. Water (200 mL) was added to the reaction mixture, and the product was extracted three times with EtOAc (50 mL). The organic layer was washed three times with brine (200 mL), dried over Na₂SO₄, filtered, and concentrated to obtain 2-bromo-4,4,4-trifluorobutanal (4.5 g, crude).

### Step 2: Preparation of 8-bromo-6-chloro-3-(2,2,2-trifluoroethyl)imidazo[1,2-b]pyridazine

The product obtained in Step 1 (4.5 g, 21.95 mmol) was dissolved in ethanol (50 mL), then 4-bromo-6-chloro-pyridazin-3-amine (915 mg, 4.39 mmol) was added and stirred at 120 °C for 16 hours. Water (300 mL) was added to the reaction mixture, and the product was extracted three times with EtOAc (100 mL). The organic layer was washed three times with brine (300 mL), dried over Na₂SO₄, filtered, and concentrated, and then the concentrate was purified by silica gel chromatography to obtain 8-bromo-6-chloro-3-(2,2,2-trifluoroethyl)imidazo[1,2-b]pyridazine (890 mg, 64.45 % yield). MS: m/z = 314.2 (M+1, ESI+).

### Step 3: Preparation of tert-butyl (6-chloro-3-(2,2,2-trifluoroethyl)imidazo[1,2-b]pyridazin-8-yl)glycinate

The product obtained in Step 2 (890 mg, 2.83 mmol) was dissolved in tert-butyl glycinate (10 mL), and then stirred at 120 °C for 16 hours. Water (100 mL) was added to the reaction mixture, and the product was extracted three times with EtOAc (40 mL). The organic layer was washed three times with brine (100 mL), dried over Na₂SO₄, filtered, and concentrated, and then the concentrate was purified by silica gel chromatography to obtain tert-butyl (6-chloro-3-(2,2,2-trifluoroethyl)imidazo[1,2-b]pyridazin-8-yl)glycinate (770 mg, 74.60 % yield). MS: m/z = 365.1 (M+1, ESI+).

### Step 4: Preparation of tert-butyl N-(6-chloro-3-(2,2,2-trifluoroethyl)imidazo[1,2-blpyridazin-8-yl)-N-(4-methoxybenzyl)glycinate

The product obtained in Step 3 (770 mg, 2.11 mmol) and 4-methoxybenzyl chloride (496 mg, 3.17 mmol) were dissolved in MeCN (20 mL), then cesium carbonate (2.06 g, 6.33 mmol) was added thereto, and the mixture was stirred at 60 °C for 16 hours. Water (200 mL) was added to the reaction mixture, and the product was extracted three times with EtOAc (40 mL). The organic layer was washed three times with brine (200 mL), dried over Na₂SO₄, filtered, and concentrated, and then the concentrate was purified by silica gel chromatography to obtain *tert-*butyl N-(6-chloro-3-(2,2,2-trifluoroethyl)imidazo[1,2-b]pyridazin-8-yl)-N-(4-methoxybenzyl)glycinate (470 mg, 45.91 % yield). MS: m/z = 485.1 (M+1, ESI+).

### Step 5: Preparation of tert-butyl N-(4-methoxybenzyl)-N-(6-morpholino-3-(2,2,2-trifluoroethyl)imidazo[1.2-b]pyridazin-8-yl)glycinate

The product obtained in Step 4 (470 mg, 969 umol) and morpholine (0.85 mL, 9.69 mmol) were dissolved in 1,4-dioxane (20 mL), then cesium carbonate (948 mg, 2.91 mmol) and RuPhos-Pd-G3 (81 mg, 97 umol) were added thereto, and the mixture was stirred at 110 °C for 16 hours. Water (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain *tert-butyl N-(4-methoxybenzyl)-N-(6-morpholino-3-(2,2,2-trifluoroethyl)imidazo[1,2-*b]pyridazin-8-yl)glycinate (290 mg, 55.87 % yield). MS: m/z = 536.2 (M+1, ESI+).

### Step 6: Preparation of N-(4-methoxybenzyl)-N-(6-morpholino-3-(2,2,2-trifluoroethyl)imidazo[1,2-b]pyridazin-8-yl)glycine

The product obtained in Step 5 (290 mg, 541.5 umol) was dissolved in 1,4-dioxane (5 mL) and water (2 mL), then sodium hydroxide (109 mg, 2.71 mmol) was added thereto, and the mixture was stirred at 100 °C for 48 hours. 1 N hydrochloric acid (50 mL) was added to the reaction mixture, and the product was extracted three times with EtOAc (15 mL). The organic layer was washed three times with brine (50 mL), dried over Na₂SO₄, filtered, and concentrated, and then the concentrate was purified by silica gel chromatography to obtain N-(4-methoxybenzyl)-N-(6-morpholino-3-(2,2,2-trifluoroethyl)imidazo[1,2-b]pyridazin-8-yl)glycine (220 mg, 84.74 % yield). MS: m/z = 480.1 (M+1, ESI+).

### Step 7: Preparation of N-(6-amino-2,3-difluorophenyl)-2-((4-methoxybenzyl)(6-morpholino-3-(2,2,2-trifluoroethyl)imidazo[1,2-b]pyridazin-8-yl)amino)acetamide

The product obtained in Step 6 (220 mg, 459 umol) and **Intermediate B1** (2.20 g, 15.26 mmol) were dissolved in DMF (10 mL), then HOBT (310 mg, 2.29 mmol), EDCI (440 mg, 2.29 mmol), and DIEA (0.4 mL, 2.29 mmol) were added thereto, and the mixture was stirred at 25 °C for 16 hours. Water (100 mL) was added to the reaction mixture, and the product was extracted three times with EtOAc (40 mL). The organic layer was washed three times with brine (100 mL), dried over Na₂SO₄, filtered, and concentrated, and then the concentrate was purified by silica gel chromatography to obtain N-(6-amino-2,3-difluorophenyl)-2-((4-methoxybenzyl)(6-morpholino-3-(2,2,2-trifluoroethyl)imidazo[1,2-b]pyridazin-8-yl)amino)acetamide (230 mg, 82.77 % yield). MS: m/z = 606.2 (M+1, ESI+).

### Step 8: Preparation of N-((6,7-difluoro-1H-benzo[d]imidazol-2-yl)methyl)-N-(4-methoxybenzyl)-6-morpholino-3-(2,2,2-trifluoroethyl)imidazo[1,2-b]pyridazin-8-amine

The product obtained in Step 7 (230 mg, 380 umol) was dissolved in AcOH (10 mL), and then the mixture was stirred at 70 °C for 16 hours. Sodium bicarbonate solution (60 mL) was added to the reaction mixture, and the product was extracted three times with EtOAc (20 mL). The organic layer was washed three times with brine (60 mL), dried over Na₂SO₄, filtered, and concentrated, and then the concentrate was purified by silica gel chromatography to obtain N-((6,7-difluoro-1H-benzo[d]imidazol-2-yl)methyl)-N-(4-methoxybenzyl)-6-morpholino-3-(2,2,2-trifluoroethyl)imidazo[1,2-b]pyridazin-8-amine (300 mg, crude). MS: m/z = 588.2 (M+1, ESI+).

### Step 9: Preparation of N-((6,7-difluoro-1H-benzo[d]imidazol-2-yl)methyl)-6-morpholino-3-(2,2,2-trifluoroethyl)imidazo[1,2-b]pyridazin-8-amine

The product obtained in Step 8 (300 mg, 509 umol) was dissolved in DCM (10 mL), then TFA (5 mL) was added and stirred at 25 °C for 16 hours. Sodium bicarbonate solution (100 mL) was added to the reaction mixture, and the product was extracted three times with DCM (40 mL). The organic layer was washed three times with brine (100 mL), dried over Na₂SO₄, filtered, and concentrated, and then the concentrate was purified by Prep-HPLC to obtain **the compound of Example** 2 (80 mg, 33.49 % yield) as a white solid. ¹H NMR (400 MHz, DMSO-d₆) δ 12.68 (s, 1H), 7.65 (t, 1H), 7.39 (s, 1H), 7.26-7.16 (m, 2H), 6.04 (s, 1H), 4.82 (d, 2H), 3.95 (dd, 2H), 3.67 (t, 4H), 3.32 (t, 4H); MS: m/z = 468.1 (M+1, ESI+).

### Example 3: Preparation of N-((6,7-difluoro-1H-benzo[d]imidazol-2-yl)methyl)-3-isopropyl-6-morpholinoimidazo[1,2-b]pyridazin-8-amine

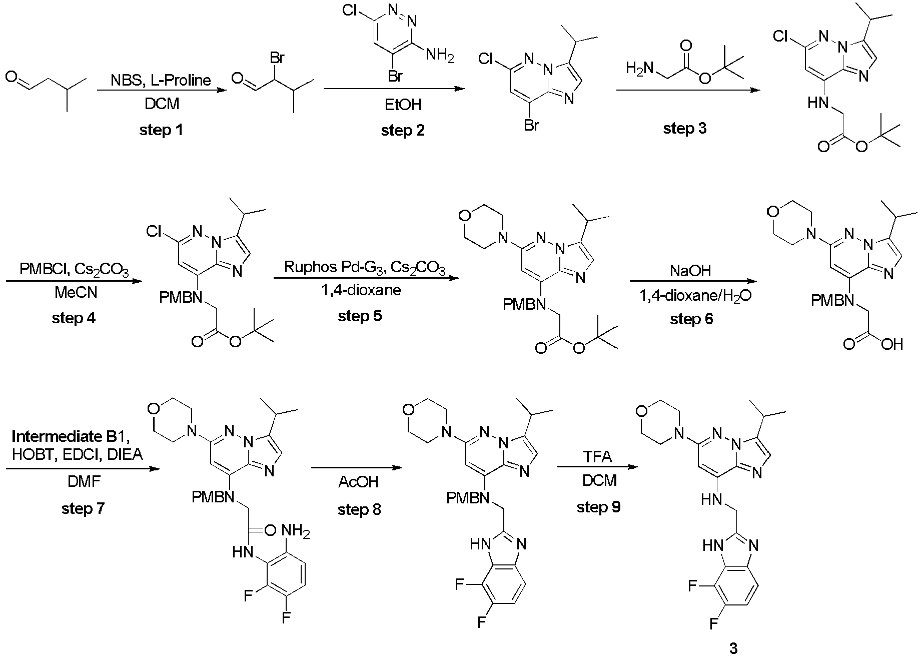

### Step 1: Preparation of 2-bromo-3-methylbutanal

2-bromo-methylbutanal (14.3 g, crude) was obtained in the same method as Step 1 of Example 2, except that 3-methylbutanal (11 g, 127.71 mmol) was dissolved in DCM (300 mL), L-proline (1.47 g, 12.77 mmol) and NBS (29.55 g, 166.02 mmol) were used instead of bromine (7.61 g, 47.59 mmol), and the mixture was stirred for 16 hours.

### Step 2: Preparation of 8-bromo-6-chloro-3-isopropylimidazo[1,2-b]pyridazine

8-bromo-6-chloro-3-isopropylimidazo[1,2-*b*]pyridazine (4.3 g, 90.37 % yield) was obtained in the same method as Step 2 of Example 2, except that the reaction temperature was changed to 100 °C and stirred for 72 hours using the product obtained in Step 1 (14.3 g, 86.65 mmol). MS: m/z = 274.2 (M+1, ESI+).

### Step 3 to 9: Preparation of N-((6,7-difluoro-1H-benzo[dlimidazol-2-yl)methyl)-3-isopropyl-6-morpholinoimidazo[1,2-b]pyridazin-8-amine

**The compound of Example** 3 (210 mg, 67.26 % yield) was obtained as a white solid in the same method as Steps 3 to 9 of Example 2, using the product obtained in Step 2 as a starting material. However, the reaction times of Step 6, Step 7, and Step 9 of Example 2 were changed to 16 hours, 2 hours, and 1 hour, respectively. ¹H NMR (400 MHz, DMSO-d₆) δ 12.66 (s, 1H), 7.50 (t, 1H), 7.22-7.13 (m, 3H), 5.97 (s, 1H), 4.79 (d, 2H), 3.68 (t, 4H), 3.29 (t, 4H), 3.25-3.19 (m, 1H), 1.31 (d, 6H); MS: m/z = 428.2 (M+1, ESI+).

### Example 4: Preparation of N-((6,7-difluoro-1H-benzo[d]imidazol-2-yl)methyl)-3-methyl-6-morpholinoimidazo[1,2-b]pyridazin-8-amine

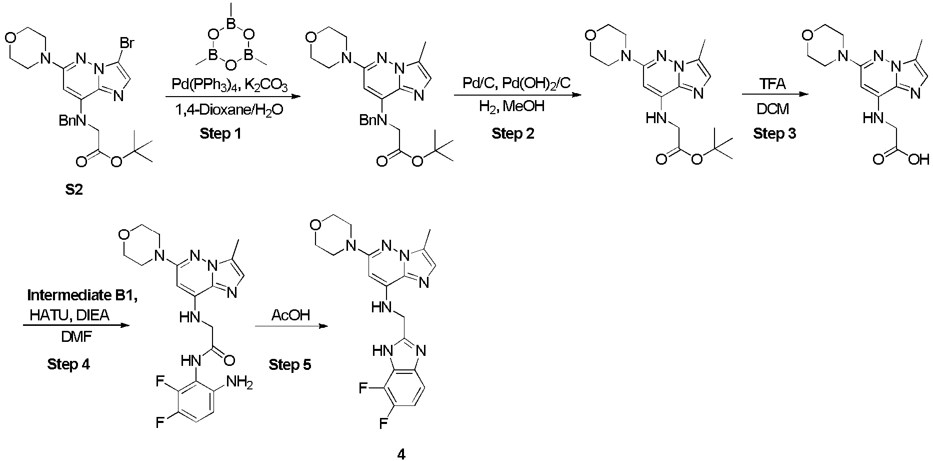

### Step 1: Preparation of tert-butyl N-benzyl-N-(3-methyl-6-morpholinoimidazo[1,2-b]pyridazin-8-yl)glycinate

**Intermediate** S2 (2.1 g, 4.18 mmol) and 2,4,6-trimethyl-1,3,5,2,4,6-trioxatriborinane (1.05 g, 8.36 mmol) were dissolved in 1,4-dioxane (20 mL) and water (5 mL), then Pd(PPh₃)₄ (483 mg, 418 umol) and potassium carbonate (1.73 g, 12.54 mmol) were added thereto, and the mixture was stirred at 100 °C for 16 hours. Water (100 mL) was added to the reaction mixture, and the product was extracted three times with EtOAc (30 mL). The organic layer was washed three times with brine (30 mL), dried over Na₂SO₄, filtered, and concentrated, and then the concentrate was purified by silica gel chromatography to obtain *tert-butyl* N-benzyl-N-(3-methyl-6-morpholinoimidazo[1,2-*b*]pyridazin-8-yl)glycinate (4.1 g, crude). MS: m/z = 438.2 (M+1, ESI+).

### Step 2: Preparation of tert-butyl (3-methyl-6-morpholinoimidazo[1,2-b]pyridazin-8-yl)glycinate

The product obtained in Step 1 (1.15 g, 2.63 mmol) was dissolved in methanol (20 mL), then Pd/C (200 mg) and Pd(OH)₂/C (200 mg) were added and stirred at 50 °C for 48 hours under H₂ atmosphere. The reaction mixture was filtered and concentrated, and then purified by silica gel chromatography to obtain *tert-butyl* (3-methyl-6-morpholinoimidazo[1,2-*b*]pyridazin-8-yl)glycinate (550 mg, 60.23 % yield). MS: m/z = 348.2 (M+1, ESI+).

### Step 3: Preparation of (3-methyl-6-morpholinoimidazo[1,2-b]pyridazin-8-yl)glycine

The product obtained in Step 2 (550 mg, 1.58 mmol) was dissolved in DCM (10 mL), then TFA (1.81 g, 15.83 mmol) was added and stirred at 25 °C for 16 hours. The reaction mixture was concentrated to obtain (3-methyl-6-morpholinoimidazo[1,2-*b*]pyridazin-8-yl)glycine (830 mg, crude). MS: m/z = 292.1 (M+1, ESI+).

### Step 4: Preparation of N-(6-amino-2,3-difluorophenyl)-2-((3-methyl-6-morpholinoimidazo[1,2-b]pyridazin-8-yl)amino)acetamide

The product obtained in Step 3 (830 mg, 2.85 mmol) and Intermediate **B1** (493 mg, 3.42 mmol) were dissolved in anhydrous DMF (10 mL), then HATU (1.63 g, 4.27 mmol) and DIEA (1.48 mL, 8.55 mmol) were added thereto and stirred at 25 °C for 16 hours. Water (100 mL) was added to the reaction mixture, and the product was extracted three times with EtOAc (30 mL). The organic layer was washed three times with brine (100 mL), dried over Na₂SO₄, filtered, and concentrated, and then the concentrate was purified by silica gel chromatography to obtain N-(6-amino-2,3-difluorophenyl)-2-((3-methyl-6-morpholinoimidazo[1,2-*b*]pyridazin-8-yl)amino)acetamide (195 mg, 16.40 % yield). MS: m/z = 418.1 (M+1, ESI+).

### Step 5: Preparation of N-((6,7-difluoro-1H-benzo[d]imidazol-2-yl)methyl)-3-methyl-6-morpholinoimidazo[1,2-b]pyridazin-8-amine

The product obtained in Step 4 (195 mg, 467 umol) was dissolved in AcOH (5 mL), then the mixture was stirred at 70 °C for 4 hours. Sodium bicarbonate solution (50 mL) was added to the reaction mixture, and the product was extracted three times with DCM (20 mL). The organic layer was washed three times with brine (50 mL), dried over Na₂SO₄, filtered, and concentrated, and then the concentrate was purified by Prep-HPLC to obtain the compound of Example 4 (70 mg, 37.52 % yield) as a white solid. ¹H NMR (400 MHz, DMSO-d₆) δ 12.72 (s, 1H), 7.47 (s, 1H), 7.24-7.16 (m, 3H), 5.97 (s, 1H), 4.81 (d, 2H), 3.68 (t, 4H), 3.31 (t, 4H), 2.33 (s, 3H); MS: m/z = 400.1 (M+1, ESI+).

### Example 5: Preparation of N-((6,7-difluoro-1H-benzo[d]imidazol-2-yl)methyl)-3-ethyl-6-morpholinoimidazo[1,2-b]pyridazin-8-amine

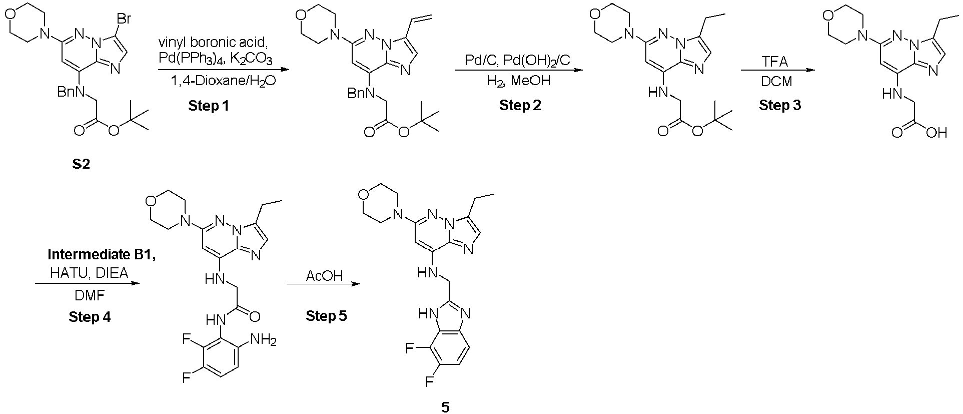

**The compound** of Example 5 (35 mg, 25.72 % yield) was obtained as a white solid in the same method as Steps 1 to 5 of Example 4, except that **Intermediate** S2 (1.55 g, 3.09 mmol) was used as a starting material and vinylboronic acid (266 mg, 3.70 mmol) was used instead of 2,4,6-trimethyl-1,3,5,2,4,6-trioxatriborinane (1.05 g, 8.36 mmol) in Step 1 of Example 4. ¹H NMR (400 MHz, DMSO-d₆) δ 12.66 (s, 1H), 7.48 (t, 1H), 7.24-7.15 (m, 3H), 5.96 (s, 1H), 4.80 (d, 2H), 3.67 (t, 4H), 3.31 (t, 4H), 2.78 (dd, 2H), 1.26 (t, 3H); MS: m/z = 414.0 (M+1, ESI+).

### Example 6: Preparation of N-((6,7-difluoro-1H-benzo[d]imidazol-2-yl)methyl)-6-morpholino-3-(trifluoromethyl)imidazo[1,2-b]pyridazin-8-amine

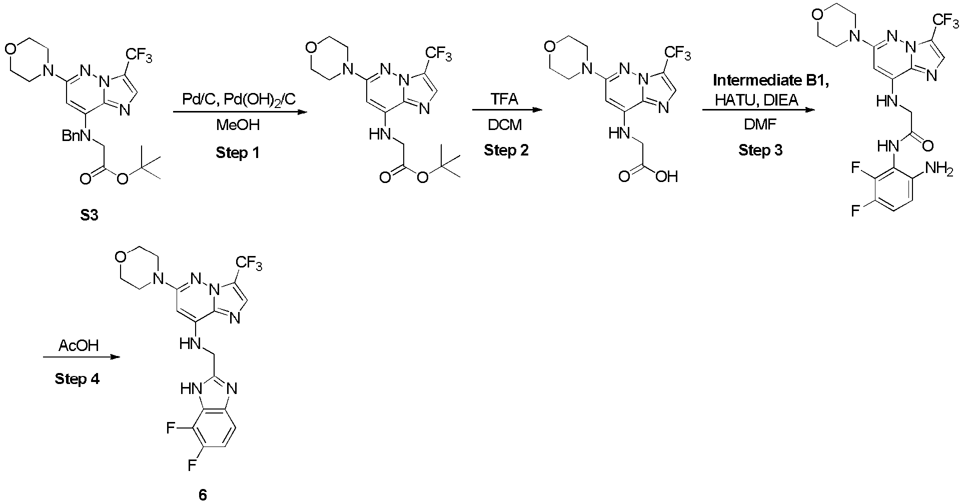

### Step 1: Preparation of tert-butyl (6-morpholino-3-(trifluoromethyl)imidazo[1,2-b]pyridazin-8-yl)glycinate

Intermediate S3 (1.4 g, 2.85 mmol) was dissolved in methanol (20 mL), then Pd/C (200 mg) and Pd(OH)₂/C (200 mg) were added thereto, and the mixture was stirred at 50 °C for 48 hours. The reaction mixture was filtered and concentrated, and then purified by silica gel chromatography to obtain *tert-butyl* (6-morpholino-3-(trifluoromethyl)imidazo[1,2-b]pyridazin-8-yl)glycinate (910 mg, 79.59 % yield). MS: m/z = 402.1 (M+1, ESI+).

### Step 2: Preparation of (6-morpholino-3-(trifluoromethyl)imidazo[1,2-b]pyridazin-8-yl)glycine

The product obtained in Step 1 (910 mg, 2.27 mmol) was dissolved in DCM (10 mL), then TFA (1.74 mL, 22.67 mmol) was added thereto, and the mixture was stirred at 25 °C for 16 hours. The reaction mixture was concentrated, and the concentrate was purified by silica gel chromatography to obtain (6-morpholino-3-(trifluoromethyl)imidazo[1,2-b]pyridazin-8-yl)glycine (970 mg, crude). MS: m/z = 346.1 (M+1, ESI+).

### Step 3: Preparation of N-(6-amino-2,3-difluorophenyl)-2-((6-morpholino-3-(trifluoromethyl)imidazo[1,2-b]pyridazin-8-yl)amino)acetamide

The product obtained in Step 2 (970 mg, 2.81 mmol) and **Intermediate B1** (486 mg, 3.37 mmol) were dissolved in anhydrous DMF (20 mL), then HATU (1.60 g, 4.21 mmol) and DIEA (1.47 mL, 8.43 mmol) were added thereto, and the mixture was stirred at 25 °C for 16 hours. Water (200 mL) was added to the reaction mixture, and the product was extracted three times with EtOAc (40 mL). The organic layer was washed three times with brine (200 mL), dried over Na₂SO₄, filtered, and concentrated, and then the concentrate was purified by silica gel chromatography to obtain N-(6-amino-2,3-difluorophenyl)-2-((6-morpholino-3-(trifluoromethyl)imidazo[1,2-b]pyridazin-8-yl)amino)acetamide (890 mg, 67.21 % yield). MS: m/z = 472.1 (M+1, ESI+).

### Step 4: Preparation of N-((6,7-difluoro-1H-benzo[d]imidazol-2-yl)methyl)-6-morpholino-3-(trifluoromethyl)imidazo[1,2-b]pyridazin-8-amine

The product obtained in Step 3 (890 mg, 1.89 mmol) was dissolved in AcOH (10 mL), and then the mixture was stirred at 70 °C for 4 hours. Sodium bicarbonate solution (80 mL) was added to the reaction mixture, and the product was extracted three times with EtOAc (30 mL). The organic layer was washed three times with brine (100 mL), dried over Na₂SO₄, filtered, and concentrated, and then the concentrate was purified by Prep-HPLC to obtain **the compound of Example** 6 (570 mg, 66.59 % yield) as a white solid. ¹H NMR (400 MHz, DMSO-d₆) δ 12.83 (s, 1H), 7.88-7.84 (m, 2H), 7.28-7.15 (m, 2H), 6.21 (s, 1H), 4.84 (d, 2H), 3.67 (t, 4H), 3.35 (t, 4H); MS: m/z = 454.0 (M+1, ESI+).

### Example 7: Preparation of 6-(3-oxa-8-azabicyclo[3.2.1]octan-8-yl)-N-((6,7-difluoro-1H-benzo[d]imidazol-2-yl)methyl)-3-(trifluoromethyl)imidazo[1,2-b]pyridazin-8-amine

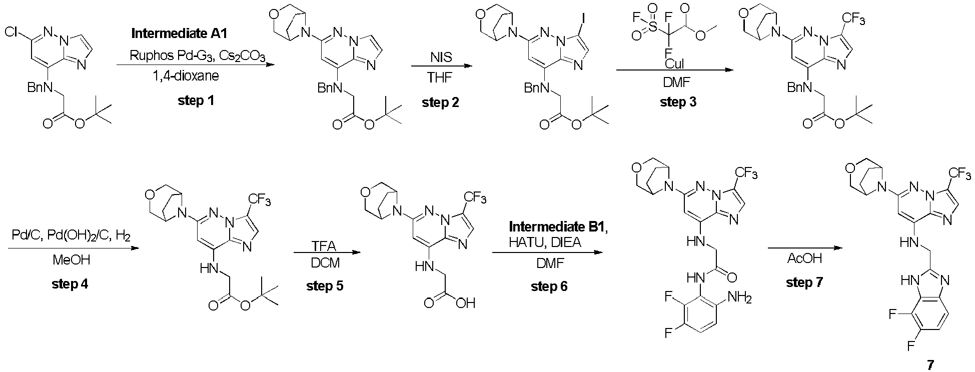

### Step 1: Preparation of tert-butyl-N-(6-(3-oxa-8-azabicyclo[3.2.1]octan-8-yl)imidazo[1,2-b]pyridazin-8-yl)-N-benzylglycinate

The product obtained in Step 2 of Intermediate S1 (6 g, 16 mmol) and **Intermediate A1** (2.73 g, 24 mmol) were dissolved in 1,4-dioxane (60 mL), then RuPhos-Pd-G3 (674 mg, 805 umol) and cesium carbonate (15.73 g, 48.28 mmol) were added thereto, and the mixture was stirred at 110 °C for 16 hours under N₂ atmosphere. Water (400 mL) was added to the reaction mixture, and the product was extracted three times with EtOAc (80 mL). The organic layer was washed three times with brine (400 mL), dried over Na₂SO₄, filtered, and concentrated, and then the concentrate was purified by silica gel chromatography to obtain tert-butyl-N-(6-(3-oxa-8-azabicyclo[3.2.1]octan-8-yl)imidazo[1,2-*b*]pyridazin-8-yl)-*N*-benzylglycinate (5.1 g, 70.50 % yield). MS: m/z = 450.2 (M+1, ESI+).

### Step 2: Preparation of tert-butyl N-(6-(3-oxa-8-azabicyclo[3.2.1]octan-8-yl)-3-iodoimidazo[1,2-b]pyridazin-8-yl)-N-benzylglycinate

The product obtained in Step 1 (5.1 g, 11.34 mmol) was dissolved in THF (30 mL), then NIS (3.06 g, 13.61 mmol) was added at 0 °C and stirred at 25 °C for 16 hours. Water (300 mL) was added to the reaction mixture, and the product was extracted three times with DCM (80 mL). The organic layer was washed three times with brine (400 mL), dried over Na₂SO₄, filtered, and concentrated, and then the concentrate was purified by silica gel chromatography to obtain *tert-*butyl *N*-(6-(3-oxa-8-azabicyclo[3.2.1]octan-8-yl)-3-iodoimidazo[1,2-*b*]pyridazin-8-yl)-*N-*benzylglycinate (5.2 g, 79.65 % yield). MS: m/z = 576.1 (M+1, ESI+).

### Step 3: Preparation of tert-butyl N-(6-(3-oxa-8-azabicyclo[3.2.1]octan-8-yl)-3-(trifluoromethyl)imidazo[1,2-b]pyridazin-8-yl)-N-benzylglycinate

The product obtained in Step 2 (5.2 g, 9.04 mmol) and methyl 2,2-difluoro-2-(fluorosulfonyl)acetate (17.36 g, 90.4 mmol) were dissolved in DMF (30 mL), then CuI (8.61 g, 45.2 mmol) was added and stirred at 100 °C for 16 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain *tert-butyl N-(6-(3-oxa-8-*azabicyclo[3.2.1]octan-8-yl)-3-(trifluoromethyl)imidazo[1,2-b]pyridazin-8-yl)-N-benzylglycinate (2.8 g, 59.87 % yield). MS: m/z = 518.5 (M+1, ESI+).

### Step 4 to 7: Preparation of 6-(3-oxa-8-azabicyclo[3.2.1]octan-8-yl)-N-((6,7-difluoro-1H-benzo[d]imidazol-2-yl)methyl)-3-(trifluoromethyl)imidazo[1.2-b]pyridazin-8-amine

**The compound** of Example 7 (58 mg, 14.33 % yield) was obtained as a white solid in the same method as Steps 1 to 4 of Example 6, using the product of Step 3 as a starting material. However, the reaction time of Step 7 of Example 7 was changed to 2 hours. ¹H NMR (400 MHz, DMSO-d₆) δ 12.75 (s, 1H), 7.89-7.87 (m, 2H), 7.28-7.16 (m, 2H), 6.13 (s, 1H), 4.84 (d, 2H), 4.28 (s, 2H), 3.61 (d, 2H), 3.44 (d, 2H), 1.94-1.83 (m, 4H); MS: m/z = 480.1 (M+1, ESI+).

Hereinafter, the compounds of Examples 8 to 22 were prepared using the appropriate intermediates among the Intermediate A series in [Table 1] according to Scheme I below.

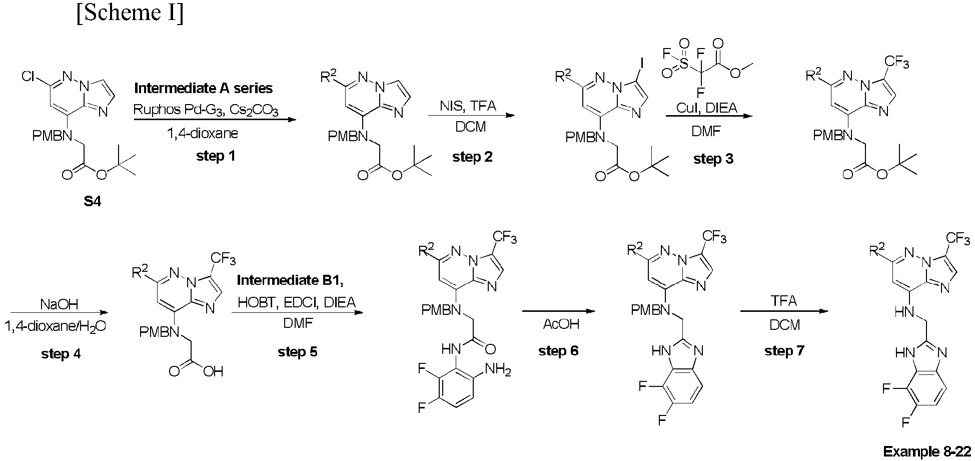

In the above Scheme I, R² has the same structure as R² of each example compound. Intermediates referred to as Intermediate A1 to Intermediate A52 described in [Table 1] below have the structures shown in [Table 1] below, throughout the examples as well as Examples 8 to 22.

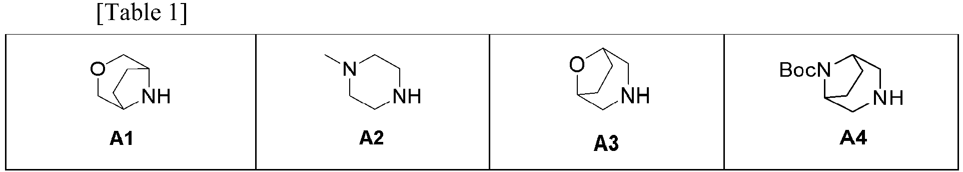

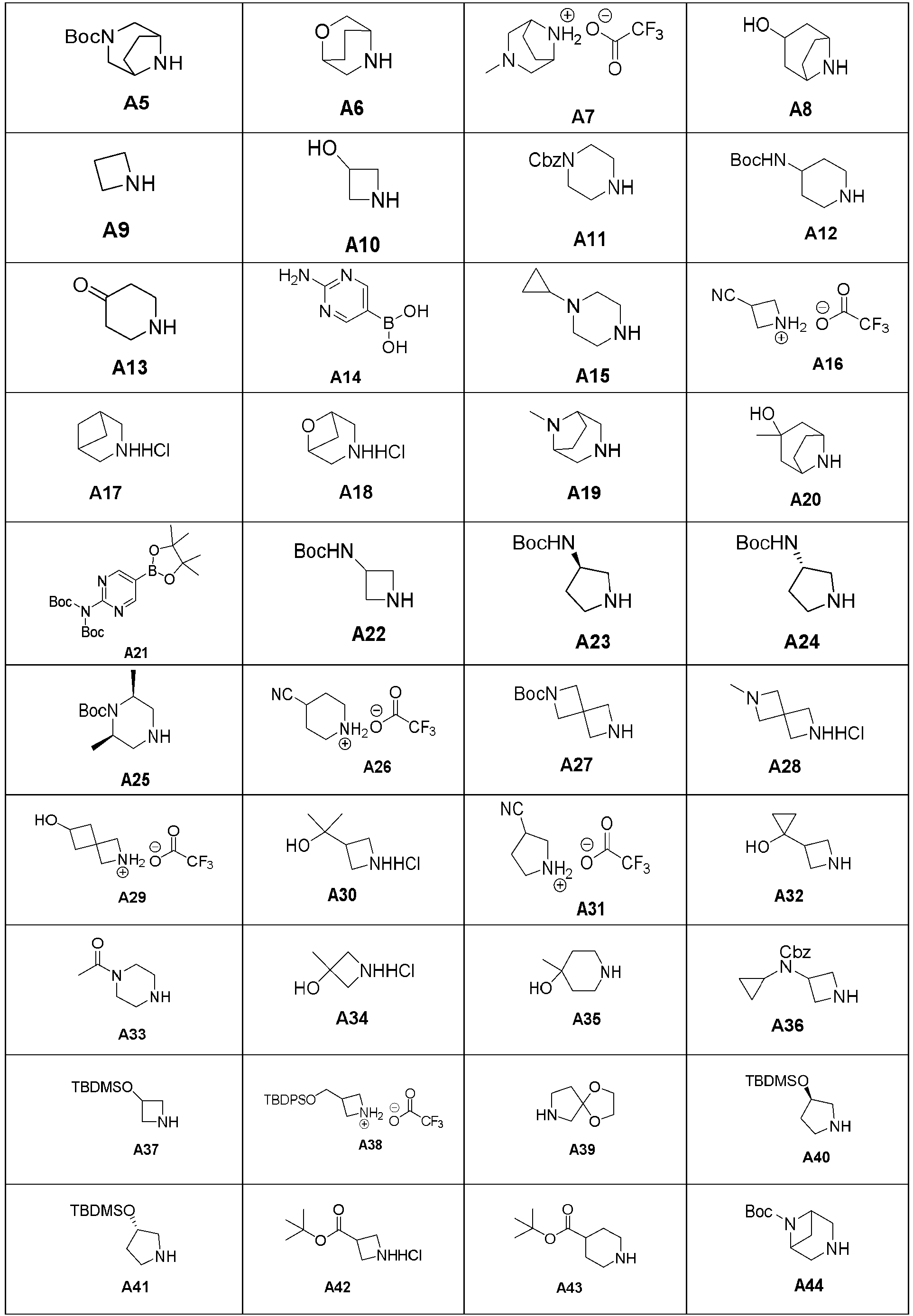

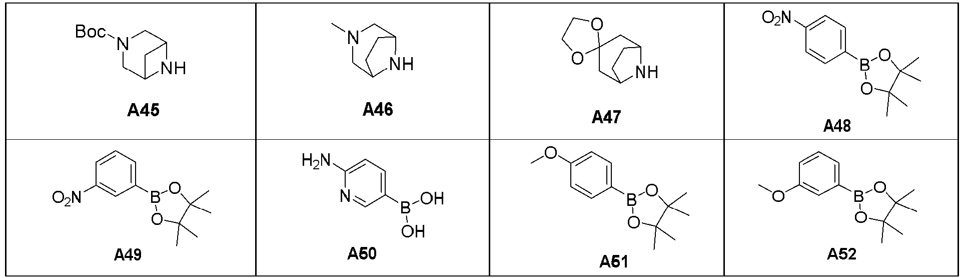

### Example 8: Preparation of N-((6,7-difluoro-1H-benzo[d]imidazol-2-yl)methyl)-6-(4-methylpiperazin-1-yl)-3-(trifluoromethyl)imidazo[1,2-b]pyridazin-8-amine

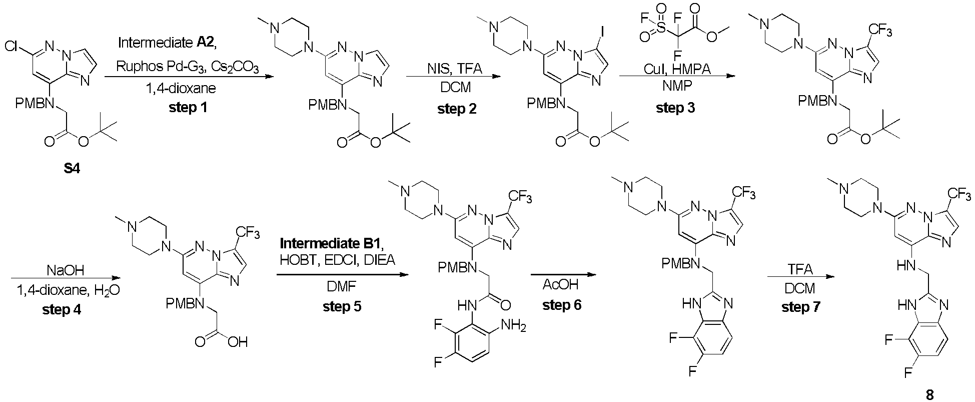

### Step 1: Preparation of tert-butyl N-(4-methoxybenzyl)-N-(6-(4-methylpiperazin-1-yl)imidazo[1.2-b]pyridazin-8-yl)glycinate

**Intermediate** S4 (6.1 g, 15.14 mmol) and **Intermediate** A2 (4.54 g, 45.42 mmol) were dissolved in 1,4-dioxane (100 mL), then cesium carbonate (9.87 g, 30.28 mmol) and Ruphos-Pd-G3 (1.27 g, 1.51 mmol) were added thereto, and the mixture was stirred at 100 °C for 16 hours under N₂ atmosphere. Water (500 mL) was added to the reaction mixture, and the product was extracted three times with EtOAc (100 mL). The organic layer was washed three times with brine (500 mL), dried over Na₂SO₄, filtered, and concentrated, and then the concentrate was purified by silica gel chromatography to obtain *tert-butyl N-(4-methoxybenzyl)-N-(6-(4-methylpiperazin-1-*yl)imidazo[1,2-b]pyridazin-8-yl)glycinate (3.9 g, 55.21 % yield). MS: m/z = 467.3 (M+1, ESI+).

### Step 2: Preparation of tert-butyl N-(3-iodo-6-(4-methylpiperazin-1-yl)imidazo[1,2-b]pyridazin-8-yl)-N-(4-methoxybenzyl)glycinate

The product obtained in Step 1 (3.8 g, 8.14 mmol) was dissolved in DCM (80 mL), then NIS (1.03 g, 4.59 mmol) and TFA (1.25 mL, 16.28 mmol) were added thereto at 0 °C, and the mixture was stirred at 25 °C for 2 hours under N₂ atmosphere. Water (300 mL) was added to the reaction mixture, and the product was extracted three times with DCM (60 mL). This organic layer was washed again three times with sodium sulfite solution (300 mL), dried over Na₂SO₄, filtered, and concentrated, and then the concentrate was purified by silica gel chromatography to obtain *tert-butyl N-(3-iodo-6-(4-methylpiperazin-1-yl)imidazo[1,2-b]pyridazin-8-yl)-N-(4-*methoxybenzyl)glycinate (2.3 g, 47.66 % yield). MS: m/z = 593.2 (M+1, ESI+).

### Step 3: Preparation of tert-butyl N-(4-methoxybenzyl)-N-(6-(4-methylpiperazin-1-yl)-3-(trifluoromethyl)imidazo[1,2-b]pyridazin-8-yl)glycinate

The product obtained in Step 2 (2.1 g, 3.85 mmol) and methyl 2,2-difluoro-2-(fluorosulfonyl)acetate (7.40 g, 38.50 mmol) were dissolved in N-methyl-2-pyrrolidone (35 mL), then CuI (3.67 g, 19.25 mmol) and HMPA (3.45 g, 19.25 mmol) were added thereto, and the mixture was stirred at 50 °C for 16 hours. Water (400 mL) was added to the reaction mixture, and the product was extracted three times with EtOAc (80 mL). The organic layer was washed three times with brine (400 mL), dried over Na₂SO₄, filtered, and concentrated, and then the concentrate was purified by silica gel chromatography to obtain tert-butyl N-(4-methoxybenzyl)-N-(6-(4-methylpiperazin-1-yl)-3-(trifluoromethyl)imidazo[1,2-b]pyridazin-8-yl)glycinate (850 mg, 41.30 % yield). MS: m/z = 535.3 (M+1, ESI+).

### Step 4: Preparation of N-(4-methoxybenzyl)-N-(6-(4-methylpiperazin-1-yl)-3-(trifluoromethyl)imidazo[1,2-b]pyridazin-8-yl)glycine

The product obtained in Step 3 (850 mg, 1.59 mmol) was dissolved in 1,4-dioxane (10 mL) and water (10 mL), then sodium hydroxide (1.27 g, 31.80 mmol) was added thereto, and the mixture was stirred at 100 °C for 16 hours. 0.5 N hydrochloric acid (80 mL) was added to the reaction mixture, and the product was extracted three times with EtOAc (30 mL). The organic layer was washed three times with brine (100 mL), dried over Na₂SO₄, filtered, and concentrated, and then the concentrate was purified by silica gel chromatography to obtain N-(4-methoxybenzyl)-N-(6-(4-methylpiperazin-1-yl)-3-(trifluoromethyl)imidazo[1,2-b]pyridazin-8-yl)glycine (500 mg, 65.72 % yield). MS: m/z = 479.2 (M+1, ESI+).

### Step 5: Preparation of N-(6-amino-2,3-difluorophenyl)-2-((4-methoxybenzyl)(6-(4-methylpiperazin-1-yl)-3-(trifluoromethyl)imidazo[1,2-b]pyridazin-8-yl)amino)acetamide

The product obtained in Step 4 (500 mg, 1.05 mmol) and **Intermediate B1** (181 mg, 1.26 mmol) were dissolved in DMF (15 mL), then HOBT (706 mg, 5.23 mmol), EDCI (1.00 g, 5.23 mmol), and DIEA (1.82 mL, 10.45 mmol) were added thereto, and the mixture was stirred at 25 °C for 2 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain N-(6-amino-2,3-difluorophenyl)-2-((4-methoxybenzyl)(6-(4-methylpiperazin-1-yl)-3-(trifluoromethyl)imidazo[1,2-b]pyridazin-8-yl)amino)acetamide (380 mg, 60.15 % yield). MS: m/z = 605.2 (M+1, ESI+).

### Step 6: Preparation of N-((6,7-difluoro-1H-benzo[d]imidazol-2-yl)methyl)-N-(4-methoxybenzyl)-6-(4-methylpiperazin-1-yl)-3-(trifluoromethyl)imidazo[1.2-b]pyridazin-8-amine

The product obtained in Step 5 (380 mg, 629 umol) was dissolved in AcOH (10 mL), and then the mixture was stirred at 100 °C for 2 hours. Sodium bicarbonate solution (100 mL) was added to the reaction mixture, and the product was extracted three times with EtOAc (40 mL). The organic layer was washed three times with brine (100 mL), dried over Na₂SO₄, filtered, and concentrated, and then the concentrate was purified by silica gel chromatography to obtain N-((6,7-difluoro-1H-benzo[d]imidazol-2-yl)methyl)-N-(4-methoxybenzyl)-6-(4-methylpiperazin-1-yl)-3-(trifluoromethyl)imidazo[1,2-b]pyridazin-8-amine (330 mg, crude). MS: m/z = 587.2 (M+1, ESI+).

### Step 7: Preparation of N-((6,7-difluoro-1H-benzo[d]imidazol-2-yl)methyl)-6-(4-methylpiperazin-1-yl)-3-(trifluoromethyl)imidazo[1,2-b]pyridazin-8-amine

The product obtained in Step 6 (330 mg, 562.60 umol) was dissolved in DCM (10 mL), then TFA (5 mL) was added thereto, and the mixture was stirred at 25 °C for 1 hour. The reaction mixture was concentrated and then purified by Prep-HPLC to obtain **the compound of Example** 8 (55 mg, 20.96 % yield) as a white solid. ¹H NMR (400 MHz, DMSO-d₆) δ 12.71 (s, 1H), 7.86 (s, 1H), 7.82 (t, 1H), 7.27-7.15 (m, 2H), 6.19 (s, 1H), 4.83 (d, 2H), 3.37 (t, 4H), 2.35 (t, 4H), 2.18 (s, 3H); MS: m/z = 467.2 (M+1, ESI+).

### Examples 9 to 18

The compounds of Examples 9 to 18 were prepared in the same method as Example 8, using the intermediates described in [Table 2].

**[Table 2]**

| Example | Structure (Intermediate) | IUPAC Name | NMR and MS Data |
|---|---|---|---|
| 9 | 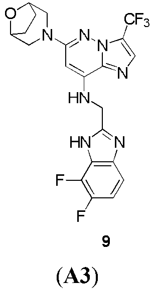 | 6-(8-oxa-3-azabicyclo[3.2.1]octan-3-yl)-N-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 12.69 (s, 1H), 7.86-7.84 (m, 2H), 7.25-7.17 (m, 2H), 6.13 (s, 1H), 4.84 (d, 2H), 4.40 (s, 2H), 3.65 (d, 2H), 2.93 (dd, 2H), 1.81-1.66 (m, 4H); MS: m/z = 480.1 (M+1, ESI+). |
| 10 | 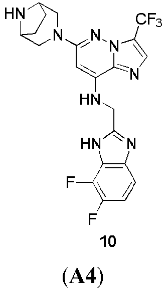 | 6-(3,8-diazabicyclo[3.2.1]octan-3-yl)-*N*-((6,7-difluoro-1*H-*benzo[d]imidazol-2-yl)methyl)-3-(trifluoromethyl)imidazo[1,2-b]pyridazin-8-amine | ¹H NMR (400 MHz, DMSO-d₆) δ 12.64 (s, 1H), 7.83-7.77 (m, 2H), 7.26-7.18 (m, 2H), 6.05 (s, 1H), 4.82 (d, 2H), 3.61 (d, 2H), 2.45 (s, 2H), 2.82 (d, 2H), 1.63-1.52 (m, 4H); MS: m/z = 479.2 (M+1, ESI+). |
| 11 | 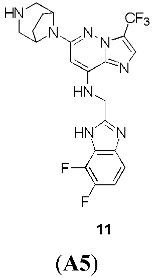 | 6-(3,8-diazabicyclo[3.2.1]octan-8-yl)-*N*-((6,7-difluoro-1*H-*benzo[d]imidazol-2-yl)methyl)-3-(trifluoromethyl)imidazo[1,2-b]pyridazin-8-amine | ¹H NMR (400 MHz, DMSO-d₆) δ 12.74 (s, 1H), 7.83-7.78 (m, 2H), 7.27-7.18 (m, 2H), 6.01 (s, 1H), 4.80 (d, 2H), 4.18 (s, 2H), 2.77 (d, 2H), 2.36 (d, 2H), 1.88-1.79 (m, 4H); MS: m/z = 479.2 (M+1, ESI+). |
| 12 | 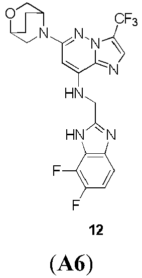 | 6-(2-oxa-5-azabicyclo[2.2.2]octan-5-yl)-N-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 12.71 (s, 1H), 7.86-7.83 (m, 2H), 7.25-7.17 (m, 2H), 5.94 (s, 1H), 4.84 (d, 2H), 4.24 (s, 1H), 3.98-3.91 (m, 3H), 3.66 (d, 1H), 3.44 (d, 1H), 2.00-1.97 (m, 1H), 1.87-1.86 (m, 2H), 1.68-1.61 (m, 1H); MS: m/z = 480.2 (M+1, ESI+). |
| 13 | 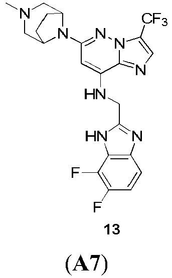 | N-((6,7-difluoro-1H-benzo[d]imidazol-2-yl)methyl)-6-(3-methyl-3,8-diazabicyclo[3.2.1]octan-8-yl)-3-(trifluoromethyl)imidazo[1,2-blpyridazin-8-amine | ¹H NMR (400 MHz, DMSO-d₆) δ 12.83 (s, 1H), 7.89-7.81 (m, 2H), 7.26-7.17 (m, 2H), 6.08 (s, 1H), 4.84 (d, 2H), 4.35 (s, 2H), 2.52 (s, 2H), 2.09-1.77 (m, 9H); MS: m/z = 493.2 (M+1, ESI+). |
| 14 | 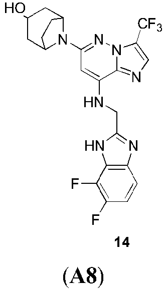 | 8-(8-(((6,7-difluoro-1H-benzo[d]imidazol-2-yl)methyl)amino)-3-(trifluoromethyl)imidazo[1,2-b]pyridazin-6-yl)-8-azabicyclo[3.2.1]octan-3-ol | ¹H NMR (400 MHz, DMSO-d₆) δ 7.83-7.80 (m, 2H), 7.28-7.19 (m, 2H), 5.99 (s, 1H), 4.80 (d, 2H), 4.22 (s, 2H), 3.70 (t, 1H), 2.21 (d, 2H), 1.88-1.85 (m, 4H), 1.44 (d, 2H); MS: m/z = 494.2 (M+1, ESI+). |
| 15 | 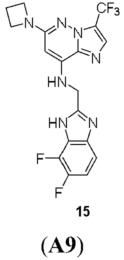 | 6-(azetidin-1-yl)-*N*-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 12.74 (s, 1H), 7.87-7.84 (m, 2H), 7.28-7.16 (m, 2H), 5.58 (s, 1H), 4.79 (d, 2H), 3.87 (t, 4H), 2.30-2.22 (m, 2H); MS: m/z = 424.1 (M+1, ESI+). |
| 16 | 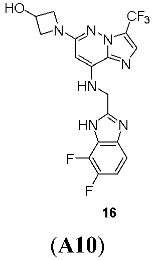 | 1-(8-(((6,7-difluoro-1*H-*benzo[d]imidazol-2-yl)methyl)amino)-3-(trifluoromethyl)imidazo[1,2-b]pyridazin-6-yl)azetidin-3-ol | ¹H NMR (400 MHz, DMSO-d₆) δ 12.70 (s, 1H), 7.88-7.84 (m, 2H), 7.23-7.19 (m, 2H), 5.61 (s, 2H), 4.79 (d, 2H), 4.50 (s, 2H), 4.07 (t, 2H), 3.60 (dd, 2H); MS: m/z = 440.1 (M+1, ESI+). |
| 17 | 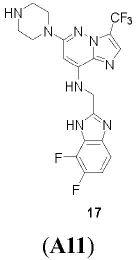 | N-((6,7-difluoro-1H-benzo[d]imidazol-2-yl)methyl)-6-(piperazin-1-yl)-3-(trifluoromethyl)imidazo[1,2-blpyridazin-8-amine | ¹H NMR (400 MHz, DMSO-d₆) δ 7.86-7.82 (m, 2H), 7.28-7.16 (m, 2H), 6.17 (s, 1H), 4.86 (d, 2H), 3.31 (t, 4H), 2.75 (t, 4H); MS: m/z = 453.1 (M+1, ESI+). |
| 18 | 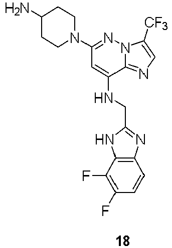 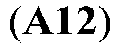 | 6-(4-aminopiperidin-1-yl)-N-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 7.84 (s, 1H), 7.77 (s, 1H), 7.26-7.13 (m, 2H), 6.17 (s, 1H), 4.82 (s, 2H), 3.96 (d, 2H), 2.88-2.75 (m, 3H), 1.70 (d, 2H), 1.22-1.13 (m, 2H); MS: m/z = 467.2 (M+1, ESI+). |

### Example 19: Preparation of 1-(8-(((6,7-difluoro-1H-benzo[d]imidazol-2-yl)methyl)amino)-3-(trifluoromethyl)imidazo[1,2-b]pyridazin-6-yl)piperidin-4-ol

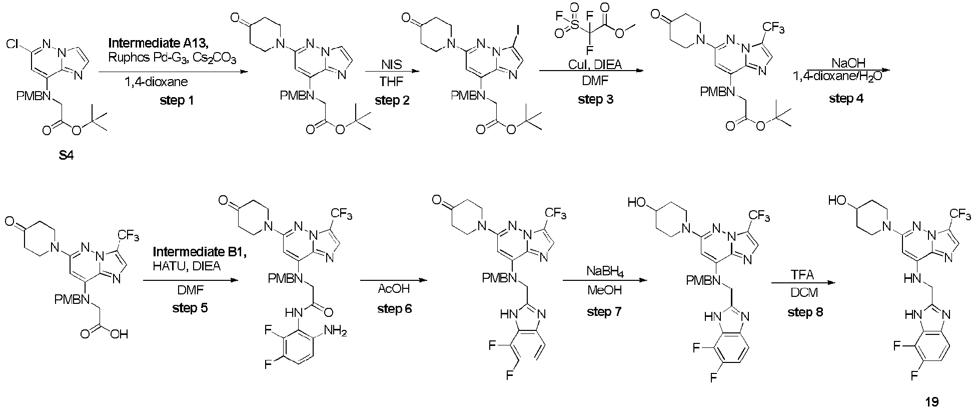

### Step 1: Preparation of tert-butyl N-(4-methoxybenzyl)-N-(6-(4-oxopiperidin-1-yl)imidazo[1,2-b]pyridazin-8-yl)glycinate

*tert-butyl N-(4-methoxybenzyl)-N-(6-(4-oxopiperidin-1-yl)imidazo[1,2-b]pyridazin-8-*yl)glycinate (5.4 g, 51.92 % yield) was obtained in the same method as Step 1 of Example 8, except that the reaction was carried out at 110 °C using **Intermediate** S4 (9 g, 22.34 mmol) and **Intermediate A13** (3.32 g, 33.51 mmol). MS: m/z = 466.2 (M+1, ESI+).

### Step 2: Preparation of tert-butyl N-(3-iodo-6-(4-oxopiperidin-1-yl)imidazo[1,2-b]pyridazin-8-yl)-N-(4-methoxybenzyl)glycinate

*tert-butyl N-(3-iodo-6-(4-oxopiperidin-1-yl)imidazo[1,2-b]pyridazin-8-yl)-N-(4-*methoxybenzyl)glycinate (2.8 g, 40.81 % yield) was obtained in the same method as Step 2 of Example 8, except that THF (50 mL) was used instead of DCM (80 mL), TFA was not added, and the mixture was stirred at 0 °C for 1 hour. MS: m/z = 592.3 (M+3, ESI+).

### Step 3: Preparation of tert-butyl N-(4-methoxybenzyl)-N-(6-(4-oxopiperidin-1-yl)-3-(trifluoromethyl)imidazo[1.2-b]pyridazin-8-yl)glycinate

*tert-butyl N-(4-methoxybenzyl)-N-(6-(4-oxopiperidin-1-yl)-3-*(trifluoromethyl)imidazo[1,2-b]pyridazin-8-yl)glycinate (1.7 g, 67.30 % yield) was obtained in the same method as Step 3 of Example 8, except that DMF (80 mL) was used instead of NMP (35 mL), and DIEA (11.3 mL, 64.67 mmol) was used instead of HMPA (3.45 g, 19.25 mmol) at 100 °C. MS: m/z = 534.3 (M+1, ESI+).

### Step 4: Preparation of N-(4-methoxybenzyl)-N-(6-(4-oxopiperidin-1-yl)-3-(trifluoromethyl)imidazo[1,2-b]pyridazin-8-yl)glycine

*N*-(4-methoxybenzyl)-*N*-(6-(4-oxopiperidin-1-yl)-3-(trifluoromethyl)imidazo[1,2-b]pyridazin-8-yl)glycine (1.2 g, 78.88 % yield) was obtained in the same method as Step 4 of Example 8, except that the reaction time was changed to 2 hours. MS: m/z = 492.2 (M+1, ESI+).

### Step 5: Preparation of N-(6-amino-2,3-difluorophenyl)-2-((4-methoxybenzyl)(6-(4-oxopiperidin-1-yl)-3-(trifluoromethyl)imidazo[1,2-b]pyridazin-8-yl)amino)acetamide

N-(6-amino-2,3-difluorophenyl)-2-((4-methoxybenzyl)(6-(4-oxopiperidin-1-yl)-3-(trifluoromethyl)imidazo[1,2-b]pyridazin-8-yl)amino)acetamide (1.1 g, 72.85 % yield) was obtained in the same method as Step 5 of Example 8, except that HATU (1.14 g, 3.01 mmol) was used instead of HOBT (706 mg, 5.23 mmol) and EDCI (1.00 g, 5.23 mmol). MS: m/z = 604.1 (M+1, ESI+).

### Step 6: Preparation of 1-(8-(((6,7-difluoro-1H-benzo[d]imidazol-2-yl)methyl)(4-methoxybenzyl)amino)-3-(trifluoromethyl)imidazo[1,2-b]pyridazin-6-yl)piperidin-4-one

1-(8-(((6,7-difluoro-1H-benzo[d]imidazol-2-yl)methyl)(4-methoxybenzyl)amino)-3-(trifluoromethyl)imidazo[1,2-b]pyridazin-6-yl)piperidin-4-one (700 mg, 65.60 % yield) was obtained in the same method as Step 6 of Example 8, except that the reaction temperature was changed to 70 °C and the mixture was stirred for 16 hours. MS: m/z = 586.2 (M+1, ESI+).

### Step 7: Preparation of 1-(8-(((6,7-difluoro-1H-benzo[d]imidazol-2-yl)methyl)(4-methoxybenzyl)amino)-3-(trifluoromethyl)imidazo[1.2-b]pyridazin-6-yl)piperidin-4-ol

The product obtained in Step 6 (700 mg, 1.2 mmol) was dissolved in methanol (10 mL), then sodium borohydride (55 mg, 1.44 mmol) was added at 0 °C and stirred at 25 °C for 2 hours. The reaction mixture was concentrated, and the concentrate was purified by Prep-HPLC to obtain 1-(8-(((6,7-difluoro-1H-benzo[d]imidazol-2-yl)methyl)(4-methoxybenzyl)amino)-3-(trifluoromethyl)imidazo[1,2-b]pyridazin-6-yl)piperidin-4-ol (550 mg, 78.57 % yield). MS: m/z = 588.5 (M+1, ESI+).

### Step 8: Preparation of 1-(8-(((6,7-difluoro-1H-benzo[d]imidazol-2-yl)methyl)amino)-3-(trifluoromethyl)imidazo[1,2-b]pyridazin-6-yl)piperidin-4-ol

**The compound** of Example **19** (200 mg, 45.76 % yield) was obtained as a white solid in the same method as Step 7 of Example 8, except that the reaction time was changed to 2 hours. ¹H NMR (400 MHz, DMSO-d₆) δ 12.73 (s, 1H), 7.85-7.80 (m, 2H), 7.25-7.16 (m, 2H), 6.16 (s, 1H), 4.83 (d, 2H), 4.67 (d, 1H), 3.80 (d, 2H), 3.66-3.63 (m, 1H), 3.02 (t, 2H), 1.71 (t, 2H), 1.34-1.27 (m, 2H); MS: m/z = 468.3 (M+1, ESI+).

### Example 20: Preparation of 6-(2-aminopyrimidin-5-yl)-N-((6,7-difluoro-1H-benzo[d]imidazol-2-yl)methyl)-3-(prop-1-yn-1-yl)imidazo[1,2-b]pyridazin-8-amine

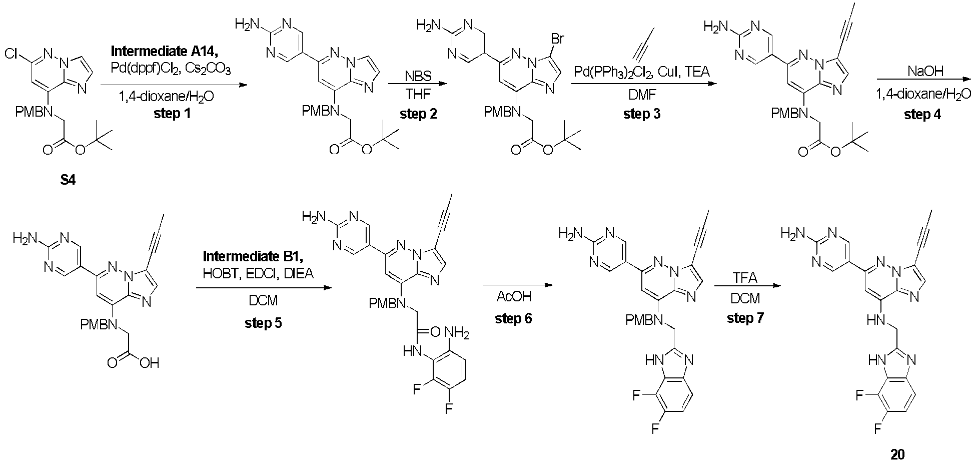

### Step 1: Preparation of tert-butyl N-(6-(2-aminopyrimidin-5-yl)imidazo[1,2-b]pyridazin-8-yl)-N-(4-methoxybenzyl)glycinate

**Intermediate** S4 (2.6 g, 6.45 mmol) and **Intermediate A14** (1.34 g, 9.68 mmol) were dissolved in 1,4-dioxane (50 mL) and water (50 mL), then [1,1'-bis(diphenylphosphino)ferrocene]dichloropalladium(II) (469 mg, 645 umol) and cesium carbonate (4.21 g, 12.91 mmol) were added thereto, and the mixture was stirred at 100 °C for 16 hours under N₂ atmosphere. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain *tert-butyl* N-(6-(2-aminopyrimidin-5-yl)imidazo[1,2-b]pyridazin-8-yl)-N-(4-methoxybenzyl)glycinate (2.5 g, 83.94 % yield). MS: m/z = 462.2 (M+1, ESI+).

### Step 2: Preparation of tert-butyl N-(6-(2-aminopyrimidin-5-yl)-3-bromoimidazo[1,2-b]pyridazin-8-yl)-N-(4-methoxybenzyl)glycinate

The product obtained in Step 1 (2.5 g, 5.42 mmol) was dissolved in THF (50 mL), then NBS (964 mg, 5.42 mmol) was added and the mixture was stirred at -60 °C for 3 hours under N₂ atmosphere. Water (200 mL) was added to the reaction mixture, and the product was extracted three times with EtOAc (50 mL). This organic layer was washed again three times with sodium sulfite solution (200 mL), dried over Na₂SO₄, filtered, and concentrated, and then the concentrate was purified by silica gel chromatography to obtain *tert-butyl* N-(6-(2-aminopyrimidin-5-yl)-3-bromoimidazo[1,2-b]pyridazin-8-yl)-N-(4-methoxybenzyl)glycinate (2.5 g, 85.40 % yield). MS: m/z = 542.2 (M+3, ESI+).

### Step 3: Preparation of tert-butyl N-(6-(2-aminopyrimidin-5-yl)-3-(prop-1-yn-1-yl)imidazo[1,2-b]pyridazin-8-yl)-N-(4-methoxybenzyl)glycinate

The product obtained in Step 2 (2.5 g, 4.63 mmol) and prop-1-yne (3.71 g, 92.52 mmol) were dissolved in DMF (20 mL), then CuI (176 mg, 925 umol), Pd(PPh₃)₂Cl₂ (325 mg, 463 umol), and TEA (0.65 mL, 4.63 mmol) were added thereto, and the mixture was stirred at 40 °C for 72 hours under N₂ atmosphere. Water (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain tert-butyl N-(6-(2-aminopyrimidin-5-yl)-3-(prop-1-yn-1-yl)imidazo[1,2-b]pyridazin-8-yl)-N-(4-methoxybenzyl)glycinate (1.1 g, 47.60 % yield). MS: m/z = 500.2 (M+1, ESI+).

### Step 4: Preparation of N-(6-(2-aminopyrimidin-5-yl)-3-(prop-1-yn-1-yl)imidazo[1,2-b]pyridazin-8-yl)-N-(4-methoxybenzyl)glycine

The product obtained in Step 3 (1.1 g, 2.20 mmol) was used to obtain N-(6-(2-aminopyrimidin-5-yl)-3-(prop-1-yn-1-yl)imidazo[1,2-*b*]pyridazin-8-yl)-*N*-(4-methoxybenzyl)glycine (300 mg, 30.72 % yield) in the same method as Step 4 of Example 8. MS: m/z = 442.2 (M+1, ESI+).

### Step 5: Preparation of N-(6-amino-2,3-difluorophenyl)-2-((6-(2-aminopyrimidin-5-yl)-3-(prop-1-yn-1-yl)imidazo[1,2-b]pyridazin-8-yl)(4-methoxybenzyl)amino)acetamide

N-(6-amino-2,3-difluorophenyl)-2-((6-(2-aminopyrimidin-5-yl)-3-(prop-1-yn-1-yl)imidazo[1,2-b]pyridazin-8-yl)(4-methoxybenzyl)amino)acetamide (270 mg, 70.07 % yield) was obtained in the same method as Step 5 of Example 8, except that DMF (15 mL) was replaced with DCM (20 mL) and the mixture was stirred for 16 hours. MS: m/z = 570.3 (M+1, ESI+).

### Step 6: Preparation of 6-(2-aminopyrimidin-5-yl)-N-((6,7-difluoro-1H-benzo[d]imidazol-2-yl)methyl)-N-(4-methoxybenzyl)-3-(prop-1-yn-1-yl)imidazo[1,2-b]pyridazin-8-amine

6-(2-aminopyrimidin-5-yl)-N-((6,7-difluoro-1H-benzo[d]imidazol-2-yl)methyl)-N-(4-methoxybenzyl)-3-(prop-1-yn-1-yl)imidazo[1,2-*b*]pyridazin-8-amine (250 mg, 95.62 % yield) was obtained in the same method as Step 6 of Example 8, except that the reaction temperature was changed to 70 °C and the mixture was stirred for 16 hours. MS: m/z = 552.2 (M+1, ESI+).

### Step 7: Preparation of 6-(2-aminopyrimidin-5-yl)-N-((6,7-difluoro-1H-benzo[d]imidazol-2-yl)methyl)-3-(prop-1-yn-1-yl)imidazo[1,2-b]pyridazin-8-amine

**The compound of Example 20** (8 mg, 4.09 % yield) was obtained as a white solid in the same method as Step 7 of Example 8. ¹H NMR (400 MHz, DMSO-d₆) δ 12.73 (s, 1H), 8.79 (s, 2H), 8.07 (s, 1H), 7.73 (s, 1H), 7.22-7.08 (m, 4H), 6.72 (s, 1H), 4.94 (d, 2H), 2.21 (s, 3H); MS: m/z = 432.1 (M+1, ESI+).

### Example 21: Preparation of 6-(2-aminopyrimidin-5-yl)-3-(cyclopropylethynyl)-N-((6,7-difluoro-1H-benzo[d]imidazol-2-yl)methyl)imidazo[1,2-b]pyridazin-8-amine

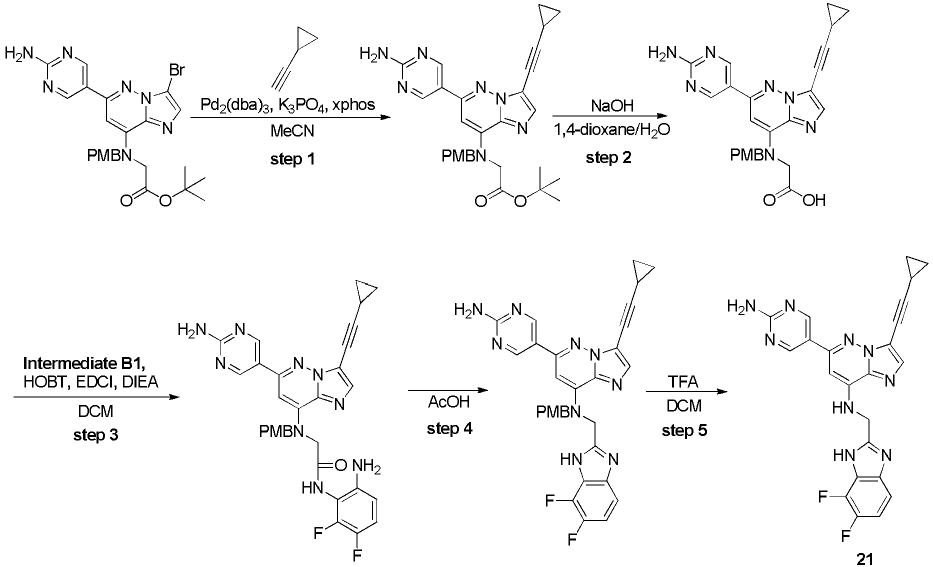

### Step 1: Preparation of tert-butyl N-(6-(2-aminopyrimidin-5-yl)-3-(cyclopropylethynyl)imidazo[1,2-b]pyridazin-8-yl)-N-(4-methoxybenzyl)glycinate

The product obtained in Step 2 of Example 20 (2.6 g, 4.81 mmol) and ethynylcyclopropane (318 mg, 4.81 mmol) were dissolved in MeCN (20 mL), then tris(dibenzylideneacetone)dipalladium(0) (441 mg, 481 umol), 2-dicyclohexylphosphino-2',4',6'-triisopropylbiphenyl (X-phos, 459 mg, 962 umol), and potassium phosphate (K₃PO₄, 3.06 g, 14.43 mmol) were added thereto, and the mixture was stirred at 70 °C for 48 hours under N₂ atmosphere. Water (200 mL) was added to the reaction mixture, and the product was extracted three times with EtOAc (60 mL). The organic layer was washed three times with brine (200 mL), dried over Na₂SO₄, filtered, and concentrated, and then the concentrate was purified by silica gel chromatography to obtain *tert-butyl N*-(6-(2-aminopyrimidin-5-yl)-3-(cyclopropylethynyl)imidazo[1,2*-b*]pyridazin-8-yl)-N-(4-methoxybenzyl)glycinate (900 mg, 35.59 % yield). MS: m/z = 526.3 (M+1, ESI+).

### Step 2: Preparation of N-(6-(2-aminopyrimidin-5-yl)-3-(cyclopropylethynyl)imidazo[1,2-b]pyridazin-8-yl)-N-(4-methoxybenzyl)glycine

*N*-(6-(2-aminopyrimidin-5-yl)-3-(cyclopropylethynyl)imidazo[1,2-*b*]pyridazin-8-yl)-*N-*(4-methoxybenzyl)glycine (780 mg, 67.17 % yield) was obtained in the same method as Step 4 of Example 8. MS: m/z = 470.2 (M+1, ESI+).

### Step 3: Preparation of N-(6-amino-2,3-difluorophenyl)-2-((6-(2-aminopyrimidin-5-yl)-3-(cyclopropylethynyl)imidazo[1,2-b]pyridazin-8-yl)(4-methoxybenzyl)amino)acetamide

N-(6-amino-2,3-difluorophenyl)-2-((6-(2-aminopyrimidin-5-yl)-3-(cyclopropylethynyl)imidazo[1,2-*b*]pyridazin-8-yl)(4-methoxybenzyl)amino)acetamide (660 mg, 66.70 % yield) was obtained in the same method as Step 5 of Example 8, except that DMF (15 mL) was replaced with DCM (20 mL) and the mixture was stirred for 16 hours. MS: m/z = 596.2 (M+1, ESI+).

### Step 4: Preparation of 6-(2-aminopyrimidin-5-yl)-3-(cyclopropylethynyl)-N-((6,7-difluoro-1H-benzo[d]imidazol-2-yl)methyl)-N-(4-methoxybenzyl)imidazo[1,2-b]pyridazin-8-amine

6-(2-aminopyrimidin-5-yl)-3-(cyclopropylethynyl)-*N*-((6,7-difluoro-1*H-*benzo[d]imidazol-2-yl)methyl)-N-(4-methoxybenzyl)imidazo[1,2-b]pyridazin-8-amine (580 mg, 90.62 % yield) was obtained in the same method as Step 6 of Example 8, except that the reaction temperature was changed to 70 °C and the mixture was stirred for 16 hours. MS: m/z = 578.2 (M+1, ESI+).

### Step 5: Preparation of 6-(2-aminopyrimidin-5-yl)-3-(cyclopropylethynyl)-N-((6,7-difluoro-1H-benzo[dlimidazol-2-yl)methyl)imidazo[1,2-b]pyridazin-8-amine

**The compound** of Example **21** (25 mg, 5.44 % yield) was obtained as a white solid in the same method as Step 7 of Example 8. ¹H NMR (400 MHz, DMSO-d₆) δ 12.73 (s, 1H), 8.80 (s, 2H), 8.07-8.03 (m, 1H), 7.72 (s, 1H), 7.24-7.17 (m, 2H), 7.08 (s, 2H), 6.73 (s, 1H), 4.93 (s, 2H), 1.73-1.69 (m, 1H), 0.99-0.95 (m, 2H), 0.84-0.80 (m, 2H); MS: m/z = 458.2 (M+1, ESI+).

### Example 22: Preparation of 6-(2-aminopyrimidin-5-yl)-N-((6,7-difluoro-1H-benzo[d]imidazol-2-yl)methyl)-3-ethynylimidazo[1,2-b]pyridazin-8-amine

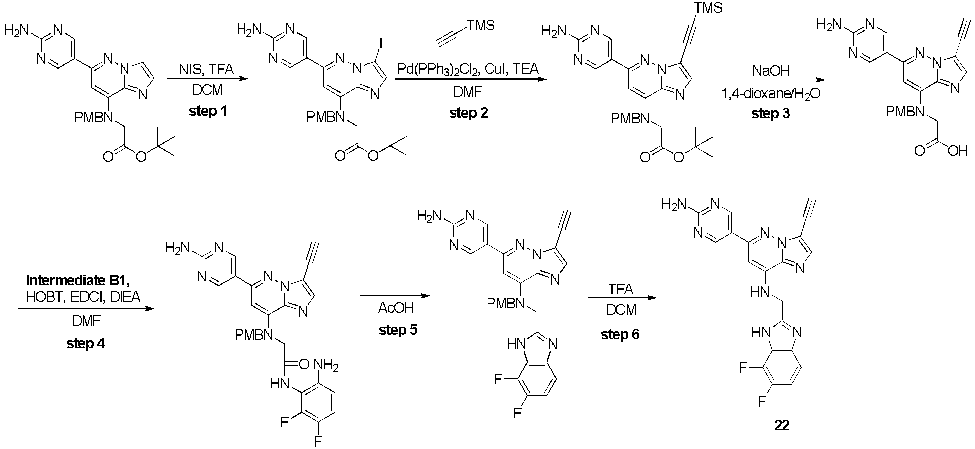

### Step 1: Preparation of tert-butyl N-(6-(2-aminopyrimidin-5-yl)-3-iodoimidazo[1,2-blpyridazin-8-yl)-N-(4-methoxybenzyl)glycinate

*tert-butyl N*-(6-(2-aminopyrimidin-5-yl)-3-iodoimidazo[1,2-*b*]pyridazin-8-yl)-*N*-(4-methoxybenzyl)glycinate (3.7 g, 72.68 % yield) was obtained in the same method as Step 2 of Example 8, except that the product obtained in Step 1 of Example 20 (4 g, 8.67 mmol) was used as a starting material and the reaction time was changed to 1 hour. MS: m/z = 588.2 (M+1, ESI+).

### Step 2: Preparation of tert-butyl N-(6-(2-aminopyrimidin-5-yl)-3-((trimethylsilyl)ethynyl)imidazo[1,2-b]pyridazin-8-yl)-N-(4-methoxybenzyl)glycinate

The product obtained in Step 1 (3.7 g, 6.30 mmol) and ethynyl(trimethyl)silane (928 mg, 9.45 mmol) were dissolved in DMF (50 mL), then CuI (240 mg, 1.26 mmol), Pd(PPh₃)₂Cl₂ (577 mg, 630 umol), and TEA (2.63 mL, 18.90 mmol) were added thereto, and the mixture was stirred at 50 °C for 2 hours under N₂ atmosphere. Water (500 mL) was added to the reaction mixture, and the product was extracted three times with EtOAc (100 mL). The organic layer was washed three times with brine (500 mL), dried over Na₂SO₄, filtered, and concentrated, and then the concentrate was purified by silica gel chromatography to obtain tert-butyl N-(6-(2-aminopyrimidin-5-yl)-3-((trimethylsilyl)ethynyl)imidazo[1,2-b]pyridazin-8-yl)-N-(4-methoxybenzyl)glycinate (2.9 g, 82.55 % yield). MS: m/z = 558.5 (M+1, ESI+).

### Step 3 to 6: Preparation of 6-(2-aminopyrimidin-5-yl)-N-((6,7-difluoro-1H-benzo[d]imidazol-2-yl)methyl)-3-ethynylimidazo[1.2-b]pyridazin-8-amine

The compound of Example 22 (30 mg, 9.66 % yield) was obtained as a white solid in the same method as Steps 4 to 7 of Example 8. However, the reaction time was changed to 16 hours in Step 5 of Example 8, and the reaction temperature was changed to 40 °C and stirred for 16 hours in Step 6 of Example 8. ¹H NMR (400 MHz, DMSO-d₆) δ 12.74 (s, 1H), 8.80 (s, 2H), 8.15 (s, 1H), 7.88 (s, 1H), 7.22-7.11 (m, 4H), 6.78 (s, 1H), 4.95 (d, 2H), 4.86 (s, 1H); MS: m/z = 418.1 (M+1, ESI+).

### Example 23: Preparation of 3-cyclopropyl-N-((6,7-difluoro-lH-benzo[d]imidazol-2-yl)methyl)-6-morpholinoimidazo[1,2-b]pyridazin-8-amine

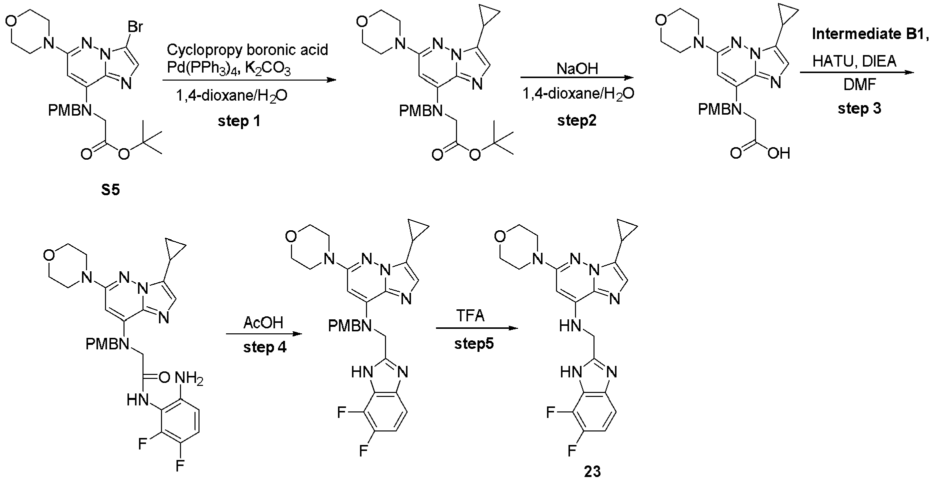

### Step 1: Preparation of tert-butylylN-(3-cyclopropyl-6-morpholinoimidazo[1,2-b]pyridazin-8-yl)-N-(4-methoxybenzyl)glycinate

**Intermediate S5** (2.6 g, 4.88 mmol) and cyclopropylboronic acid (1.26 g, 14.65 mmol) were dissolved in 1,4-dioxane/water (40 mL/10 mL), then Pd(PPh₃)₄ (564 mg, 488 umol) and potassium carbonate (K₂CO₃, 2.02 g, 14.65 mmol) were added thereto, and the mixture was stirred at 110 °C for 16 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain *tert*-butyl *N*-(3-cyclopropyl-6-morpholinoimidazo[1,2-b]pyridazin-8-yl)-N-(4-methoxybenzyl)glycinate (1.47 g, 60.99 % yield). MS: m/z = 494.2 (M+1, ESI+).

### Step 2: Preparation of N-(3-cyclopropyl-6-morpholinoimidazo[1,2-b]pyridazin-8-yl)-N-(4-methoxybenzyl)glycine

The product obtained in Step 1 (1.47 g, 2.98 mmol) was dissolved in 1,4-dioxane/water (10 mL/4 mL), and then *N*-(3-cyclopropyl-6-morpholinoimidazo[1,2-*b*]pyridazin-8-yl)-*N*-(4-methoxybenzyl)glycine (620 mg, 47.59 % yield) was obtained in the same method as Step 4 of Example 8. MS: m/z = 438.2 (M+1, ESI+).

### Step 3: Preparation of N-(6-amino-2,3-difluorophenyl)-2-((3-cyclopropyl-6-morpholinoimidazo[1,2-b]pyridazin-8-yl)(4-methoxybenzyl)amino)acetamide

The product obtained in Step 2 (620 mg, 1.42 mmol) and **Intermediate B1** (245 mg, 1.70 mmol) were dissolved in DMF (15 mL), then HATU (802 mg, 2.13 mmol) and DIEA (550 mg, 4.25 mmol) were added thereto, and the mixture was stirred at 70 °C for 16 hours. Water (150 mL) was added to the reaction mixture, and the product was extracted three times with DCM (40 mL). The organic layer was washed three times with brine (150 mL), dried over Na₂SO₄, filtered, and concentrated, and then the concentrate was purified by silica gel chromatography to obtain N-(6-amino-2,3-difluorophenyl)-2-((3-cyclopropyl-6-morpholinoimidazo[1,2-*b*]pyridazin-8-yl)(4-methoxybenzyl)amino)acetamide (410 mg, 51.33 % yield). MS: m/z = 564.2 (M+1, ESI+).

### Step 4: Preparation of 3-cyclopropyl-N-((6,7-difluoro-1H-benzo[d]imidazol-2-yl)methyl)-N-(4-methoxybenzyl)-6-(morpholinoimidazo[1,2-b]pyridazin-8-amine

3-cyclopropyl-N-((6,7-difluoro-1H-benzo[d]imidazol-2-yl)methyl)-N-(4-methoxybenzyl)-6-(morpholinoimidazo[1,2-*b*]pyridazin-8-amine (350 mg, 87.88 % yield) was obtained in the same method as Step 6 of Example 8, except that the reaction temperature was changed to 70 °C and the mixture was stirred for 16 hours. MS: m/z = 546.2 (M+1, ESI+).

### Step 5: Preparation of 3-cyclopropyl-N-((6,7-difluoro-1H-benzo[d]imidazol-2-yl)methyl)-6-morpholinoimidazo[1,2-b]pyridazin-8-amine

The product obtained in Step 4 (350 mg, 642 umol) was dissolved in TFA (5 mL), and then stirred at 25 °C for 1 hour. The reaction mixture was concentrated, and the concentrate was purified by Prep-HPLC to obtain **the compound of Example 23** (120 mg, 43.97 % yield) as a white solid. ¹H NMR (400 MHz, DMSO-d₆) δ 12.70 (s, 1H), 7.47 (s, 1H), 7.26-7.16 (m, 2H), 7.09 (s, 1H), 6.00 (s, 1H), 4.80 (d, 2H), 3.68 (t, 4H), 3.34 (t, 4H), 2.09-2.04 (m, 1H), 0.96-0.92 (m, 2H), 0.80-0.76 (m, 2H); MS: m/z = 426.2 (M+1, ESI+).

### Example 24: Preparation of N-((6,7-difluoro-1H-benzo[d]imidazol-2-yl)methyl)-6-morpholino-3-(prop-1-yn-1-yl)imidazo[1,2-b]pyridazin-8-amine

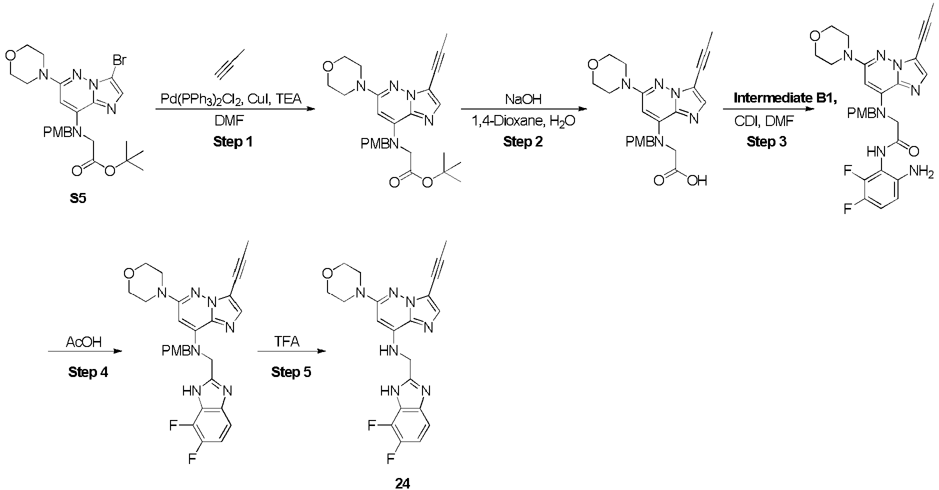

### Step 1: Preparation of tert-butyl N-(4-methoxybenzyl)-N-(6-morpholino-3-(prop-1-yn-1-yl)imidazo[1,2-b]pyridazin-8-yl)glycinate

**Intermediate S5** (4 g, 7.51 mmol) and prop-1-yne (3 g, 75.1 mmol) were dissolved in DMF (30 mL), then CuI (429 mg, 2.25 mmol), Pd(PPh₃)₂Cl₂ (527 mg, 751 umol), and TEA (3.14 mL, 22.54 mmol) were added thereto, and the mixture was stirred at 40 °C for 48 hours under N₂ atmosphere. Water (300 mL) was added to the reaction mixture, and the product was extracted three times with EtOAc (80 mL). The organic layer was washed three times with brine (80 mL), dried over Na₂SO₄, filtered, and concentrated, and then the concentrate was purified by silica gel chromatography to obtain *tert-butyl N-(4-methoxybenzyl)-N-(6-morpholino-3-(prop-1-yn-1-*yl)imidazo[1,2-b]pyridazin-8-yl)glycinate (1.45 g, 39.26 % yield). MS: m/z = 492.2 (M+1, ESI+).

### Step 2: Preparation of N-(4-methoxybenzyl)-N-(6-morpholino-3-(prop-1-yn-1-yl)imidazo[1,2-b]pyridazin-8-yl)glycine

The product obtained in Step 1 (1.45 g, 2.95 mmol) was dissolved in 1,4-dioxane/water (15 mL/15 mL), and then N-(4-methoxybenzyl)-N-(6-morpholino-3-(prop-1-yn-1-yl)imidazo[1,2-b]pyridazin-8-yl)glycine (950 mg, 73.96 % yield) was obtained in the same method as Step 4 of Example 8. MS: m/z = 436.2 (M+1, ESI+).

### Step 3: Preparation of N-(6-amino-2,3-difluorophenyl)-2-((4-methoxybenzyl)(6-morpholino-3-(prop-1-yn-1-yl)imidazo[1,2-b]pyridazin-8-yl)amino)acetamide

The product obtained in Step 2 (900 mg, 2.07 mmol) was dissolved in DMF (15 mL), then 1,1-carbonyldiimidazole (312 mg, 2.17 mmol) was added thereto, and the mixture was stirred at 25 °C for 2 hours. Intermediate **B1** (298 mg, 2.07 mmol) was added to this reaction solution, and the mixture was stirred at 50 °C for 16 hours. Water (150 mL) was added to the reaction mixture, and the product was extracted three times with DCM (40 mL). The organic layer was washed three times with brine (150 mL), dried over Na₂SO₄, filtered, and concentrated, and then the concentrate was purified by silica gel chromatography to obtain N-(6-amino-2,3-difluorophenyl)-2-((4-methoxybenzyl)(6-morpholino-3-(prop-1-yn-1-yl)imidazo[1,2-*b*]pyridazin-8-yl)amino)acetamide (470 mg, 40.50 % yield). MS: m/z = 562.2 (M+1, ESI+).

### Step 4: Preparation of N-((6,7-difluoro-1H-benzo[d]imidazol-2-yl)methyl)-N-(4-methoxybenzyl)-6-(morpholino-3 -(prop-1-yn-1-yl)imidazo[1,2-b]pyridazin-8-amine

N-((6,7-difluoro-1H-benzo[d]imidazol-2-yl)methyl)-N-(4-methoxybenzyl)-6-(morpholino-3-(prop-1-yn-1-yl)imidazo[1,2-*b*]pyridazin-8-amine (410 mg, 90.13 % yield) was obtained in the same method as Step 6 of Example 8, except that the reaction mixture was stirred for 1 hour. MS: m/z = 520.2 (M+1, ESI+).

### Step 5: Preparation of N-((6,7-difluoro-1H-benzo[d]imidazol-2-yl)methyl)-6-morpholino-3-(prop-1-yn-1-yl)imidazo[1,2-b]pyridazin-8-amine

The product obtained in Step 4 (410 mg, 754 umol) was dissolved in TFA (10 mL), then stirred at 25 °C for 1 hour. The reaction mixture was concentrated, and the concentrate was purified by Prep-HPLC to obtain **the compound of Example 24** (50 mg, 15.67 % yield) as a white solid. ¹H NMR (400 MHz, DMSO-d₆) δ 7.63 (t, 1H), 7.55 (s, 1H), 7.28-7.19 (m, 2H), 6.06 (s, 1H), 4.81 (d, 2H), 3.68 (t, 4H), 3.39 (t, 4H), 2.15 (s, 3H); MS: m/z = 424.2 (M+1, ESI+).

### Example 25: Preparation of 3-(cyclopropylethynyl)-N-((6,7-difluoro-1H-benzo[d]imidazol-2-yl)methyl)-6-morpholinoimidazo[1,2-b]pyridazin-8-amine

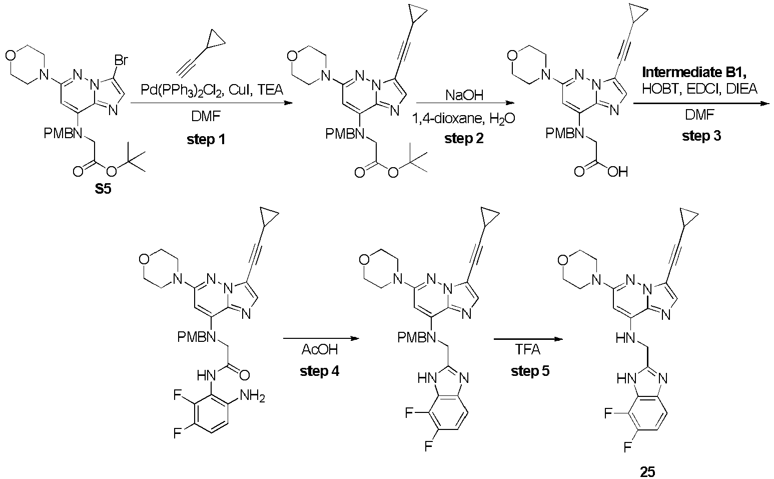

### Step 1: Preparation of tert-butyl N-(3-(cyclopropylethynyl)-6-morpholinoimidazo[1,2-blpyridazin-8-yl)-N-(4-methoxybenzyl)glycinate

*tert*-butyl *N*-(3-(cyclopropylethynyl)-6-morpholinoimidazo[1,2-*b*]pyridazin-8-yl)-*N*-(4-methoxybenzyl)glycinate (2.66 g, 65.15 % yield) was obtained in the same method as Step 1 of Example 24, except that ethynylcyclopropane (782 mg, 11.83 mmol) was used instead of prop-1-yne (3 g, 75.1 mmol) in Step 1 of Example 24, starting from **Intermediate S5** (4.2 g, 7.89 mmol) and stirring at 90 °C. MS: m/z = 518.2 (M+1, ESI+).

### Step 2: Preparation of N-(3-(cyclopropylethynyl)-6-morpholinoimidazo[1,2-b]pyridazin-8-yl)-N-(4-methoxybenzyl)glycine

The product of Step 1 (2.6 g, 5.02 mmol) was used to obtain N-(3-(cyclopropylethynyl)-6-morpholinoimidazo[1,2-*b*]pyridazin-8-yl)-*N*-(4-methoxybenzyl)glycine (1.5 g, 64.71 % yield) in the same method as Step 4 of Example 8. MS: m/z = 462.2 (M+1, ESI+).

### Step 3: Preparation of N-(6-amino-2,3-difluorophenyl)-2-((3-(cyclopropylethynyl)-6-morpholinoimidazo[1,2-b]pyridazin-8-yl)(4-methoxybenzyl)amino)acetamide

*N*-(6-amino-2,3-difluorophenyl)-2-((3-(cyclopropylethynyl)-6-morpholinoimidazo[1,2-b]pyridazin-8-yl)(4-methoxybenzyl)amino)acetamide (500 mg, 29.09 % yield) was obtained in the same method as Step 5 of Example 8, except that the reaction time was changed to 16 hours. MS: m/z = 588.2 (M+1, ESI+).

### Step 4 and 5: Preparation of 3-(cyclopropylethynyl)-N-((6,7-difluoro-1H-benzo[d]imidazol-2-yl)methyl)-6-morpholinoimidazo[1,2-b]pyridazin-8-amine

**The compound of Example 25** (58 mg, 11.97 % yield) was obtained as a white solid in the same method as Step 6 and Step 7 of Example 8, except that the reaction times were changed to 4 hours and 2 hours, respectively. ¹H NMR (400 MHz, DMSO-d₆) δ 7.62 (t, 1H), 7.53 (s, 1H), 7.28-7.16 (m, 2H), 6.05 (s, 1H), 4.81 (d, 2H), 3.68 (s, 4H), 3.34 (s, 4H), 1.66-1.62 (m, 1H), 0.95-0.92 (m, 2H), 0.77-0.76 (m, 2H); MS: m/z = 450.4 (M+1, ESI+).

### Example 26: Preparation of N-((6,7-difluoro-1H-benzo[d]imidazol-2-yl)methyl)-3-iodo-6-morpholinoimidazo[1.2-b]pyridazin-8-amine

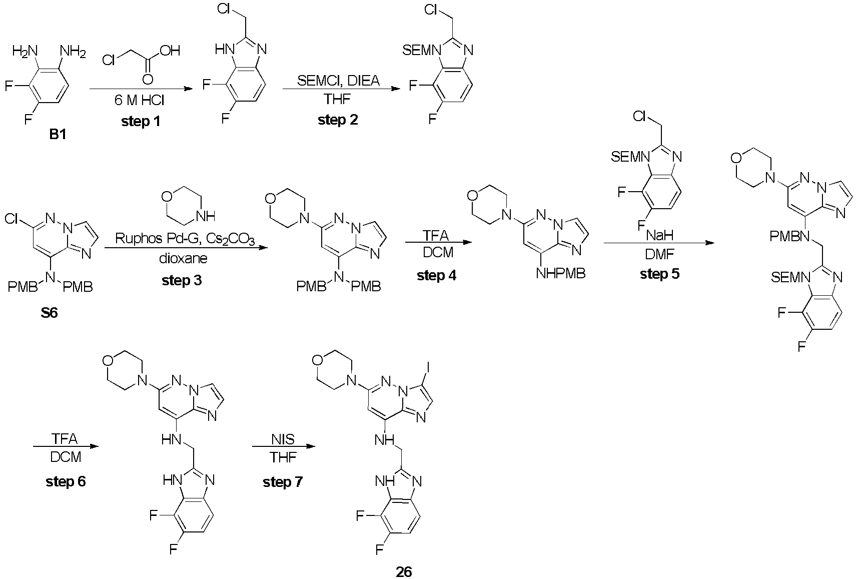

### Step 1: Preparation of 2-(chloromethyl)-6,7-difluoro-1H-benzo[dlimidazole

**Intermediate B1** (10 g, 69.44 mmol) was dissolved in 6 M hydrochloric acid solution (100 mL), then 2-chloroacetic acid (9.84 g, 104.2 mmol) was added and stirred at 100 °C for 16 hours. Water (200 mL) was added to the reaction mixture, and the product was extracted three times with EtOAc (80 mL). The organic layer was washed three times with brine (200 mL), dried over Na₂SO₄, filtered, and concentrated, and then the concentrate was purified by silica gel chromatography to obtain 2-(chloromethyl)-6,7-difluoro-1H-benzo[d]imidazole (10 g, 71.42 % yield). MS: m/z = 203.2 (M+1, ESI+).

### Step 2: Preparation of 2-(chloromethyl)-6,7-difluoro-1-((2-(trimethylsilyl)ethoxy)methyl)-1H-benzo[d]imidazole

The product obtained in Step 1 (10 g, 49.50 mmol) and DIEA (17.2 mL, 99 mmol) were dissolved in THF, then TMSC1 (11.85 g, 71.4 mmol) was added at 0 °C and stirred at 25 °C for 16 hours. Water (200 mL) was added to the reaction mixture, and the product was extracted three times with EtOAc (80 mL). The organic layer was washed three times with brine (200 mL), dried over Na₂SO₄, filtered, and concentrated, and then the concentrate was purified by silica gel chromatography to obtain 2-(chloromethyl)-6,7-difluoro-1-((2-(trimethylsilyl)ethoxy)methyl)-1*H*-benzo[*d*]imidazole (16 g, 97.56 % yield). MS: m/z = 333.2 (M+1, ESI+).

### Step 3: Preparation of N,Nbis(4-methoxybenzyl)-6-morpholinoimidazo[1,2-b]pyridazin-8-amine

*N,N*-bis(4-methoxybenzyl)-6-morpholinoimidazo[1,2-*b*]pyridazin-8-amine (4 g, 88.98 % yield) was obtained in the same method as Step 5 of Example 2, starting from Intermediate S6 (4 g, 9.78 mmol). MS: m/z = 460.2 (M+1, ESI+).

### Step 4: Preparation of N-(4-methoxybenzyl)-6-morpholinoimidazo[1,2-b]pyridazin-8-amine

The product obtained in Step 3 (4 g, 9.78 mmol) was dissolved in DCM (20 mL), then TFA (10 mL) was added thereto, and the mixture was stirred at 25 °C for 3 hours. 2 N sodium bicarbonate solution (100 mL) was added to the reaction mixture, and the product was extracted three times with DCM (40 mL). The organic layer was washed three times with brine (100 mL), dried over Na₂SO₄, filtered, and concentrated, and then the concentrate was purified by silica gel chromatography to obtain *N*-(4-methoxybenzyl)-6-morpholinoimidazo[1,2-*b*]pyridazin-8-amine (2.8 g, 84.33 % yield). MS: m/z = 340.1 (M+1, ESI+).

### Step 5: Preparation of N-((6,7-difluoro-1-((2-(trimethylsilyl)ethoxy)methyl)-1H-benzo[d]imidazol-2-yl)methyl)-N-(4-methoxybenzyl)-6-morpholinoimidazo[1,2-b]pyridazin-8-amine

The product obtained in Step 4 (2.8 g, 8.25 mmol) was dissolved in DMF (30 mL), then NaH (990 mg, 24.75 mmol, 60 % purity) was added thereto in portions at 0 °C and stirred for 30 minutes. The product of Step 2 (4.12 g, 12.38 mmol) was added to this reaction solution, and the mixture was stirred at 25 °C for 16 hours. Water (300 mL) was added to the reaction mixture, and the product was extracted three times with EtOAc (80 mL). The organic layer was washed three times with brine (300 mL), dried over Na₂SO₄, filtered, and concentrated, and then the concentrate was purified by silica gel chromatography to obtain N-((6,7-difluoro-1-((2-(trimethylsilyl)ethoxy)methyl)-1H-benzo[d]imidazol-2-yl)methyl)-N-(4-methoxybenzyl)-6-morpholinoimidazo[1,2-*b*]pyridazin-8-amine (2.6 g, 49.57 % yield). MS: m/z = 636.5 (M+1, ESI+).

### Step 6: Preparation of N-((6,7-difluoro-1H-benzo[d]imidazol-2-yl)methyl)-6-morpholinoimidazo[1,2-b]pyridazin-8-amine

The product obtained in Step 5 (2.6 g, 4.09 mmol) was dissolved in DCM (20 mL), then TFA (10 mL) was added thereto, and the mixture was stirred at 25 °C for 1 hour. 2 N sodium bicarbonate solution (200 mL) was added to the reaction mixture, and the product was extracted three times with DCM (60 mL). The organic layer was washed three times with brine (200 mL), dried over Na₂SO₄, filtered, and concentrated, and then the concentrate was purified by silica gel chromatography to obtain N-((6,7-difluoro-1H-benzo[d]imidazol-2-yl)methyl)-6-morpholinoimidazo[1,2-*b*]pyridazin-8-amine (1 g, 63.45 % yield). MS: m/z = 386.2 (M+1, ESI+).

### Step 7: Preparation of N-((6,7-difluoro-1H-benzo[d]imidazol-2-yl)methyl)-3-iodo-6-morpholinoimidazo[1,2-b]pyridazin-8-amine

The product obtained in Step 6 (500 mg, 1.30 mmol) was dissolved in THF (10 mL), then NIS (263 mg, 1.17 mmol) was added at 0 °C and the mixture was stirred for 1 hour. Water (80 mL) was added to the reaction mixture, and the product was extracted three times with EtOAc (20 mL). This organic layer was washed again three times with sodium sulfite solution (80 mL), dried over Na₂SO₄, filtered, and concentrated, and then the concentrate was purified by Prep-HPLC to obtain the compound of Example 26 (56 mg, 8.44 % yield) as a yellow solid. ¹H NMR (400 MHz, DMSO-d₆) δ 7.96 (d, 1H), 7.43 (d, 1H), 7.24-7.14 (m, 2H), 7.06 (t, 1H), 5.72 (d, 2H), 3.79 (t, 4H), 3.12 (t, 4H); MS: m/z = 512.0 (M+1, ESI+).

### Example 27: Preparation of 3-chloro-N-((6,7-difluoro-1H-benzo[d]imidazol-2-yl)methyl)-6-morpholinoimidazo[1,2-b]pyridazin-8-amine

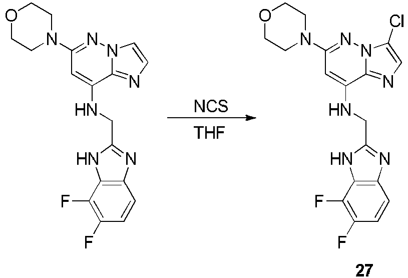

The product obtained in Step 6 of Example 26 (300 mg, 778 umol) was dissolved in THF (5 mL), then NCS (135 mg, 1.01 mmol) was added at 0 °C and stirred for 1 hour. Water (50 mL) was added to the reaction mixture, and the product was extracted three times with EtOAc (15 mL). This organic layer was washed again three times with sodium sulfite solution (50 mL), dried over Na₂SO₄, filtered, and concentrated, and then the concentrate was purified by silica gel chromatography to obtain **the compound** of Example **27** (155 mg, 47.43 % yield) as a white solid. ¹H NMR (400 MHz, DMSO-d₆) δ 7.92 (d, 1H), 7.56 (t, 1H), 7.42 (s, 1H), 7.25-7.13 (m, 2H), 5.69 (d, 2H), 3.78 (t, 4H), 3.17 (t, 4H); MS: m/z = 421.7 (M+1, ESI+).

### Example 28: Preparation of 6-(4-cyclopropylpiperazin-1-yl)-N-((6,7-difluoro-1H-benzo[d]imidazol-2-yl)methyl)-3-(trifluoromethyl)imidazo[1,2-b]pyridazin-8-amine

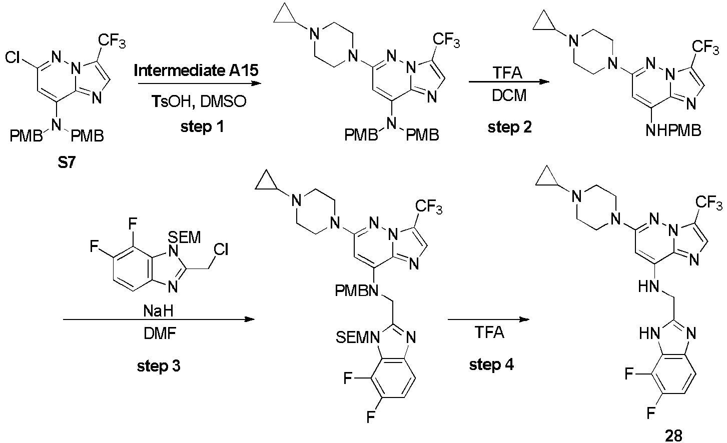

### Step 1: Preparation of 6-(4-cyclopropylpiperazin-1-yl)-N,N-bis(4-methoxybenzyl)-3-(trifluoromethyl)imidazo[1,2-b]pyridazin-8-amine

**Intermediate** S7 (2.1 g, 4.52 mmol) and **Intermediate A15** (1.17 g, 6.77 mmol) were dissolved in DMSO (15 mL), then TsOH (722 mg, 4.19 mmol) was added thereto and stirred at 140 °C for 16 hours. Water (150 mL) was added to the reaction mixture, and the product was extracted three times with EtOAc (50 mL). The organic layer was washed three times with brine (150 mL), dried over Na₂SO₄, filtered, and concentrated, and then the concentrate was purified by silica gel chromatography to obtain 6-(4-cyclopropylpiperazin-1-yl)-*N,N*-bis(4-methoxybenzyl)-3-(trifluoromethyl)imidazo[1,2-b]pyridazin-8-amine (1.33 g, 55.97 % yield). MS: m/z = 567.5 (M+1, ESI+).

### Step 2: Preparation of 6-(4-cyclopropylpiperazin-1-yl)-N-(4-methoxybenzyl)-3-(trifluoromethyl)imidazo[1,2-b]pyridazin-8-amine

The product obtained in Step 1 (800 mg, 1.41 mmol) was dissolved in DCM (6 mL) and TFA (2 mL), and then stirred at 25 °C for 3 hours. Sodium bicarbonate solution (100 mL) was added to the reaction mixture, and the product was extracted three times with DCM (40 mL). The organic layer was washed three times with brine (100 mL), dried over Na₂SO₄, filtered, and concentrated, and then the concentrate was purified by silica gel chromatography to obtain 6-(4-cyclopropylpiperazin-1-yl)-*N*-(4-methoxybenzyl)-3-(trifluoromethyl)imidazo[1,2-*b*]pyridazin-8-amine (500 mg, 79.32 % yield). MS: m/z = 447.2 (M+1, ESI+).

### Step 3: Preparation of 6-(4-cyclopropylpiperazin-1-yl)-N-((6,7-difluoro-1-((2-(trimethylsilyl)ethoxy)methyl)-1H-benzo[d]imidazol-2-yl)methyl)-N-(4-methoxybenzyl)-3-(trifluoromethyl)imidazo[1,2-b]pyridazin-8-amine

6-(4-cyclopropylpiperazin-1-yl)-*N*-((6,7-difluoro-1-((2-(trimethylsilyl)ethoxy)methyl)-1*H*-benzo[*d*]imidazol-2-yl)methyl)-*N*-(4-methoxybenzyl)-3-(trifluoromethyl)imidazo[1,2-b]pyridazin-8-amine (650 mg, 78.13 % yield) was obtained in the same method as Step 5 of Example 26. MS: m/z = 743.5 (M+1, ESI+).

### Step 4: Preparation of 6-(4-cyclopropylpiperazin-1-yl)-N-((6,7-difluoro-1H-benzo[d]imidazol-2-yl)methyl)-3-(trifluoromethyl)imidazo[1,2-b]pyridazin-8-amine

The product obtained in Step 3 (650 mg, 875 umol) was dissolved in TFA (10 mL) and then stirred at 25 °C for 1 hour. The reaction mixture was concentrated and then purified by Prep-HPLC to obtain **the compound of Example 28** (140 mg, 32.49 % yield) as a white solid. ¹H NMR (400 MHz, DMSO-d₆) δ 12.70 (s, 1H), 7.87 (s, 2H), 7.26-7.17 (m, 2H), 6.20 (s, 1H), 4.85 (d, 2H), 3.33 (s, 4H), 2.57 (s, 4H), 1.60 (s, 1H), 0.42 (s, 2H), 0.33 (s, 2H); MS: m/z = 493.2 (M+1, ESI+).

Hereinafter, the compounds of Examples 29 to 67 were prepared using the appropriate intermediates from the Intermediate A series in [Table 1] and Intermediate B series in [Table 3] according to Scheme II below.

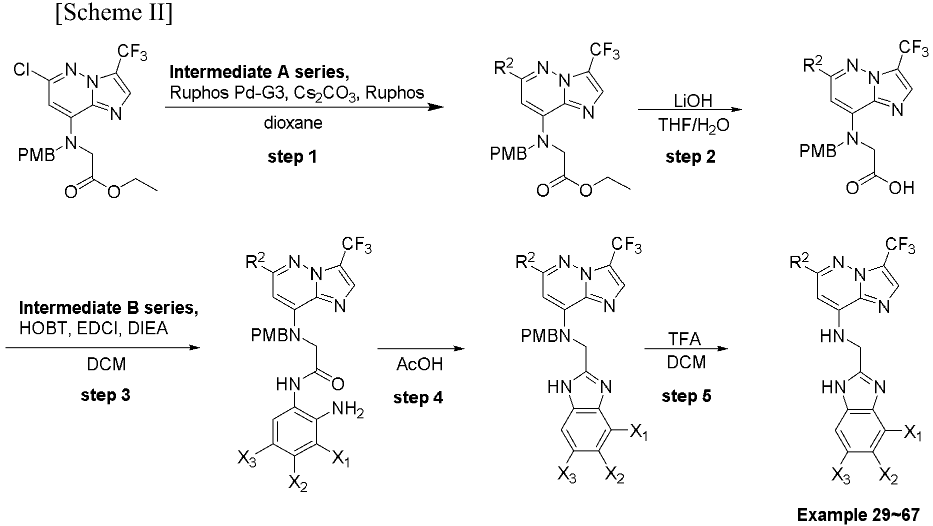

In the Scheme II, R² has the same structure as R² of each example compound, and X₁, X₂ and X₃ also represent the substituent structures at the corresponding positions of each example compound. Intermediates referred to as Intermediate B1 to Intermediate B30 described in [Table 3] below have the structures shown in [Table 3] below, throughout the examples as well as Examples 29 to 67.

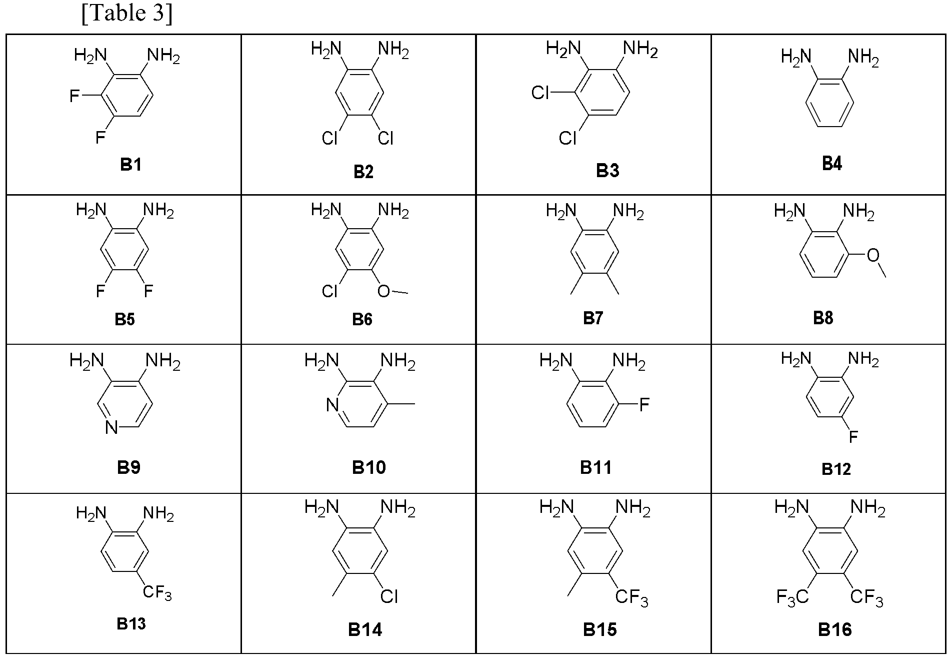

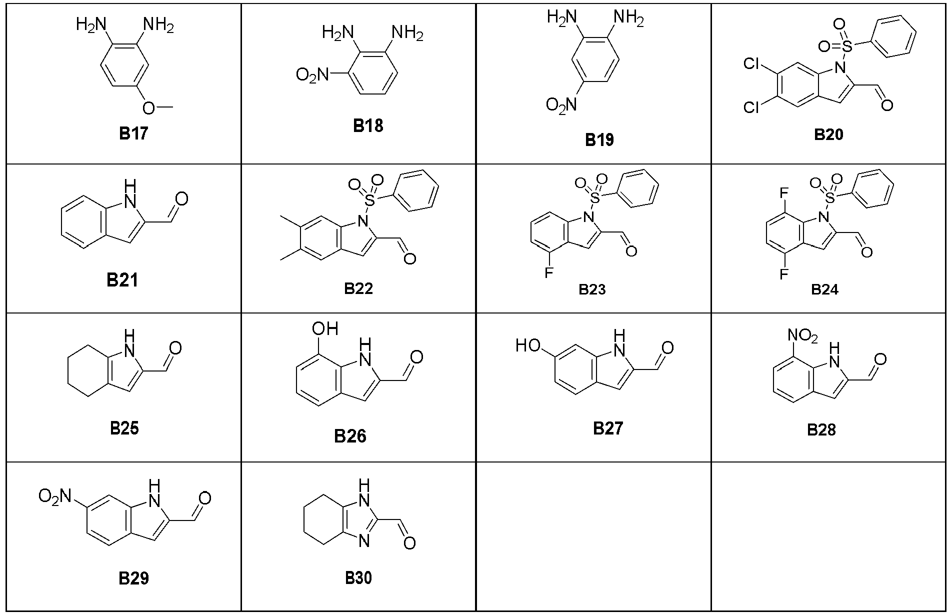

### Example 29: Preparation of N-((5,6-dichloro-1H-benzo[d]imidazol-2-yl)methyl)-6-(4-methylpiperazin-1-yl)-3-(trifluoromethyl)imidazo[1,2-b]pyridazin-8-amine

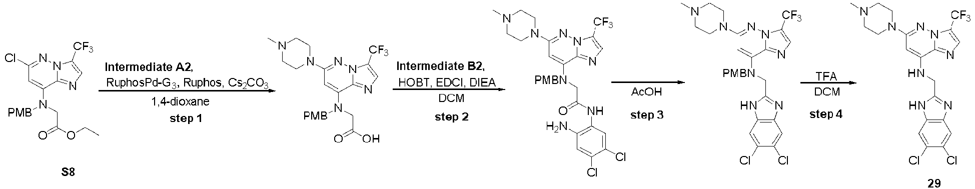

### Step 1: Preparation of N-(4-methoxybenzyl)-N-(6-(4-methylpiperazin-1-yl)-3-(trifluoromethyl)imidazo[1,2-b]pyridazin-8-yl)glycine

**Intermediate** S8 (1 g, 2.26 mmol) and **Intermediate** A2 (1.13 g, 11.29 mmol) were dissolved in 1,4-dioxane (20 mL), then Ruphos-Pd-G3 (189 mg, 226 umol), 2-dicyclohexylphosphino-2',6'-diisopropoxybiphenyl (RuPhos, 106 mg, 226 umol), and cesium carbonate (3.68 g, 11.29 mmol) were added thereto, and the mixture was stirred at 110 °C for 16 hours. 1 N hydrochloric acid (150 mL) was added to the reaction mixture, and the product was extracted three times with EtOAc (50 mL). The organic layer was washed three times with brine (200 mL), dried over Na₂SO₄, filtered, and concentrated, and then the concentrate was purified by silica gel chromatography to obtain N-(4-methoxybenzyl)-N-(6-(4-methylpiperazin-1-yl)-3-(trifluoromethyl)imidazo[1,2-b]pyridazin-8-yl)glycine (400 mg, 37.02 % yield). MS: m/z = 479.2 (M+1, ESI+).

### Step 2: Preparation of N-(2-amino-4,5-dichlorophenyl)-2-((4-methoxybenzyl)(6-(4-methylpiperazin-1-yl)-3-(trifluoromethyl)imidazo[1,2-b]pyridazin-8-yl)amino)acetamide

The product obtained in Step 1 (340 mg, 711 umol) and **Intermediate B2** (629 mg, 3.55 mmol) were dissolved in DCM (10 mL), then HOBT (480 mg, 3.55 mmol), EDCI (681 mg, 3.55 mmol), and DIEA (1.24 mL, 7.11 mmol) were added thereto, and the mixture was stirred at 25 °C for 16 hours. Water (80 mL) was added to the reaction mixture, and the product was extracted three times with DCM (30 mL). The organic layer was washed three times with brine (80 mL), dried over Na₂SO₄, filtered, and concentrated, and then the concentrate was purified by silica gel chromatography to obtain N-(2-amino-4,5-dichlorophenyl)-2-((4-methoxybenzyl)(6-(4-methylpiperazin-1-yl)-3-(trifluoromethyl)imidazo[1,2-b]pyridazin-8-yl)amino)acetamide (200 mg, 44.15 % yield). MS: m/z = 637.1 (M+1, ESI+).

### Step 3: Preparation of N-((5,6-dichloro-1H-benzo[d]imidazol-2-yl)methyl)-N-(4-methoxybenzyl)-6-(4-methylpiperazin-1-yl)-3-(trifluoromethyl)imidazo[1,2-b]pyridazin-8-amine

The product obtained in Step 2 (150 mg, 235 umol) was dissolved in TFA (8 mL), then the mixture was stirred at 70 °C for 16 hours. Sodium bicarbonate solution (100 mL) was added to the reaction mixture, and the product was extracted three times with EtOAc (40 mL). The organic layer was washed three times with brine (100 mL), dried over Na₂SO₄, filtered, and concentrated, and then the concentrate was purified by silica gel chromatography to obtain N-((5,6-dichloro-1H-benzo[d]imidazol-2-yl)methyl)-N-(4-methoxybenzyl)-6-(4-methylpiperazin-1-yl)-3-(trifluoromethyl)imidazo[1,2-b]pyridazin-8-amine (130 mg, 89.19 % yield). MS: m/z = 619.2 (M+1, ESI+).

### Step 4: Preparation of N-((5,6-dichloro-1H-benzo[d]imidazol-2-yl)methyl)-6-(4-methylpiperazin-1-yl)-3-(trifluoromethyl)imidazo[1,2-b]pyridazin-8-amine

The product obtained in Step 3 (139 mg, 204 umol) was dissolved in DCM (4 mL), then TFA (1 mL) was added thereto, and the mixture was stirred at 25 °C for 1 hour. The reaction mixture was concentrated and then purified by Prep-HPLC to obtain **the compound of Example 29** (35 mg, 34.37 % yield) as a white solid. ¹H NMR (400 MHz, DMSO-d₆) δ 7.86 (s, 1H), 7.81 (t, 1H), 7.76 (s, 2H), 6.16 (s, 1H), 4.83 (d, 2H), 3.36 (t, 4H), 2.35 (t, 4H), 2.17 (s, 3H); MS: m/z = 499.1 (M+1, ESI+).

### Example 30: Preparation of 1-(8-(((5,6-dichloro-1H-benzol[d]imidazol-2-yl)methyl)amino)-3-(trifluoromethyl)imidazo[1,2-b]pyridazin-6-yl)azetidine-3-carbonitrile

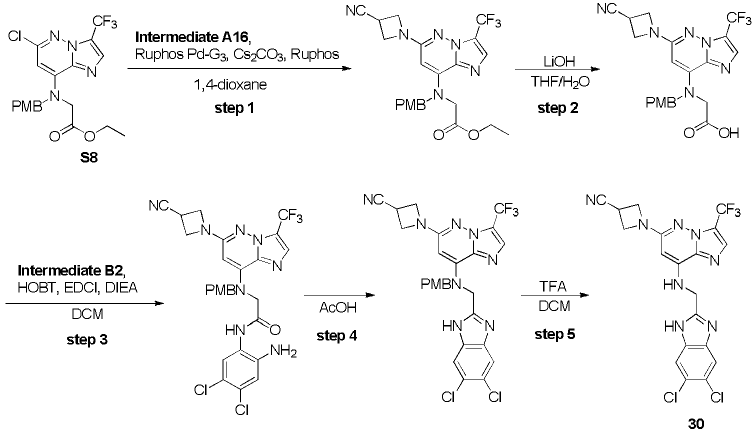

### Step 1: Preparation of ethyl N-(6-(3-cyanoazetidin-1-yl)-3-(trifluoromethyl)imidazo[1,2-blpyridazin-8-yl)-N-(4-methoxybenzyl)glycinate

**Intermediate** S8 (2 g, 4.52 mmol) and **Intermediate A16** (742 mg, 9.03 mmol) were dissolved in 1,4-dioxane (20 mL), then Ruphos-Pd-G3 (189 mg, 226 umol), 2-dicyclohexylphosphino-2',6'-diisopropoxybiphenyl (RuPhos, 106 mg, 226 umol), and cesium carbonate (2.94 g, 9.03 mmol) were added thereto, and the mixture was stirred at 110 °C for 48 hours. Water (200 mL) was added to the reaction mixture, and the product was extracted three times with EtOAc (60 mL). The organic layer was washed three times with brine (20 mL), dried over Na₂SO₄, filtered, and concentrated, and then the concentrate was purified by silica gel chromatography to obtain ethyl *N*-(6-(3-cyanoazetidin-1-yl)-3-(trifluoromethyl)imidazo[1,2-b]pyridazin-8-yl)-N-(4-methoxybenzyl)glycinate (1.5 g, 67.99 % yield). MS: m/z = 489.1 (M+1, ESI+).

### Step 2: Preparation of N-(6-(3-cyanoazetidin-1-yl)-3-(trifluoromethyl)imidazo[1,2-b]pyridazin-8-yl)-N-(4-methoxybenzyl)glycine

The product obtained in Step 1 (1.5 g, 3.07 mmol) was dissolved in THF (10 mL) and water (5 mL), then lithium hydroxide (368 mg, 15.35 mmol) was added thereto and stirred at 25 °C for 2 hours. 1 N hydrochloric acid (100 mL) was added to the reaction mixture, and the product was extracted three times with DCM (30 mL). The organic layer was washed three times with brine (100 mL), dried over Na₂SO₄, filtered, and concentrated, and then the concentrate was purified by silica gel chromatography to obtain *N*-(6-(3-cyanoazetidin-1-yl)-3-(trifluoromethyl)imidazo[1,2-b]pyridazin-8-yl)-N-(4-methoxybenzyl)glycine (900 mg, 63.66 % yield). MS: m/z = 461.1 (M+1, ESI+).

### Step 3: Preparation of N-(2-amino-4,5-dichlorophenyl)-2-((6-(3-cyanoazetidin-1-yl)-3-(trifluoromethyl)imidazo[1,2-b]pyridazin-8-yl)(4-methoxybenzyl)amino)acetamide

The product obtained in Step 2 (900 mg, 1.95 mmol) and **Intermediate B2** (1.38 g, 7.8 mmol) were dissolved in DCM (20 mL), then HOBT (1.32 g, 9.77 mmol), EDCI (1.87 g, 9.77 mmol), and DIEA (3.41 mL, 19.55 mmol) were added thereto, and the mixture was stirred at 25 °C for 2 hours. Water (80 mL) was added to the reaction mixture, and the product was extracted three times with DCM (30 mL). The organic layer was washed three times with brine (80 mL), dried over Na₂SO₄, filtered, and concentrated, and then the concentrate was purified by silica gel chromatography to obtain *N*-(2-amino-4,5-dichlorophenyl)-2-((6-(3-cyanoazetidin-1-yl)-3-(trifluoromethyl)imidazo[1,2-b]pyridazin-8-yl)(4-methoxybenzyl)amino)acetamide (600 mg, 49.55 % yield). MS: m/z = 619.2 (M+1, ESI+).

### Step 4: Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidine-3-carbonitrile

The product obtained in Step 3 (600 mg, 969 umol) was dissolved in AcOH (10 mL) and then stirred at 100 °C for 1 hour. Sodium bicarbonate solution (100 mL) was added to the reaction mixture, and the product was extracted three times with EtOAc (40 mL). The organic layer was washed three times with brine (100 mL), dried over Na₂SO₄, filtered, and concentrated, and then the concentrate was purified by silica gel chromatography to obtain 1-(8-(((5,6-dichloro-1H-benzo[*d*]imidazol-2-yl)methyl)(4-methoxybenzyl)amino)-3-(trifluoromethyl)imidazo[1,2-b]pyridazin-6-yl)azetidine-3-carbonitrile (400 mg, 68.66 % yield). MS: m/z = 601.2 (M+1, ESI+).

### Step 5: Preparation of 1-(8-(((5,6-dichloro-1H-benzo[d]imidazol-2-yl)methyl)amino)-3-(trifluoromethyl)imidazo[1,2-b]pyridazin-6-yl)azetidine-3-carbonitrile

The product obtained in Step 4 (400 mg, 665 umol) was dissolved in DCM (6 mL), then TFA (3 mL) was added thereto, and the mixture was stirred at 25 °C for 2 hours. The reaction mixture was concentrated and then purified by Prep-HPLC to obtain **the compound of Example 30** (55 mg, 17.18 % yield) as a white solid. ¹H NMR (400 MHz, DMSO-d₆) δ 12.61 (s, 1H), 7.98 (t, 1H), 7.89 (s, 1H), 7.77 (s, 2H), 5.71 (s, 1H), 4.81 (d, 2H), 4.18 (t, 2H), 4.04 (dd, 2H), 3.85-3.79 (m, 1H); MS: m/z = 481.1 (M+1, ESI+).

### Examples 31 to 56

The compounds of Examples 31 to 56 were prepared in the same method as Example 30, using Intermediate A of [Table 1] and Intermediate B of [Table 3] corresponding to the structure of the target compound, while appropriately changing the amounts of reagents, catalysts, and reaction conditions.

**[Table 4]**

| Example | Structure (Intermediate) | IUPAC Name | NMR and MS Data |
|---|---|---|---|
| 31 | 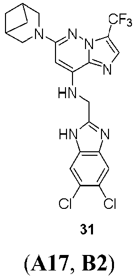 | 6-(3-azabicyclo[3.1.1]heptan-3-yl)-*N*-((5,6-dichloro-1*H-*benzo[d]imidazol-2-yl)methyl)-3-(trifluoromethyl)imidazo[1,2-b]pyridazin-8-amine | ¹H NMR (400 MHz, DMSO-d₆) δ 12.61 (s, 1H), 7.82-7.77 (m, 4H), 5.89 (s, 1H), 4.83 (d, 2H), 3.51 (s, 4H), 2.70-2.65 (m, 2H), 2.12 (d, 2H), 1.32 (dd, 2H); MS: m/z = 496.0 (M+1, ESI+). |
| 32 | 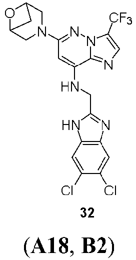 | 6-(6-oxa-3-azabicyclo[3.1.1]heptan-3-yl)-*N-*((5,6-dichloro-1*H-*benzo[d]imidazol-2-yl)methyl)-3-(trifluoromethyl)imidazo[1,2-blpyridazin-8-amine | ¹H NMR (400 MHz, DMSO-d₆) δ 12.62 (s, 1H), 7.88-7.70 (m, 4H), 5.96 (s, 1H), 4.85 (d, 2H), 4.67 (d, 2H), 3.60 (d, 2H), 3.48 (d, 2H), 3.08 (dd, 1H), 1.84 (d, 1H); MS: m/z = 498.1 (M+1, ESI+). |
| 33 | 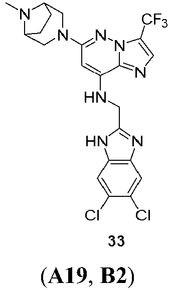 | *N*-((5,6-dichloro-1*H-*benzo[d]imidazol-2-yl)methyl)-6-(8-methyl-3,8-diazabicyclo[3.2.1]octan-3-yl)-3-(trifluoromethyl)imidazo[1,2-blpyridazin-8-amine | ¹H NMR (400 MHz, DMSO-d₆) δ 7.83 (s, 1H), 7.75 (s, 3H), 6.04 (s, 1H), 4.81 (d, 2H), 3.59 (d, 2H), 3.16 (s, 2H), 2.90 (d, 2H), 2.19 (s, 3H), 1.88-1.85 (m, 2H), 1.46 (d, 2H); MS: m/z = 525.1(M+1, ESI+). |
| 34 | 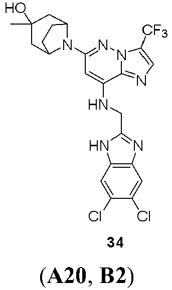 | 8-(8-(((5,6-dichloro-1*H-*benzo[d]imidazol-2-yl)methyl)amino)-3-(trifluoromethyl)imidazo[1,2-b]pyridazin-6-yl)-8-azabicyclo[3.2.1]octan-3-ol | ¹H NMR (400 MHz, DMSO-d₆) δ 12.55 (s, 1H), 7.84-7.81 (m, 2H), 7.75 (s, 2H), 5.82 (s, 1H), 4.80 (d, 2H), 4.20 (s, 2H), 4.15 (s, 1H), 2.18 (dd, 2H), 1.78-1.76 (m, 2H), 1.52 (d, 2H), 1.37 (d, 2H), 0.75 (s, 3H); MS: m/z = 540.2 (M+1, ESI+). |
| 35 | 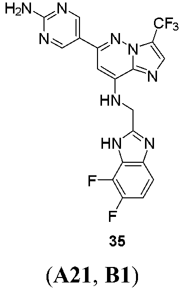 | 6-(2-aminopyrimidin-5-yl)-N-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 12.73 (s, 1H), 8.80 (s, 2H), 8.35 (t, 1H), 8.11 (s, 1H), 7.24-7.16 (m, 4H), 6.91 (s, 1H), 4.99 (d, 2H); MS: m/z = 462.1 (M+1, ESI+). |
| 36 | 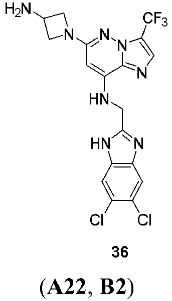 | 6-(3-aminoazetidin-1-yl)-*N*-((5,6-dichloro-1*H*-benzo[*d*]imidazol-2-yl)methyl)-3-(trifluoromethyl)imidazo[1,2-blpyridazin-8-amine | ¹H NMR (400 MHz, DMSO-d₆) δ 7.83 (t, 2H), 7.77 (s, 2H), 5.56 (s, 1H), 4.78 (d, 2H), 4.02 (t, 2H), 3.75-3.70 (m, 1H), 3.46 (dd, 2H); MS: m/z = 471.0 (M+1, ESI+). |
| 37 | 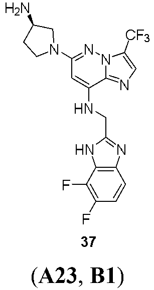 | *(R)*-6-(3-aminopyrrolidin-1-yl)-N-((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 7.80-7.76 (m, 2H), 7.27-7.14 (m, 2H), 5.73 (s, 1H), 4.81 (d, 2H), 3.52-3.50 (m, 1H), 3.45-3.38 (m, 2H), 3.31-3.25 (m, 1H), 2.97 (dd, 1H), 2.01-1.97 (m, 1H), 1.67-1.63 (m, 1H); MS: m/z = 453.2 (M+1, ESI+). |
| 38 | 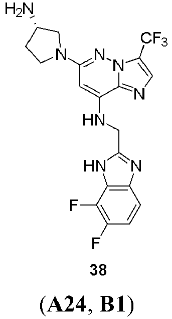 | *(S)*-6-(3-aminopyrrolidin-1-yl)-*N-*((6,7-difluoro-1H-benzo[d]imidazol-2-yl)methyl)-3-(trifluoromethyl)imidazo[1,2-blpyridazin-8-amine | ¹H NMR (400 MHz, DMSO-d₆) δ 7.80-7.76 (m, 2H), 7.27-7.14 (m, 2H), 5.73 (s, 1H), 4.81 (d, 2H), 3.60-3.48 (m, 1H), 3.45-3.4 (m, 2H), 3.38-3.25 (m, 1H), 2.97 (dd, 1H), 2.03-1.95 (m, 1H), 1.67-1.61 (m, 1H); MS: m/z = 453.1 (M+1, ESI+). |
| 39 | 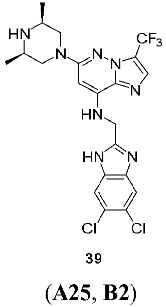 | *N*-((5,6-dichloro-1*H-*benzo[d]imidazol-2-yl)methyl)-6-((3S,5R)-3,5-dimethylpiperazin-1-yl)-3-(trifluoromethyl)imidazo[1,2-b]pyridazin-8-amine | ¹H NMR (400 MHz, DMSO-d₆) δ 7.84 (s, 1H), 7.78 (t, 1H), 7.75 (s, 2H), 6.10 (s, 1H), 4.83 (d, 2H), 3.84 (d, 2H), 2.70-2.65 (m, 2H), 2.20 (t, 2H), 0.95 (d, 6H); MS: m/z = 513.1 (M+1, ESI+). |
| 40 | 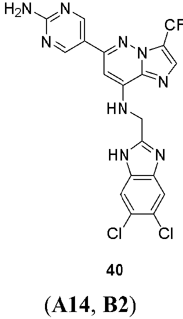 | 6-(2-aminopyrimidin-5-yl)-N-((5,6-dichloro-1*H-*benzo[d]imidazol-2-yl)methyl)-3-(trifluoromethyl)imidazo[1,2-blpyridazin-8-amine | ¹H NMR (400 MHz, DMSO-d₆) δ 12.64 (s, 1H), 8.79 (s, 2H), 8.32 (t, 1H), 8.10 (s, 1H), 7.84 (s, 1H), 7.69 (s, 1H), 7.15 (s, 2H), 6.89 (s, 1H), 4.98 (d, 2H); MS: m/z = 494.1 (M+1, ESI+). |
| 41 | 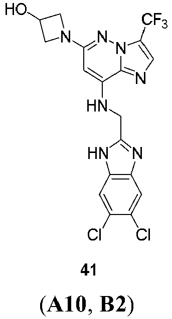 | 1-(8-(((5,6-dichloro-1*H-*benzo[d]imidazol-2-yl)methyl)amino)-3-(trifluoromethyl)imidazo[1,2-b]pyridazin-6-yl)azetidin-3-ol | ¹H NMR (400 MHz, DMSO-d₆) δ 12.61 (s, 1H), 7.88-7.77 (m, 4H), 5.62 (d, 1H), 5.59 (s, 1H), 4.79 (d, 2H), 4.52-4.48 (m, 1H), 4.07 (t, 2H), 3.60 (dd, 2H); MS: m/z = 472.0 (M+1, ESI+). |
| 42 | 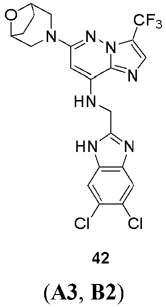 | 6-(8-oxa-3-azabicyclo[3.2.1]octan-3-yl)-*N-*((5,6-dichloro-1*H-*benzo[d]imidazol-2-yl)methyl)-3-(trifluoromethyl)imidazo[1,2-blpyridazin-8-amine | ¹H NMR (400 MHz, DMSO-d₆) δ 12.60 (s, 1H), 7.85-7.80 (m, 3H), 7.69 (s, 1H), 6.09 (s, 1H), 4.83 (d, 2H), 4.39 (s, 2H), 3.63 (d, 2H), 2.92 (dd, 2H), 1.80-1.67 (m, 4H); MS: m/z = 512.1 (M+1, ESI+). |
| 43 | 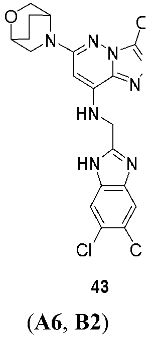 | 6-(2-oxa-5-azabicyclo[2.2.2]octan-5-yl)-*N*-((5,6-dichloro-1*H-*benzo[d]imidazol-2-yl)methyl)-3-(trifluoromethyl)imidazo[1, 2-b]pyridazin-8-amine | ¹H NMR (400 MHz, DMSO-d₆) δ 7.83-7.77 (m, 4H), 5.90 (s, 1H), 4.82 (d, 2H), 4.23 (s, 1H), 3.97 (s, 1H), 3.92 (s, 2H), 3.65 (d, 1H), 3.42 (d, 1H), 2.03-1.98 (m, 1H), 1.96-1.85 (m, 2H), 1.68-1.62 (m, 1H); MS: m/z = 512.2 (M+1, ESI+). |
| 44 | 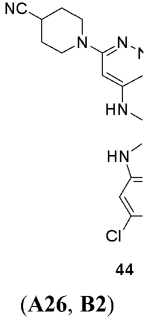 | 1-(8-(((5,6-dichloro-1*H-*benzo[d]imidazol-2-yl)methyl)amino)-3-(trifluoromethyl)imidazo[1, 2-b]pyridazin-6-yl)piperidine-4-carbonitrile | ¹H NMR (400 MHz, DMSO-d₆) δ 12.60 (s, 1H), 7.88-7.84 (m, 2H), 7.76 (s, 2H), 6.19 (s, 1H), 4.85 (d, 2H), 3.70-3.64 (m, 2H), 3.26-3.20 (m, 2H), 3.09-3.05 (m, 1H), 1.93-1.88 (m, 2H), 1.74-1.66 (m, 2H); MS: m/z = 509.2 (M+1, ESI+). |
| 45 | 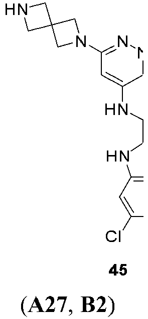 | *N*-((5,6-dichloro-1*H-*benzo[d]imidazol-2-yl)methyl)-6-(2,6-diazaspiro[3.3]heptan-2-yl)-3-(trifluoromethyl)imidazo[1, 2-b]pyridazin-8-amine | ¹H NMR (400 MHz, DMSO-d₆) δ 7.87-7.84 (m, 2H). 7.77 (s, 2H), 5.59 (s, 1H), 4.79 (s, 2H), 3.95 (s, 4H), 3.65 (s, 4H); MS: m/z = 497.1 (M+1, ESI+). |
| 46 | 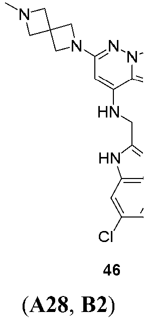 | *N*-((5,6-dichloro-1*H-*benzo[d]imidazol-2-yl)methyl)-6-(6-methyl-2,6-diazaspiro[3.3]heptan-2-yl)-3-(trifluoromethyl)imidazo[1, 2-b]pyridazin-8-amine | ¹H NMR (400 MHz, DMSO-d₆) δ 12.58 (s, 1H), 7.84 (s, 2H), 7.77 (s, 2H), 5.58 (s, 1H), 4.78 (d, 2H), 3.92 (s, 4H), 3.21 (s, 4H), 2.15 (s, 3H); MS: m/z = 511.2 (M+1, ESI+). |
| 47 | 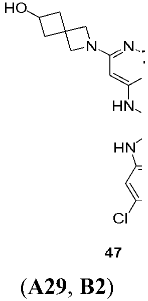 | 2-(8-(((5,6-dichloro-1*H-*benzo[d]imidazol-2-yl)methyl)amino)-3-(trifluoromethyl)imidazo[1, 2-b]pyridazin-6-yl)-2-azaspiro[3.3]heptan-6-ol | ¹H NMR (400 MHz, DMSO-d₆) δ 12.58 (s, 1H), 7.86-7.83 (m, 2H), 7.77 (s, 2H), 5.54 (s, 1H), 5.02 (d, 1H), 4.77 (d, 2H), 3.99-3.94 (m, 1H), 3.84 (s, 2H), 3.79 (s, 2H), 2.42-2.38 (m, 2H), 1.98-1.93 (m, 2H); MS: m/z = 512.0 (M+1, ESI+). |
| 48 | 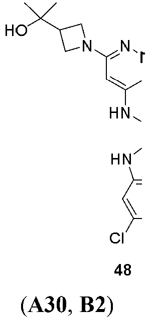 | 2-(1-(8-(((5,6-dichloro-1*H-*benzo[d]imidazol-2-yl)methyl)amino)-3-(trifluoromethyl)imidazo[1, 2-b]pyridazin-6-yl)azetidin-3-yl)propan-2-ol | ¹H NMR (400 MHz, DMSO-d₆) δ 12.61 (s, 1H), 7.82-7.77 (m, 4H), 5.56 (s, 1H), 4.78 (d, 2H), 4.44 (s, 1H), 3.77 (d, 4H), 2.68-2.62 (m, 1H), 1.01 (s, 6H); MS: m/z = 514.1 (M+1, ESI+). |
| 49 | 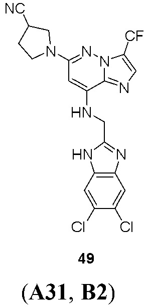 | 1-(8-(((5,6-dichloro-1*H-*benzo[d]imidazol-2-yl)methyl)amino)-3-(trifluoromethyl)imidazo[1, 2-b]pyridazin-6-yl)pyrrolidine-3-carbonitrile | ¹H NMR (400 MHz, DMSO-d₆) δ 7.88-7.84 (m, 2H), 7.76 (s, 2H), 5.83 (s, 1H), 4.83 (d, 2H), 3.74-3.61 (m, 1H), 3.57-3.42 (m, 4H), 2.34-2.30 (m, 1H), 2.24-2.18 (m, 1H); MS: m/z = 495.0 (M+1, ESI+). |
| 50 | 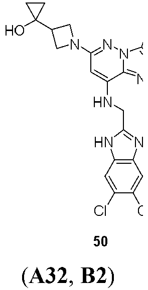 | 1-(1-(8-(((5,6-dichloro-1*H-*benzo[d]imidazol-2-yl)methyl)amino)-3-(trifluoromethyl)imidazo[1, 2-b]pyridazin-6-yl)azetidin-3-yl)cyclopropan-1-ol | ¹H NMR (400 MHz, DMSO-d₆) δ 12.61 (s, 1H), 7.83 (t, 2H), 7.77 (s, 2H), 5.57 (s, 1H), 5.36 (s, 1H), 4.78 (d, 2H), 3.86 (t, 2H), 3.73 (t, 2H), 2.66-2.59 (m, 1H), 0.57 (t, 2H), 0.40 (t, 2H); MS: m/z = 512.2 (M+1, ESI+). |
| 51 | 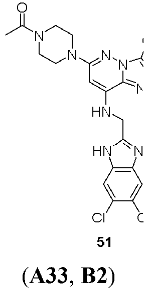 | 1-(4-(8-(((5,6-dichloro-1*H-*benzo[d]imidazol-2-yl)methyl)amino)-3-(trifluoromethyl)imidazo[1, 2-b]pyridazin-6-yl)piperazin-1-yl)ethan-1-one | ¹H NMR (400 MHz, DMSO-d₆) δ 12.64 (s, 1H), 7.88-7.86 (m, 3H), 7.69 (s, 1H), 6.21 (s, 1H), 4.85 (d, 2H), 3.52-3.50 (m, 4H), 3.43-3.36 (m, 4H), 2.02 (s, 3H); MS: m/z = 527.0 (M+1, ESI+). |
| 52 | 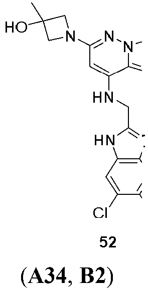 | 1-(8-(((5,6-dichloro-1*H-*benzo[d]imidazol-2-yl)methyl)amino)-3-(trifluoromethyl)imidazo[1, 2-b]pyridazin-6-yl)-3-methylazetidin-3-ol | ¹H NMR (400 MHz, DMSO-d₆) δ 7.88-7.84 (m, 2H), 7.77 (s, 2H), 5.61 (s, 1H), 4.80 (d, 2H), 3.74 (dd, 4H), 1.40 (s, 3H); MS: m/z = 486.0 (M+1, ESI+). |
| 53 | 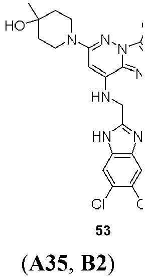 | 1-(8-(((5,6-dichloro-1*H-*benzo[d]imidazol-2-yl)methyl)amino)-3-(trifluoromethyl)imidaz o[1,2-*b*]pyridazin-6-yl)-4-methylpiperidin-4-ol | ¹H NMR (400 MHz, DMSO-d₆) δ 12.56 (s, 1H), 7.84 (s, 1H), 7.79-7.76 (m, 3H), 6.09 (s, 1H), 4.82 (d, 2H), 4.31 (s, 1H), 3.62 (d, 2H), 3.26-3.16 (m, 2H), 1.44-1.43 (m, 4H), 1.07 (s, 3H); MS: m/z = 514.1 (M+1, ESI+). |
| 54 | 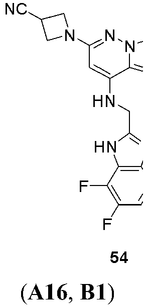 | 1-(8-(((6,7-difluoro-1*H-*benzo[d]imidazol-2-yl)methyl)amino)-3-(trifluoromethyl)imidazo[1, 2-b]pyridazin-6-yl)azetidine-3-carbonitrile | ¹H NMR (400 MHz, DMSO-d₆) δ 12.68 (s, 1H), 8.01 (s, 1H), 7.89 (s, 1H), 7.23-7.19 (m, 2H), 5.72 (s, 1H), 4.81 (d, 2H), 4.18 (t, 2H), 4.04 (dd, 2H), 3.85-3.79 (m, 1H); MS: m/z = 449.1 (M+1, ESI+). |
| 55 | 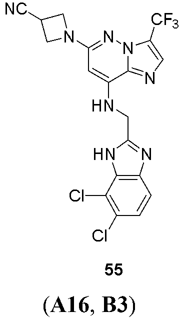 | 1-(8-(((6,7-dichloro-1*H-*benzo[d]imidazol-2-yl)methyl)amino)-3-(trifluoromethyl)imidazo[1,2-b]pyridazin-6-yl)azetidine-3-carbonitrile | ¹H NMR (400 MHz, DMSO-d₆) δ 12.82 (s, 1H), 8.00 (s, 1H), 7.89 (s, 1H), 7.46 (d, 1H), 7.36 (d, 1H), 5.76 (s, 1H), 4.83 (d, 2H), 4.19 (t, 2H), 4.04 (dd, 2H), 3.84-3.82 (m, 1H); MS: m/z = 481.0 (M+1, ESI+). |
| 56 | 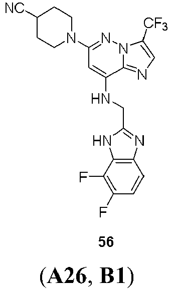 | 1-(8-(((6,7-difluoro-1*H-*benzo[d]imidazol-2-yl)methyl)amino)-3-(trifluoromethyl)imidazo[1,2-b]pyridazin-6-yl)piperidine-4-carbonitrile | ¹H NMR (400 MHz, DMSO-d₆) δ 12.77 (s, 1H), 7.88 (s, 2H), 7.25-7.16 (m, 2H), 6.22 (s, 1H), 4.87 (d, 2H), 3.71-3.68 (m, 2H), 3.28-3.22 (m, 2H), 3.11-3.05 (m, 1H), 1.93-1.90 (m, 2H), 1.75-1.66 (m, 2H); MS: m/z = 477.1 (M+1, ESI+). |

### Example 57: Preparation of N-((5,6-dichloro-1H-benzo[d]imidazol-2-yl)methyl)-6-(piperazin-1-yl)-3-(trifluoromethyl)imidazo[1,2-b]pyridazin-8-amine

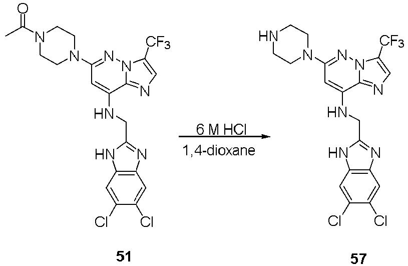

**The** compound of Example 51 (650 mg, 1.23 mmol) was dissolved in 1,4-dioxane (10 mL), then 6 M hydrochloric acid solution (2 mL) was added and the mixture was stirred at 100 °C for 48 hours. After concentrating the reaction mixture, the concentrate was purified by Prep-HPLC to obtain the compound of Example 57 (120 mg, 20.06 % yield) as a grayish-white solid. ¹H NMR (400 MHz, DMSO-d₆) δ 7.86 (s, 1H), 7.82-7.77 (m, 3H), 6.13 (s, 1H), 4.83 (d, 2H), 3.28 (s, 4H), 2.73 (s, 4H); MS: m/z = 485.0 (M+1, ESI+).

### Example 58: Preparation of 6-(3-(cyclopropylamino)azetidin-1-yl)-N-((5,6-dichloro-1H-benzo[d]imidazol-2-yl)methyl)-3-(trifluoromethyl)imidazo[1,2-b]pyridazin-8-amine

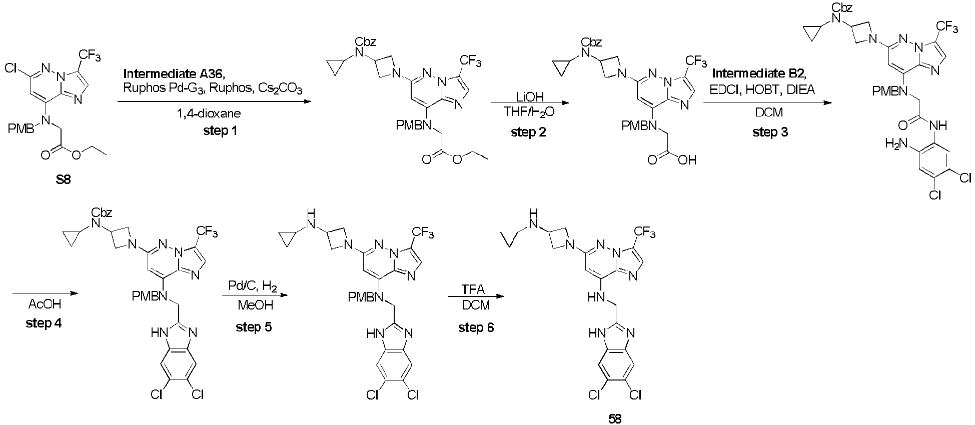

### Steps 1 to 4: Preparation of benzyl cyclopropyl(1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidin-3-yl)carbamate

benzyl cyclopropyl(1-(8-(((5,6-dichloro-1H-benzo[d]imidazol-2-yl)methyl)(4-methoxybenzyl)amino)-3-(trifluoromethyl)imidazo[1,2-*b*]pyridazin-6-yl)azetidin-3-yl)carbamate (450 mg, 32.90 % yield) was obtained in the same method as Steps 1 to 4 of Example 30. However, **Intermediate** S8 (3 g, 6.77 mmol) and **Intermediate A36** (4.17 g, 16.94 mmol) were used and stirred for 16 hours in Step 1 of Example 30, and the reaction time was changed to 16 hours in Step 3 of Example 30. MS: m/z = 765.3 (M+1, ESI+).

### Step 5: Preparation of 6-(3-(cyclopropylamino)azetidin-1-yl)-N-((5,6-dichloro-1H-benzo[d]imidazol-2-yl)methyl)-N-(4-methoxybenzyl)-3-(trifluoromethyl)imidazo[1,2-b]pyridazin-8-amine

The product obtained in Step 4 (450 mg, 588 umol) was dissolved in methanol (10 mL), then Pd/C (45 mg) was added thereto, and the mixture was stirred at 25 °C for 3 hours under H₂ atmosphere. The reaction mixture was filtered and concentrated, and then the concentrate was purified by silica gel chromatography to obtain 6-(3-(cyclopropylamino)azetidin-1-yl)-N-((5,6-dichloro-1*H*-benzo[*d*]imidazol-2-yl)methyl)-*N*-(4-methoxybenzyl)-3-(trifluoromethyl)imidazo[1,2-b]pyridazin-8-amine (250 mg, 67.36 % yield). MS: m/z = 631.2 (M+1, ESI+).

### Step 6: Preparation of 6-(3-(cyclopropylamino)azetidin-1-yl)-N-((5,6-dichloro-1H-benzo[d]imidazol-2-yl)methyl)-3-(trifluoromethyl)imidazo[1,2-b]pyridazin-8-amine

**The compound of Example 58** (65 mg, 32.11 % yield) was obtained as a white solid in the same method as Step 5 of Example 30, except that the reaction time was changed to 1 hour. ¹H NMR (400 MHz, DMSO-d₆) δ 12.63 (s, 1H), 7.86-7.83 (m, 2H), 7.77 (s, 2H), 5.57 (s, 1H), 4.78 (d, 2H), 4.01 (t, 2H), 3.69-3.66 (m, 1H), 3.57 (t, 2H), 2,91 (br s, 1H), 2.03-1.99 (m, 1H), 0.36-0.31 (m, 2H), 0.20-0.17 (m, 2H); MS: m/z = 511.1 (M+1, ESI+).

### Example 59: Preparation of 1-(8-(((5,6-dichloro-1H-benzo[d]imidazol-2-yl)methyl)amino)-3-(trifluoromethyl)imidazo[1,2-b]pyridazin-6-yl)-3-hydroxyazetidine-3-carbonitrile

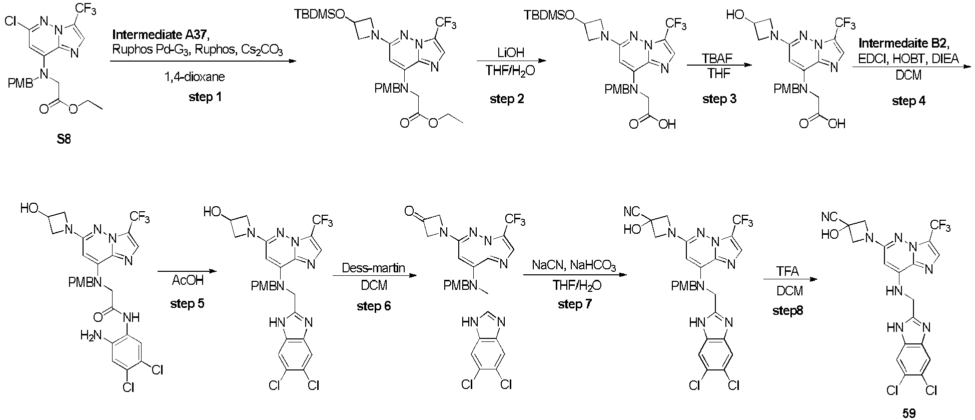

### Steps 1 and 2: Preparation of N-(6-(3-((tert-butyldimethylsilyl)oxy)azetidin-1-yl)-3-(trifluoromethyl)imidazo[1,2-b]pyridazin-8-yl)-N-(4-methoxybenzyl)glycine

N-(6-(3-((tert-butyldimethylsilyl)oxy)azetidin-1-yl)-3-(trifluoromethyl)imidazo[1,2-b]pyridazin-8-yl)-N-(4-methoxybenzyl)glycine (2.5 g, crude) was obtained in the same method as Steps 1 and 2 of Example 30. However, in Step 1, **Intermediate** S8 (5 g, 11.29 mmol) and **Intermediate** A37 (5.29 g, 28.23 mmol) were used and the mixture was stirred for 16 hours, and in Step 2, the reaction time was changed to 16 hours. MS: m/z = 566.2 (M+1, ESI+).

### Step 3: Preparation of N-(6-hydroxyazetidin-1-yl)-3-(trifluoromethyl)imidazo[1,2-b]pyridazin-8-yl)-N-(4-methoxybenzyl)glycine

The product obtained in Step 2 (1.5 g, 2.65 mmol) was dissolved in THF (20 mL), then 1 M TBAF solution (10.6 mmol, 10.6 mL) was added thereto and the mixture was stirred at 25 °C for 16 hours. Water (100 mL) was added to the reaction mixture, and the product was extracted three times with EtOAc (30 mL). The organic layer was washed three times with brine (100 mL), dried over Na₂SO₄, filtered, and concentrated, and then the concentrate was purified by silica gel chromatography to obtain N-(6-hydroxyazetidin-1-yl)-3-(trifluoromethyl)imidazo[1,2-b]pyridazin-8-yl)-N-(4-methoxybenzyl)glycine (700 mg, 58.48 % yield). MS: m/z = 452.1 (M+1, ESI+).

### Steps 4 and 5: Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidin-3-ol

1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidin-3-ol (780 mg, 90.31 % yield) was obtained in the same method as Steps 3 and 4 of Example 30. However, the reaction time was changed to 16 hours in Step 3 of Example 30, and the reaction temperature was changed to 70 °C and stirred for 3 hours in Step 4 of Example 30. MS: m/z = 592.0 (M+1, ESI+).

### Step 6: Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidin-3-one

The product obtained in Step 5 (730 mg, 1.23 mmol) was dissolved in DCM (20 mL), then Dess-Martin periodinane (1.57 g, 3.70 mmol) was added thereto and the mixture was stirred at 25 °C for 16 hours. Water (100 mL) was added to the reaction mixture, and the product was extracted three times with DCM (20 mL). The organic layer was washed three times with brine (100 mL), dried over Na₂SO₄, filtered, and concentrated, and then the concentrate was purified by silica gel chromatography to obtain 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidine (550 mg, 75.60 % yield). MS: m/z = 590.0 (M+1, ESI+).

### Step 7. Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)-3-hydroxyazetidine-3-carbonitrile

The product obtained in Step 6 (400 mg, 678 umol) and sodium cyanide (166 mg, 3.39 mmol) were dissolved in THF/water (10 mL/10 mL), then sodium bicarbonate (285 mg, 3.39 mmol) was added thereto and the mixture was stirred at 25 °C for 16 hours. Water (100 mL) was added to the reaction mixture, and the product was extracted three times with EtOAc (30 mL). The organic layer was washed three times with brine (100 mL), dried over Na₂SO₄, filtered, and concentrated, and then the concentrate was purified by silica gel chromatography to obtain 1-(8-(((5,6-dichloro-1H-benzo[d]imidazol-2-yl)methyl)(4-methoxybenzyl)amino)-3-(trifluoromethyl)imidazo[1,2-*b*]pyridazin-6-yl)-3-hydroxyazetidine-3-carbonitrile (250 mg, 59.76% yield). MS: m/z = 617.1 (M+1, ESI+).

### Step 8: Preparation of 1-(8-(((5,6-dichloro-1H-benzo[d]imidazol-2-yl)methyl)amino)-3-(trifluoromethyl)imidazo[1,2-b]pyridazin-6-yl)-3-hydroxyazetidine-3-carbonitrile

**The compound of Example 59** (30 mg, 14.90 % yield) was obtained as a white solid in the same method as Step 5 of Example 30, except that the reaction time was changed to 1 hour. ¹H NMR (400 MHz, DMSO-d₆) δ 12.62 (s, 1H), 8.00 (t, 1H), 7.90 (s, 1H), 7.77 (s, 2H), 7.53 (s, 1H), 5.78 (s, 1H), 4.82 (d, 2H), 4.38 (d, 2H), 3.96 (d, 2H); MS: m/z = 497.1 (M+1, ESI+).

### Example 60: Preparation of (1-(8-(((5,6-dichloro-1H-benzo[d]imidazol-2-yl)methyl)amino)-3-(trifluoromethyl)imidazo[1,2-b]pyridazin-6-yl)azetidin-3-yl)methanol

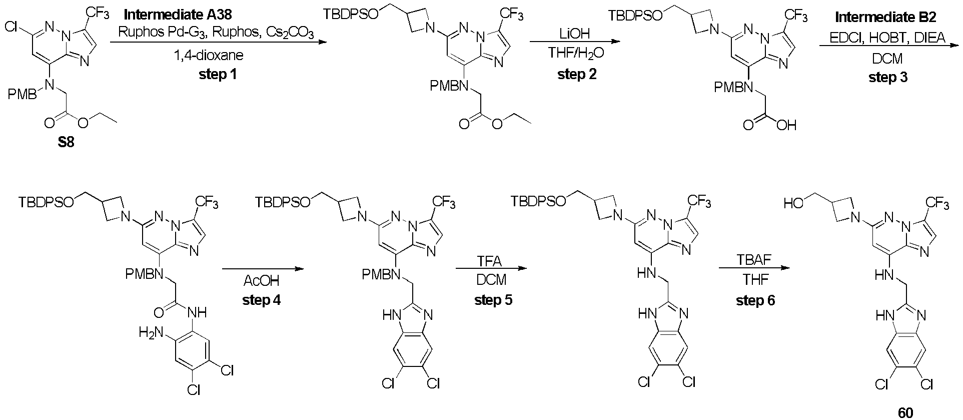

### Steps 1 to 5: Preparation of 6-(3-(((tert-butyldiphenylsilyl)oxy)methyl)azetidin-1-yl)-N-((5,6-dichloro-1H-benzo[d]imidazol-2-yl)methyl)-3-(trifluoromethyl)imidazo[1,2-b]pyridazin-8-amine

6-(3-(((tert-butyldiphenylsilyl)oxy)methyl)azetidin-1-yl)-N-((5,6-dichloro-1H-benzo[d]imidazol-2-yl)methyl)-3-(trifluoromethyl)imidazo[1,2-b]pyridazin-8-amine (350 mg, 86.83 % yield) was obtained in the same method as Steps 1 to 5 of Example 30, except that Intermediate S8 (2 g, 4.52 mmol) and Intermediate A38 (7.35 g, 22.58 mmol) were used in Step 1 and the mixture was stirred for 16 hours. MS: m/z = 724.2 (M+1, ESI+).

### Step 6: Preparation of (1-(8-(((5,6-dichloro-1H-benzo[d]imidazol-2-yl)methyl)amino)-3-(trifluoromethyl)imidazo[1,2-b]pyridazin-6-yl)azetidin-3-yl)methanol

The product obtained in Step 5 (350 mg, 484 umol) was dissolved in THF (10 mL), then 1 M TBAF solution (2.42 mL, 2.42 mmol) was added thereto and the mixture was stirred at 25 °C for 1 hour. Water (100 mL) was added to the reaction mixture, and the product was extracted three times with EtOAc (60 mL). The organic layer was washed three times with brine (100 mL), dried over Na₂SO₄, filtered, and concentrated, and then the concentrate was purified by Prep-HPLC to obtain **the compound of Example 60** (100 mg, 42.61 % yield) as a white solid. ¹H NMR (400 MHz, DMSO-d₆) δ 12.60 (s, 1H), 7.84-7.77 (m, 4H), 5.56 (s, 1H), 4.79-4.73 (m, 3H), 3.87 (t, 2H), 3.62 (dd, 2H), 3.52 (t, 2H), 2.77-2.70 (m, 1H); MS: m/z = 486.0 (M+1, ESI+).

### Example 61: Preparation of 1-(8-(((5,6-dichloro-1H-benzo[d]imidazol-2-yl)methyl)amino)-3-(trifluoromethyl)imidazo[1,2-b]pyridazin-6-yl)-3-hydroxypyrrolidine-3-carbonitrile

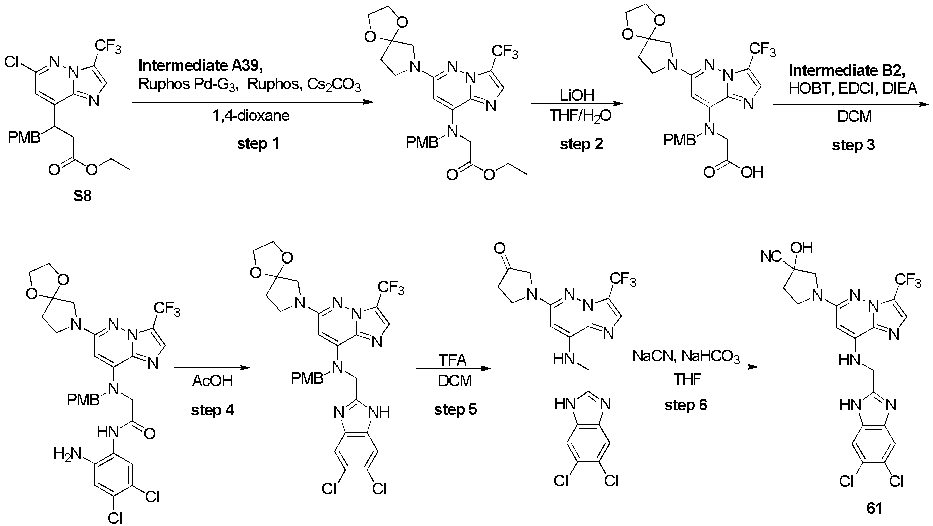

### Step 1: Preparation of ethyl N-(6-(1,4-dioxa-7-azaspiro[4.4]nonan-7-yl)-3-(trifluoromethyl)imidazo[1,2-b]pyridazin-8-yl)-N-(4-methoxybenzyl)glycinate

ethyl *N*-(6-(1,4-dioxa-7-azaspiro[4.4]nonan-7-yl)-3-(trifluoromethyl)imidazo[1,2-b]pyridazin-8-yl)-N-(4-methoxybenzyl)glycinate (780 mg, 17.92 % yield) was obtained in the same method as Step 1 of Example 30, except that **Intermediate S8** (3.6 g, 8.13 mmol) and **Intermediate A39** (3.15 g, 24.29 mmol) were used and the mixture was stirred for 16 hours. MS: m/z = 535.2 (M+1, ESI+).

### Step 2: Preparation of N-(6-(1,4-dioxa-7-azaspiro[4.4]nonan-7-yl)-3-(trifluoromethyl)imidazo[1,2-b]pyridazin-8-yl)-N-(4-methoxybenzyl)glycine

The product obtained in Step 1 (780 mg, 1.46 mmol) was dissolved in THF/water (10 mL/5 mL), then lithium hydroxide (185 mg, 4.38 mmol) was added thereto and the mixture was stirred at 50 °C for 2 hours. 0.5 N hydrochloric acid solution (80 mL) was added to the reaction mixture, and the product was extracted three times with DCM (20 mL). The organic layer was washed three times with brine (80 mL), dried over Na₂SO₄, filtered, and concentrated, and then the concentrate was purified by silica gel chromatography to obtain N-(6-(1,4-dioxa-7-azaspiro[4.4]nonan-7-yl)-3-(trifluoromethyl)imidazo[1,2-b]pyridazin-8-yl)-N-(4-methoxybenzyl)glycine (410 mg, 55.25 % yield). MS: m/z = 507.2 (M+1, ESI+).

### Steps 3 to 5: Preparation of 1-(8-(((5,6-dichloro-1H-benzo[d]imidazol-2-yl)methyl)amino)-3-(trifluoromethyl)imidazo[1,2-b]pyridazin-6-yl)pyrrolidin-3-one

1-(8-(((5,6-dichloro-1H-benzo[d]imidazol-2-yl)methyl)amino)-3-(trifluoromethyl)imidazo[1,2-*b*]pyridazin-6-yl)pyrrolidin-3-one (200 mg, 62.30 % yield) was obtained in the same method as Steps 3 to 5 of Example 30, except that the reaction times were changed to 16 hours and 1 hour in Step 3 and Step 5 of Example 30, respectively. MS: m/z = 484.0 (M+1, ESI+).

### Step 6: Preparation of 1-(8-(((5,6-dichloro-1H-benzo[d]imidazol-2-yl)methyl)amino)-3-(trifluoromethyl)imidazo[1,2-b]pyridazin-6-yl)-3-hydroxypyrrolidine-3-carbonitrile

**The compound of Example 61** (95 mg, 42.03 % yield) was obtained as a white solid in the same method as Step 7 of Example 59, using the product obtained in Step 5 (200 mg, 413 umol). ¹H NMR (400 MHz, DMSO-d₆) δ 7.89 (t, 1H), 7.85 (s, 1H), 7.79 (s, 2H), 6.96 (s, 1H), 5.83 (s, 1H), 4.87 (d, 2H), 3.74 (d, 1H), 3.64 (d, 1H), 3.54-3.49 (m, 1H), 3.44-3.38 (m, 1H), 2.46-2.39 (m, 1H), 2.33-2.28 (m, 1H); MS: m/z = 511.1 (M+1, ESI+).

### Example 62: Preparation of (R)-1-(8-(((5,6-dichloro-1H-benzo[d]imidazol-2-yl)methyl)amino)-3-(trifluoromethyl)imidazo[1,2-b]pyridazin-6-yl)pyrrolidin-3-ol

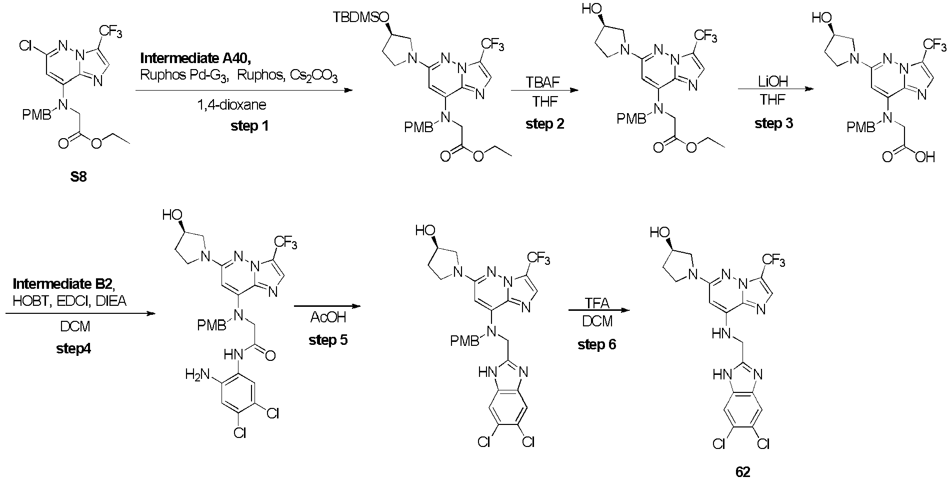

### Step 1: Preparation of ethyl (R)-N-(6-(3-((tert-butyldimethylsilyl)oxy)pyrrolidin-1-yl)-3-(trifluoromethyl)imidazo[1,2-b]pyridazin-8-yl)-N-(4-methoxybenzyl)glycinate

Ethyl (R)-N-(6-(3-((tert-butyldimethylsilyl)oxy)pyrrolidin-1-yl)-3-(trifluoromethyl)imidazo[1,2-b]pyridazin-8-yl)-N-(4-methoxybenzyl)glycinate (560 mg, 20.40 % yield) was obtained in the same method as in Step 1 of Example 30, except that **Intermediate S8** (2 g, 4.52 mmol) and **Intermediate A40** (2.73 g, 13.55 mmol) were used and the mixture was stirred for 16 hours. MS: m/z = 608.3 (M+1, ESI+).

### Step 2: Preparation of ethyl (R)-N-(6-(3-hydroxypyrrolidin-1-yl)-3-(trifluoromethyl)imidazo[1,2-b]pyridazin-8-yl)-N-(4-methoxybenzyl)glycinate

The product obtained in Step 1 (560 mg, 921 umol) was dissolved in THF (5 mL), and then 1 M TBAF solution (1.84 mL, 1.84 mmol) was added thereto, followed by stirring at 25 °C for 3 hours. Saturated aqueous ammonium chloride solution (50 mL) was added to the reaction mixture, and the product was extracted three times with EtOAc (10 mL). The organic layer was washed three times with brine (50 mL), dried over Na₂SO₄, filtered, and concentrated. The concentrate was purified by silica gel chromatography to obtain ethyl (R)-N-(6-(3-hydroxypyrrolidin-1-yl)-3-(trifluoromethyl)imidazo[1,2-b]pyridazin-8-yl)-N-(4-methoxybenzyl)glycinate (400 mg, 87.97 % yield). MS: m/z = 494.0 (M+1, ESI+).

### Steps 3 to 6: Preparation of (R)-1-(8-(((5,6-dichloro-1H-benzo[d]imidazol-2-yl)methyl)amino)-3-(trifluoromethyl)imidazo[1,2-b]pyridazin-6-yl)pyrrolidin-3-ol

**The compound of Example 62** (56 mg, 25.93 % yield) was obtained as a white solid in the same method as in Steps 2 to 5 of Example 30, except that the reaction times of Step 2, Step 3, and Step 5 of Example 30 were changed to 16 hours, 16 hours, and 3 hours, respectively, and the reaction temperature in Step 4 was changed to 65 °C with stirring for 5 hours. ¹H NMR (400 MHz, DMSO-d₆) δ 12.63 (s, 1H), 7.86 (s, 1H), 7.80-7.76 (m, 2H), 7.68 (s, 1H), 5.72 (s, 1H), 4.93 (d, 1H), 4.81 (d, 2H), 4.33 (s, 1H), 3.39-3.35 (m, 2H), 3.19 (d, 1H), 1.98-1.92 (m, 1H), 1.85-1.81 (m, 1H); MS: m/z = 486.2 (M+1, ESI+).

### Example 63: Preparation of (S)-1-(8-(((5,6-dichloro-1H-benzo[d]imidazol-2-yl)methyl)amino)-3-(trifluoromethyl)imidazo[1,2-b]pyridazin-6-yl)pyrrolidin-3-ol

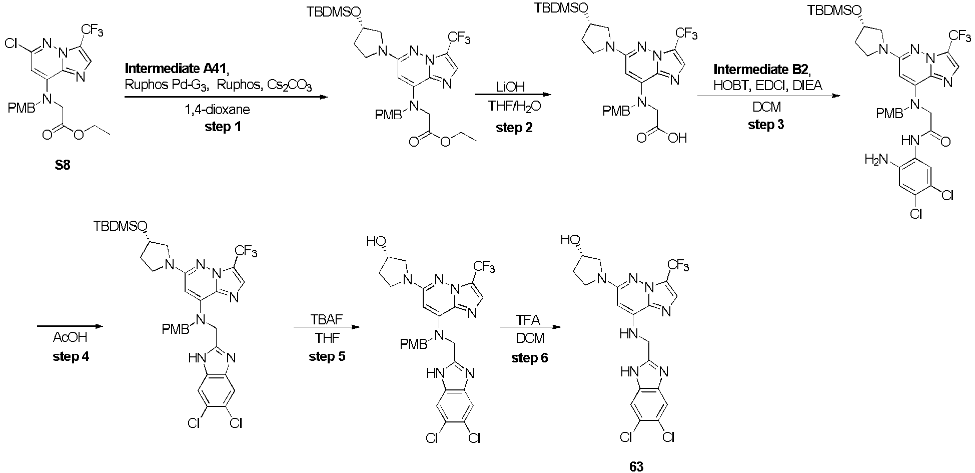

### Steps 1 to 4: Preparation of (S)-6-(3-((tert-butyldimethylsilyl)oxy)pyrrolidin-1-yl)-N-((5,6-dichloro-1H-benzo[d]imidazol-2-yl)methyl)-N-(4-methoxybenzyl)-3-(trifluoromethyl)imidazo[1,2-b]pyridazin-8-amine

*(S)*-6-(3-((*tert*-butyldimethylsilyl)oxy)pyrrolidin-1-yl)-*N*-((5,6-dichloro-1*H-*benzo[d]imidazol-2-yl)methyl)-N-(4-methoxybenzyl)-3-(trifluoromethyl)imidazo[1,2-b]pyridazin-8-amine (850 mg, 87.12 % yield) was obtained in the same method as in Steps 1 to 4 of Example 30. However, in Step 1, **Intermediate S8** (3.1 g, 7.0 mmol) and **Intermediate A41** (2.82 g, 14.0 mmol) were used and stirred for 16 hours, and the reaction times in Step 2 and Step 3 were changed to 16 hours. MS: m/z = 720.2 (M+1, ESI+).

### Step 5: Preparation of (S)-1-(8-(((5,6-dichloro-1H-benzo[d]imidazol-2-yl)methyl)(4-methoxybenzyl)amino)-3-(trifluoromethyl)imidazo[1,2-b]pyridazin-6-yl)pyrrolidin-3-ol

(S)-1-(8-(((5,6-dichloro-1H-benzo[d]imidazol-2-yl)methyl)(4-methoxybenzyl)amino)-3-(trifluoromethyl)imidazo[1,2-b]pyridazin-6-yl)pyrrolidin-3-ol (600 mg, 83.89 % yield) was obtained in the same method as in Step 2 of Example 62, except that the reaction time was changed to 16 hours. MS: m/z = 606.1 (M+1, ESI+).

### Step 6: Preparation of (S)-1-(8-(((5,6-dichloro-1H-benzo[d]imidazol-2-yl)methyl)amino)-3-(trifluoromethyl)imidazo[1,2-b]pyridazin-6-yl)pyrrolidin-3-ol

**The compound of Example 63** (290 mg, 59.86 % yield) was obtained as a white solid in the same method as in Step 5 of Example 30, except that the reaction time was changed to 16 hours. ¹H NMR (400 MHz, DMSO-d₆) δ 12.63 (s, 1H), 7.80-7.76 (m, 4H), 5.72 (s, 1H), 4.93 (d, 1H), 4.81 (d, 2H), 4.33 (s, 1H), 3.39-3.33 (m, 2H), 3.20 (d, 1H), 1.98-1.94 (m, 1H), 1.85-1.82 (m, 1H); MS: m/z = 486.2 (M+1, ESI+).

### Example 64: Preparation of 1-(8-(((5,6-dichloro-1H-benzo[d]imidazol-2-yl)methyl)amino)-3-(trifluoromethyl)imidazo[1,2-b]pyridazin-6-yl)azetidine-3-carboxylic acid

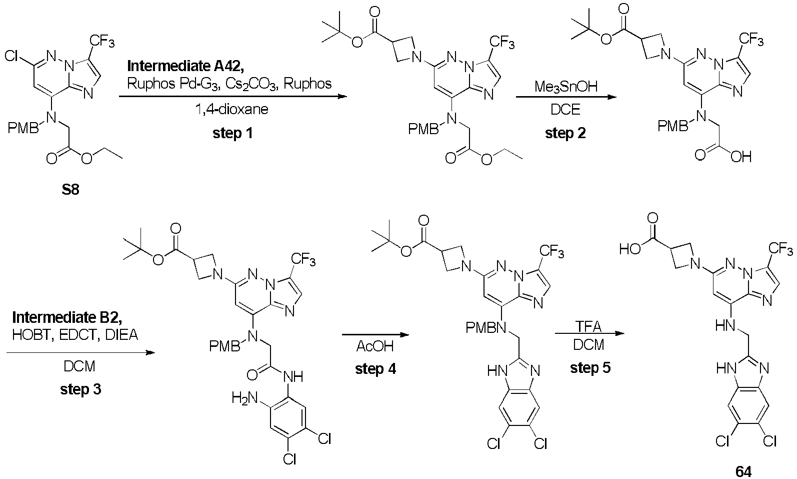

### Step 1: Preparation of tert-butyl 1-(8-((2-ethoxy-2-oxoethyl)(4-methoxybenzyl)amino)-3-(trifluoromethyl)imidazo[1,2-b]pyridazin-6-yl)azetidine-3-carboxylate

*tert-Butyl* 1-(8-((2-ethoxy-2-oxoethyl)(4-methoxybenzyl)amino)-3-(trifluoromethyl)imidazo[1,2-b]pyridazin-6-yl)azetidine-3-carboxylate (1 g, 39.29 % yield) was obtained in the same method as in Step 1 of Example 30, except for stirring for 16 hours using **Intermediate S8** (2 g, 4.52 mmol) and **Intermediate A42** (3.55 g, 22.58 mmol). MS: m/z = 564.2 (M+1, ESI+).

### Step 2: Preparation of N-(6-(3-(tert-butoxycarbonyl)azetidin-1-yl)-3-(trifluoromethyl)imidazo[1,2-b]pyridazin-8-yl)-N-(4-methoxybenzyl)glycine

The product obtained in Step 1 (1 g, 1.77 mmol) was dissolved in 1,2-dichloroethane (20 mL), and then trimethyltin hydroxide (1.60 g, 8.87 mmol) was added thereto, followed by stirring at 70 °C for 16 hours. 0.5 N hydrochloric acid solution (100 mL) was added to the reaction mixture, and the mixture was extracted three times with DCM (30 mL). The organic layer was washed three times with brine (100 mL), dried over Na₂SO₄, filtered, and concentrated. The concentrate was purified by silica gel chromatography to obtain *N*-(6-(3-(*tert*-butoxycarbonyl)azetidin-1-yl)-3-(trifluoromethyl)imidazo[1,2-*b*]pyridazin-8-yl)-*N*-(4-methoxybenzyl)glycine (750 mg, 78.93 % yield). MS: m/z = 536.1 (M+1, ESI+).

### Steps 3 to 5: Preparation of 1-(8-(((5,6-dichloro-1H-benzo[d]imidazol-2-yl)methyl)amino)-3-(trifluoromethyl)imidazo[1,2-b]pyridazin-6-yl)azetidine-3-carboxylic acid

**The compound of Example 64** (70 mg, 31.56 % yield) was obtained as a white solid in the same method as in Steps 3 to 5 of Example 30, except for changing the reaction time in Step 5 of Example 30 to 1 hour. ¹H NMR (400 MHz, DMSO-d₆) δ 7.90 (t, 1H), 7.85 (s, 1H), 7.77 (s, 2H), 5.65 (s, 1H), 4.80 (d, 2H), 4.05 (t, 2H), 3.93 (t, 2H), 3.48-3.41 (m, 1H); MS: m/z = 500.0 (M+1, ESI+).

### Example 65: Preparation of 1-(8-(((5,6-dichloro-1H-benzo[d]imidazol-2-yl)methyl)amino)-3-(trifluoromethyl)imidazo[1,2-b]pyridazin-6-yl)azetidine-3-carboxamide

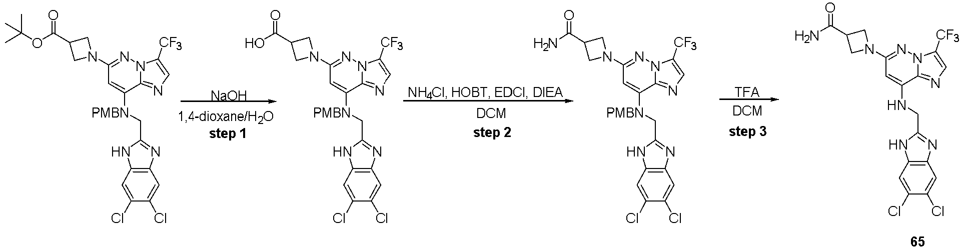

### Step 1: Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidine-3-carboxylic acid

The product obtained in Step 4 of Example 64 (310 mg, 458 umol) was dissolved in 1,4-dioxane (8 mL) and water (4 mL), and then sodium hydroxide (92 mg, 2.29 mmol) was added thereto, followed by stirring at 100 °C for 16 hours. 1 N hydrochloric acid solution (80 mL) was added to the reaction mixture, and the product was extracted three times with DCM (30 mL). The organic layer was washed three times with brine (100 mL), dried over Na₂SO₄, filtered, and concentrated. The concentrate was purified by silica gel chromatography to obtain 1-(8-(((5,6-dichloro-1*H*-benzo[*d*]imidazol-2-yl)methyl)(4-methoxybenzyl)amino)-3-(trifluoromethyl)imidazo[1,2-*b*]pyridazin-6-yl)azetidine-3-carboxylic acid (250 mg, 87.94 % yield). MS: m/z = 620.1 (M+1, ESI+).

### Step 2: Preparation of 1-(8-(((5,6-dichloro-1H-benzo[d]imidazol-2-yl)methyl)(4-methoxybenzyl)amino)-3-(trifluoromethyl)imidazo[1,2-b]pyridazin-6-yl)azetidine-3-carboxamide

1-(8-(((5,6-dichloro-1*H*-benzo[d]imidazol-2-yl)methyl)(4-methoxybenzyl)amino)-3-(trifluoromethyl)imidazo[1,2-*b*]pyridazin-6-yl)azetidine-3-carboxamide (150 mg, 60.10 % yield) was obtained in the same method as in Step 3 of Example 30, except that ammonium chloride (108 mg, 2.01 mmol) was used instead of **Intermediate B2** (1.38 g, 7.8 mmol). MS: m/z = 619.2 (M+1, ESI+).

### Step 3: Preparation of 1-(8-(((5,6-dichloro-1H-benzo[d]imidazol-2-yl)methyl)amino)-3-(trifluoromethyl)imidazo[1,2-b]pyridazin-6-yl)azetidine-3-carboxamide

**The compound of Example 65** (50 mg, 41.35 % yield) was obtained as a white solid in the same method as in Step 5 of Example 30, except that the reaction time was changed to 1 hour. ¹H NMR (400 MHz, DMSO-d₆) δ 12.61 (s, 1H), 7.90-7.76 (m, 4H), 7.45 (s, 1H), 7.01 (s, 1H), 5.62 (s, 1H), 4.80 (d, 2H), 3.98 (t, 2H), 3.89 (t, 2H), 3.42-3.36 (m, 1H); MS: m/z = 499.0 (M+1, ESI+).

### Example 66: Preparation of 4-(8-(((5,6-dichloro-1H-benzo[d]imidazol-2-yl)methyl)amino)-3-(trifluoromethyl)imidazo[1,2-b]pyridazin-6-yl)piperazine-1-carboxamide

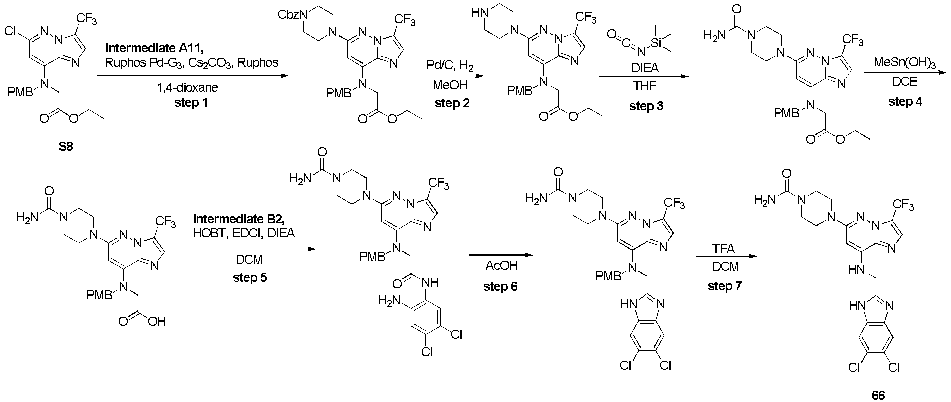

### Step 1: Preparation of benzyl 4-(8-((2-ethoxy-2-oxoethyl)(4-methoxybenzyl)amino)-3-(trifluoromethyl)imidazo[1,2-b]pyridazin-6-yl)piperazine-1-carboxylate

Benzyl 4-(8-((2-ethoxy-2-oxoethyl)(4-methoxybenzyl)amino)-3-(trifluoromethyl)imidazo[1,2-*b*]pyridazin-6-yl)piperazine-1-carboxylate (700 mg, 24.73 % yield) was obtained in the same method as in Step 1 of Example 30, except for stirring for 16 hours using **Intermediate S8** (2 g, 4.52 mmol) and **Intermediate A11** (4.97 g, 22.58 mmol). MS: m/z = 627.3 (M+1, ESI+).

### Step 2: Preparation of ethyl N-(4-methoxybenzyl)-N-(6-(piperazin-1-yl)-3-(trifluoromethyl)imidazo[1,2-b]pyridazin-8-yl)glycinate

The product obtained in Step 1 (700 mg, 1.12 mmol) was dissolved in methanol (15 mL), and then Pd/C (100 mg) was added thereto, followed by stirring under hydrogen gas charging at 25 °C for 48 hours. The reaction mixture was filtered and concentrated, and then the concentrate was purified by silica gel chromatography to obtain ethyl *N*-(4-methoxybenzyl)-*N*-(6-(piperazin-1-yl)-3-(trifluoromethyl)imidazo[1,2-*b*]pyridazin-8-yl)glycinate (500 mg, 90.88 % yield). MS: m/z = 493.2 (M+1, ESI+).

### Step 3: Preparation of ethyl N-(6-(4-carbamoylpiperazin-1-yl)-3-(trifluoromethyl)imidazo[1,2-b]pyridazin-8-yl)-N-(4-methoxybenzyl)glycinate

The product obtained in Step 2 (500 mg, 1.02 mmol) and isocyanatotrimethylsilane (351 mg, 3.05 mmol) were dissolved in THF (10 mL), and then DIEA (0.53 mL, 3.05 mmol) was added thereto, followed by stirring at 25 °C for 4 hours. Water (80 mL) was added to the reaction mixture, and the product was extracted three times with DCM (30 mL). The organic layer was washed three times with brine (100 mL), dried over Na₂SO₄, filtered, and concentrated. The concentrate was purified by silica gel chromatography to obtain ethyl *N*-(6-(4-carbamoylpiperazin-1-yl)-3-(trifluoromethyl)imidazo[1,2-*b*]pyridazin-8-yl)-*N*-(4-methoxybenzyl)glycinate (450 mg, 82.77 % yield). MS: m/z = 536.3 (M+1, ESI+).

### Step 4: Preparation of N-(6-(4-carbamoylpiperazin-1-yl)-3-(trifluoromethyl)imidazo[1,2-b]pyridazin-8-yl)-N-(4-methoxybenzyl)glycine

*N*-(6-(4-carbamoylpiperazin-1-yl)-3-(trifluoromethyl)imidazo[1,2-*b*]pyridazin-8-yl)-*N-*(4-methoxybenzyl)glycine (280 mg, 65.66 % yield) was obtained in the same method as in Step 2 of Example 64. MS: m/z = 508.2 (M+1, ESI+).

### Steps 5 to 7: Preparation of 4-(8-(((5,6-dichloro-1H-benzo[d]imidazol-2-yl)methyl)amino)-3-(trifluoromethyl)imidazo[1,2-b]pyridazin-6-yl)piperazine-1-carboxamide

**The compound of Example 66** (39 mg, 28.16 % yield) was obtained as a white solid in the same method as in Steps 3 to 5 of Example 30, except for changing the reaction temperature in Step 4 of Example 30 to 70 °C with stirring for 3 hours, and changing the reaction time in Step 5 to 1 hour. ¹H NMR (400 MHz, DMSO-d₆) δ 12.58 (s, 1H), 7.88 (s, 1H), 7.84 (t, 1H), 7.77 (s, 2H), 6.22 (s, 1H), 6.05 (s, 2H), 4.84 (d, 2H), 3.35 (s, 8H); MS: m/z = 528.1 (M+1, ESI+).

### Example 67: Preparation of 1-(8-(((5,6-dichloro-1H-benzo[d]imidazol-2-yl)methyl)amino)-3-(trifluoromethyl)imidazo[1,2-b]pyridazin-6-yl)piperidine-4-carboxamide

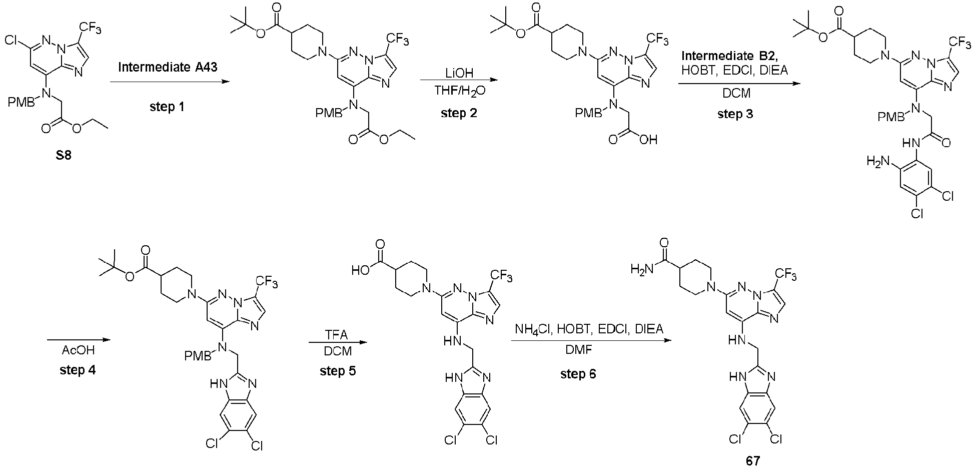

### Step 1: Preparation of tert-butyl 1-(8-((2-ethoxy-2-oxoethyl)(4-methoxybenzyl)amino)-3-(trifluoromethyl)imidazo[1,2-b]pyridazin-6-yl)piperidine-4-carboxylate

**Intermediate S8** (1.1 g, 2.48 mmol) was dissolved in **Intermediate A43** (15 mL) and stirred at 120 °C for 16 hours. Water (100 mL) was added to the reaction mixture, and the product was extracted three times with EtOAc (30 mL). The organic layer was washed three times with brine (100 mL), dried over Na₂SO₄, filtered, and concentrated. The concentrate was purified by silica gel chromatography to obtain *tert*-butyl 4-(8-((2-ethoxy-2-oxoethyl)(4-methoxybenzyl)amino)-3-(trifluoromethyl)imidazo[1,2-*b*]pyridazin-6-yl)piperazine-1-carboxylate (1.05 g, 71.42 % yield). MS: m/z = 592.1 (M+1, ESI+).

### Steps 2 to 5: Preparation of 1-(8-(((5,6-dichloro-1H-benzo[d]imidazol-2-yl)methyl)amino)-3-(trifluoromethyl)imidazo[1,2-b]pyridazin-6-yl)piperidine-4-carboxylic acid

1-(8-(((5,6-dichloro-1*H*-benzo[*d*]imidazol-2-yl)methyl)amino)-3-(trifluoromethyl)imidazo[1,2-*b*]pyridazin-6-yl)piperidine-4-carboxylic acid (200 mg, 76.05 % yield) was obtained in the same method as in Steps 2 to 5 of Example 30, except that the reaction times in Step 2, Step 3, and Step5 of Example 30 were changed to 1 hour, 16 hours, and 1 hour, respectively, and the reaction temperature in Step 4 was changed to 70 °C with stirring for 4 hours. MS: m/z = 528.2 (M+1, ESI+).

### Step 6: Preparation of 1-(8-(((5,6-dichloro-1H-benzo[d]imidazol-2-yl)methyl)amino)-3-(trifluoromethyl)imidazo[1,2-b]pyridazin-6-yl)piperidine-4-carboxamide

**The compound of Example 67** (10 mg, 5.00 % yield) was obtained as a white solid in the same method as in Step 3 of Example 30, except that ammonium chloride (102 mg, 1.90 mmol) was used instead of **Intermediate B2** (1.38 g, 7.8 mmol) and the mixture was stirred for 16 hours. ¹H NMR (400 MHz, DMSO-d₆) δ 12.56 (s, 1H), 7.85 (s, 1H), 7.79-7.72 (m, 3H), 7.24 (d, 1H), 6.76 (s, 1H), 6.16 (s, 1H), 4.83 (d, 2H), 4.06-4.01 (m, 2H), 2.82-2.67 (m, 2H), 2.44-2.28 (m, 1H), 1.71-1.66 (m, 2H), 1.53-1.44 (m, 2H); MS: m/z = 527.1 (M+1, ESI+).

Hereinafter, the compounds of Examples 68 to 81 were prepared using appropriate intermediates among the Intermediate B series in [Table 3] according to the following Scheme III.

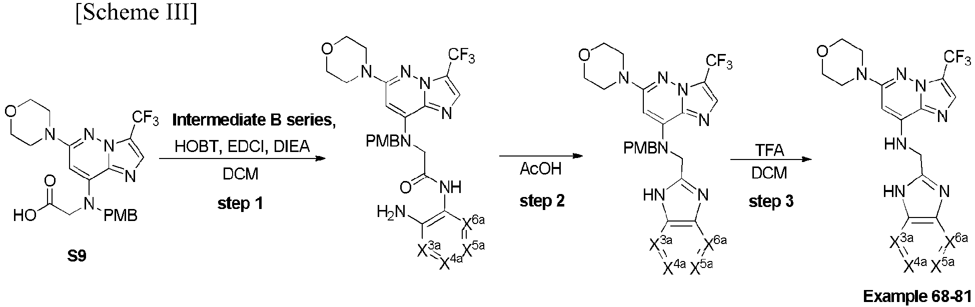

(In Scheme III, not more than one of X^{3a}, X^{4a}, X^{5a}, and X^{6a} is N, and the others are CR^{3a}, where each R^{3d} is independently H, halo, cyano, hydroxy, C₁-C₆ alkoxy, or C₁-C₆ alkyl, provided that any C₁-C₆ alkoxy group and C₁-C₆ alkyl group in R^{3d} may be optionally substituted with halo, cyano, or hydroxy.)

### Example 68: Preparation of N-((5,6-dichloro-1H-benzo[d]imidazol-2-yl)methyl)-6-morpholino-3-(trifluoromethyl)imidazo[1,2-b]pyridazin-8-amine

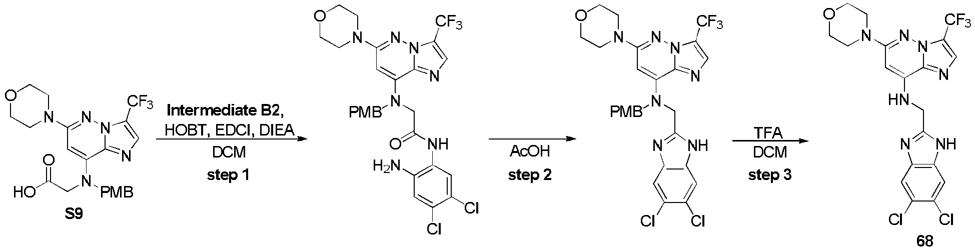

### Step 1: Preparation of N-(2-amino-4,5-dichlorophenyl)-2-((4-methoxybenzyl)(6-morpholino-3-(trifluoromethyl)imidazo[1,2-b]pyridazin-8-yl)amino)acetamide

*N*-(2-amino-4,5-dichlorophenyl)-2-((4-methoxybenzyl)(6-morpholino-3-(trifluoromethyl)imidazo[1,2-*b*]pyridazin-8-yl)amino)acetamide (1.7 g, 66.69 % yield) was obtained in the same method as in Step 3 of Example 30, except that **Intermediate S9** (1.9 g, 4.08 mmol) was used as a starting material and the reaction time was changed to 16 hours. MS: m/z = 624.2 (M +1, ESI+).

### Step 2: Preparation of N-((5,6-dichloro-1H-benzo[d]imidazol-2-yl)methyl)-N-(4-methoxybenzyl)-6-morpholino-3-(trifluoromethyl)imidazo[1,2-b]pyridazin-8-amine

*N*-((5,6-dichloro-1*H*-benzo[*d*]imidazol-2-yl)methyl)-*N*-(4-methoxybenzyl)-6-morpholino-3-(trifluoromethyl)imidazo[1,2-*b*]pyridazin-8-amine (1.6 g, 96.91 % yield) was obtained in the same method as in Step 4 of Example 30, except for stirring for 16 hours by changing the reaction temperature to 70 °C. MS: m/z = 606.2 (M + 1, ESI+).

### Step 3: Preparation of N-((5,6-dichloro-1H-benzo[d]imidazol-2-yl)methyl)-6-morpholino-3-(trifluoromethyl)imidazo[1,2-b]pyridazin-8-amine

**The compound of Example 68** (720 mg, 56.12 % yield) was obtained as a white solid in the same method as in Step 5 of Example 30. ¹H NMR (400 MHz, DMSO-d₆) δ 7.88-7.77 (m, 4H), 6.18 (s, 1H), 4.84 (d, 2H), 3.67 (t, 4H), 3.34 (t, 4H); MS: m/z = 486.1 (M+1, ESI+).

### Example 69 to 81

The compounds of Examples 69 to 81 were prepared in a similar method to Example 68, by using intermediates corresponding to the structure of the target compound from the Intermediate B series and appropriately changing the amounts of reagents, catalysts, and reaction conditions.

**[Table 5]**

| Example | Structure (Intermediate) | IUPAC Name | NMR and MS Data | |
|---|---|---|---|---|
| 69 | 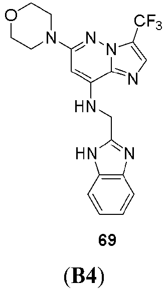 | *N*-((1*H*-benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.29 (s, 1H), 7.88-7.83 (m, 2H), 7.50 (s, 2H), 7.14 (dd, 2H), 6.19 (s, 1H), 4.82 (d, 2H), 3.66 (t, 4H), 3.33 (s, 4H); MS: m/z = 418.1 (M+1, ESI+). | |
| 70 | 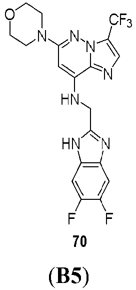 | *N*-((5,6-difluoro-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.47 (s, 1H), 7.88-7.82 (m, 2H), 7.55 (s, 2H), 6.18 (s, 1H), 4.81 (d, 2H), 3.67 (t, 4H), 3.35 (s, 4H); MS: m/z = 454.1 (M+1, ESI+). | |
| 71 | 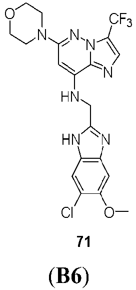 | *N*-((6-chloro-5-methoxy-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 7.89-7.83 (m, 2H), 7.64 (s, 1H), 7.22 (s, 1H), 6.20 (s, 1H), 4.86 (d, 2H), 3.87 (s, 3H), 3.67 (s, 4H), 3.35 (s, 4H); MS: m/z = 482.1 (M+1, ESI+). | |
| 72 | 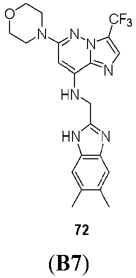 | *N*-((5,6-dimethyl-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.03 (s, 1H), 7.87 (s, 1H), 7.78 (t, 1H), 7.32 (s, 1H), 7.19 (s, 1H), 6.17 (s, 1H), 4.76 (d, 2H), 3.67 (t, 4H), 3.33 (s, 4H), 2.28 (s, 6H); MS: m/z = 446.2 (M+1, ESI+). | |
| 73 | 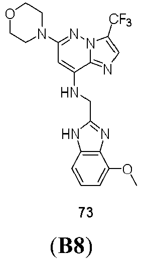 | *N*-((4-methoxy-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.28 (s, 1H), 7.87-7.77 (m, 2H), 7.06 (t, 2H), 6.69 (d, 1H), 6.22 (s, 1H), 4.77 (d, 2H), 3.91 (s, 3H), 3.67 (t, 4H), 3.34 (t, 4H); MS: m/z = 448.1 (M+1, ESI+). | |
| 74 | 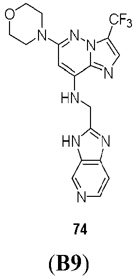 | *N*-((3*H*-imidazo[4,5-*c*]pyridin-2-yl)methyl)-6-morpholino-3-(trifluoromethyl)imidazo[1,2-*b*]pyridazin-8-amine | ¹H NMR (400 MHz, DMSO-d₆) δ 12.71 (s, 1H), 8.85 (s, 1H), 8.26 (d, 1H), 7.88 (d, 2H), 7.47 (s, 1H), 6.19 (s, 1H), 4.89 (d, 2H), 3.66 (t, 4H), 3.35 (s, 4H); MS: m/z = 419.1 (M+1, ESI+). | |
| 75 | 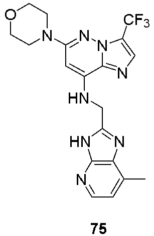 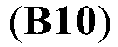 | *N*-((7-methyl-3*H*-imidazo[4,5-*b*]pyridin-2-yl)methyl)-6-morpholino-3-(trifluoromethyl)imidazo[1,2-*b*]pyridazin-8-amine | ¹H NMR (400 MHz, DMSO-d₆) δ 12.94-12.70 (m, 1H), 8.20-8.11 (m, 1H), 7.87 (s, 1H), 7.81 (t, 1H), 7.02 (t, 1H), 6.24-6.19 (m, 1H), 4.87-4.81 (m, 2H), 3.67 (d, 4H), 3.33 (s, 4H), 2.53 (s, 3H); MS: m/z = 433.1 (M+1, ESI+). | |
| 76 | 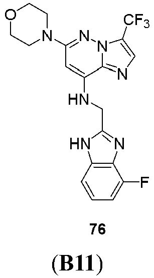 | *N*-((4-fluoro-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.65 (s, 1H), 7.89-7.86 (m, 2H), 7.30 (d, 1H), 7.16-7.11 (m, 1H), 6.96 (dd, 1H), 6.22 (s, 1H), 4.86 (d, 2H), 3.68 (t, 4H), 3.36 (t, 4H); MS: m/z = 436.2 (M+1, ESI+). | |
| 77 | 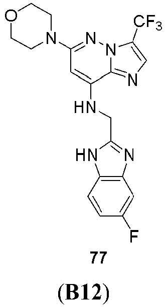 | *N*-((5-fluoro-1*H-*benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.40 (s, 1H), 7.88-7.84 (m, 2H), 7.56-7.22 (m, 2H), 7.00 (dd, 1H), 6.18 (s, 1H), 4.82 (s, 2H), 3.67 (t, 4H), 3.35 (s, 4H); MS: m/z = 436.1 (M+1, ESI+). | |
| 78 | 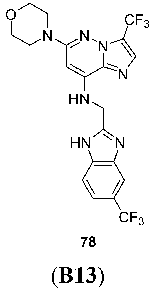 | 6-morpholino-3-(trifluoromethyl)-N-((5-(trifluoromethyl)-1*H-*benzo[*d*]imidazol-2-yl)methyl)imidazo[1,2-*b*]pyridazin-8-amine | | ¹H NMR (400 MHz, DMSO-d₆) δ 12.72 (d, 1H), 7.93-7.62 (m, 4H), 7.50-7.48 (m, 1H), 6.19 (s, 1H), 4.89 (d, 2H), 3.66 (t, 4H), 3.34 (s, 4H); MS: m/z = 486.1 (M+1, ESI+). |
| 79 | 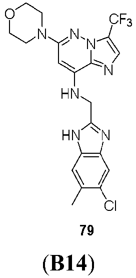 | *N*-((5-chloro-6-methyl-1*H-*benzo[*d*]imidazol-2-yl)methyl)-6-morpholino-3-(trifluoromethyl)imidazo[1,2-*b*]pyridazin-8-amine | | ¹H NMR (400 MHz, DMSO-d₆) δ 12.32 (s, 1H), 7.88 (s, 1H), 7.83 (t, 1H), 7.55 (s, 1H), 7.45 (s, 1H), 6.17 (s, 1H), 4.80 (d, 2H), 3.67 (t, 4H), 3.34 (s, 4H), 2.39 (s, 3H); MS: m/z = 466.1 (M+1, ESI+). |
| 80 | 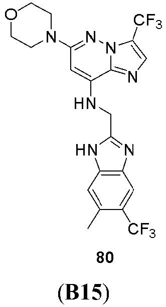 | *N*-((6-methyl-5-(trifluoromethyl)-1*H*-benzo[*d*]imidazol-2-yl)methyl)-6-morpholino-3-(trifluoromethyl)imidazo[1,2-*b*]pyridazin-8-amine | ¹H NMR (400 MHz, DMSO-d₆) δ 12.55 (s, 1H), 7.88-7.82 (m, 3H), 7.51 (s, 1H), 6.18 (s, 1H), 4.86 (d, 2H), 3.66 (t, 4H), 3.33 (t, 4H), 2.51 (s, 3H); MS: m/z = 500.2 (M+1, ESI+). | |
| 81 | 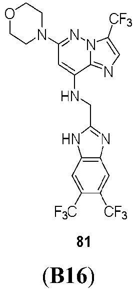 | *N*-((5,6-bis(trifluoromethyl)-1*H-*benzo[*d*]imidazol-2-yl)methyl)-6-morpholino-3-(trifluoromethyl)imidazo[1,2-*b*]pyridazin-8-amine | | ¹H NMR (400 MHz, DMSO-d₆) δ13.11 (s, 1H), 8.15 (s, 1H), 7.95 (t, 1H), 7.89 (s, 1H), 6.20 (s, 1H), 4.96 (d, 2H), 3.66 (t, 4H), 3.34 (t, 4H); MS: m/z = 554.1 (M+1, ESI+). |

### Example 82: Preparation of N-(imidazo[1,2-a]pyridin-2-ylmethyl)-6-morpholino-3-(trifluoromethyl)imidazo[1,2-b]pyridazin-8-amine

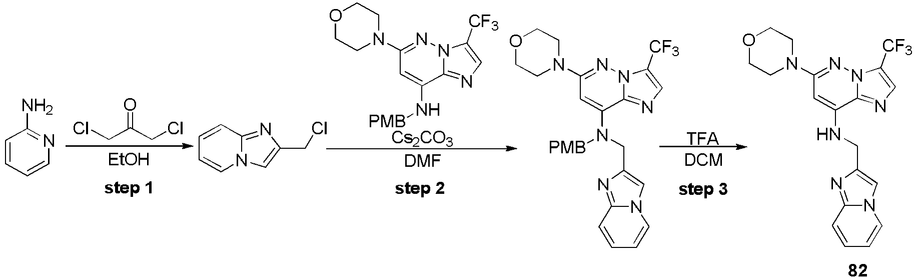

### Step 1: Preparation of 2-(chloromethyl)imidazo[1,2-a]pyridine

Pyridin-2-amine (2.2 g, 23.38 mmol) and 1,3-dichloropropan-2-one (2.97 g, 23.38 mmol) were dissolved in ethanol (30 mL), and then the mixture was stirred at 90 °C for 16 hours. The reaction mixture was concentrated, and the concentrate was purified by silica gel chromatography to obtain 2-(chloromethyl)imidazo[1,2-*a*]pyridine (400 mg, 10.27 % yield). MS: m/z = 167.0 (M+1, ESI+).

### Step 2: Preparation of N-(imidazo[1,2-a]pyridin-2-ylmethyl)-N-(4-methoxybenzyl)-6-morpholino-3-(trifluoromethyl)imidazo[1,2-b]pyridazin-8-amine

The product obtained in Step 1 (245 mg, 1.47 mmol) and the product obtained in Step 2 for the preparation of Intermediate S9 (400 mg, 982 umol) were dissolved in DMF (10 mL), and then cesium carbonate (960 mg, 2.95 mmol) was added thereto, followed by stirring at 90 °C for 3 hours. Saturated aqueous ammonium chloride solution (100 mL) was added to the reaction mixture, and the product was extracted three times with EtOAc (30 mL). The organic layer was washed three times with brine (100 mL), dried over Na₂SO₄, filtered, and concentrated. The concentrate was purified by silica gel chromatography to obtain *N*-(imidazo[1,2-*a*]pyridin-2-ylmethyl)-*N*-(4-methoxybenzyl)-6-morpholino-3-(trifluoromethyl)imidazo[1,2-b]pyridazin-8-amine (270 mg, 51.16 % yield). MS: m/z = 538.3 (M+1, ESI+).

### Step 3: Preparation of N-(imidazo[1,2-a]pyridin-2-ylmethyl)-6-morpholino-3-(trifluoromethyl)imidazo[1,2-b]pyridazin-8-amine

The product obtained in Step 2 (270 mg, 502.3 umol) was dissolved in DCM (6 mL), and then TFA (3 mL) was added thereto, followed by stirring at 25 °C for 3 hours. The reaction mixture was concentrated, and the concentrate was purified by silica gel chromatography to obtain **the compound of Example 82** (90 mg, 42.93 % yield) as a white solid. ¹H NMR (400 MHz, DMSO-d₆) δ 8.48 (d, 1H), 7.84 (d, 2H), 7.74 (t, 1H), 7.50 (d, 1H), 7.23-7.19 (m, 1H), 6.87-6.83 (m, 1H), 6.20 (s, 1H), 4.68 (d, 2H), 3.68 (t, 4H), 3.36 (t, 4H); MS: m/z = 418.2 (M+1, ESI+).

### Example 83: Preparation of N-(imidazo[1,2-a]pyrimidin-2-ylmethyl)-6-morpholino-3-(trifluoromethyl)imidazo[1,2-b]pyridazin-8-amine

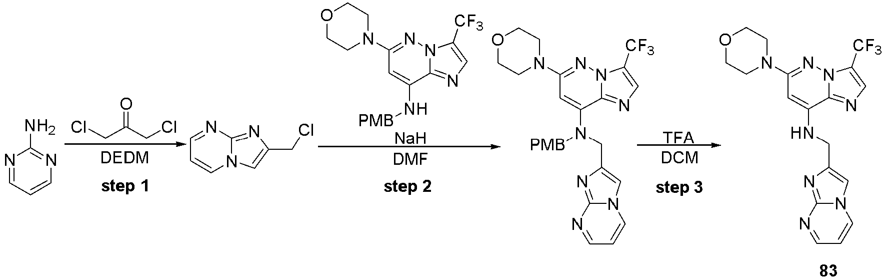

### Step 1: Preparation of 2-(chloromethyl)imidazo[1,2-a]pyrimidine

Pyrimidin-2-amine (5 g, 52.57 mmol) was dissolved in diethylene glycol dimethyl ether (20 mL), and then 1,3-dichloropropan-2-one (20.03 g, 157.72 mmol) was added thereto, followed by stirring at 70 °C for 16 hours. Water (200 mL) was added to the reaction mixture, and the product was extracted three times with EtOAc (50 mL). The organic layer was washed three times with brine (200 mL), dried over Na₂SO₄, filtered, and concentrated, and then the concentrate was purified by silica gel chromatography to obtain 2-(chloromethyl)imidazo[1,2-*a*]pyrimidine (2.5 g, 28.37 % yield). MS: m/z = 167.8 (M+1, ESI+).

### Step 2: Preparation of N-(imidazo[1,2-a]pyrimidin-2-ylmethyl)-N-(4-methoxybenzyl)-6-morpholino-3-(trifluoromethyl)imidazo[1,2-b]pyridazin-8-amine

The product obtained in Step 2 for the preparation of Intermediate S9 (1 g, 2.45 mmol) was dissolved in DMF (5 mL), and then NaH (295 mg, 7.36 mmol, 60 % purity) was added thereto at 0 °C, followed by stirring for 30 minutes. To this reaction solution, the product of Step 1 (411 mg, 2.45 mmol) was added, and the mixture was stirred at 25 °C for 3 hours. Ice water (50 mL) was added to the reaction mixture, and the product was washed three times with EtOAc (10 mL). The organic layer was washed three times with brine (50 mL), dried over Na₂SO₄, filtered, and concentrated, and then the concentrate was purified by silica gel chromatography to obtain *N-*(imidazo[1,2-a]pyrimidin-2-ylmethyl)-*N-*(4-methoxybenzyl)-6-morpholino-3-(trifluoromethyl)imidazo[1,2-*b*]pyridazin-8-amine (680 mg, 51.44 % yield). MS: m/z = 539.3 (M+1, ESI+).

### Step 3: Preparation of N-(imidazo[1,2-a]pyrimidin-2-ylmethyl)-6-morpholino-3-(trifluoromethyl)imidazo[1,2-b]pyridazin-8-amine

The product obtained in Step 2 (680 mg, 1.26 mmol) was dissolved in DCM (10 mL), and then TFA (5 mL) was added thereto, followed by stirring at 25 °C for 16 hours. The reaction mixture was concentrated, and the concentrate was purified by Prep-HPLC to obtain **the compound of Example 83** (70 mg, 13.25 % yield) as a white solid. ¹H NMR (400 MHz, DMSO-d₆) δ 8.89 (dd, 1H), 8.49 (dd, 1H), 7.86 (s, 1H), 7.81 (t, 1H), 7.78 (s, 1H), 7.02 (dd, 1H), 6.18 (s, 1H), 4.72 (d, 2H), 3.68 (t, 4H), 3.35 (t, 4H); MS: m/z = 419.1 (M+1, ESI+).

### Example 84: Preparation of N-((1H-pyrrolo[2,3-b]pyridin-2-yl)methyl)-6-morpholino-3-(trifluoromethyl)imidazo[1,2-b]pyridazin-8-amine

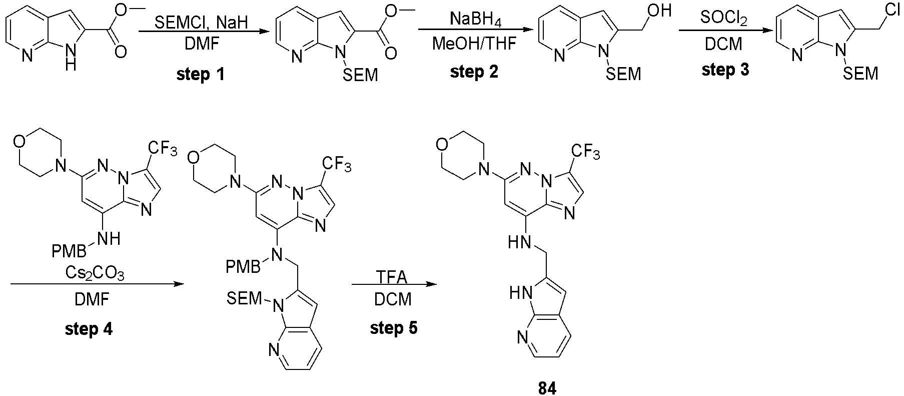

### Step 1: Preparation of methyl 1-((2-(trimethylsilyl)ethoxy)methyl)-1H-pyrrolo[2,3-b]pyridine-2-carboxylate

Methyl 1*H*-pyrrolo[2,3-*b*]pyridine-2-carboxylate (3.2 g, 18.16 mmol) was dissolved in DMF (50 mL), and then NaH (1.09 g, 27.25 mmol, 60% purity) was added thereto in several portions at 0 °C, followed by stirring at 25 °C for 1 hour. To this reaction solution, 2-(trimethylsilyl)ethoxymethyl chloride (3.86 mL, 21.80 mmol) was added, and the mixture was stirred at 25 °C for 16 hours. Saturated aqueous ammonium chloride solution (500 mL) was added to the reaction mixture, and the product was washed three times with EtOAc (100 mL). The organic layer was washed with brine (100 mL), dried over Na₂SO₄, filtered, and concentrated. The concentrate was purified by silica gel chromatography to obtain methyl 1-((2-(trimethylsilyl)ethoxy)methyl)-1*H*-pyrrolo[2,3-*b*]pyridine-2-carboxylate (2.8 g, 50.31 % yield). MS: m/z = 307.1 (M+1, ESI+).

### Step 2: Preparation of (1-((2-(trimethylsilyl)ethoxy)methyl)-1H-pyrrolo[2,3-b]pyridin-2-yl)methanol

The product obtained in Step 1 (2.8 g, 9.14 mmol) was dissolved in THF (50 mL) and methanol (10 mL), and then sodium borohydride (491 mg, 13.71 mmol) was added thereto, followed by stirring at 50 °C for 5 hours. Saturated aqueous ammonium chloride solution (200 mL) was added to the reaction mixture, and the product was extracted three times with EtOAc (60 mL). The organic layer was washed three times with brine (200 mL), dried over Na₂SO₄, filtered, and concentrated. The concentrate was purified by silica gel chromatography to obtain (1-((2-(trimethylsilyl)ethoxy)methyl)-1*H*-pyrrolo[2,3-*b*]pyridin-2-yl)methanol (2.1 g, 82.55% yield). MS: m/z = 279.1 (M+1, ESI+).

### Step 3: Preparation of 2-(chloromethyl)-1-((2-(trimethylsilyl)ethoxy)methyl)-1H-pyrrolo[2,3-b]pyridine

The product obtained in Step 2 (2.1 g, 7.54 mmol) was dissolved in DCM (15 mL), and then thionyl chloride (1.64 mL, 22.63 mmol) was slowly added thereto at 0 °C, followed by stirring at 25 °C for 3 hours. The reaction mixture was concentrated, and then the concentrate was purified by silica gel chromatography to obtain 2-(chloromethyl)-1-((2-(trimethylsilyl)ethoxy)methyl)-1*H-*pyrrolo[2,3-*b*]pyridine (1.5 g, 66.99 % yield). MS: m/z = 297.1 (M+1, ESI+).

### Steps 4 and 5: Preparation of N-((1H-pyrrolo[2,3-b]pyridin-2-yl)methyl)-6-morpholino-3-(trifluoromethyl)imidazo[1,2-b]pyridazin-8-amine

**The compound of Example 84** (60 mg, 34.28 % yield) was obtained as a white solid in the same method as in Steps 2 and 3 of Example 82, except that the reaction time in Step 2 of Example 82 was changed to 5 hours. ¹H NMR (400 MHz, DMSO-d₆) δ 11.55 (s, 1H), 8.13 (dd, 1H), 7.86-7.83 (m, 2H), 7.70 (t, 1H), 7.00 (dd, 1H), 6.36 (s, 1H), 6.18 (s, 1H), 4.74 (d, 2H), 3.68 (t, 4H), 3.36 (t, 4H); MS: m/z = 418.3 (M+1, ESI+).

### Example 85: Preparation of N-(imidazo[1,2-b]pyridazin-2-ylmethyl)-6-morpholino-3-(trifluoromethyl)imidazo[1,2-b]pyridazin-8-amine

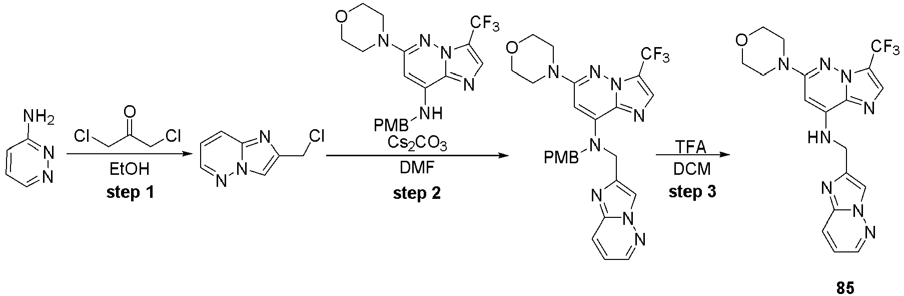

**The compound of Example 85** (70 mg, 20.02 % yield) was obtained as a white solid in the same method as in Steps 1 to 3 of Example 82. However, in Step 1 of Example 82, pyridazin-3-amine (5.2 g, 54.68 mmol) was used as a starting material with stirring at 85 °C for 3 hours, and the reaction time in Step 2 was changed to 5 hours. ¹H NMR (400 MHz, DMSO-d₆) δ 8.48 (dd, 1H), 8.21 (s, 1H), 8.07 (dd, 1H), 7.85 (s, 1H), 7.76 (t, 1H), 7.21 (dd, 1H), 6.21 (s, 1H), 4.72 (d, 2H), 3.69 (t, 4H), 3.36 (t, 4H); MS: m/z = 419.0 (M+1, ESI+).

### Example 86: Preparation of 6-morpholino-N-(pyrazolo[1,5-a]pyridin-2-ylmethyl)-3-(trifluoromethyl)imidazo [1,2-b]pyridazin-8-amine

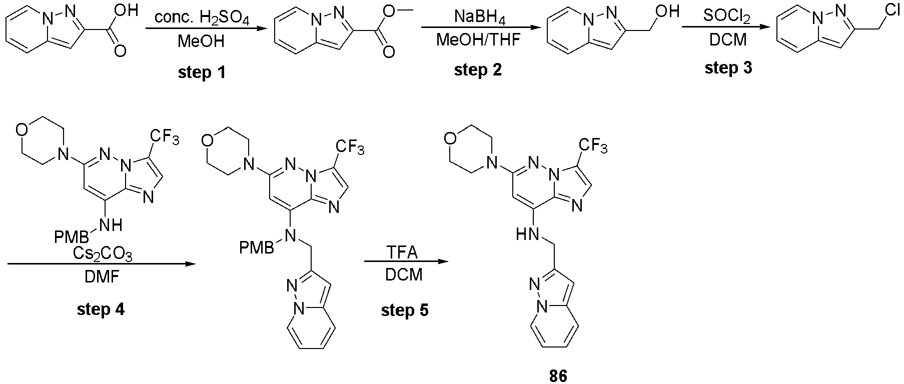

### Step 1: Preparation of methyl pyrazolo[1,5-a]pyridine-2-carboxylate

Pyrazolo[1,5-*a*]pyridine-2-carboxylic acid (1.1 g, 6.78 mmol) was dissolved in methanol (30 mL), and then concentrated sulfuric acid (0.11 mL, 2.04 mmol) was added thereto, followed by stirring at 75 °C for 5 hours. After the reaction mixture was concentrated, sodium bicarbonate solution (100 mL) was added, and the product was washed three times with EtOAc (30 mL). The organic layer was washed three times with brine (100 mL), dried over Na₂SO₄, filtered, and concentrated, and then the concentrate was purified by silica gel chromatography to obtain methyl pyrazolo[1,5-*a*]pyridine-2-carboxylate (950 mg, 79.49 % yield). MS: m/z = 177.1 (M+1, ESI+).

### Steps 2 to 5: Preparation of 6-morpholino-N-(pyrazolo[1,5-a]pyridin-2-ylmethyl)-3-(trifluoromethyl)imidazo[1,2-b]pyridazin-8-amine

**The compound of Example 86** (100 mg, 41.54 % yield) was obtained as a white solid in the same method as in Steps 2 to 5 of Example 84, except for changing the reaction times in Step 3 and Step 4 of Example 84 to 2 hours and 3 hours, respectively. ¹H NMR (400 MHz, DMSO-d₆) δ 8.62 (dd, 1H), 7.85-7.82 (m, 2H), 7.60 (d, 1H), 7.20-7.15 (m, 1H), 6.86-6.82 (m, 1H), 6.51 (s, 1H), 6.18 (s, 1H), 4.73 (d, 2H), 3.68 (t, 4H), 3.34 (t, 4H); MS: m/z = 418.1 (M+1, ESI+).

### Example 87: Preparation of N-((1H-pyrrolo[3,2-b]pyridin-2-yl)methyl)-6-morpholino-3-(trifluoromethyl)imidazo[1,2-b]pyridazin-8-amine

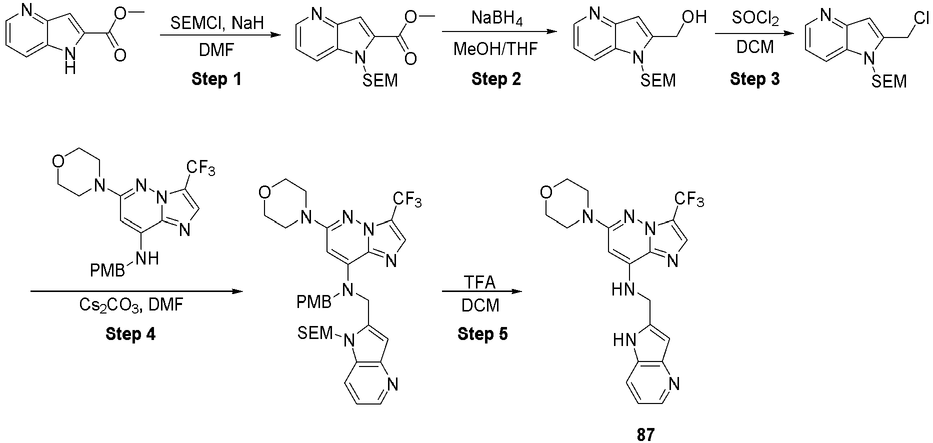

**The compound of Example 87** (74 mg, 34.26 % yield) was obtained as a white solid in the same method as in Steps 1 to 5 of Example 84. ¹H NMR (400 MHz, DMSO-d₆) δ 11.19 (s, 1H), 8.24 (d, 1H), 7.86 (s, 1H), 7.79 (s, 1H), 7.69 (d, 1H), 7.05-7.00 (m, 1H), 6.51 (s, 1H), 6.16 (d, 1H), 4.78 (s, 2H), 3.77-3.62 (m, 4H), 3.40-3.24 (m, 4H); MS: m/z = 418.1 (M+1, ESI+).

### Example 88: Preparation of N-((5,7-dimethylimidazo[1,2-a]pyrimidin-2-yl)methyl)-6-morpholino-3-(trifluoromethyl)imidazo[1,2-b]pyridazin-8-amine

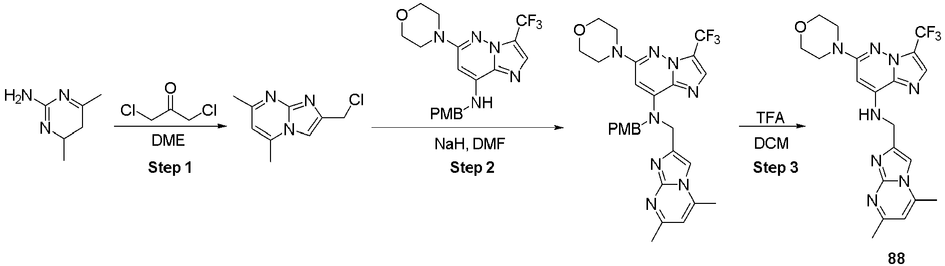

**The compound of Example 88** (28 mg, 10.14 % yield) was obtained as a white solid in the same method as in Steps 1 to 3 of Example 82. However, in Step 1 of Example 82, 4,6-dimethyl-4,5-dihydropyrimidin-2-amine (6 g, 50 mmol) was dissolved in DME (80 mL) and stirred at 50 °C; in Step 2, the mixture was stirred at 25 °C for 2 hours using NaH (145 mg, 6.03 mmol, 60 % purity); and in Step 3, the reaction time was changed to 1 hour. ¹H NMR (400 MHz, DMSO-d₆) δ 7.85 (s, 1H), 7.73-7.67 (m, 1H), 7.66 (s, 1H), 6.84 (s, 1H), 6.19 (s, 1H), 4.68 (d, 2H), 3.72-3.64 (m, 4H), 3.38-3.33 (m, 4H), 2.56 (s, 3H), 2.48 (s, 3H); MS: m/z = 447.2 (M+1, ESI+).

### Example 89: Preparation of 6-morpholino-N-(pyrazolo[1,5-a]pyrimidin-2-ylmethyl)-3-(trifluoromethyl)imidazo[1,2-b]pyridazin-8-amine

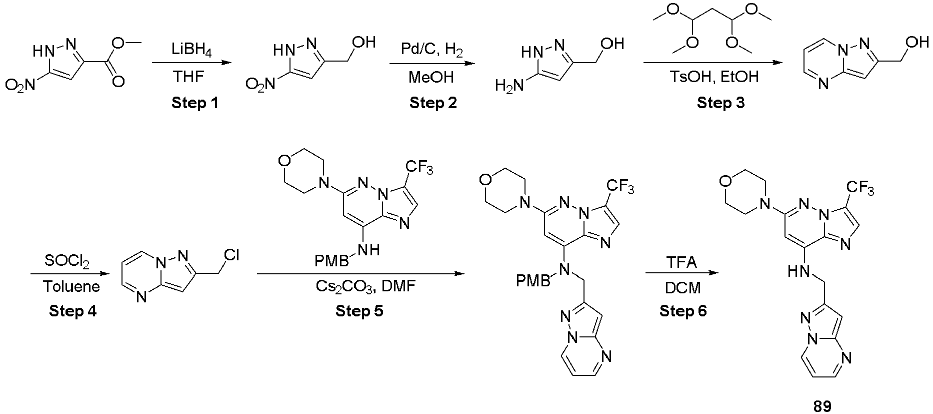

### Step 1: Preparation of (5-nitro-1H-pyrazol-3-yl)methanol

Methyl 5-nitro-1*H*-pyrazole-3-carboxylate (9 g, 52.60 mmol) was dissolved in THF (200 mL), and then lithium borohydride (LiBH₄, 5.73 g, 263 mmol) was added in several portions at 0 °C within 1 hour, followed by stirring at 40 °C for 48 hours. The reaction mixture was slowly cooled to room temperature by pouring ice water (300 mL), and the product was extracted three times with EtOAc (60 mL). The organic layer was washed three times with brine (300 mL), dried over Na₂SO₄, filtered, and concentrated. The concentrate was purified by silica gel chromatography to obtain (5-nitro-1*H*-pyrazol-3-yl)methanol (4.5 g, 59.84 % yield). MS: m/z = 144.1 (M+1, ESI+).

### Step 2: Preparation of (5-amino-1H-pyrazol-3-yl)methanol

The product obtained in Step 1 (4.50 g, 31.45 mmol) was dissolved in methanol (50 mL), and then Pd/C (1 g) was added thereto, followed by stirring at 25 °C for 16 hours under H₂ atmosphere. The reaction mixture was filtered and concentrated to obtain (5-amino-1*H*-pyrazol-3-yl)methanol (3.5 g, 98.39% yield). MS: m/z = 113.9 (M+1, ESI+).

### Step 3: Preparation of pyrazolo[1,5-a]pyrimidin-2-ylmethanol

The product obtained in Step 2 (3.5 g, 30.94 mmol) and 1,1,3,3-tetramethoxypropane (6.10 g, 37.13 mmol) were dissolved in ethanol (50 mL), and then TsOH (2.66 g, 15.47 mmol) was added thereto, followed by stirring at 80 °C for 1 hour. The reaction mixture was concentrated, and the concentrate was purified by silica gel chromatography to obtain pyrazolo[1,5-a]pyrimidin-2-ylmethanol (360 mg, 7.80% yield). MS: m/z = 150.2 (M+1, ESI+).

### Step 4: Preparation of 2-(chloromethyl)pyrazolo[1,5-a]pyrimidine

The product obtained in Step 3 (360 mg, 2.41 mmol) was dissolved in toluene (5 mL), and then thionyl chloride (861 mg, 7.24 mmol, 0.53 mL) was added thereto, followed by stirring at 50 °C for 1 hour. The reaction mixture was concentrated to obtain 2-(chloromethyl)pyrazolo[1,5-*a*]pyrimidine (360 mg, 2.15 mmol, 88.99% yield). MS: m/z = 168.0 (M+1, ESI+).

### Step 5 and 6: Preparation of 6-morpholino-N-(pyrazolo[1,5-a]pyrimidin-2-ylmethyl)-3-(trifluoromethyl)imidazo[1,2-b]pyridazin-8-amine

**The compound of Example 89** (290 mg, 31.12% yield) was obtained as a white solid in the same method as in Steps 4 and 5 of Example 84. ¹H NMR (400 MHz, DMSO-d₆) δ 9.09-9.06 (m, 1H), 8.51 (dd, 1H), 7.89-7.85 (m, 2H), 7.00 (dd, 1H), 6.64 (s, 1H), 6.17 (s, 1H), 4.77 (d, 2H), 3.68 (t, 4H), 3.35 (t, 4H); MS: m/z = 419.2 (M+1, ESI+).

### Example 90: Preparation of N-((5,6-dichloro-1H-indol-2-yl)methyl)-6-morpholino-3-(trifluoromethyl)imidazo[1,2-b]pyridazin-8-amine

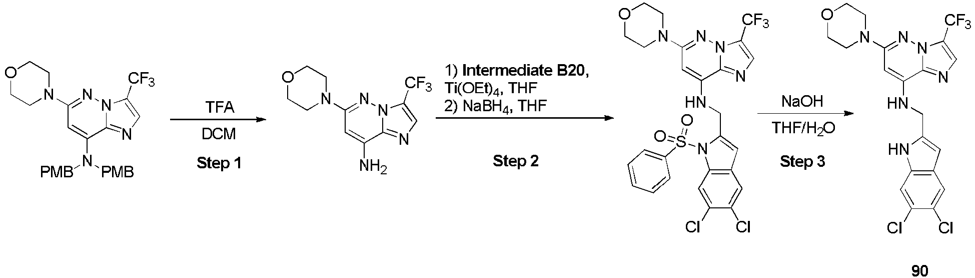

### Step 1: Preparation of 6-morpholino-3-(trifluoromethyl)imidazo[1,2-b]pyridazin-8-amine

The product of Step 1 for the preparation of **Intermediate S9** (36 g, 68.31 mmol) was dissolved in DCM (300 mL), and then TFA (300 mL) was added thereto, followed by stirring at 25 °C for 16 hours. Sodium bicarbonate solution (2000 mL) was added to the reaction mixture, and the product was extracted three times with DCM (300 mL). The organic layer was washed three times with brine (2000 mL), dried over Na₂SO₄, filtered, and concentrated. The concentrate was purified by silica gel chromatography to obtain 6-morpholino-3-(trifluoromethyl)imidazo[1,2-*b*]pyridazin-8-amine (18 g, 91.83 % yield) as a pale yellow solid. ¹H NMR (400 MHz, DMSO-d₆) δ 7.85 (s, 1H), 6.90 (br s, 2H), 6.09 (s, 1H), 3.71 (t, 4H), 3.33 (t, 4H). MS: m/z = 288.1 (M+1, ESI+).

### Step 2: Preparation of N-((5,6-dichloro-1-(phenylsulfonyl)-1H-indol-2-yl)methyl)-6-morpholino-3-(trifluoromethyl)imidazo[1,2-b]pyrazin-8-amine

The product obtained in Step 1 (1.22 g, 4.23 mmol) and **Intermediate B20** (3 g, 8.47 mmol) were dissolved in THF (50 mL), and then Ti(OEt)₄ (2.90 g, 12.70 mmol) was added thereto, followed by stirring at 90 °C for 16 hours. Thereafter, the reaction mixture was cooled to 0 °C, and NaBH₄ (641 mg, 16.94 mmol) was added thereto, followed by stirring at 0 °C for 1 hour. Water (400 mL) was added to the reaction mixture, and the product was extracted three times with EtOAc (80 mL). The organic layer was washed three times with brine (400 mL), dried over Na₂SO₄, filtered, and concentrated. The concentrate was purified by silica gel chromatography to obtain N-((5,6-dichloro-1-(phenylsulfonyl)-1H-indol-2-yl)methyl)-6-morpholino-3-(trifluoromethyl)imidazo[1,2-b]pyrazin-8-amine (250 mg, crude) as a yellow solid. MS: m/z = 625.2 (M+1, ESI+).

### Step 3: Preparation of N-((5,6-dichloro-1H-indol-2-yl)methyl)-6-morpholino-3-(trifluoromethyl)imidazo[1,2-b]pyridazin-8-amine

The product obtained in Step 2 (250 mg, crude) was dissolved in THF (10 mL), and then 4 N NaOH aqueous solution (10 mL) was added thereto, followed by stirring at 90 °C for 16 hours. After the reaction mixture was cooled to room temperature, water (20 mL) was added thereto, and the mixture was extracted three times with DCM (5 mL). The organic layer was washed three times with brine (20 mL), dried over Na₂SO₄, filtered, and concentrated. The concentrate was purified by silica gel chromatography to obtain *N*-((5,6-dichloro-1*H*-indol-2-yl)methyl)-6-morpholino-3-(trifluoromethyl)imidazo[1,2-*b*]pyridazin-8-amine (30 mg, 15.47 % yield) as a pale yellow solid. ¹H NMR (400 MHz, DMSO-d₆) δ 11.27 (s, 1H), 7.86 (s, 1H), 7.78 (t, 1H), 7.71 (s, 1H), 7.59 (s, 1H), 6.40 (s, 1H), 6.15 (s, 1H), 4.73 (d, 2H), 3.68 (t, 4H), 3.60-3.34 (m, 4H); MS: m/z = 485.1 (M+1, ESI+).

### Example 91: Preparation of N-((5,6-dichlorobenzo[d]oxazol-2-yl)methyl)-6-morpholino-3-(trifluoromethyl)imidazo[1,2-b]pyridazin-8-amine

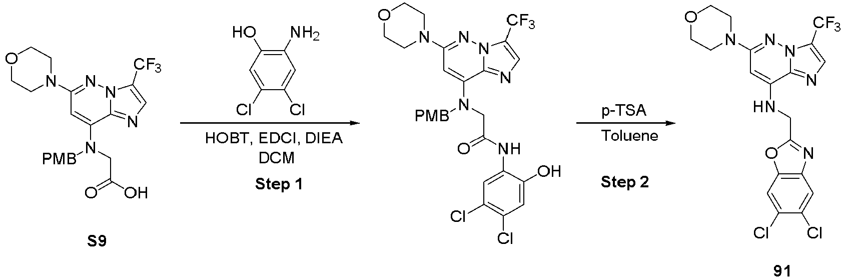

### Step 1: Preparation of N-(4,5-dichloro-2-hydroxyphenyl)-2-((4-methoxybenzyl)(6-morpholino-3-(trifluoromethyl)imidazo[1,2-b]pyridazin-8-yl)amino)acetamide

*N*-(4,5-dichloro-2-hydroxyphenyl)-2-((4-methoxybenzyl)(6-morpholino-3-(trifluoromethyl)imidazo[1,2-*b*]pyridazin-8-yl)amino)acetamide (1.3 g, 48.37 % yield) was obtained as a yellow solid in the same method as in Step 3 of Example 30, except that **Intermediate S9** (2 g, 4.34 mmol) and 2-amino-4,5-dichlorophenol (1.91 g, 10.74 mmol) were used and the mixture was stirred for 16 hours. MS: m/z = 625.1 (M+1, ESI+).

### Step 2: Preparation of N-((5,6-dichlorobenzo[d]oxazol-2-yl)methyl)-6-morpholino-3-(trifluoromethyl)imidazo[1,2-b]pyridazin-8-amine

The product obtained in Step 1 (1.3 g, 2.08 mmol) was dissolved in toluene (15 mL), and then TsOH (1.79 g, 10.39 mmol) was added thereto, followed by stirring at 120 °C for 2 hours. The reaction mixture was cooled to room temperature, and ice water (200 mL) was added thereto, and the product was extracted three times with EtOAc (50 mL). The organic layer was washed three times with brine (200 mL), dried over Na₂SO₄, filtered, and concentrated. The concentrate was purified by silica gel chromatography to obtain **the compound of Example 91** (30 mg, 2.68 % yield) as a white solid. ¹H NMR (400 MHz, DMSO-d₆) δ 8.22 (s, 1H), 8.09 (s, 1H), 7.94 (t, 1H), 7.88 (s, 1H), 6.31 (s, 1H), 4.97 (d, 2H), 3.68 (t, 4H), 3.37 (t, 4H); MS: m/z = 487.1 (M+1, ESI+).

### Example 92: Preparation of 4-(8-(((6,7-difluoro-1H-benzo[d]imidazol-2-yl)methyl)amino)-3-(trifluoromethyl)imidazo[1,2-b]pyridazin-6-yl)piperazine-1-carboxamide

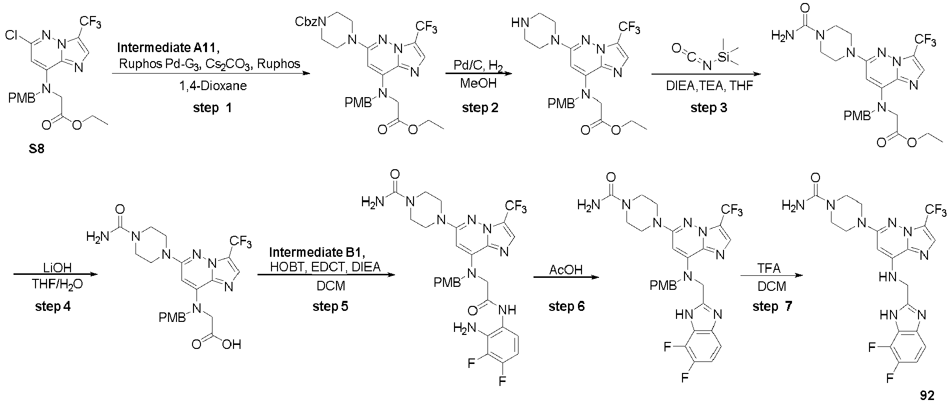

### Step 1: Preparation of benzyl 4-(8-((2-ethoxy-2-oxoethyl)(4-methoxybenzyl)amino)-3-(trifluoromethyl)imidazo[1,2-b]pyridazin-6-yl)piperazine-1-carboxylate

Benzyl 4-(8-((2-ethoxy-2-oxoethyl)(4-methoxybenzyl)amino)-3-(trifluoromethyl)imidazo[1,2-*b*]pyridazin-6-yl)piperazine-1-carboxylate (1.5 g, 35.29 % yield) was obtained as a yellow solid in the same method as in Step 1 of Example 30, except that **Intermediate A11** (4.48 g, 20.37 mmol) was used and the mixture was stirred for 16 hours. MS: m/z = 627.1 (M+1, ESI+).

### Step 2: Preparation of ethyl N-(4-methoxybenzyl)-N-(6-(piperazin-1-yl)-3-(trifluoromethyl)imidazo[1,2-b]pyridazin-8-yl)glycinate

The product obtained in Step 1 (1.5 g, 2.40 mmol) was dissolved in methanol (20 mL), and then Pd/C (200 mg) was added thereto, followed by stirring at 25 °C for 48 hours under hydrogen gas charging. The reaction mixture was filtered and concentrated, and the concentrate was purified by silica gel chromatography to obtain ethyl *N*-(4-methoxybenzyl)-*N*-(6-(piperazin-1-yl)-3-(trifluoromethyl)imidazo[1,2-*b*]pyridazin-8-yl)glycinate (970 mg, 82.20 % yield) as a yellow solid. MS: m/z = 493.1 (M+1, ESI+).

### Step 3: Preparation of ethyl N-(6-(4-carbamoylpiperazin-1-yl)-3-(trifluoromethyl)imidazo[1,2-b]pyridazin-8-yl)-N-(4-methoxybenzyl)glycinate

The product obtained in Step 2 (970 mg, 1.97 mmol) and trimethylsilyl isocyanate (295 mg, 2.56 mmol) were dissolved in THF (30 mL), and then DIEA (763 mg, 5.91 mmol, 1.04 mL) was added thereto, followed by stirring at 25 °C for 16 hours. Water (100 mL) was added to the reaction mixture, and the mixture was extracted three times with EtOAc (40 mL). The organic layer was washed three times with brine (100 mL), dried over Na₂SO₄, filtered, and concentrated. The concentrate was purified by silica gel chromatography to obtain ethyl *N*-(6-(4-carbamoylpiperazin-1-yl)-3-(trifluoromethyl)imidazo[1,2-*b*]pyridazin-8-yl)-*N*-(4-methoxybenzyl)glycinate (450 mg, 42.73 % yield) as a yellow solid. MS: m/z = 536.1 (M+1, ESI+).

### Steps 4 to 7: Preparation of 4-(8-(((6,7-difluoro-1H-benzo[d]imidazol-2-yl)methyl)amino)-3-(trifluoromethyl)imidazo[1,2-b]pyridazin-6-yl)piperazine-1-carboxamide

**The compound of Example 92** (60 mg, 31.09 % yield) was obtained as a white solid in the same method as in Steps 2 to 5 of Example 30. However, in Step 5, the product of Step 3 (450 mg, 840 umol) and **Intermediate B1** (583 mg, 4.05 mmol) were dissolved in DMF (15 mL) and stirred for 16 hours, and in Step 7, the reaction temperature was changed to 70 °C with stirring for 16 hours. ¹H NMR (400 MHz, DMSO-d₆) δ 12.69 (s, 1H), 7.88-7.85 (m, 2H), 7.25-7.16 (m, 2H), 6.23 (s, 1H), 6.05 (s, 2H), 4.85 (d, 2H), 3.36 (s, 8H); MS: m/z = 496.0 (M+1, ESI+).

### Example 93: Preparation of N-((5,6-dichlorobenzo[d]thiazol-2-yl)methyl)-6-morpholino-3-(trifluoromethyl)imidazo[1,2-b]pyridazin-8-amine

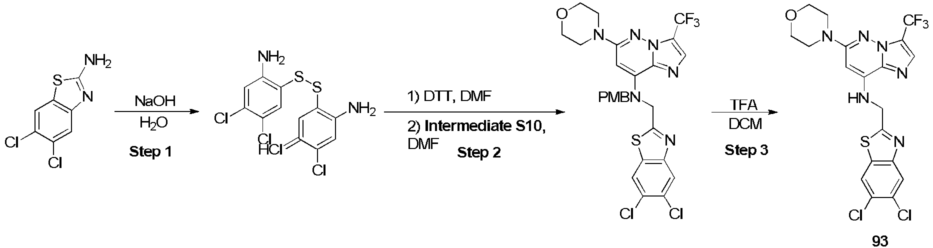

### Step 1: Preparation of 6,6'-disulfanedialbis(3,4-dichloroaniline)

5,6-dichlorobenzo[*d*]thiazol-2-amine (3.6 g, 16.43 mmol) was dissolved in water (40 mL), and then NaOH (19.7 g, 493 mmol) was added thereto at 0 °C, followed by stirring at 130 °C for 16 hours. The reaction mixture was concentrated, methanol was added thereto, and then the mixture was filtered and concentrated to obtain 6,6'-disulfanedialbis(3,4-dichloroaniline) (1.9 g, 29.94 % yield) as a yellow solid. MS: m/z = 386.8 (M+1, ESI+).

### Step 2: Preparation of N-((5,6-dichlorobenzo[d]thiazol-2-yl)methyl)-N-(4-methoxybenzyl)-6-morpholino-3-(trifluoromethyl)imidazo[1,2-b]pyridazin-8-amine

The product obtained in Step 1 (480 mg, 1.24 mmol) was dissolved in DMF (10 mL), and then 1,4-dithiothreitol (DTT, 959 mg, 6.22 mmol) was added thereto, followed by stirring at 25 °C for 30 minutes. Thereafter, **Intermediate S10** (600 mg, crude) was added thereto, and the mixture was stirred at 25 °C for 16 hours. Water (100 mL) was added to the reaction mixture, and the product was extracted three times with DCM (30 mL). The organic layer was washed three times with brine (100 mL), dried over Na₂SO₄, filtered, and concentrated. The concentrate was purified by silica gel chromatography to obtain N-((5,6-dichlorobenzo[d]thiazol-2-yl)methyl)-N-(4-methoxybenzyl)-6-morpholino-3-(trifluoromethyl)imidazo[1,2-b]pyridazin-8-amine (330 mg, 42.58 % yield) as a yellow solid. MS: m/z = 623.1 (M+1, ESI+).

### Step 3: Preparation of N-((5,6-dichlorobenzo[d]thiazol-2-yl)methyl)-6-morpholino-3-(trifluoromethyl)imidazo[1,2-b]pyridazin-8-amine

**The compound of Example 93** (85 mg, 31.91 % yield) was obtained as a white solid in the same method as in Step 5 of Example 30, except that the reaction time was changed to 2 hours. ¹H NMR (400 MHz, DMSO-d₆) δ 8.41 (s, 1H), 8.29 (s, 1H), 8.25 (t, 1H), 7.90 (s, 1H), 6.29 (s, 1H), 5.07 (d, 2H), 3.67 (t, 4H), 3.35 (t, 4H); MS: m/z = 502.9 (M+1, ESI+).

### Example 94 to 108

The compounds of Examples 94 to 108 were prepared in a similar method to Example 30 by using Intermediate B2 and intermediates corresponding to the structure of the target compound among the Intermediate S series and Intermediate A series in [Table 1], and appropriately changing the amounts of reagents, catalysts, and reaction conditions.

**[Table 6]**

| Example | Structure (Intermediate) | IUPAC Name | NMR and MS Data |
|---|---|---|---|
| 94 | 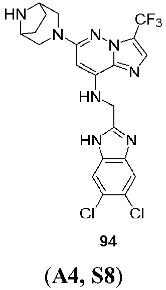 | 6-(3,8-diazabicyclo[3.2.1]octan-3-yl)-*N*-((5,6-dichloro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 7.83 (s, 1H), 7.77-7.74 (m, 3H), 6.00 (s, 1H), 4.81 (d, 2H), 3.60 (d, 2H), 3.44 (s, 2H), 2.81 (d, 2H), 1.62-1.51 (m, 4H); MS: m/z = 511.0 (M+1, ESI+). |
| 95 | 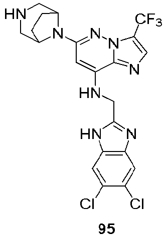 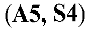 | 6-(3,8-diazabicyclo[3.2.1]octan-8-yl)-*N*-((5,6-dichloro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 7.84-7.76 (m, 4H), 5.99 (s, 1H), 4.80 (d, 2H), 4.18 (s, 2H), 2.77 (d, 2H), 2.37 (d, 2H), 1.88-1.79 (m, 4H); MS: m/z = 511.0 (M+1, ESI+). |
| 96 | 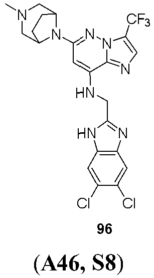 | *N*-((5,6-dichloro-1*H-*benzo[d]imidazol-2-yl)methyl)-6-(3-methyl-3,8-diazabicyclo[3.2.1]octan-8-yl)-3-(trifluoromethyl)imidazo[1,2-*b*]pyridazin-8-amine | ¹H NMR (400 MHz, DMSO-d₆) δ 12.58 (s, 1H), 7.85-7.71 (m, 4H), 5.97 (s, 1H), 4.81 (d, 2H), 4.28 (s, 2H), 2.39 (d, 2H), 2.04 (d, 2H), 1.98 (s, 3H), 1.86-1.76 (m, 4H); MS: m/z = 525.0 (M+1, ESI+). |
| 97 | 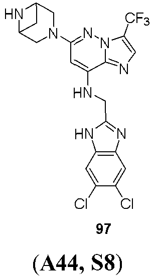 | 6-(3,6-diazabicyclo[3.1.1]heptan-3-yl)-*N*-((5,6-dichloro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 7.83 (s, 1H), 7.79 (t, 1H), 7.76 (s, 2H), 5.90 (s, 1H), 4.82 (d, 2H), 3.63 (d, 2H), 3.53-3.46 (m, 4H), 2.45-2.43 (m, 1H), 1.41 (d, 1H); MS: m/z = 497.0 (M+1, ESI+). |
| 98 | 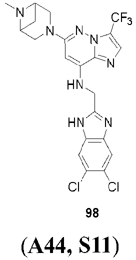 | *N*-((5,6-dichloro-1*H-*benzo[*d*]imidazol-2-yl)methyl)-6-(6-methyl-3,6-diazabicyclo[3.1.1]heptan-3-yl)-3-(trifluoromethyl)imidazo[1,2-*b*]pyridazin-8-amine | ¹H NMR (400 MHz, DMSO-d₆) δ 12.62 (s, 1H), 7.84-7.77 (m, 4H), 5.95 (s, 1H), 4.85 (d, 2H), 3.53-3.50 (m, 4H), 3.32-3.30 (m, 2H), 2.40-2.38 (m, 1H), 1.98 (s, 3H), 1.45 (d, 1H); MS: m/z = 511.1 (M+1, ESI+). |
| 99 | 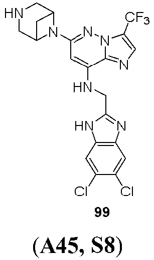 | 6-(3,6-diazabicyclo[3.1.1]heptan-6-yl)-*N*-((5,6-dichloro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 7.85-7.76 (m, 2H), 7.68 (d, 2H), 5.79 (d, 1H), 4.72 (dd, 2H), 4.28 (d, 1H), 4.13 (d, 1H), 3.87-3.83 (m, 1H), 3.24-3.12 (m, 2H), 2.67-2.55 (m, 2H), 1.68-1.48 (m, 1H); MS: m/z = 496.9 (M+1, ESI+). |
| 100 | 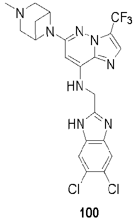 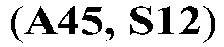 | *N*-((5,6-dichloro-1*H-*benzo[*d*]imidazol-2-yl)methyl)-6-(3-methyl-3,6-diazabicyclo[3.1.1]heptan-6-yl)-3-(trifluoromethyl)imidazo[1,2-*b*]pyridazin-8-amine | ¹H NMR (400 MHz, DMSO-d₆) δ 12.58 (s, 1H), 7.90 (t, 1H), 7.85 (s, 1H), 7.77 (s, 2H), 5.55 (s, 1H), 4.77 (d, 2H), 4.14 (d, 2H), 2.77 (d, 2H), 2.51-2.48 (m, 2H), 2.40 (dd, 1H), 1.94 (s, 3H), |
| 101 | 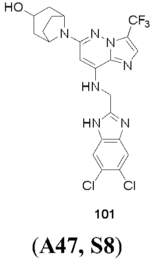 | 8-(8-(((5,6-dichloro-1*H-*benzo[*d*]imidazol-2-yl)methyl)amino)-3-(trifluoromethyl)imidazo[1,2-*b*]pyridazin-6-yl)-8-azabicyclo[3.2.1]octan-3-ol | MS: m/z = 525.9 (M+1, ESI+). |
| 102 | 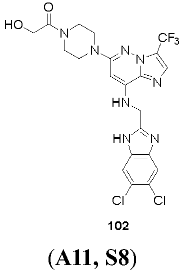 | 1-(4-(8-(((5,6-dichloro-1*H-*benzo[*d*]imidazol-2-yl)methyl)amino)-3-(trifluoromethyl)imidazo[1,2-*b*]pyridazin-6-yl)piperazin-1-yl)-2-hydroxyethan-1-one | ¹H NMR (400 MHz, DMSO-d₆) δ 12.63 (s, 1H), 7.88-7.86 (m, 2H), 7.76 (s, 2H), 6.21 (s, 1H), 4.84 (d, 2H), 4.56 (s, 1H), 4.11 (s, 2H), 3.54 (s, 2H), 3.50-3.41 (m, 6H); MS: m/z = 543.0 (M+1, ESI+). |
| 103 | 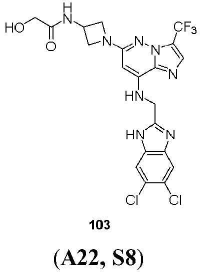 | *N*-(1-(8-(((5,6-dichloro-1*H-*benzo[*d*]imidazol-2-yl)methyl)amino)-3-(trifluoromethyl)imidazo[1,2-*b*]pyridazin-6-yl)azetidin-3-yl)-2-hydroxyacetamide | ¹H NMR (400 MHz, DMSO-d₆) δ 8.41 (d, 1H), 7.87-7.84 (m, 2H), 7.76 (s, 2H), 5.62 (s, 1H), 5.42 (t, 1H), 4.78 (d, 2H), 4.64-4.58 (m, 1H), 4.11 (t, 2H), 3.85-3.76 (m, 4H); MS: m/z = 529.0 (M+1, ESI+). |
| 104 | 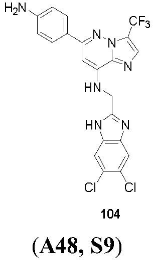 | 6-(4-aminophenyl)-*N*-((5,6-dichloro-1*H*-benzo[*d*]imidazol-2-yl)methyl)-3-(trifluoromethyl)imidazo[1,2-*b*]pyridazin-8-amine | ¹H NMR (400 MHz, DMSO-d₆) δ 12.63 (s, 1H), 8.12 (t, 1H), 8.05 (s, 1H), 7.77 (br s, 2H), 7.64 (d, 2H), 6.74 (s, 1H), 6.62 (d, 2H), 5.56 (s, 2H), 4.96 (d, 2H); MS: m/z = 492.0 (M+1, ESI+). |
| 105 | 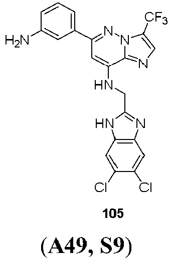 | 6-(3-aminophenyl)-*N*-((5,6-dichloro-1*H*-benzo[d]imidazol-2-yl)methyl)-3-(trifluoromethyl)imidazo[1,2-*b*]pyridazin-8-amine | ¹H NMR (400 MHz, DMSO-d₆) δ 12.63 (s, 1H), 8.28 (t, 1H), 8.12 (s, 1H), 7.77 (br s, 2H), 7.14-7.09 (m, 2H), 6.98 (d, 1H), 6.73 (s, 1H), 6.68 (dd, 1H), 5.28 (s, 2H), 4.96 (d, 2H); MS: m/z = 492.1 (M+1, ESI+). |
| 106 | 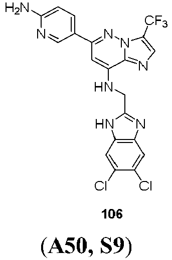 | 6-(6-aminopyridin-3-yl)-*N*-((5,6-dichloro-1*H*-benzo[*d*]imidazol-2-yl)methyl)-3-(trifluoromethyl)imidazo[1,2-*b*]pyridazin-8-amine | ¹H NMR (400 MHz, DMSO-d₆) δ 12.64 (s, 1H), 8.50 (d, 1H), 8.22 (t, 1H), 8.08 (s, 1H), 7.92 (dd, 1H), 7.76 (s, 2H), 6.81 (s, 1H), 6.53 (d, 1H), 6.42 (s, 2H), 4.97 (d, 2H); MS: m/z = 493.0 (M+1, ESI+). |
| 107 | 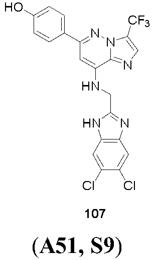 | 4-(8-(((5,6-dichloro-1*H-*benzo[*d*]imidazol-2-yl)methyl)amino)-3-(trifluoromethyl)imidazo[1,2-*b*]pyridazin-6-yl)phenol | ¹H NMR (400 MHz, DMSO-d₆) δ 12.63 (s, 1H), 9.88 (s, 1H), 8.23 (t, 1H), 8.09 (s, 1H), 7.78 (d, 4H), 6.87 (d, 2H), 6.81 (s, 1H), 4.97 (d, 2H); MS: m/z = 493.0 (M+1, ESI+). |
| 108 | 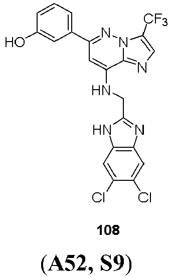 | 3-(8-(((5,6-dichloro-1*H-*benzo[*d*]imidazol-2-yl)methyl)amino)-3-(trifluoromethyl)imidazo[1,2-*b*]pyridazin-6-yl)phenol | ¹H NMR (400 MHz, DMSO-d₆) δ 12.65 (s, 1H), 9.68 (s, 1H), 8.33 (t, 1H), 8.14 (s, 1H), 7.77 (s, 2H), 7.35-7.27 (m, 3H), 6.90-6.86 (m, 1H), 6.82 (s, 1H), 4.99 (d, 2H); MS: m/z = 493.0 (M+1, ESI+). |

### Examples 109 to 113

The compounds of Examples 109 to 113 were prepared in a similar method to Example 30 by selecting and using appropriate intermediates corresponding to the structure of the target compound among the products obtained during the processes for preparing the compounds of the above Examples, along with Intermediate B5, and appropriately changing the amounts of reagents, catalysts, and reaction conditions.

**[Table 7]**

| Example | Structure (Intermediate) | IUPAC Name | NMR and MS Data |
|---|---|---|---|
| 109 | 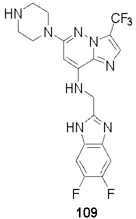 (Product of Step 1 of Example 105) | *N*-((5,6-difluoro-1*H-*benzo[*d*]imidazol-2-yl)methyl)-6-(piperazin-1-yl)-3-(trifluoromethyl)imidazo*[*1,2-blpyridazin-8-amine | ¹H NMR (400 MHz, DMSO-d₆) δ 7.85 (s, 1H), 7.77 (t, 1H), 7.54 (t, 2H), 6.12 (s, 1H), 4.79 (d, 2H), 3.27 (t, 4H), 2.72 (t, 4H); MS: m/z = 453.0 (M+1, ESI+). |
| 110 | 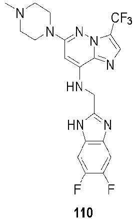 (Product of Step 4 of Example 8) | *N*-((5,6-difluoro-1*H-*benzo[*d*]imidazol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 12.48 (s, 1H), 7.86 (s, 1H), 7.79 (t, 1H), 7.54 (s, 2H), 6.16 (s, 1H), 4.81 (d, 2H), 3.36 (t, 4H), 2.35 (t, 4H), 2.18 (s, 3H); MS: m/z = 467.1 (M+1, ESI+). |
| 111 | 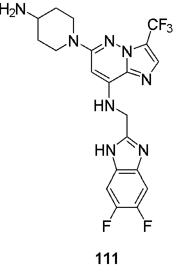 (Product of Step 4 of Example 18) | 6-(4-aminopiperidin-1-yl)-*N-*((5,6-difluoro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 7.84 (s, 1H), 7.74 (t, 1H), 7.54 (t, 2H), 6.13 (s, 1H), 4.79 (d, 2H), 3.93 (d, 2H), 2.88-2.81 (m, 2H), 2.77-2.67 (m, 1H), 1.71-1.67 (m, 2H), 1.20-1.10 (m, 2H); MS: m/z = 467.1 (M+1, ESI+). |
| 112 | 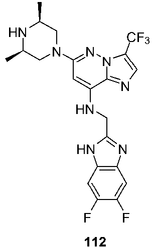 (Product of Step 2 of Example 39) | *N*-((5,6-difluoro-1*H-*benzo[*d*]imidazol-2-yl)methyl)-6-((3*S,* 5*R*)-3,5-dimethylpiperazin-1-yl)-3-(trifluoromethyl)imidazo[1,2-*b*]pyridazin-8-amine | ¹H NMR (400 MHz, DMSO-d₆) δ 12.52 (s, 1H), 7.84 (s, 1H), 7.77 (t, 1H), 7.54 (s, 2H), 6.09 (s, 1H), 4.80 (d, 2H), 3.84 (d, 2H), 2.68-2.61 (m, 2H), 2.20 (t, 2H), 0.95 (d, 6H); MS: m/z = 481.2 (M+1, ESI+). |
| 113 | 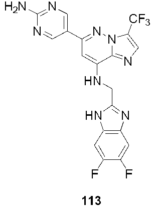 (Product of Step 2 of Example 40) | 6-(2-aminopyrimidin-5-yl)-*N-*((5,6-difluoro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 8.79 (s, 2H), 8.31-8.27 (m, 1H), 8.10 (s, 1H), 7.52 (t, 2H), 7.16 (s, 2H), 6.88 (s, 1H), 4.94 (s, 2H); MS: m/z = 462.1 (M+1, ESI+). |

### Example 114: Preparation of 1-(1-(8-(((5,6-dichloro-1H-benzo[d]imidazol-2-yl)methyl)amino)-3-(trifluoromethyl)imidazo[1,2-b]pyridazin-6-yl)piperidin-4-yl)-2-hydroxyethan-1-one

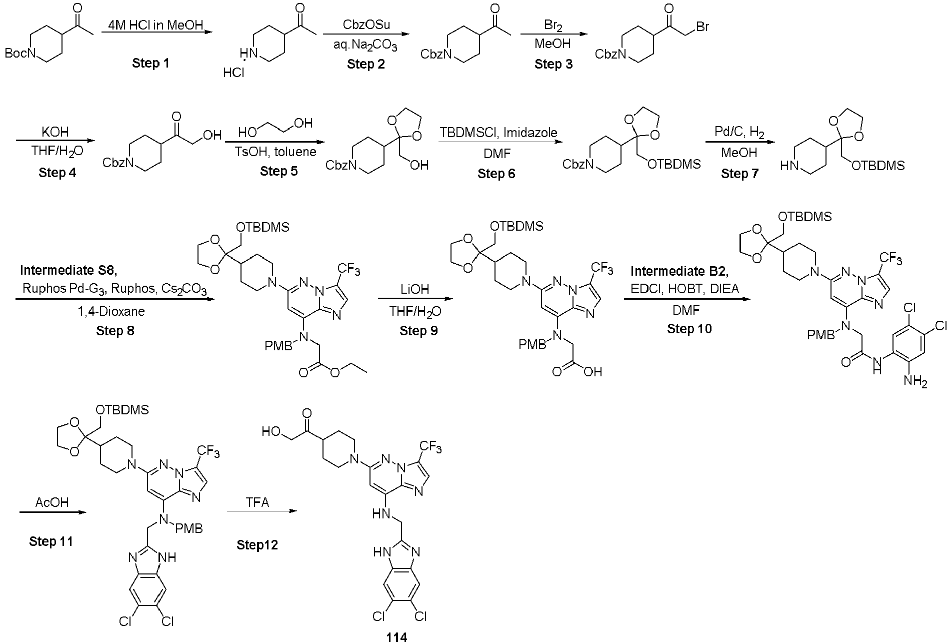

### Step 1: Preparation of 1-(piperidin-4-yl)ethan-1-one hydrochloride

To *tert*-butyl 4-acetylpiperidine-1-carboxylate (50 g, 219.97 mmol) was added 4 M hydrochloric acid solution (219.97 mmol, 200 mL), and the mixture was stirred at 25 °C for 16 hours. The reaction mixture was concentrated to obtain 1-(piperidin-4-yl)ethan-1-one hydrochloride (70 g, crude) as a light yellow solid. MS: m/z = 128.1 (M+1, ESI+).

### Step 2: Preparation of benzyl 4-acetylpiperidine-1-carboxylate

The product obtained in Step 1 (70 g, 428 mmol) was dissolved in aqueous Na₂CO₃ solution (500 mL), and then CbzOSu (170.57 g, 684 mmol) was added thereto, followed by stirring at 25 °C for 16 hours. The reaction mixture was filtered, and the resulting solid was dried to obtain benzyl 4-acetylpiperidine-1-carboxylate (55 g, 49.20 % yield) as a light yellow solid. MS: m/z = 262.0 (M+1, ESI+).

### Step 3: Preparation of benzyl 4-(2-bromoacetyl)piperidine-1-carboxylate

The product obtained in Step 2 (55 g, 210.47 mmol) was dissolved in methanol (500 mL), and then bromine (Br₂, 10.8 mL, 210.47 mmol) was slowly added thereto at 0 °C, followed by stirring at 25 °C for 16 hours. Water (2000 mL) was added to the reaction mixture, and the product was extracted three times with EtOAc (500 mL). The organic layer was washed three times with brine (2000 mL), dried over Na₂SO₄, filtered, and concentrated. The concentrate was purified by silica gel chromatography to obtain benzyl 4-(2-bromoacetyl)piperidine-1-carboxylate (30 g, 41.90 % yield) as a light yellow solid. MS: m/z = 340.0 (M+1, ESI+).

### Step 4: Preparation of benzyl 4-(2-hydroxyacetyl)piperidine-1-carboxylate

The product obtained in Step 3 (30 g, 88.18 mmol) was dissolved in THF (80 mL), and then potassium hydroxide (KOH, 9.90 g, 176.36 mmol) was added thereto, followed by stirring at 25 °C for 1 hour. Water (600 mL) was added to the reaction mixture, and the product was extracted three times with EtOAc (200 mL). The organic layer was washed three times with brine (600 mL), dried over Na₂SO₄, filtered, and concentrated. The concentrate was purified by silica gel chromatography to obtain benzyl 4-(2-hydroxyacetyl)piperidine-1-carboxylate (12 g, 49.07 % yield) as a light yellow solid. MS: m/z = 278.0 (M+1, ESI+).

### Step 5: Preparation of benzyl 4-(2-(hydroxymethyl)-1,3-dioxolan-2-yl)piperidine-1-carboxylate

The product obtained in Step 4 (12 g, 43.32 mmol) was dissolved in toluene (150 mL), and then TsOH (225 mg, 4.33 mmol) and ethane-1,2-diol (13.42 g, 216.6 mmol) were added thereto, followed by stirring at 120 °C for 48 hours. After the reaction mixture was cooled, water (800 mL) was added thereto, and the product was extracted three times with EtOAc (200 mL). The organic layer was washed three times with brine (800 mL), dried over Na₂SO₄, filtered, and concentrated. The concentrate was purified by silica gel chromatography to obtain benzyl 4-(2-(hydroxymethyl)-1,3-dioxolan-2-yl)piperidine-1-carboxylate (8.8 g, 63.31 % yield) as a light yellow solid. MS: m/z = 321.9 (M+1, ESI+).

### Step 6: Preparation of benzyl 4-(2-(((tert-butyldimethylsilyl)oxy)methyl)-1,3-dioxolan-2-yl)piperidine-1-carboxylate

The product obtained in Step 5 (8.8 g, 33.72 mmol) was dissolved in DMF (100 mL), and then imidazole (4.6 g, 67.44 mmol) and TBDMSCl (8.8 g, 67.44 mmol) were added thereto, followed by stirring at 25 °C for 16 hours. Water (1000 mL) was added to the reaction mixture, and the product was extracted three times with EtOAc (300 mL). The organic extract was washed three times with brine (1000 mL), dried over Na₂SO₄, filtered, and concentrated. The concentrate was purified by silica gel chromatography to obtain benzyl 4-(2-(((*tert*butyldimethylsilyl)oxy)methyl)-1,3-dioxolan-2-yl)piperidine-1-carboxylate (8.4 g, 57.53 % yield) as a light yellow solid. MS: m/z = 436.1 (M+1, ESI+).

### Step 7: Preparation of 4-(2-(((tert-butyldimethylsilyl)oxy)methyl)-1,3-dioxolan-2-yl)piperidine

The product obtained in Step 6 (8.4 g, 19.31 mmol) was dissolved in methanol (100 mL), and then Pd/C (1.16 g, 9.66 mmol) was added thereto, followed by stirring at 25 °C under H₂ atmosphere for 2 hours. The reaction mixture was filtered and concentrated to obtain 4-(2-(((*tert*butyldimethylsilyl)oxy)methyl)-1,3-dioxolan-2-yl)piperidine (5.26 g, 90.53 % yield) as a light yellow solid. MS: m/z = 302.1 (M+1, ESI+).

### Steps 8 to 12: Preparation of 1-(1-(8-(((5,6-dichloro-1H-benzo[d]imidazol-2-yl)methyl)amino)-3-(trifluoromethyl)imidazo[1,2-b]pyridazin-6-yl)piperidin-4-yl)-2-hydroxyethan-1-one

**The compound of Example 114** (70 mg, 30.3 % yield) was obtained as a yellow solid in the same method as in Steps 1 to 5 of Example 30. However, in Step 8, the product obtained in Step 7 (5.26 g, 17.48 mmol) was used and the mixture was stirred for 96 hours. ¹H NMR (400 MHz, DMSO-d₆) δ 12.58 (s, 1H), 7.86 (s, 1H), 7.82-7.76 (m, 3H), 6.15 (s, 1H), 5.01 (t, 1H), 4.83 (d, 2H), 4.16 (d, 2H), 4.04 (d, 2H), 2.85 (t, 2H), 2.78-2.72 (m, 1H), 1.74 (d, 2H), 1.45-1.35 (m, 2H); MS: m/z = 542.2 (M+1, ESI+).

### Example 115: Preparation of N-(1-(8-(((5,6-dichloro-1H-benzo[d]imidazol-2-yl)methyl)amino)-3-(trifluoromethyl)imidazo[1,2-b]pyridazin-6-yl)piperidin-4-yl)-2-hydroxyacetamide

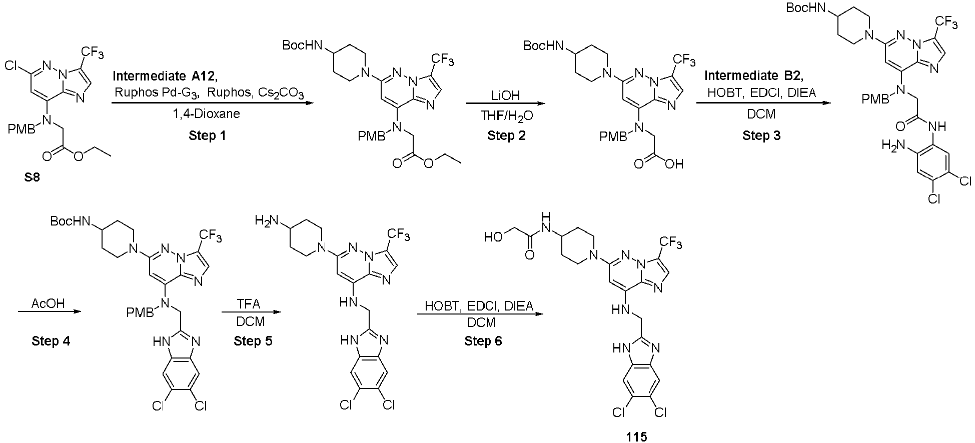

### Steps 1 to 5: Preparation of 6-(4-aminopiperidin-1-yl)-N-((5,6-dichloro-1H-benzo[d]imidazol-2-yl)methyl)-3-(trifluoromethyl)imidazo[1,2-b]pyridazin-8-amine

6-(4-aminopiperidin-1-yl)-*N*-((5,6-dichloro-1*H*-benzo[*d*]imidazol-2-yl)methyl)-3-(trifluoromethyl)imidazo[1,2-*b*]pyridazin-8-amine (150 mg, 71.43 % yield) was obtained as a yellow solid in the same method as in Steps 1 to 5 of Example 30. However, in Step 1 of Example 30, **Intermediate A12** (4.61 g, 23.07 mmol) was used and the mixture was stirred for 16 hours; in Step 2, the reaction temperature was changed to 50 °C with stirring for 2 hours; and in Step 4, the reaction conditions were changed to stirring at 70 °C for 4 hours. MS: m/z = 499.0 (M+1, ESI+).

### Step 6: Preparation of N-(1-(8-(((5,6-dichloro-1H-benzo[d]imidazol-2-yl)methyl)amino)-3-(trifluoromethyl)imidazo[1,2-b]pyridazin-6-yl)piperidin-4-yl)-2-hydroxyacetamide

The product obtained in Step 5 (300 mg, 595 umol) and 2-hydroxyacetamide (46 mg, 0.26 mmol) were dissolved in DCM (10 mL), and then HOBT (176 mg, 1.3 mmol), EDCI (248 mg, 1.3 mmol), and DIEA (336 mg, 2.6 mmol, 0.45 mL) were added thereto, followed by stirring at 25 °C for 16 hours. Water (80 mL) was added to the reaction mixture, and the product was extracted three times with DCM (30 mL). The organic layer was washed three times with brine (80 mL), dried over Na₂SO₄, filtered, and concentrated. The concentrate was purified by silica gel chromatography to obtain **the compound of Example 115** (32 mg, 22.22 % yield) as a white solid. ¹H NMR (400 MHz, DMSO-d₆) δ 12.60 (s, 1H), 7.85 (d, 1H), 7.82-7.71 (m, 3H), 7.59 (d, 1H), 6.18 (s, 1H), 5.36 (t, 1H), 4.83 (d, 2H), 4.02 (d, 2H), 3.88-3.80 (m, 1H), 3.77 (d, 2H), 2.88 (t, 2H), 1.70 (d, 2H), 1.52-1.42 (m, 2H); MS: m/z = 557.0 (M+1, ESI+).

### Example 116: Preparation of N-((1H-indol-2-yl)methyl)-6-(4-methylpiperazin-1-yl)-3-(trifluoromethyl)imidazo[1,2-b]pyridazin-8-amine

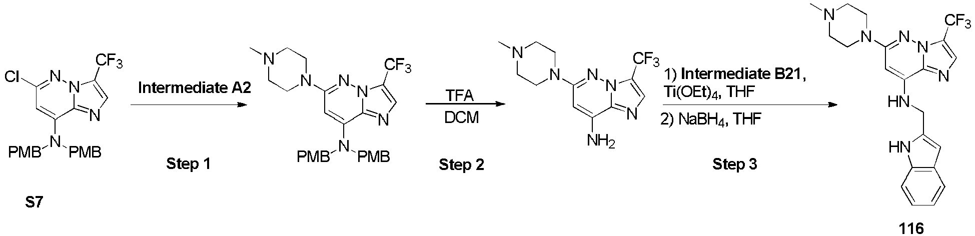

### Step 1: Preparation of N,N-bis(4-methoxybenzyl)-6-(4-methylpiperazin-1-yl)-3-(trifluoromethyl)imidazo[1,2-b]pyridazin-8-amine

**Intermediate S7** (6 g, 12.58 mmol) was dissolved in **Intermediate A2** (15 mL), and then the mixture was stirred at 120 °C for 16 hours. Water (250 mL) was added to the reaction mixture, and the product was extracted three times with EtOAc (50 mL). The organic layer was washed three times with brine (200 mL), dried over Na₂SO₄, filtered, and concentrated, and then the concentrate was purified by silica gel chromatography to obtain *N,N*-bis(4-methoxybenzyl)-6-(4-methylpiperazin-1-yl)-3-(trifluoromethyl)imidazo[1,2-*b*]pyridazin-8-amine (6 g, 88.22 % yield). MS: m/z = 541.2 (M+1, ESI+).

### Step 2: Preparation of 6-(4-methylpiperazin-1-yl)-3-(trifluoromethyl)imidazo[1,2-b]pyridazin-8-amine

The product obtained in Step 1 (2 g, 3.7 mmol) was dissolved in DCM (15 mL), and then TFA (5 mL) was added thereto, followed by stirring at 25 °C for 16 hours. The reaction mixture was concentrated, adjusted to pH 8-9 using sodium bicarbonate solution, and extracted three times with DCM (50 mL). The organic layer was washed three times with brine (200 mL), dried over Na₂SO₄, filtered, and concentrated, and then the concentrate was purified by silica gel chromatography to obtain 6-(4-methylpiperazin-1-yl)-3-(trifluoromethyl)imidazo[1,2-*b*]pyridazin-8-amine (750 mg, 67.57 % yield) as a yellow solid. MS: m/z = 301.1 (M+1, ESI+).

### Step 3: Preparation of N-((1H-indol-2-yl)methyl)-6-(4-methylpiperazin-1-yl)-3-(trifluoromethyl)imidazo[1,2-b]pyridazin-8-amine

The product obtained in Step 2 (600 mg, 2 mmol) and **Intermediate B21** (580.64 mg, 4 mmol) were dissolved in THF (10 mL), and then Ti(OEt)₄ (1.7 g, 6 mmol) was added thereto, followed by stirring at 90 °C for 16 hours. Thereafter, the reaction mixture was cooled to 0 °C, and NaBH₄ (302.64 mg, 8 mmol) was added thereto, followed by stirring at 0 °C for 1 hour. Water (150 mL) was added to the reaction mixture, and the product was extracted three times with EtOAc (50 mL). The organic layer was washed three times with brine (400 mL), dried over Na₂SO₄, filtered, and concentrated, and then the concentrate was purified by Prep-HPLC to obtain **the compound of Example 116** (60 mg, 5.55 % yield) as a white solid. ¹H NMR (400 MHz, DMSO-d₆) δ 10.97 (s, 1H), 7.84 (s, 1H), 7.64 (t, 1H), 7.44 (d, 1H), 7.33 (d, 1H), 7.04-7.00 (m, 1H), 6.96-6.92 (m, 1H), 6.36 (s, 1H), 6.15 (s, 1H), 4.71 (d, 2H), 3.37 (t, 4H), 2.36 (t, 4H), 2.18 (s, 3H); MS: m/z = 430.2 (M+1, ESI+).

### Example 117 to 125

The compounds of Examples 117 to 125 were prepared in a similar method to Example 116 by using intermediates corresponding to the structure of the target compound among the Intermediate B series, and appropriately changing the amounts of reagents, catalysts, and reaction conditions.

**[Table 8]**

| Example | Structure (Intermediate) | IUPAC Name | NMR and MS Data |
|---|---|---|---|
| 117 | 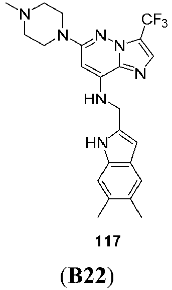 | *N*-((5,6-dimethyl-1*H*-indol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 10.68 (s, 1H), 7.83 (s, 1H), 7.57 (t, 1H), 7.18 (s, 1H), 7.09 (s, 1H), 6.22 (s, 1H), 6.13 (s, 1H), 4.66 (d, 2H), 3.37 (t, 4H), 2.36 (t, 4H), 2.25 (s, 3H), 2.22 (s, 3H), 2.18 (s, 3H); MS: m/z = 458.3 (M+1, ESI+). |
| 118 | 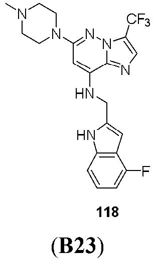 | *N*-((4-fluoro-1*H*-indol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 11.30 (s, 1H), 7.84 (s, 1H), 7.67 (t, 1H), 7.18 (d, 1H), 7.03-6.97 (m, 1H), 6.72 (dd, 1H), 6.44 (s, 1H), 6.16 (s, 1H), 4.73 (d, 2H), 3.40-3.32 (m, 4H), 2.39-2.32 (m, 4H), 2.18 (s, 3H); MS: m/z = 448.3 (M+1, ESI+). |
| 119 | 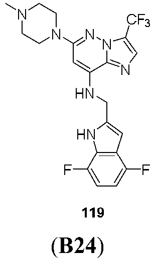 | *N*-((4,7-difluoro-1*H*-indol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 11.87 (s, 1H), 7.84 (s, 1H), 7.62 (t, 1H), 6.89-6.84 (m, 1H), 6.73-6.67 (m, 1H), 6.50 (d, 1H), 6.19 (s, 1H), 4.73 (d, 2H), 3.40 (t, 4H), 2.37 (t, 4H), 2.18 (s, 3H); MS: m/z = 466.3 (M+1, ESI+). |
| 120 | 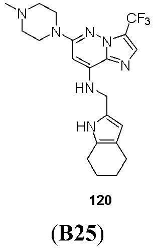 | 6-(4-methylpiperazin-1-yl)-*N-*((4,5,6,7-tetrahydro-1*H*-indol-2-yl)methyl)-3-(trifluoromethyl)imidazo[1,2-*b*]pyridazin-8-amine | ¹H NMR (400 MHz, DMSO-d₆) δ 10.14 (s, 1H), 7.80 (s, 1H), 7.17 (t, 1H), 6.10 (s, 1H), 5.72 (d, 1H), 4.37 (d, 2H), 3.41 (t, 4H), 2.45 (t, 2H), 2.39 (t, 4H), 2.33 (t, 2H), 2.21 (s, 3H), 1.69-1.58 (m, 4H); MS: m/z = 434.2 (M+1, ESI+). |
| 121 | 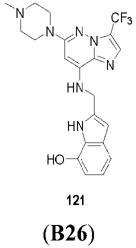 | 2-(((6-(4-methylpiperazin-1-yl)-3-(trifluoromethyl)imidazo[1,2-*b*]pyridazin-8-yl)amino)methyl)-1*H*-indol-7-ol | ¹H NMR (400 MHz, DMSO-d₆) δ 10.74 (s, 1H), 9.48 (s, 1H), 7.77 (s, 1H), 7.58 (t, 1H), 7.32 (d, 1H), 7.20 (d, 1H), 6.77 (t, 1H), 6.48 (d, 1H), 6.12 (s, 1H), 4.63 (d, 2H), 3.36-3.34 (m, 4H), 2.37-2.32 (m, 4H), 2.07 (s, 3H); MS: m/z = 446.2 (M+1, ESI+). |
| 122 | 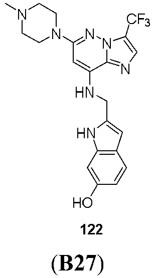 | 2-(((6-(4-methylpiperazin-1-yl)-3-(trifluoromethyl)imidazo[1,2-*b*]pyridazin-8-yl)amino)methyl)-1*H*-indol-6-ol | ¹H NMR (400 MHz, DMSO-d₆) δ 10.52 (s, 1H), 8.87 (s, 1H), 7.77 (s, 1H), 7.56-7.50 (m, 2H), 7.21 (d, 1H), 6.69 (d, 1H), 6.50 (dd, 1H), 6.12 (s, 1H), 4.60 (d, 2H), 3.37 (t, 4H), 2.39 (s, 4H), 2.21 (s, 3H); MS: m/z = 446.2 (M+1, ESI+). |
| 123 | 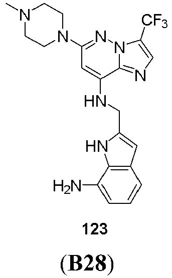 | *N*-((7-amino-1*H*-indol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 10.51 (s, 1H), 7.77 (s, 1H), 7.52 (t, 1H), 7.33 (d, 1H), 6.99 (d, 1H), 6.72 (t, 1H), 6.31 (d, 1H), 6.10 (s, 1H), 4.99 (s, 2H), 4.61 (d, 2H), 3.36 (t, 4H), 2.37 (t, 4H), 2.19 (s, 3H); MS: m/z = 445.2 (M+1, ESI+). |
| 124 | 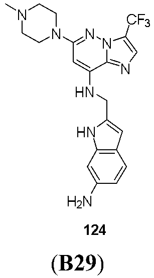 | *N*-((6-amino-1*H*-indol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 10.32 (s, 1H), 7.77 (s, 1H), 7.48 (t, 1H), 7.39 (d, 1H), 7.07 (d, 1H), 6.50 (d, 1H), 6.36 (dd, 1H), 6.12 (s, 1H), 4.72 (br s, 2H), 4.56 (d, 2H), 3.37 (t, 4H), 2.39 (t, 4H), 2.21 (s, 3H); MS: m/z = 445.2 (M+1, ESI+). |
| 125 | 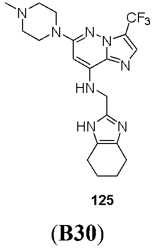 | 6-(4-methylpiperazin-1-yl)-*N-*((4,5,6,7-tetrahydro-1*H-*benzo[*d*]imidazol-2-yl)methyl)-3-(trifluoromethyl)imidazo[1,2-*b*]pyridazin-8-amine | ¹H NMR (400 MHz, DMSO-d₆) δ 11.50 (s, 1H), 7.82 (s, 1H), 7.49 (t, 1H), 6.19 (s, 1H), 4.45 (d, 2H), 3.39 (s, 4H), 2.454-2.334 (m, 8H), 2.20 (s, 3H), 1.69 (s, 4H); MS: m/z =435.2 (M+1, ESI+). |

### Example 126: Preparation of 2-(((6-(4-methylpiperazin-1-yl)-3-(trifluoromethyl)imidazo[1,2-b]pyridazin-8-yl)amino)methyl)-1H-benzo[d]imidazol-7-ol

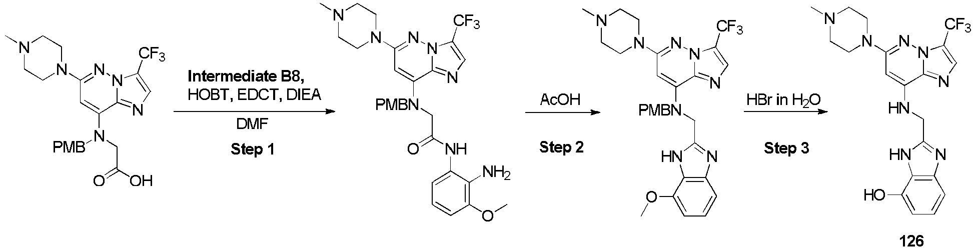

### Steps 1 and 2: Preparation of N-((7-methoxy-1H-benzo[d]imidazol-2-yl)methyl)-N-(4-methoxybenzyl)-6-(4-methylpiperazin-1-yl)-3-(trifluoromethyl)imidazo[1,2-b]pyridazin-8-amine

*N*-((7-methoxy-1*H*-benzo[*d*]imidazol-2-yl)methyl)-*N*-(4-methoxybenzyl)-6-(4-methylpiperazin-1-yl)-3-(trifluoromethyl)imidazo[1,2-*b*]pyridazin-8-amine (410 mg, 82.89 % yield) was obtained as a yellow solid in the same method as in Steps 5 and 6 of Example 8, by using **Intermediate B8** (718.5 mg, 5.2 mmol). However, in Step 1, the reaction time was changed to 16 hours, and in Step 2, the reaction temperature was changed to 70 °C with stirring for 16 hours. MS: m/z = 581.3 (M+1, ESI+).

### Step 3: Preparation of 2-(((6-(4-methylpiperazin-1-yl)-3-(trifluoromethyl)imidazo[1,2-b]pyridazin-8-yl)amino)methyl)-1H-benzo[d]imidazol-7-ol

The product obtained in Step 2 (570 mg, 0.97 mmol) was dissolved in 30 % aqueous hydrobromic acid solution (HBr, 10 mL), and then the mixture was stirred at 100 °C for 16 hours. After the reaction mixture was cooled to room temperature, aqueous sodium bicarbonate solution (100 mL) was added thereto, and the product was extracted three times with EtOAc (30 mL). The organic layer was washed three times with brine (100 mL), dried over Na₂SO₄, filtered, and concentrated. The concentrate was purified by Prep-HPLC to obtain **the compound of Example 126** (65 mg, 20.62 % yield) as a white solid. ¹H NMR (400 MHz, DMSO-d₆) δ 7.85 (s, 1H), 7.72 (s, 1H), 6.92 (t, 2H), 6.51 (t, 1H), 6.18 (s, 1H), 4.77 (s, 2H), 3.36 (t, 4H), 2.35 (t, 4H), 2.17 (s, 3H); MS: m/z = 447.3 (M+1, ESI+).

### Examples 127 to 129

The compounds of Examples 127 to 129 were prepared in a similar method to Example 121, by using Intermediate B from [Table 3] corresponding to the structure of the target compound, and appropriately changing the amounts of reagents, catalysts, and reaction conditions.

**[Table 9]**

| Example | Structure (Intermediate) | IUPAC Name | NMR and MS Data |
|---|---|---|---|
| 127 | 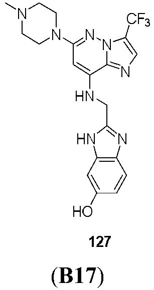 | 2-(((6-(4-methylpiperazin-1-yl)-3-(trifluoromethyl)imidazo[1,2-*b*]pyridazin-8-yl)amino)methyl)-1*H*-benzo[d]imidazol-6-ol | ¹H NMR (400 MHz, DMSO-d₆) δ 11.93 (s, 1H), 9.00 (s, 1H), 7.85 (s, 1H), 7.72 (t, 1H), 7.28 (d, 1H), 6.80 (s, 1H), 6.62 (dd, 1H), 6.16 (s, 1H), 4.72 (d, 2H), 3.36 (t, 4H), 2.35 (t, 4H), 2.18 (s, 3H); MS: m/z = 447.3 (M+1, ESI+). |
| 128 | 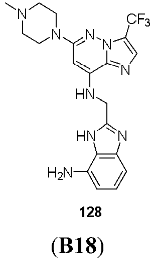 | *N*-((7-amino-1*H-*benzo[*d*]imidazol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 12.00 (s, 1H), 7.85 (s, 1H), 7.75 (s, 1H), 6.83 (t, 1H), 6.60 (d, 1H), 6.31 (d, 1H), 6.20 (s, 1H), 5.13 (s, 2H), 4.75 (s, 2H), 3.34 (t, 4H), 2.35 (t, 4H), 2.17 (s, 3H); MS: m/z = 446.1 (M+1, ESI+). |
| 129 | 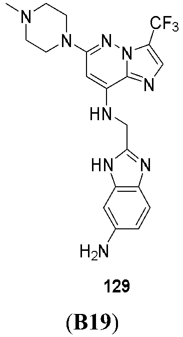 | *N*-((6-amino-1*H-*benzo[*d*]imidazol-2-yl)methyl)-6-(4-methylpiperazin-1-yl)-3-(trifluoromethyl)imidazo[1,2-*b*]pyridazin-8-amine | ¹H NMR (400 MHz, DMSO-d₆) δ 11.72 (s, 1H), 7.85 (s, 1H), 7.68 (t, 1H), 7.17 (s, 1H), 6.58 (s, 1H), 6.46 (d, 1H), 6.17 (s, 1H), 4.81 (s, 2H), 4.68 (d, 2H), 3.36 (t, 4H), 2.36 (t, 4H), 2.18 (s, 3H); MS: m/z = 446.1 (M+1, ESI+). |

### Example 130: Preparation of 6-(5-aminopyridin-2-yl)-N-((5,6-dichloro-1H-benzo[d]imidazol-2-yl)methyl)-3-(trifluoromethyl)imidazo[1,2-b]pyridazin-8-amine

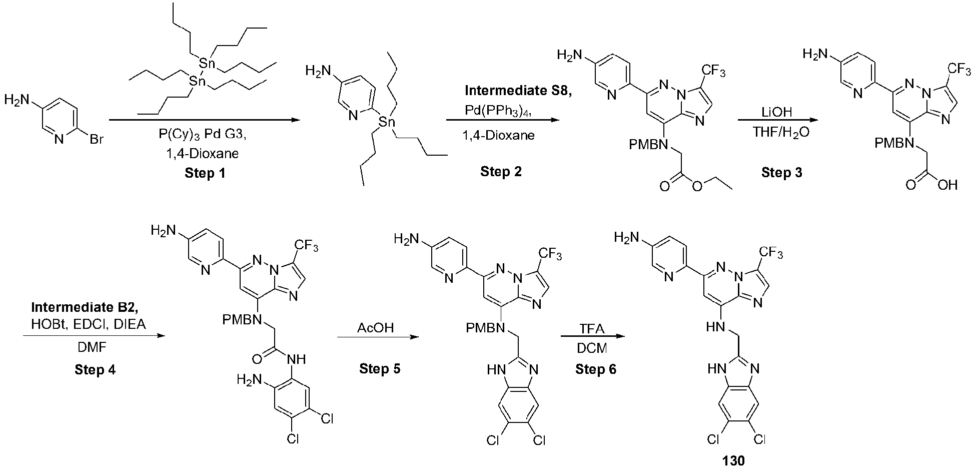

### Step 1: Preparation of 6-(tributylstannyl)pyridin-3-amine

6-Bromopyridin-3-amine (2.2 g, 12.72 mmol) and 1,1,1,2,2,2-hexabutyldistannane (25.83 g, 44.52 mmol) were dissolved in 1,4-dioxane (40 mL), and then P(Cy)₃ Pd G3 (827 mg, 1.27 mmol) was added thereto, followed by stirring at 120 °C for 48 hours under N₂ atmosphere. Water (400 mL) was added to the reaction mixture, and the product was extracted three times with EtOAc (100 mL). The organic layer was washed three times with brine (400 mL), dried over Na₂SO₄, filtered, and concentrated, and then the concentrate was purified by silica gel chromatography to obtain 6-(tributylstannyl)pyridin-3-amine (3.2 g, 65.68 % yield) as a yellow solid. MS: m/z = 384.9 (M+1, ESI+).

### Step 2: Preparation of ethyl N-(6-(5-aminopyridin-2-yl)-3-(trifluoromethyl)imidazo[1,2-b]pyridazin-8-yl)-N-(4-methoxybenzyl)glycinate

The product obtained in Step 1 (3.2 g, 8.36 mmol) and **Intermediate S8** (1.48 g, 3.34 mmol) were dissolved in 1,4-dioxane (20 mL), and then Pd(PPh₃)₄ (386 mg, 0.33 mmol) was added thereto, followed by stirring at 120 °C for 72 hours under N₂ atmosphere charging. Water (400 mL) was added to the reaction mixture, and the product was extracted three times with EtOAc (100 mL). The organic layer was washed three times with brine (400 mL), dried over Na₂SO₄, filtered, and concentrated, and then the concentrate was purified by silica gel chromatography to obtain ethyl *N*-(6-(5-aminopyridin-2-yl)-3-(trifluoromethyl)imidazo[1,2-*b*]pyridazin-8-yl)-N-(4-methoxybenzyl)glycinate (800 mg, 70.78 % yield) as a yellow solid. MS: m/z = 501.3 (M+1, ESI+).

### Steps 3 to 6: Preparation of 6-(5-aminopyridin-2-yl)-N-((5,6-dichloro-1H-benzo[d]imidazol-2-yl)methyl)-3-(trifluoromethyl)imidazo[1,2-b]pyridazin-8-amine

**The compound of Example 130** (33 mg, 18.65 % yield) was obtained as a white solid in the same method as in Steps 2 to 5 of Example 30 using the product of Step 2 (300 mg, 0.6 mmol). However, in Step 3, the reaction time was changed to 16 hours; in Step 4, the reaction solvent was changed to DCM with stirring for 16 hours; and in Step 5, the reaction temperature was changed to 70 °C with stirring for 16 hours. ¹H NMR (400 MHz, DMSO-d₆) δ 12.66 (s, 1H), 8.20 (t, 1H), 8.10 (s, 1H), 7.95 (d, 1H), 7.90 (d, 1H), 7.76 (s, 2H), 7.11 (s, 1H), 7.03 (dd, 1H), 5.82 (s, 2H), 4.92 (d, 2H); MS: m/z = 493.1 (M+1, ESI+).

### Examples 131 to 133

The compounds of Examples 131 to 133 were prepared in a similar manner to Example 130 by using appropriate starting materials corresponding to the structure of the target compound instead of 6-bromopyridin-3-amine, and appropriately changing the amounts of reagents, catalysts, and reaction conditions.

**[Table 10]**

| Example | Structure (Starting Material) | IUPAC Name | NMR and MS Data |
|---|---|---|---|
| 131 | 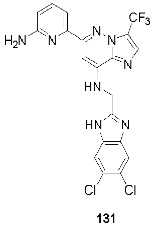 (6-bromopyridin-2-amine) | 6-(6-aminopyridin-2-yl)-*N*-((5,6-dichloro-1*H*-benzo[*d*]imidazol-2-yl)methyl)-3-(trifluoromethyl)imidazo[1,2-*b*]pyridazin-8-amine | ¹H NMR (400 MHz, DMSO-d₆) δ 12.67 (s, 1H), 8.31 (t, 1H), 8.16 (s, 1H), 7.78 (br s, 2H), 7.53 (t, 1H), 7.34 (d, 1H), 7.14 (s, 1H), 6.54 (d, 1H), 6.07 (s, 2H), 4.93 (s, 2H); MS: m/z = 493.1(M+1, ESI+). |
| 132 | 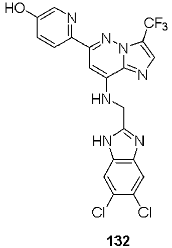 (2-bromo-5-methoxypyridine) | 6-(8-(((5,6-dichloro-1*H-*benzo[*d*]imidazol-2-yl)methyl)amino)-3-(trifluoromethyl)imidazo[1,2-*b*]pyridazin-6-yl)pyridin-3-ol | ¹H NMR (400 MHz, DMSO-d₆) δ 8.31 (t, 1H), 8.18-8.14 (m, 2H), 8.06 (d, 1H), 7.76 (s, 2H), 7.32 (dd, 1H), 7.15 (s, 1H), 4.94 (s, 2H); MS: m/z =494.0(M+1, ESI+). |
| 133 | 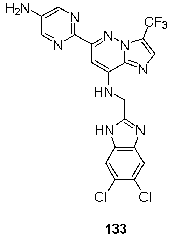 (2-chloropyrimidin-5-amine) | 6-(5-aminopyrimidin-2-yl)-*N-*((5,6-dichloro-1*H-*benzo[*d*]imidazol-2-yl)methyl)-3-(trifluoromethyl)imidazo[1,2-*b*]pyridazin-8-amine | ¹HNMR (400 MHz, DMSO-d₆) δ 12.67 (s, 1H), 8.26 (t, 1H), 8.22 (s, 1H), 8.15 (s, 1H), 7.77 (br s, 2H), 7.10 (s, 1H), 6.01 (s, 2H), 4.94 (s, 2H); MS: m/z = 494.1(M+1, ESI+). |

### Experimental Example 1: CDK12 and CDK13 kinase inhibitory activity assay

Before initiating the CDK12 and CDK13 kinase reactions, each Example compound was dissolved and diluted in DMSO as a solvent across 9 concentration ranges at room temperature. The diluted solutions were then incubated with a mixture of kinase and substrate (and cofactors, if required) for 20 minutes. Next, radioisotope-labeled ATP (³³P-gamma-ATP) was added to proceed with the kinase reaction at room temperature for 120 minutes. After the reaction was completed, the reaction mixture was transferred to a filter paper that binds the radioisotope-labeled catalytic product (³³P-substrate). Unreacted ³³P-ATP was removed by washing with 0.75 % phosphoric acid, and then the radioactive phosphorylated substrate remaining on the filter paper was measured. Kinase activity data were expressed as a percentage of the kinase activity remaining in the test sample compared to a DMSO control group. The half-maximal inhibitory concentration (IC₅₀) for each kinase was calculated using Prism (Graphpad) software.

The CDK12 and CDK13 kinase inhibitory activities of the Example compounds measured by the above method were evaluated according to the following criteria, and the results are presented in Table 11.
A : IC₅₀ ≤ 0.1 µM
B : 0.1 µM < IC₅₀ ≤ 0.5 µM
C : IC₅₀ > 0.5 µM

**[Table 11]**

| Example Number | CDK12 IC₅₀ | CDK13 IC₅₀ | Example Number | CDK12 IC₅₀ | CDK13 IC₅₀ |
|---|---|---|---|---|---|
| SR-4835 | B | B | 55 | A | A |
| 1 | B | A | 56 | A | A |
| 2 | B | B | 57 | A | A |
| 3 | C | B | 58 | C | B |
| 4 | C | B | 59 | B | B |
| 5 | B | A | 60 | B | A |
| 6 | A | A | 61 | B | B |
| 7 | B | A | 62 | B | B |
| 8 | A | A | 63 | B | A |
| 9 | A | A | 64 | B | A |
| 10 | A | A | 65 | A | A |
| 11 | A | A | 66 | A | A |
| 12 | A | A | 67 | A | A |
| 13 | A | A | 68 | B | A |
| 14 | B | A | 69 | A | A |
| 15 | B | A | 70 | A | A |
| 16 | A | A | 71 | A | A |
| 17 | A | A | 72 | A | A |
| 18 | A | A | 73 | A | A |
| 19 | A | A | 74 | A | A |
| 20 | C | B | 75 | A | A |
| 21 | C | C | 76 | A | A |
| 22 | A | A | 77 | A | A |
| 23 | B | A | 78 | A | A |
| 24 | C | B | 79 | A | A |
| 25 | C | C | 80 | A | A |
| 26 | C | C | 81 | C | B |
| 27 | C | C | 82 | A | A |
| 28 | B | B | 83 | A | A |
| 29 | A | A | 84 | A | A |
| 30 | B | A | 85 | A | A |
| 31 | C | B | 86 | A | A |
| 32 | B | B | 87 | A | A |
| 33 | A | A | 88 | A | A |
| 34 | C | B | 89 | A | A |
| 35 | A | A | 91 | C | C |
| 36 | A | A | 92 | A | A |
| 37 | A | A | 93 | C | C |
| 38 | A | A | 94 | A | A |
| 39 | A | A | 95 | A | A |
| 40 | A | A | 96 | B | A |
| 41 | A | A | 97 | A | A |
| 42 | B | A | 98 | A | A |
| 43 | B | B | 99 | B | B |
| 44 | B | B | 100 | A | A |
| 45 | B | A | 101 | B | A |
| 46 | A | A | 102 | A | A |
| 47 | B | A | 103 | A | A |
| 48 | C | B | 109 | A | A |
| 49 | A | A | 110 | A | A |
| 50 | B | B | 111 | A | A |
| 51 | A | A | 112 | A | A |
| 52 | B | A | 113 | A | A |
| 53 | B | B | 114 | A | A |
| | | | 115 | A | A |

As shown in Table 11 above, the Example compounds of the present invention demonstrated superior CDK12 and CDK13 kinase inhibitory activities compared with SR-4835.

### Experimental Example 2: Cellular CDK12 inhibitory activity assay

Cellular CDK12 inhibition by the Example compounds was confirmed by analyzing the Ser2 phosphorylation level in the carboxy-terminal domain (CTD) of RNA polymerase II subunit B1 using MDA-MB-231 breast cancer cells. MDA-MB-231 cells were dispensed into a 96-well plate at 100 µL of cell suspension per well using L-15 (Invitrogen) medium containing 10 % FBS, followed by overnight incubation in a cell incubator at 37 °C and 0 % CO₂. The next day, each Example compound dissolved in DMSO across 9 concentration ranges and a DMSO control group were treated on the MDA-MB-231 cells at 37 °C and 0 % CO₂ for 6 hours. After the compound treatment, the medium in the 96-well plate was removed, and 60 µL of a fixing solution (4.0 % paraformaldehyde in PBS) was added to each well, followed by standing for 20 minutes. After removing the fixing solution and washing with PBS, 60 µL of PBS containing 0.1 % Triton X-100 was dispensed into each well, followed by shaking at room temperature for 15 minutes. After washing with PBS, 100 µL of blocking buffer was added, followed by shaking at room temperature for 1 hour. After removing the blocking buffer, primary antibodies, which are RNA polymerase II subunit B1 phospho-Ser2 (1:500, Cell signaling technology) and RNA polymerase II subunit B1 carboxy-terminal domain (1:200, Cell signaling technology), were added at 60 µL per well and reacted overnight at 4 °C. After adding 150 µL of PBS containing 0.1 % Triton X-100 and washing at least twice by shaking at room temperature for 5 minutes, 60 µL of a secondary antibody was added, followed by shaking at room temperature for 1 hour while blocking light. After adding 150 µL of PBS containing 0.1 % Triton X-100 and washing at least twice by shaking at room temperature for 5 minutes, 150 µL of PBS was added again, followed by washing at least twice by shaking at room temperature for 5 minutes. The plate was scanned using a Li-COR Odyssey machine with the wells filled with PBS. IC₅₀ values were calculated by fitting a dose-response curve using a 4-parameter model of Prism (Graphpad) software.

The cellular CDK12 inhibitory activities of the Example compounds measured by the above method were evaluated according to the following criteria, and the results are presented in Table 12.

IC₅₀ value range
A : IC₅₀ ≤ 0.3 µM
B : 0.3 µM < IC₅₀ ≤ 3 µM
C : IC₅₀ > 3 µM

**[Table 12]**

| Example Number | pRpbl CTD S2 IC₅₀ | Example Number | pRpbl CTD S2 IC₅₀ |
|---|---|---|---|
| SR-4835 | B | 55 | B |
| 1 | B | 56 | B |
| 2 | B | 57 | A |
| 3 | B | 58 | A |
| 4 | C | 59 | A |
| 5 | B | 60 | B |
| 6 | A | 61 | A |
| 7 | A | 62 | A |
| 8 | A | 63 | A |
| 9 | A | 64 | C |
| 10 | A | 65 | A |
| 11 | A | 66 | A |
| 12 | A | 67 | A |
| 13 | A | 68 | A |
| 14 | A | 69 | B |
| 15 | B | 70 | A |
| 16 | B | 71 | A |
| 17 | A | 72 | A |
| 18 | A | 73 | B |
| 19 | B | 74 | B |
| 20 | B | 75 | A |
| 21 | C | 76 | A |
| 22 | A | 77 | A |
| 23 | B | 78 | B |
| 24 | C | 79 | A |
| 25 | C | 80 | A |
| 26 | C | 81 | B |
| 27 | C | 82 | B |
| 28 | B | 83 | B |
| 29 | A | 84 | B |
| 30 | A | 85 | C |
| 31 | B | 86 | B |
| 32 | A | 87 | B |
| 33 | A | 88 | B |
| 34 | A | 89 | C |
| 35 | A | 91 | B |
| 36 | A | 92 | B |
| 37 | A | 93 | B |
| 38 | A | 94 | A |
| 39 | A | 95 | A |
| 40 | A | 96 | A |
| 41 | A | 97 | A |
| 42 | B | 98 | A |
| 43 | B | 99 | A |
| 44 | A | 100 | A |
| 45 | A | 101 | A |
| 46 | A | 102 | A |
| 47 | A | 103 | B |
| 48 | A | 109 | A |
| 49 | A | 110 | A |
| 50 | B | 111 | A |
| 51 | A | 112 | A |
| 52 | A | 113 | A |
| 53 | A | 114 | A |
| 54 | B | 115 | A |

As shown in Table 12 above, the Example compounds of the present invention exhibited superior cellular CDK12 inhibitory activity compared to SR-4835.

### Experimental Example 3: Proliferation inhibitory activity assay against MDA-MB-231 breast cancer cells

Experiments were performed using the MDA-MB-231 breast cancer cell line to measure the cell proliferation inhibitory ability of each Example compound. MDA-MB-231 cells were dispensed into a 96-well plate at 100 µL of cell suspension per well using L-15 (Invitrogen) medium containing 10 % FBS, followed by incubation in a cell incubator at 37 °C and 0 % CO₂. The next day, each Example compound dissolved and diluted in DMSO across 9 concentration ranges and a DMSO control group were treated on the cells for 72 hours. After the compound treatment was completed, 100 µL of CellTiter Glo (Promega) reagent was added to each well of the 96-well plate, followed by shaking for 10 minutes and standing at room temperature for 10 minutes. Luminescence signals were measured after inserting the 96-well plate into an Envision plate reader (PerkinElmer). Relative IC₅₀ values were calculated by fitting a dose-response curve using XLfit (IDBS Ltd) software.

The breast cancer cell proliferation inhibitory activities of the Example compounds measured by the above method were evaluated according to the following criteria, and the results are presented in Table 13.

IC₅₀ value range
A : IC₅₀ ≤ 0.05 µM
B : 0.05 µM < IC₅₀ ≤ 0.5 µM
C : IC₅₀ > 0.5 µM

**[Table 13]**

| Example Number | MDA-MB-231 IC₅₀ | Example Number | MDA-MB-231 IC₅₀ |
|---|---|---|---|
| SR-4835 | B | 55 | B |
| 1 | B | 56 | A |
| 2 | B | 57 | A |
| 3 | B | 58 | A |
| 4 | C | 59 | A |
| 5 | B | 60 | B |
| 6 | A | 61 | A |
| 7 | A | 62 | B |
| 8 | A | 63 | B |
| 9 | A | 64 | C |
| 10 | A | 65 | B |
| 11 | A | 66 | B |
| 12 | A | 67 | B |
| 13 | A | 68 | A |
| 14 | A | 69 | B |
| 15 | A | 70 | A |
| 16 | A | 71 | A |
| 17 | A | 72 | A |
| 18 | A | 73 | A |
| 19 | A | 74 | B |
| 20 | B | 75 | B |
| 21 | C | 76 | A |
| 22 | B | 77 | A |
| 23 | B | 78 | B |
| 24 | C | 79 | A |
| 25 | C | 80 | A |
| 26 | C | 81 | B |
| 27 | C | 82 | B |
| 28 | B | 83 | B |
| 29 | A | 84 | B |
| 30 | A | 85 | C |
| 31 | C | 86 | C |
| 32 | A | 87 | B |
| 33 | A | 88 | B |
| 34 | B | 89 | C |
| 35 | A | 91 | B |
| 36 | A | 92 | B |
| 37 | A | 93 | B |
| 38 | A | 94 | A |
| 39 | A | 95 | A |
| 40 | A | 96 | A |
| 41 | B | 97 | A |
| 42 | B | 98 | A |
| 43 | B | 99 | A |
| 44 | A | 100 | A |
| 45 | A | 101 | A |
| 46 | A | 102 | A |
| 47 | A | 103 | B |
| 48 | B | 109 | A |
| 49 | A | 110 | A |
| 50 | B | 111 | A |
| 51 | A | 112 | A |
| 52 | A | 113 | A |
| 53 | A | 114 | A |
| 54 | B | 115 | A |

As shown in Table 13 above, the Example compounds of the present invention exhibited superior MDA-MB-231 breast cancer cell inhibitory effects compared to SR-4835.

### Experimental Example 4: CDK1 and CDK2 kinase inhibitory activity assay and selectivity analysis over CDK12 kinase

Before initiating the CDK1 and CDK2 kinase reactions, each Example compound was dissolved and diluted in DMSO as a solvent across 9 concentration ranges at room temperature. The diluted solutions were then incubated with a mixture of kinase and substrate (and cofactors, if required) for 20 minutes. Next, radioisotope-labeled ATP (³³P-gamma-ATP) was added to proceed with the kinase reaction at room temperature for 120 minutes. After the reaction was completed, the reaction mixture was transferred to a filter paper that binds the radioisotope-labeled catalytic product (³³P-substrate). Unreacted ³³P-ATP was removed by washing with 0.75 % phosphoric acid, and then the radioactive phosphorylated substrate remaining on the filter paper was measured. Kinase activity data were expressed as a percentage of the kinase activity remaining in the test sample compared to a DMSO control group. The half-maximal inhibitory concentration (IC₅₀) for each kinase was calculated using Prism software.

The CDK1 and CDK2 kinase inhibitory activities of the Example compounds measured by the above method were evaluated according to the following criteria, and the results are presented in Table 14.

IC₅₀ value range
A : IC₅₀ ≤ 0.1 µM
B : 0.1 µM < IC₅₀ ≤ 1 µM
C : IC₅₀ > 1 µM

The selectivity analysis criteria over CDK12 were calculated as the ratio of CDK1 IC₅₀ to CDK12 IC₅₀ or the ratio of CDK2 IC₅₀ to CDK12 IC₅₀, and were evaluated according to the following criteria, with the results summarized in Table 14 below.
A: CDK1/CDK12 or CDK2/CDK12 > 500-fold
B: 100-fold < CDK1/CDK12 or CDK2/CDK12 ≤ 500-fold
C: CDK1/CDK12 or CDK2/CDK12 ≤ 100-fold

**[Table 14]**

| Example Number | CDK1 IC₅₀ | CDK2 IC₅₀ | CDK1/CDK12 | CDK2/CDK12 |
|---|---|---|---|---|
| 7 | C | C | C | C |
| 8 | C | C | A | B |
| 11 | B | B | B | B |
| 13 | C | C | A | B |
| 14 | C | B | C | C |
| 16 | C | C | B | C |
| 17 | C | C | A | B |
| 18 | C | C | A | A |
| 19 | C | C | A | A |
| 22 | C | C | A | A |
| 23 | C | C | - | |
| 29 | C | C | - | |
| 30 | C | C | - | C |
| 35 | C | C | A | B |
| 40 | C | C | - | - |
| 41 | C | C | B | B |
| 42 | C | C | - | - |
| 44 | C | C | - | - |
| 46 | C | C | - | B |
| 68 | C | C | C | C |
| 69 | C | C | B | B |
| 71 | C | C | B | B |
| 72 | C | C | A | A |
| 73 | C | C | A | A |
| 75 | C | C | A | A |
| 76 | C | C | A | A |
| 77 | C | C | A | B |
| 115 | C | C | B | B |

As shown in Table 14 above, the Example compounds of the present invention exhibited excellent selectivity over CDK12.

### Experimental Example 5: Assay for breast cancer cell proliferation inhibitory activity

Experiments were conducted using three types of breast cancer cell lines to measure the cell proliferation inhibitory ability of each Example compound. For HCC70 cells, 100 µL of cell suspension was dispensed into a 96-well plate using RPMI1640 (Invitrogen) medium containing 10 % FBS, followed by incubation in a cell incubator at 37 °C and 5 % CO₂. For BT-20 cells, 100 µL of cell suspension was dispensed into a 96-well plate using EMEM (Invitrogen) medium containing 10 % FBS, followed by incubation in a cell incubator at 37 °C and 5 % CO₂. For MDA-MB-436 cells, 100 µL of cell suspension was dispensed into a 96-well plate using L-15 (Invitrogen) medium containing 10 % FBS, followed by incubation in a cell incubator at 37 °C and 0 % CO₂. The next day, each Example compound dissolved and diluted in DMSO across 9 concentration ranges and a DMSO control group were treated on the cells for 72 hours. After completion of the compound treatment, 100 µL of CellTiter Glo (Promega) reagent was added to each well of the 96-wellplate, followed by shaking for 10 minutes and standing at room temperature for 10 minutes. After inserting the 96-well plate into an Envision plate reader (PerkinElmer), luminescence signals were measured. Relative IC₅₀ values were calculated by fitting a dose-response curve using XL fit (IDBS Ltd) software.

The breast cancer cell proliferation inhibitory activities of the Example compounds measured by the method described above were evaluated according to the following criteria, and the results are presented in Table 15.

IC₅₀ value range
A : IC₅₀ ≤ 0.05 µM
B : 0.05 µM < IC₅₀ ≤ 1 µM
C : IC₅₀ > 1 µM

**[Table 15]**

| Example Number | HCC70 IC₅₀ | BT-20 IC₅₀ | MDA-MB-436 IC₅₀ |
|---|---|---|---|
| Palbociclib | C | C | - |
| Olaparib | C | C | C |
| SR-4835 | B | B | B |
| 8 | A | A | A |
| 29 | A | A | A |
| 30 | A | A | A |

As shown in Table 15 above, Examples 8, 29, and 30 of the present invention exhibited superior breast cancer cell proliferation inhibitory effects compared to the comparative compounds palbociclib, olaparib, and SR-4835.

### Experimental Example 6: Assay for gastric cancer cell proliferation inhibitory activity

Experiments were performed using four types of gastric cancer cell lines (AGS, SNU-1, KATO-III, and MKN45) to measure the cell proliferation inhibitory ability of each Example compound. For AGS cells, 90 µL of cell suspension was dispensed into a 96-well plate using F-12K (Gibco) medium containing 10 % FBS, followed by incubation in a cell incubator at 37 °C and 5 % CO₂. For SNU-1 and MKN45 cells, 90 µL of cell suspension was dispensed into a 96-well plate using RPMI1640 (Gibco) medium containing 10 % FBS, followed by incubation in a cell incubator at 37 °C and 5 % CO₂. For KATO-III cells, 90 uL of cell suspension was dispensed into a 96-well plate using IMDM (Gibco) medium containing 10 % FBS, followed by incubation in a cell incubator at 37 °C and 5 % CO₂. The next day, each Example compound dissolved and diluted in DMSO across 9 concentration ranges and a DMSO control group were added at 10 µL to each 96-well plate and treated for 72 hours. After completion of the compound treatment, 100 µL of CellTiter Glo (Promega) reagent was added to each well of the 96-well plate, followed by shaking for 10 minutes and standing at room temperature for 10 minutes. Luminescence signals were measured after inserting the 96-well plate into an Envision plate reader (PerkinElmer). Relative IC₅₀ values were calculated by fitting a dose-response curve using XLfit (IDBS Ltd) software.

The gastric cancer cell proliferation inhibitory activities of the Example compounds measured by the method described above were evaluated according to the following criteria, and the results are presented in Table 16.

IC₅₀ value range
A : IC₅₀ ≤ 0.2 µM
B : 0.2 µM < IC₅₀ ≤ 1 µM
C : IC₅₀ > 1 µM

**[Table 16]**

| Example Number | AGS IC₅₀ | SNU-1 IC₅₀ | KATO-III IC₅₀ | MKN45 IC₅₀ |
|---|---|---|---|---|
| Cisplatin | C | C | C | C |
| 8 | A | A | A | A |
| 29 | A | A | A | A |
| 30 | A | A | A | A |

As shown in Table 16 above, the Example compounds 8, 29, and 30 of the present invention exhibited superior gastric cancer cell proliferation inhibitory effects compared to Cisplatin, which is a comparative compound.

### Experimental Example 7: Analysis of Cyclin K degradation ability

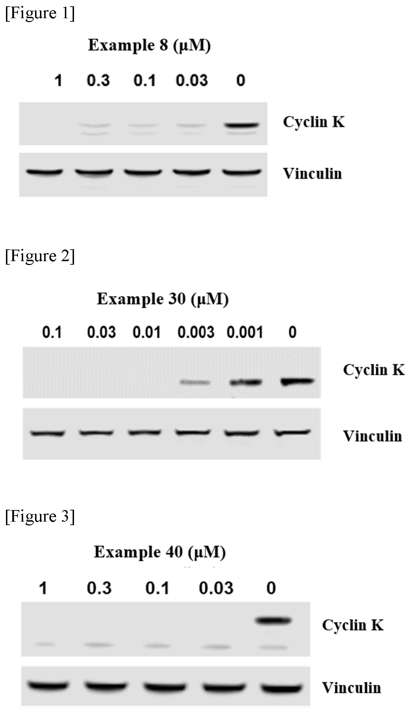

In order to confirm the Cyclin K degradation ability of the Example compounds, HCC70 breast cancer cells and MKN45 gastric cancer cells were cultured in RPMI-1640 medium supplemented with 10 % FBS, and then the cells were collected to prepare cell suspensions. The HCC70 breast cancer cell suspension was dispensed at a cell number of 2.0 X 10⁶ per 6-well and cultured in a 5 % CO₂ cell incubator at 37 °C for 24 hours, and the MKN45 gastric cancer cell suspension was dispensed at a cell number of 5.0 X 10⁶ per 6-well and cultured in a 5 % CO₂ cell incubator at 37 °C for 24 hours. The next day, each Example compound dissolved and diluted in DMSO across 4 to 5 concentration ranges and a negative control group (DMSO-treated group) were added to the 6-wells, and the HCC70 breast cancer cells were treated for 8 hours while the MKN45 gastric cancer cells were treated with the compounds for 8 hours or 24 hours. After completion of the compound treatment, the medium was removed, each cell was washed with PBS, and then 100 µL of RIPA buffer for cell lysis was added to each 6-well to collect cell lysates. The protein concentration of the cell lysates was quantified by the BSA method, and loading buffer was added to adjust the protein concentration to 30 µg, followed by reaction at 85 °C for 10 minutes, centrifugation, and standing at room temperature. After electrophoresis on a 4-12 % Bis-Tris gel, proteins were transferred to a nitrocellulose membrane. Primary antibodies, Cyclin K (1:1000, Bethyl Lab) and Vinculin (1:1000, Sigma), were attached to the membrane, respectively, and then labeled using a secondary antibody (1:10000, LI-COR). Signals were detected using an Odyssey imager (LI-COR Biosciences), and the results are shown in FIGS. 1 to 5.

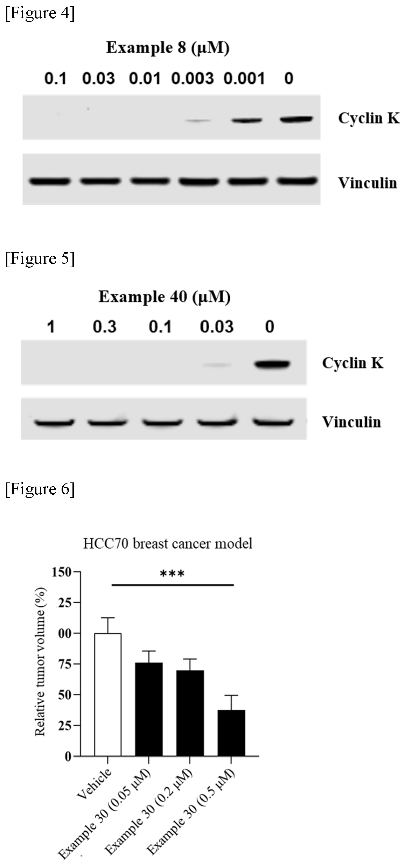

In FIGs. 1 to 3, Examples 8, 30, and 40 were shown to strongly degrade Cyclin K in HCC70 cells compared to the negative control group, and in FIGs. 4 and 5, Examples 8 and 40 were shown to strongly degrade Cyclin K in MKN45 cells compared to the negative control group.

### Experimental Example 8: Zebrafish xenograft breast cancer and gastric cancer models

Transgenic Tg(fli1:EGFP)y1 zebrafish embryos were cultured in E3 embryo medium with 0.2 mM 1-phenyl-2-thiourea (PTU) at 28 °C for 48 hours. HCC70 breast cancer cells were cultured using RPMI-1640 medium supplemented with 10 % FBS, and AGS gastric cancer cells were cultured using F-12K medium supplemented with 10 % FBS. Each cell was collected, labeled with Dil red fluorescent dye, and then subcutaneously transplanted into 2-day-old zebrafish embryos. After selecting embryos with tumors, they were classified into experimental groups (20 embryos per group), and each Example compound dissolved and diluted in DMSO across 2 to 3 concentration ranges, a DMSO control group, or a comparative compound were added, followed by incubation at 35.5 °C for 48 hours or 72 hours. In the breast cancer model, primary tumors were collected immediately after transplantation (Day 0) and after 48 hours (Day 2), and in the gastric cancer model, primary tumors were obtained immediately after transplantation (Day 0) and after 72 hours (Day 3). Images of the collected tumors were analyzed for reduction in tumor size compared to the DMSO control group using image software, and the number of disseminated cancer cells after 3 days was manually counted through the captured images, with the results shown in FIGs. 6, 7, and 8. In the breast cancer model, statistical processing for each Example compound-treated group compared to the vehicle group was performed using a one-way ANOVA test and Dunnett's multiple comparison method (*** p < 0.001). In the gastric cancer model, statistical processing for each Example compound-treated group compared to the vehicle group was performed using the Kruskal-Wallis test and Dunn's multiple comparison method (* p < 0.05). In the gastric cancer model, statistical processing for each Example compound-treated group compared to the 5-FU group, which is a comparative compound, was performed using the Mann-Whitney test (* p < 0.05).

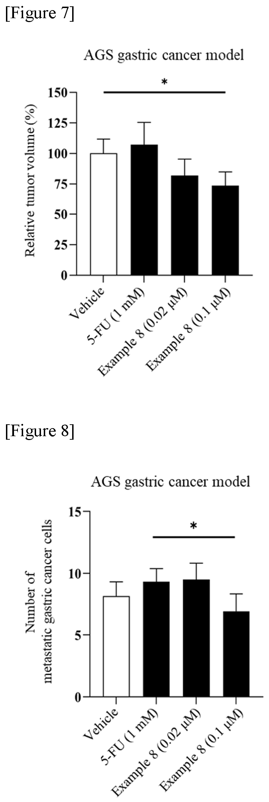

In FIG. 6, the compound of Example 30 exhibited a dose-dependent reduction in tumor size compared to the vehicle in the HCC70 breast cancer model. In FIG. 7, the compound of Example 8 exhibited a dose-dependent reduction in tumor size compared to the vehicle in the AGS gastric cancer model. In FIG. 8, in the AGS gastric cancer model, treatment with 0.1 µM of the compound of Example 8 exhibited a significant reduction in the number of metastatic gastric cancer cells compared to 1 mM of 5-FU, which is a comparative compound.

### Experimental Example 9: Assay for lung cancer cell proliferation inhibitory activity

Experiments were performed using three types of lung cancer cell lines to measure the cell proliferation inhibitory ability of the compounds of the present invention. NCI-H23 and NCI-H358 cells were dispensed into a 96-well plate at 100 µL of cell suspension per well using RPM11640 medium containing 10 % FBS and 1 % PS, followed by incubation in a cell incubator at 37 °C and 5 % CO₂. A427 cells were dispensed into a 96-well plate at 100 µL of cell suspension per well using DMEM medium containing 10 % FBS and 1 % PS, followed by incubation in a cell incubator at 37 °C and 5 % CO₂. The next day, each Example compound (such as Example 8) dissolved and diluted in DMSO across 10 concentration ranges and a DMSO control group were treated on the cells for 72 hours. After completion of the compound treatment, 10 µL of Cell Counting Kit-8 (Dojindo) reagent was added to each well of the 96-well plate, followed by incubation for 2 hours. After inserting the 96-well plate into an Envision plate reader (Thermo Fisher), luminescence signals were measured. Relative IC₅₀ values were calculated by fitting a dose-response curve using Graphpad PRISM software.

The lung cancer cell proliferation inhibitory activities of the Example compounds measured by the method described above were evaluated according to the following criteria, and the results are presented in Table 17.

IC₅₀ value range
A : IC₅₀ ≤ 0.2 µM
B : 0.2 µM < IC₅₀ ≤ 1 µM
C : IC₅₀ > 1 µM

**[Table 17]**

| Example Number | NCI-H23 IC₅₀ | NCI-H358 IC₅₀ | A-427 IC₅₀ |
|---|---|---|---|
| 8 | A | A | A |

As shown in Table 17 above, the compound of Example 8 exhibited an excellent lung cancer cell proliferation inhibitory effect.

### Experimental Example 10: Assay for pancreatic cancer cell proliferation inhibitory activity

Experiments were performed using three types of pancreatic cancer cell lines to measure the cell proliferation inhibitory ability of the compounds of the present invention. AsPC-1, MIA-PaCa2-Control, and Gemcitabine resistant-MIA-PaCa2 cells were dispensed into a 96-well plate at 100 uL of cell suspension per well using DMEM medium containing 10 % FBS and 1 % PS, followed by incubation in a cell incubator at 37 °C and 5 % CO₂. The next day, the compound of Example 8 dissolved and diluted in DMSO across 10 concentration ranges and a DMSO control group were treated on the cells for 72 hours. After completion of the compound treatment, 10 µL of Cell Counting Kit-8 (Dojindo) reagent was added to each well of the 96-well plate, followed by incubation for 2 hours. Luminescence signals were measured after inserting the 96-well plate into an Envision plate reader (Thermo Fisher). Relative IC₅₀ values were calculated by fitting a dose-response curve using Graphpad PRISM software.

The pancreatic cancer cell proliferation inhibitory activities of the Example compounds measured by the method described above were evaluated according to the following criteria, and the results are presented in Table 18.

IC₅₀ value range
A : IC₅₀ ≤ 0.2 µM
B : 0.2 µM < IC₅₀ ≤ 1 µM
C : IC₅₀ > 1 µM

**[Table 18]**

| Example Number | AsPC-1 IC₅₀ | MIA-PaCa2-Control IC₅₀ | Gemcitabine resistant-MIA-PaCa2 IC₅₀ |
|---|---|---|---|
| 8 | A | A | A |

As shown in Table 18 above, the compound of Example 8 exhibited an excellent pancreatic cancer cell proliferation inhibitory effect.

### Experimental Example 11: Assay for colorectal cancer cell proliferation inhibitory activity

Experiments were performed using seven types of colorectal cancer cell lines to measure the cell proliferation inhibitory ability of each Example compound. DLD-1, LoVo, SW620, SW480, HCT116, HCT15, and HT29 cells were dispensed into a 96-well plate at 100 µL of cell suspension per well using RPMI1640 medium containing 10 % FBS and 1 % PS, followed by incubation in a cell incubator at 37 °C and 5 % CO₂. The next day, each Example compound dissolved and diluted in DMSO across 10 concentration ranges and a DMSO control group were treated on the cells for 72 hours. After completion of the compound treatment, 10 µL of Cell Counting Kit-8 (Dojindo) reagent was added to each well of the 96-well plate, followed by incubation for 2 hours. Luminescence signals were measured after inserting the 96-well plate into an Envision plate reader (Thermo Fisher). Relative IC₅₀ values were calculated by fitting a dose-response curve using Graphpad PRISM software.

The colorectal cancer cell proliferation inhibitory activities of the Example compounds measured by the method described above were evaluated according to the following criteria, and the results are presented in Table 19.

IC₅₀ value range
A : IC₅₀ ≤ 0.2 µM
B : 0.2 µM < IC₅₀ ≤ 1 µM
C : IC₅₀ > 1 µM

**[Table 19]**

| Example Number | DLD-1 IC₅₀ | LoVo IC₅₀ | SW620 IC₅₀ | SW480 IC₅₀ | HCT116 IC₅₀ | HCT15 IC₅₀ | HT29 IC₅₀ |
|---|---|---|---|---|---|---|---|
| 8 | A | A | A | A | A | A | A |
| 40 | - | - | - | A | - | A | - |

As shown in Table 19 above, the compounds of Examples 8 and 40 exhibited an excellent colorectal cancer cell proliferation inhibitory effect.

### Experimental Example 12: Assay for gastric cancer cell proliferation inhibitory activity

Experiments were performed using six types of gastric cancer cell lines to measure the cell proliferation inhibitory ability of each Example compound. NCI-N87, SNU601, KATO-III, MKN1, and AGS cells were dispensed into a 96-well plate at 100 µL of cell suspension per well using RPMI1640 medium containing 10 % FBS and 1 % PS, followed by incubation in a cell incubator at 37 °C and 5 % CO₂. HS746T cells were dispensed into a 96-well plate at 100 µL of cell suspension per well using DMEM medium containing 10 % FBS and 1 % PS, followed by incubation in a cell incubator at 37 °C and 5 % CO₂. The next day, each Example compound dissolved and diluted in DMSO across 10 concentration ranges and a DMSO control group were treated on the cells for 72 hours. After completion of the compound treatment, 10 µL of Cell Counting Kit-8 (Dojindo) reagent was added to each well of the 96-well plate, followed by incubation for 2 hours. Luminescence signals were measured after inserting the 96-well plate into an Envision plate reader (Thermo Fisher). Relative IC₅₀ values were calculated by fitting a dose-response curve using Graphpad PRISM software.

The gastric cancer cell proliferation inhibitory activities of the Example compounds measured by the method described above were evaluated according to the following criteria, and the results are presented in Table 20.

IC₅₀ value range
A : IC₅₀ ≤ 0.2 µM
B : 0.2 µM < IC₅₀ ≤ 1 µM
C : IC₅₀ > 1 µM

**[Table 20]**

| Example Number | NCI-N87 IC₅₀ | SNU601 IC₅₀ | KATO-III IC₅₀ | MKN1 IC₅₀ | AGS IC₅₀ | HS746T IC₅₀ |
|---|---|---|---|---|---|---|
| 8 | A | A | A | A | A | A |
| 40 | - | - | - | A | A | - |

As shown in Table 20 above, the compounds of Examples 8 and 40 exhibited excellent gastric cancer cell proliferation inhibitory effects.

## Claims

1. A compound of Formula I below, a stereoisomer, hydrate, solvate, or pharmaceutically acceptable salt thereof: 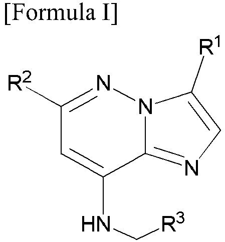 in Formula I above,
R¹ is halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-, wherein any C₁-C₆ alkoxy group, C₁-C₆ alkyl group, C₂-C₆ alkenyl group, C₂-C₆ alkynyl group, and C₃-C₆ cycloalkyl group in R¹ may be optionally substituted with halo, hydroxy, or cyano;
R² is C₆-C₁₀ aryl; a 5- or 6-membered heteroaryl containing one to two nitrogen atoms; or a monocyclic or bridged or spiro 4- to 12-membered heterocyclyl containing one nitrogen atom and linked to the imidazopyridazine ring of Formula I via said nitrogen atom, wherein the heterocyclyl may optionally contain one additional nitrogen atom or one oxygen atom, and
R² may be optionally substituted with one or more substituents selected from the group consisting of:
(i) H, halo, hydroxy, cyano, C₁-C₆ alkyl, C₃-C₆ cycloalkyl,
(ii) C₁-C₆ alkyl substituted with H, halo, hydroxy, or cyano,
(iii) C₃-C₆ cycloalkyl substituted with halo, hydroxy, or cyano,
(iv) amino, (C₁-C₆ alkyl)amino, di(C₁-C₆ alkyl)amino, (C₃-C₆ cycloalkyl)amino, (C₁-C₆ hydroxyalkyl)amino, (C₁-C₆ alkyl)carbonylamino, (C₁-C₆ hydroxyalkyl)carbonylamino, or (C₁-C₆ alkoxy)(C₁-C₆ alkyl)carbonylamino, and
(v) (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, carboxy, or (C₁-C₆ alkoxy)carbonyl; and
R³ is 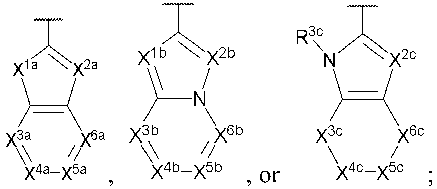
X^{1a} is NR^{3a}, O, or S, and X^{2a} is N or CR^{3a}, provided that if X^{2a} is CR^{3a}, then X^{1a} is NR^{3a}; one of X^{1b} and X^{2b} is N, and the other is CR^{3b};
X^{2c} is N or CR^{3c};
not more than one of X^{3a}, X^{4a}, X^{5a}, and X^{6a} is N, and the rest are CR^{3a};
not more than one of X^{3b}, X^{4b}, X⁵⁸, and X^{6b} is N, and the rest are CR^{3e};
not more than one of X^{3c}, X^{4c}, X^{5c} and X^{6c} is NR^{3f}, and the rest are CR^{3f}R^{3g};
R^{3a}, R^{3b}, and R^{3c} are each independently H or C₁-C₆ alkyl; and
R^{3d}, R^{3e}, R^{3f}, and R^{3g} are each independently H, halo, cyano, hydroxy, amino, (C₁-C₆ alkyl)amino, di(C₁-C₆ alkyl)amino, C₁-C₆ alkoxy, or C₁-C₆ alkyl, wherein any C₁-C₆ alkoxy group and C₁-C₆ alkyl group in R^{3d}, R^{3e}, R^{3f}, and R^{3g} may be optionally substituted with halo, cyano, or hydroxy.

2. The compound of Formula I, stereoisomer, hydrate, solvate, or pharmaceutically acceptable salt thereof according to claim 1, wherein R¹ is halo, C₁-C₆ alkyl optionally substituted with halo, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-.

3. The compound of Formula I, stereoisomer, hydrate, solvate, or pharmaceutically acceptable salt thereof according to claim 1, wherein R¹ is ethynyl, methylethynyl, cyclopropylethynyl, methyl, ethyl, isopropyl, cyclopropyl, trifluoromethyl, trifluoroethyl, iodo, or chloro.

4. The compound of Formula I, stereoisomer, hydrate, solvate, or pharmaceutically acceptable salt thereof according to claim 1, wherein R² is phenyl; pyrimidinyl, pyridinyl, pyrrolyl, or imidazolyl; azetidinyl, pyrrolidinyl, morpholinyl, piperidinyl, or piperazinyl, wherein any two non-adjacent carbon atoms of said morpholinyl, piperidinyl, or piperazinyl may be optionally linked to each other by a C₁-C₃ alkylene group to form a bridged ring; or a 7- to 11-membered azaspiro ring which contains one nitrogen atom and may optionally contain one additional nitrogen atom or one oxygen atom.

5. The compound of Formula I, stereoisomer, hydrate, solvate, or pharmaceutically acceptable salt thereof according to claim 4, wherein
the 7- to 11-membered azaspiro ring is 2-azaspiro[3.3]heptan-2-yl, 2,6-diazaspiro[3.3]heptan-2-yl, 6-oxa-2-azaspiro[3.3]heptan-2-yl, 2-azaspiro[3.4]octan-2-yl, 2,6-diazaspiro[3.4]octan-2-yl, 6-oxa-2-azaspiro[3.4]octan-2-yl, 6-azaspiro[3.4]octan-6-yl, 2,6-diazaspiro[3.4]octan-6-yl, 2-oxa-6-azaspiro[3.4]octan-6-yl, 2-azaspiro[4.4]nonan-2-yl, 2,7-diazaspiro[4.4]nonan-2-yl, or 2-oxa-7-azaspiro[4.4]nonan-7-yl, and
the bridged ring is 3-oxa-8-azabicyclo[3.2.1]octan-8-yl, 8-oxa-3-azabicyclo[3.2.1]octan-3-yl, 3,8-diazabicyclo[3.2.1]octan-3-yl, 3,8-diazabicyclo[3.2.1]octan-8-yl, 2-oxa-5-azabicyclo[2.2.2]octan-5-yl, 8-azabicyclo[3.2.1]octan-8-yl, 3-azabicyclo[3.1.1]heptan-3-yl, 6-oxa-3-azabicyclo[3.1.1]heptan-3-yl, 3,6-diazabicyclo[3.1.1]heptan-3-yl, or 3,6-diazabicyclo[3.1.1]heptan-6-yl.

6. The compound of Formula I, stereoisomer, hydrate, solvate, or pharmaceutically acceptable salt thereof according to claim 1, wherein
R² is C₆-C₁₀ aryl or a 5- or 6-membered heteroaryl containing one to two nitrogen atoms, wherein the aryl and heteroaryl are optionally substituted with R^{2d} selected from the group consisting of H, hydroxy, amino, (C₁-C₆ alkyl)amino, di(C₁-C₆ alkyl)amino, and (C₃-C₆ cycloalkyl)amino; or
R² is selected from Formula A or Formula B below: 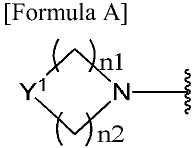
in Formula A above,
n1 and n2 are each independently 1 or 2, and a carbon atom of the ring may be optionally substituted with C₁-C₆ alkyl, and
if both n1 and n2 are 2, then Y¹ is CR^{2a}R^{2b}, NR^{2c}, or O, and any two non-adjacent carbon atoms of the ring may be optionally linked to each other by a C₁-C₃ alkylene group to form a bridged ring, and
if one or both of n1 and n2 are 1, then Y¹ is CR^{2a}R^{2b}; 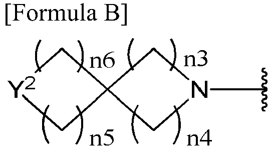
in Formula B above,
n3, n4, n5 and n6 are each independently 1 or 2, and a carbon atom of the ring may be optionally substituted with C₁-C₆ alkyl, and
Y² is CR^{2a}R^{2b}, NR^{2c} , or O,
in Formulae A and B above, R^{2a} and R^{2b} are each independently selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy; and R^{2c} is H, C₁-C₆ alkyl, C₃-C₆ cycloalkyl, (C₁-C₆ alkyl)carbonyl, (C₁-C₆ hydroxyalkyl)carbonyl, aminocarbonyl, (C₁-C₆ alkyl)aminocarbonyl, or di(C₁-C₆ alkyl)aminocarbonyl.

7. The compound of Formula I, stereoisomer, hydrate, solvate, or pharmaceutically acceptable salt thereof according to claim 6, wherein
one of R^{2a} and R^{2b} is selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy, and
the other of R^{2a} and R^{2b} is H, halo, hydroxy, cyano, or C₁-C₆ alkyl.

8. The compound of Formula I, stereoisomer, hydrate, solvate, or pharmaceutically acceptable salt thereof according to claim 6, wherein
R^{2a} and R^{2b} are selected from the group consisting of H, hydroxy, amino, cyclopropylamino, hydroxyacetamido, methyl, hydroxymethyl, 2-hydroxy-isopropyl, hydroxycyclopropyl, carboxy, carbamoyl, hydroxyacetyl, and cyano,
R^{2c} is selected from the group consisting of H, methyl, cyclopropyl, acetyl, hydroxyacetyl, and carbamoyl, and
R^{2d} is selected from the group consisting of hydroxy and amino.

9. The compound of Formula I, stereoisomer, hydrate, solvate, or pharmaceutically acceptable salt thereof according to claim 6, wherein R² is selected from the group consisting of: 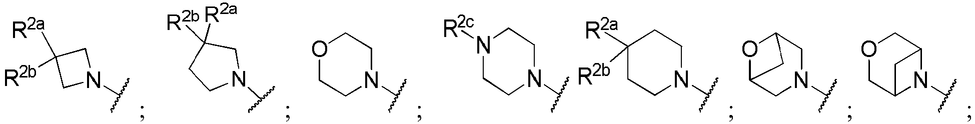 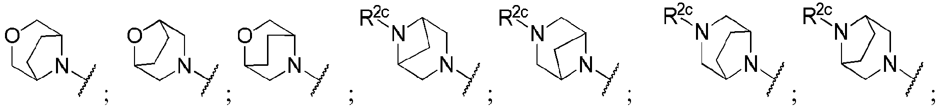 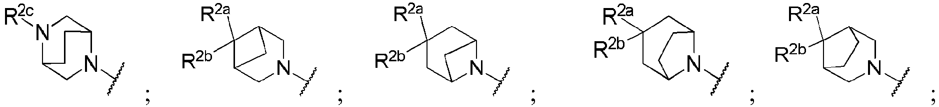 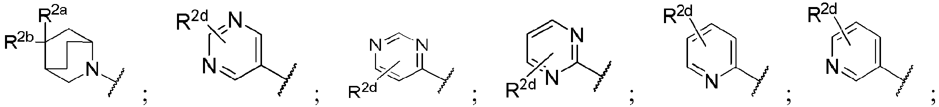 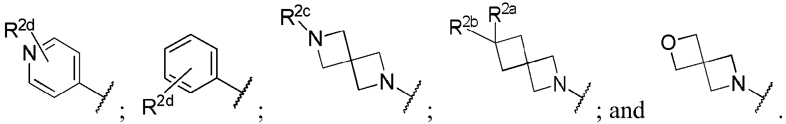 (In the above structures, R^{2a}, R^{2b}, R^{2c}, and R^{2d} are the same as described in claim 6.)

10. The compound of Formula I, stereoisomer, hydrate, solvate, or pharmaceutically acceptable salt thereof according to claim 1, wherein R² is selected from the group consisting of:

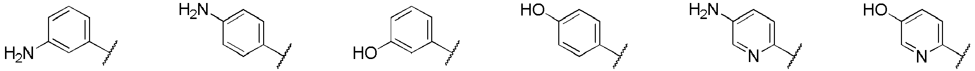

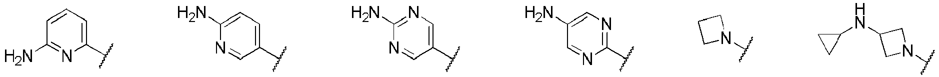

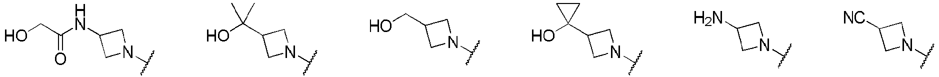

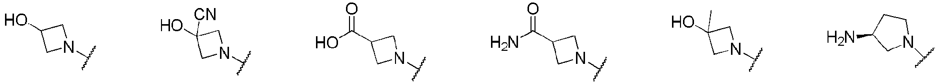

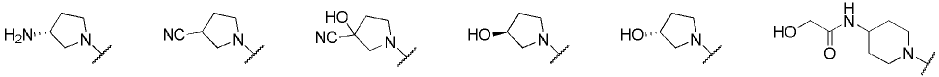

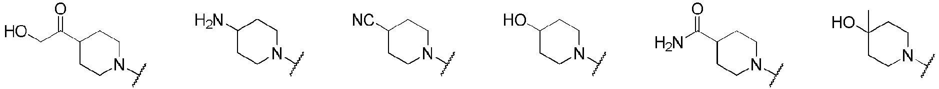

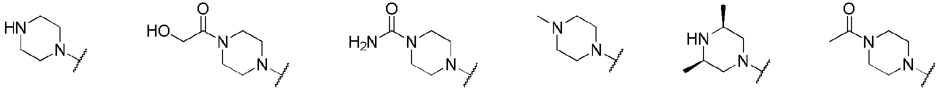

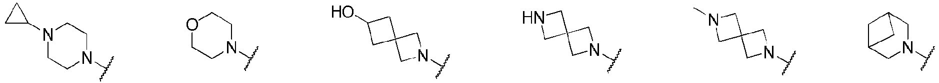

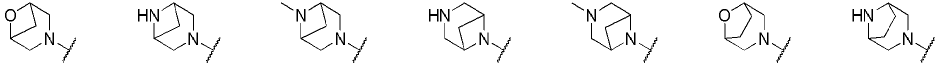

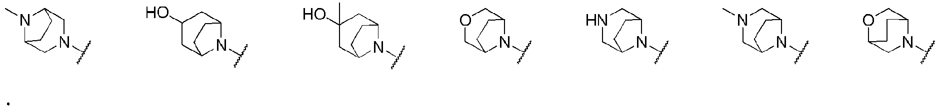

11. The compound of Formula I, stereoisomer, hydrate, solvate, or pharmaceutically acceptable salt thereof according to claim 1, wherein R³ is selected from the group consisting of: 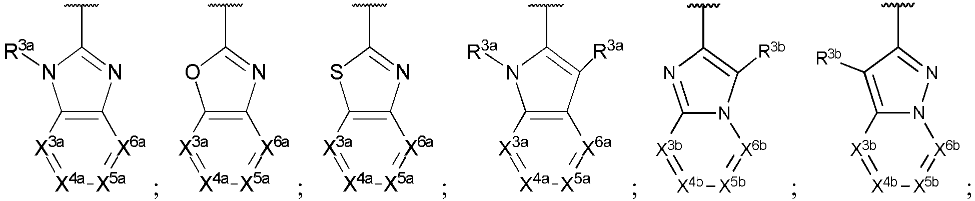 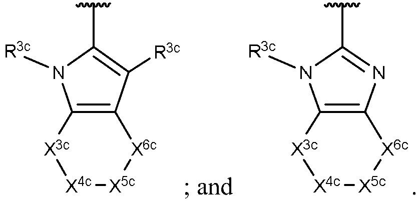
(In the above structures, not more than one of x^{3a}, X^{4a}, X^{5a}, and X^{6a} is N, and the rest are CR^{3d}; not more than one of X^{3b}, X^{4b}, X^{5b}, and X^{6b} is N, and the rest are CR^{3e}; and not more than one of X^{3c}, X^{4c} , X^{5c} and X^{6c} is NR^{3f}, and the rest are CR^{3f}R^{3g}, and
R^{3a}, R^{3b}, and R^{3c} are each independently H or C₁-C₆ alkyl; and R^{3d}, R^{3e}, R^{3f}, and R^{3g} are each independently H, halo, cyano, hydroxy, amino, (C₁-C₆ alkyl)amino, di(C₁-C₆ alkyl)amino, C₁-C₆ alkoxy, or C₁-C₆ alkyl, wherein any C₁-C₆ alkoxy group and C₁-C₆ alkyl group in R^{3d}, R^{3e}, R^{3f}, and R^{3g} may be optionally substituted with halo, cyano, or hydroxy.)

12. The compound of Formula **I,** stereoisomer, hydrate, solvate, or pharmaceutically acceptable salt thereof according to claim 11, wherein R³ is selected from the group consisting of: 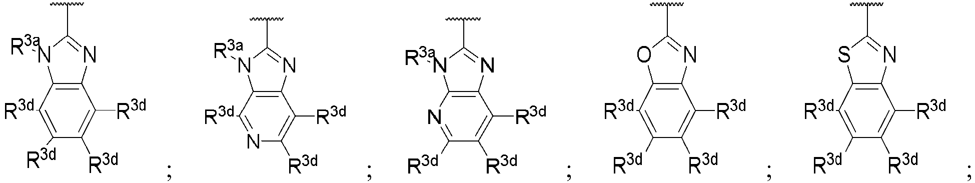 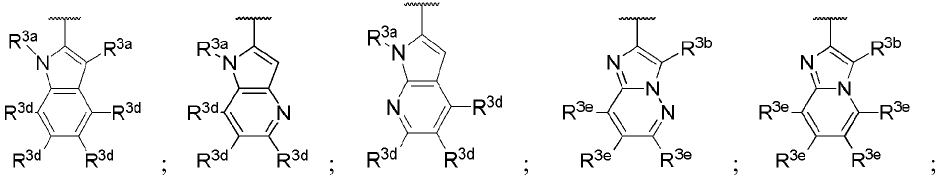 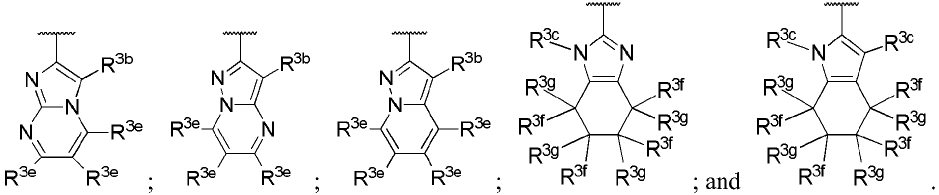
(In the above structures, R^{3a}, R^{3b}, and R^{3c} are each independently H or C₁-C₆ alkyl; and
R^{3d}, R^{3e}, R^{3f}, and R^{3g} are each independently H, halo, hydroxy, amino, C₁-C₆ alkoxy, C₁-C₆ alkyl, or C₁-C₆ haloalkyl.)

13. The compound of Formula I, stereoisomer, hydrate, solvate, or pharmaceutically acceptable salt thereof according to claim 12, wherein R^{3d}, R^{3e}, R^{3f}, and R^{3g} are each independently selected from the group consisting of H, F, Cl, hydroxy, amino, methoxy, methyl, and trifluoromethyl.

14. The compound of Formula I, stereoisomer, hydrate, solvate, or pharmaceutically acceptable salt thereof according to claim 1, wherein R³ is selected from the group consisting of:

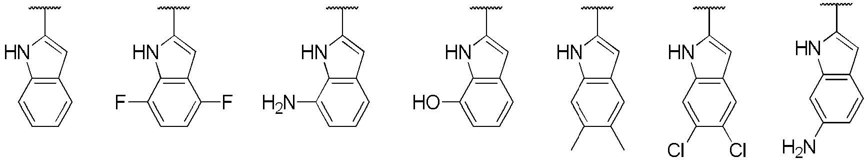

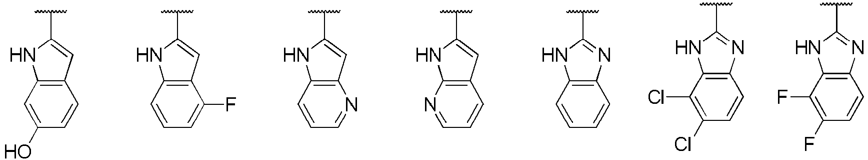

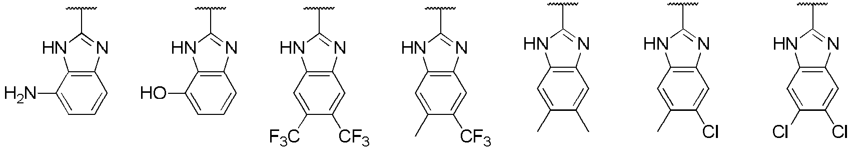

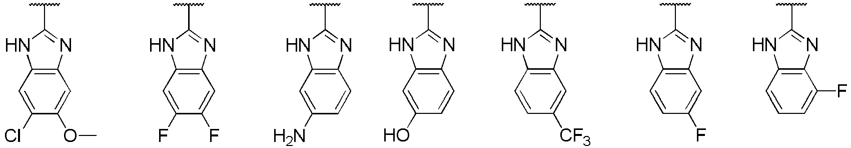

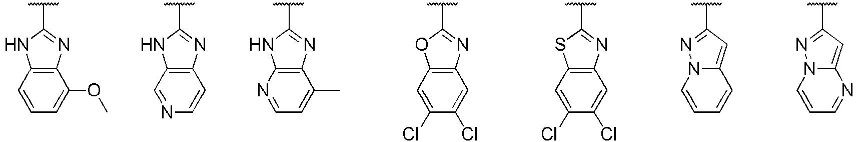

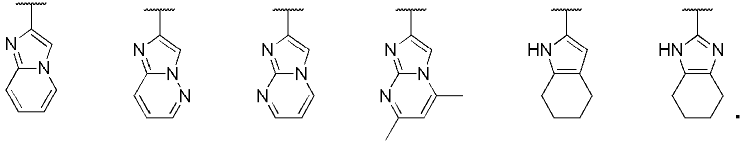

15. The compound of Formula I, stereoisomer, hydrate, solvate, or pharmaceutically acceptable salt thereof according to claim 1, wherein the compound is represented by Formula IA below: 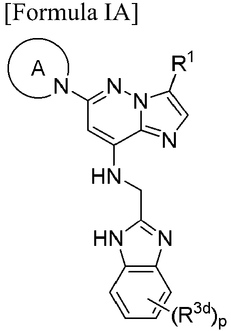 in Formula IA above,
R¹ is halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-, wherein any C₁-C₆ alkoxy group, C₁-C₆ alkyl group, C₂-C₆ alkenyl group, C₂-C₆ alkynyl group, and C₃-C₆ cycloalkyl group in R¹ may be optionally substituted with halo, hydroxy, or cyano; and
Ring A is a monocyclic or bridged or spiro 4- to 12-membered heterocyclyl which may optionally contain one additional nitrogen atom or one oxygen atom; and
Ring A may be optionally substituted with one or more substituents selected from the group consisting of:
(i) H, halo, hydroxy, cyano, C₁-C₆ alkyl, C₃-C₆ cycloalkyl,
(ii) C₁-C₆ alkyl substituted with halo, hydroxy, or cyano,
(iii) C₃-C₆ cycloalkyl substituted with halo, hydroxy, or cyano,
(iv) amino, (C₁-C₆ alkyl)amino, di(C₁-C₆ alkyl)amino, (C₃-C₆ cycloalkyl)amino, (C₁-C₆ hydroxyalkyl)amino, (C₁-C₆ alkyl)carbonylamino, (C₁-C₆ hydroxyalkyl)carbonylamino, or (C₁-C₆ alkoxy) (C₁-C₆ alkyl)carbonylamino, and
(v) (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, carboxy, or (C₁-C₆ alkoxy)carbonyl;
R^{3d} is H, halo, cyano, hydroxy, amino, (C₁-C₆ alkyl)amino, di(C₁-C₆ alkyl)amino, C₁-C₆ alkoxy, or C₁-C₆ alkyl, wherein any C₁-C₆ alkoxy group and C₁-C₆ alkyl group in R^{3d} may be optionally substituted with halo, cyano, or hydroxy; and
p is an integer from 0 to 3.

16. The compound of Formula I, stereoisomer, hydrate, solvate, or pharmaceutically acceptable salt thereof according to claim 1, wherein the compound is represented by Formula IB below: 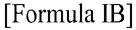 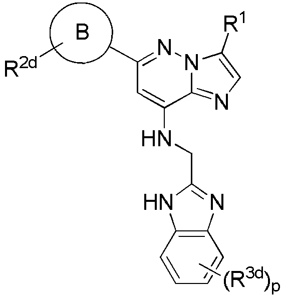 in the above formula,
R¹ is halo, hydroxy, cyano, C₁-C₆ alkoxy, C₁-C₆ alkyl, C₂-C₆ alkenyl, C₂-C₆ alkynyl, C₃-C₆ cycloalkyl, C₃-C₆ cycloalkyl-C₁-C₆ alkyl-, C₃-C₆ cycloalkyl-C₂-C₆ alkenyl-, or C₃-C₆ cycloalkyl-C₂-C₆ alkynyl-, wherein any C₁-C₆ alkoxy group, C₁-C₆ alkyl group, C₂-C₆ alkenyl group, C₂-C₆ alkynyl group, and C₃-C₆ cycloalkyl group in R¹ may be optionally substituted with halo, hydroxy, or cyano;
Ring B is selected from 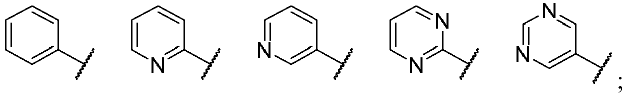
R^{2d} is H, hydroxy, amino, (C₁-C₆ alkyl)amino, di(C₁-C₆ alkyl)amino, or (C₃-C₆ cycloalkyl)amino;
R^{3d} is H, halo, cyano, hydroxy, C₁-C₆ alkoxy, or C₁-C₆ alkyl, wherein any C₁-C₆ alkoxy group and C₁-C₆ alkyl group in R^{3d} may be optionally substituted with halo, cyano, or hydroxy; and
p is an integer from 0 to 3.

17. The compound of Formula I, stereoisomer, hydrate, solvate, or pharmaceutically acceptable salt thereof according to claim 16, wherein 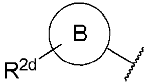 is 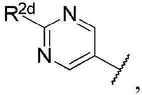 and
R^{2d} is amino, (C₁-C₆ alkyl)amino, or di(C₁-C₆ alkyl)amino.

18. The compound of Formula I, stereoisomer, hydrate, solvate, or pharmaceutically acceptable salt thereof according to claim 1, wherein the compound is represented by Formula IC-1 or Formula IC-2 below: 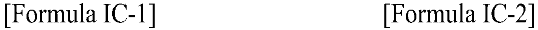 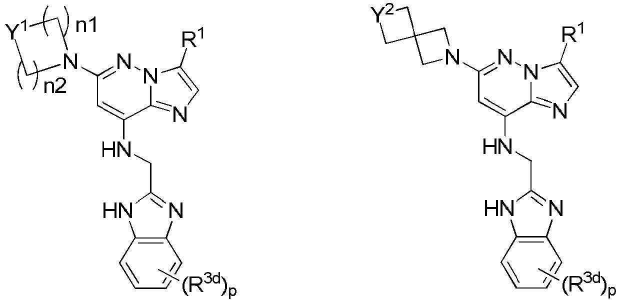 in Formula IC-1 above,
n1 and n2 are each independently 1 or 2, and a carbon atom of the ring containing Y¹ and N may be optionally substituted with C₁-C₆ alkyl, and
if both n1 and n2 are 2, then Y¹ is CR^{2a}R^{2b}, NR^{2c}, or O, and
if one or both of n1 and n2 are 1, then Y¹ is CR^{2a}R^{2b};
in Formula IC-2 above,
Y² is CR^{2a}R^{2b} , NR^{2c}, or O;
in Formulae IC-1 and IC-2 above,
R¹ is halo, hydroxy, cyano, C₁-C₆ alkoxy, C₁-C₆ alkyl, or C₁-C₆ haloalkyl,
R^{3d} is each independently H, halo, hydroxy, cyano, C₁-C₆ alkoxy, C₁-C₆ alkyl, or C₁-C₆ haloalkyl, and
p is an integer from 0 to 2;
R^{2a} and R^{2b} are each independently selected from the group consisting of H, halo, hydroxy, cyano, C₁-C₆ alkyl, or C₃-C₆ cycloalkyl; C₁-C₆ alkyl substituted with halo, hydroxy, or cyano; C₃-C₆ cycloalkyl substituted with halo, hydroxy, or cyano; amino, (C₁-C₆ alkyl)amino, di(C₁-C₆ alkyl)amino, (C₃-C₆ cycloalkyl)amino, (C₁-C₆ alkyl)carbonylamino, (C₁-C₆ hydroxyalkyl)carbonylamino; and (C₁-C₆ alkyl)carbonyl, (C₁-C₆ hydroxyalkyl)carbonyl, aminocarbonyl, (C₁-C₆ alkyl)aminocarbonyl, di(C₁-C₆ alkyl)aminocarbonyl, and carboxy; and
R^{2c} is H, C₁-C₆ alkyl, C₃-C₆ cycloalkyl, (C₁-C₆ alkyl)carbonyl, (C₁-C₆ hydroxyalkyl)carbonyl, aminocarbonyl, (C₁-C₆ alkyl)aminocarbonyl, or di(C₁-C₆ alkyl)aminocarbonyl.

19. The compound of Formula I, stereoisomer, hydrate, solvate, or pharmaceutically acceptable salt thereof according to claim 1, wherein the compound is selected from the group consisting of:

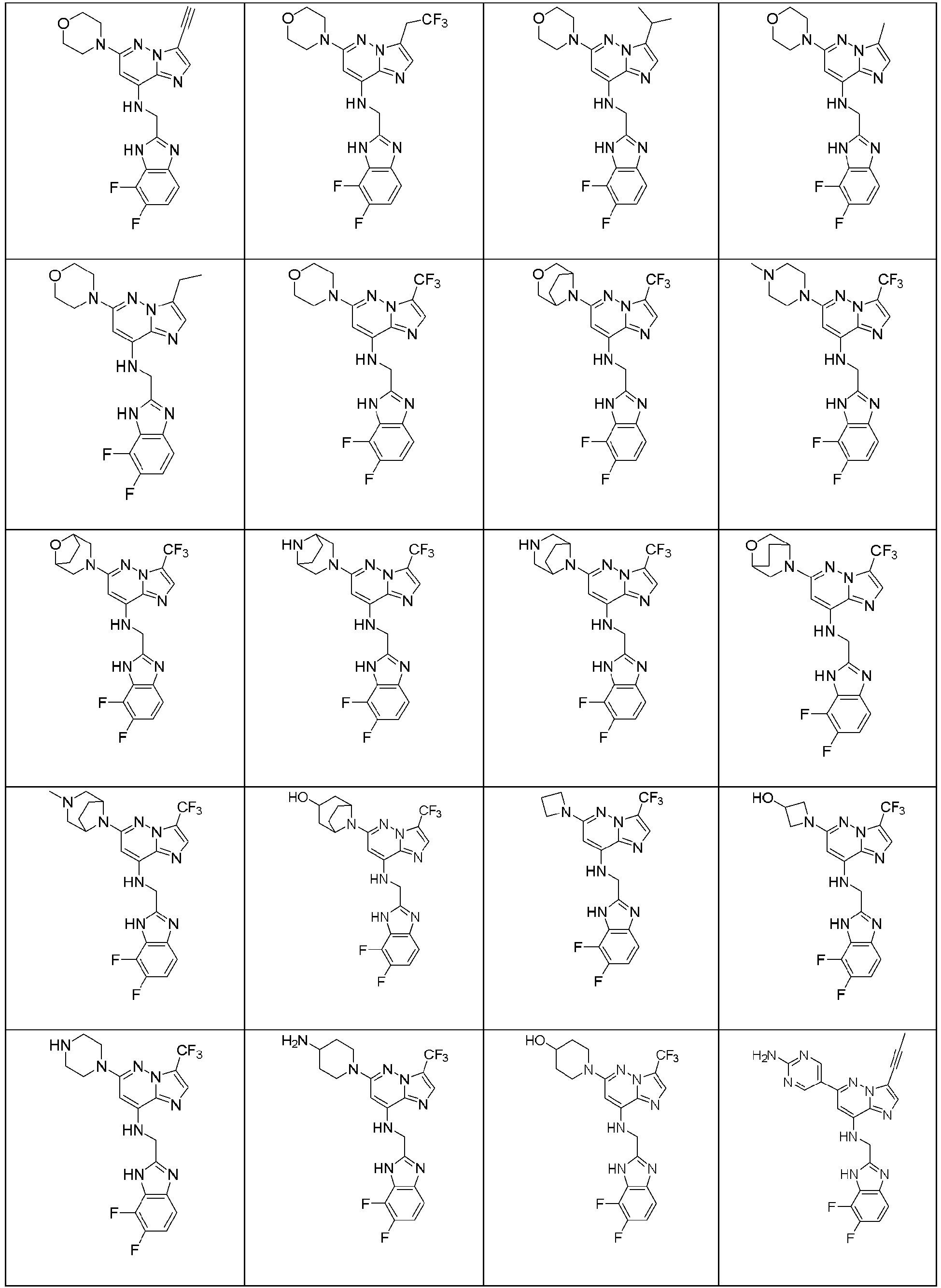

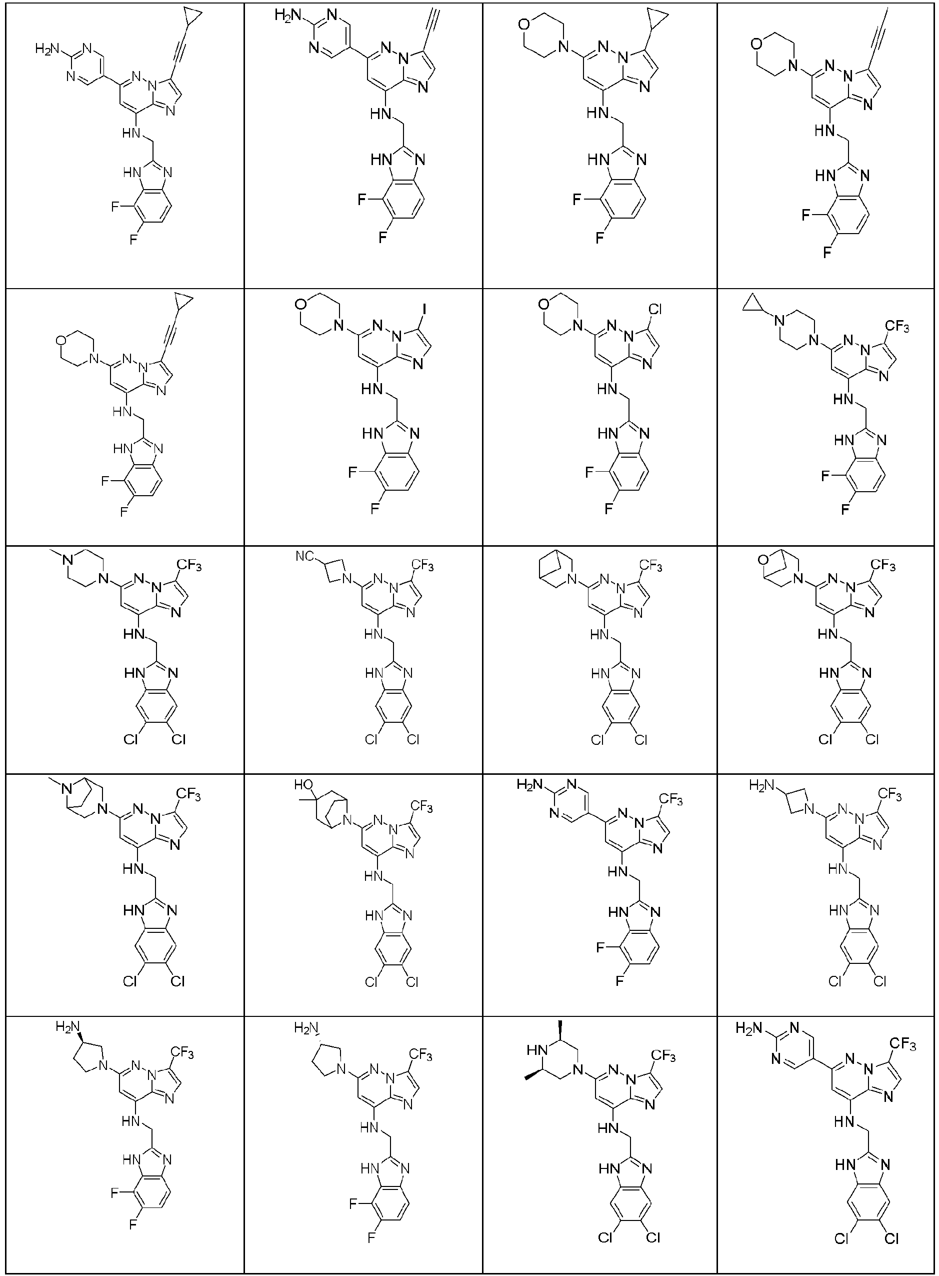

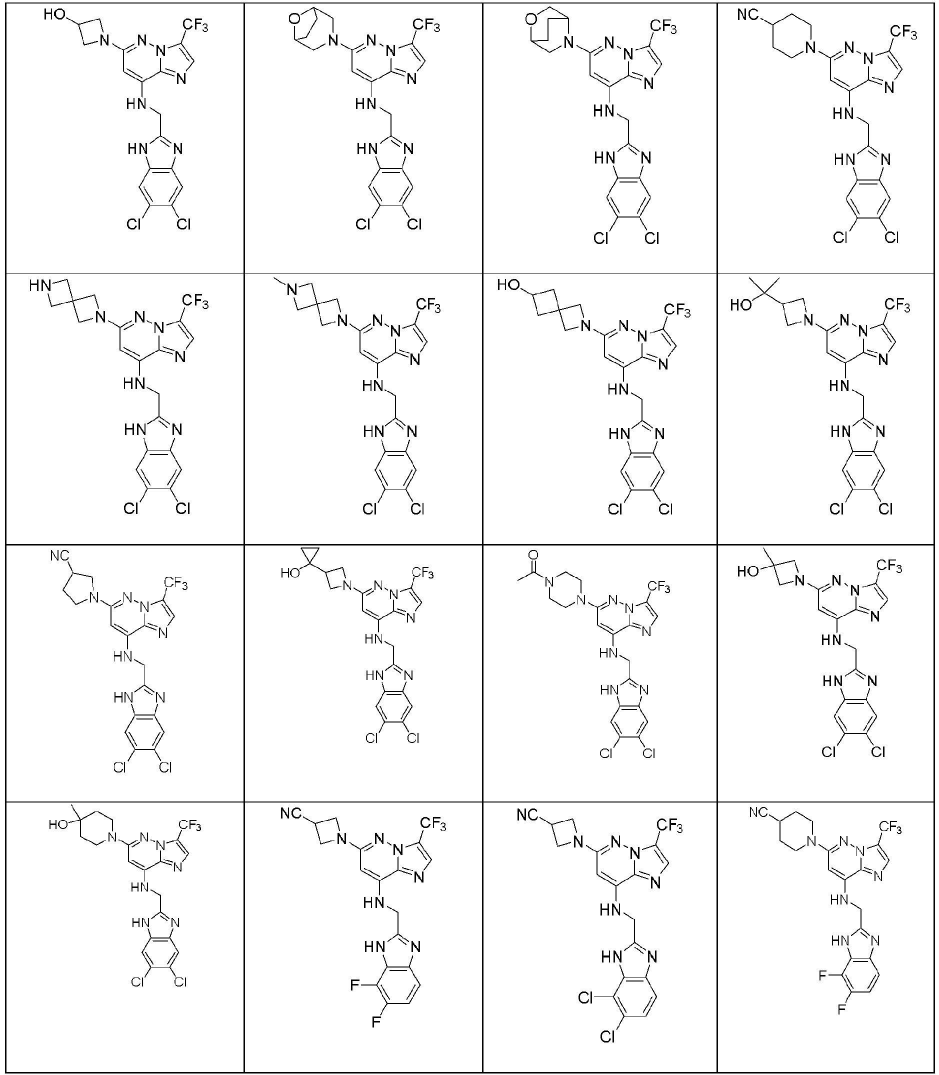

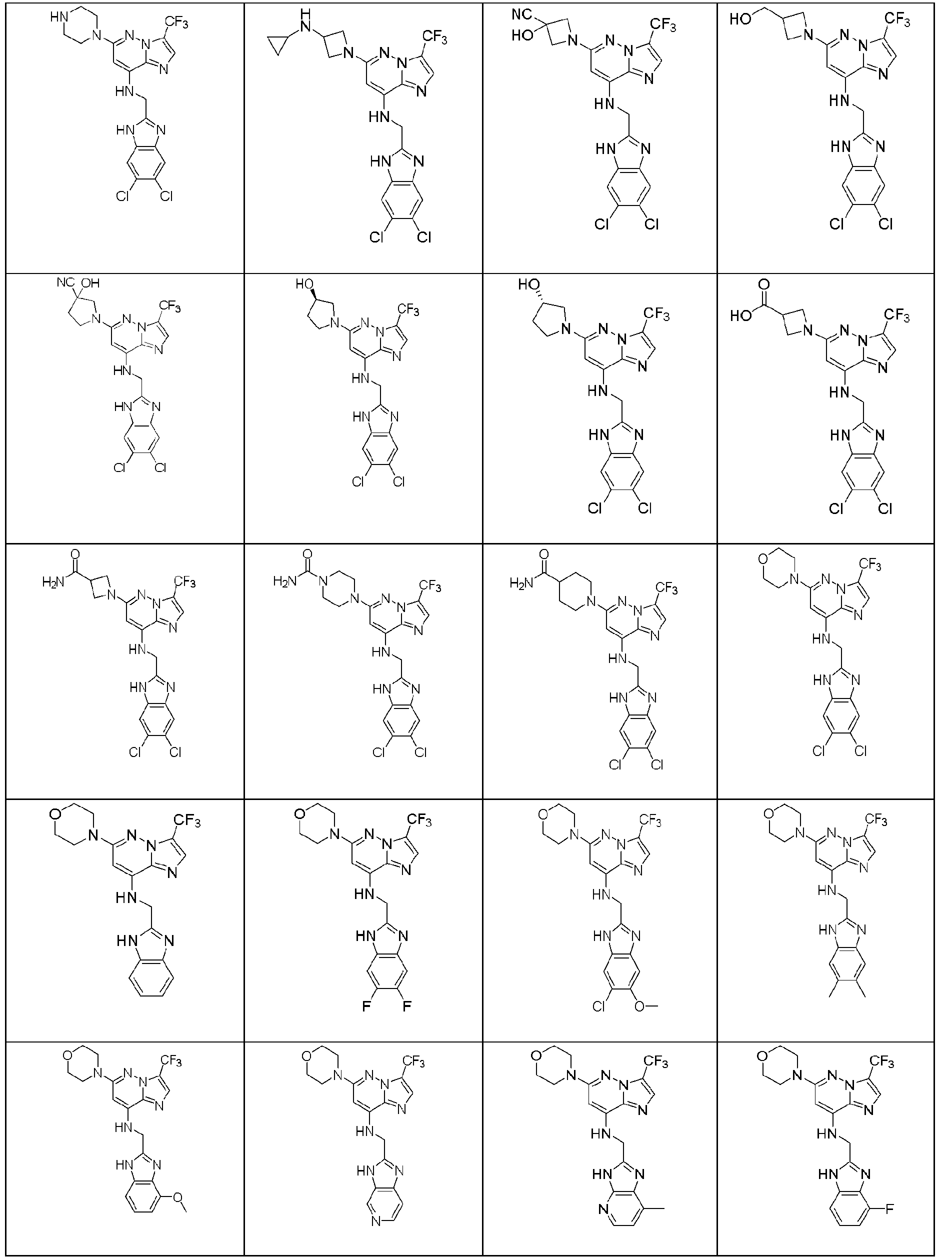

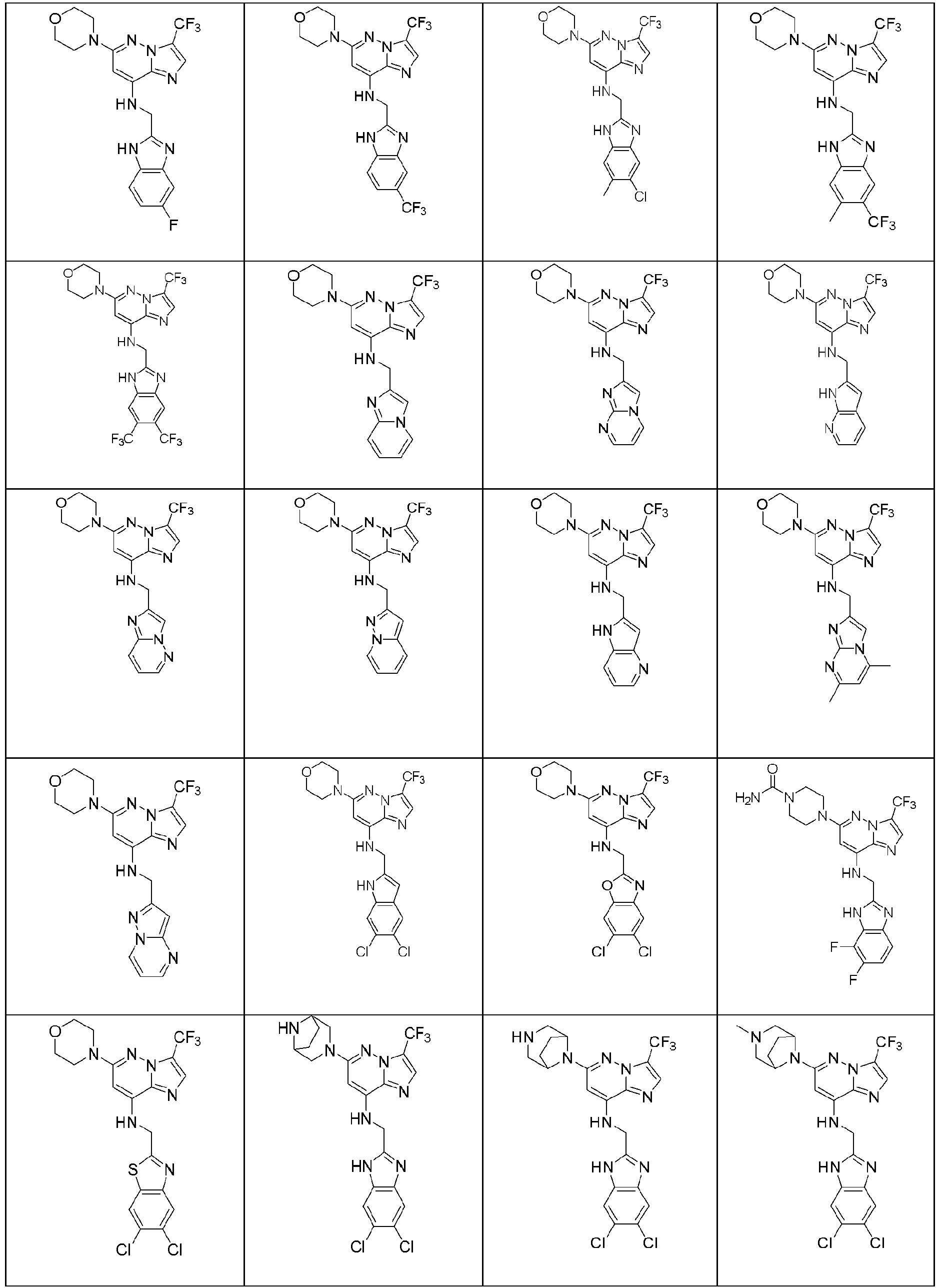

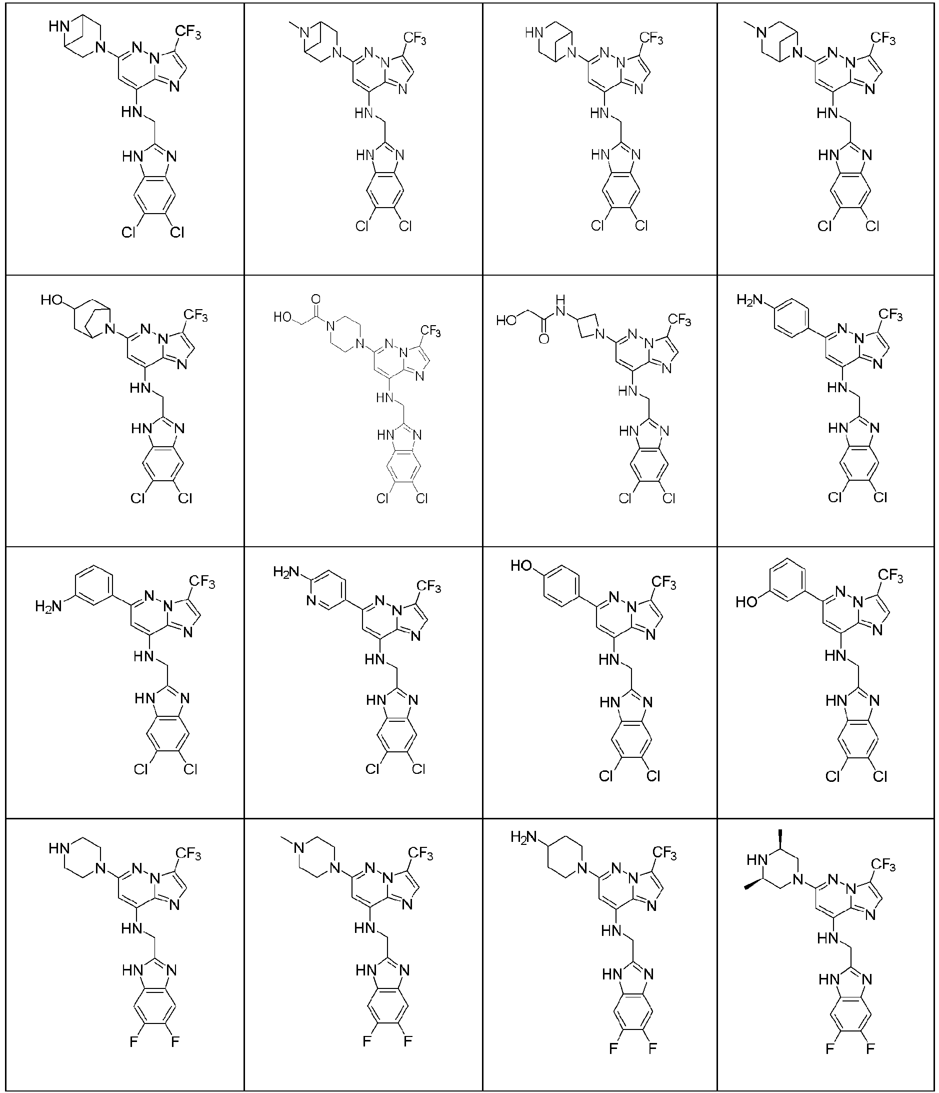

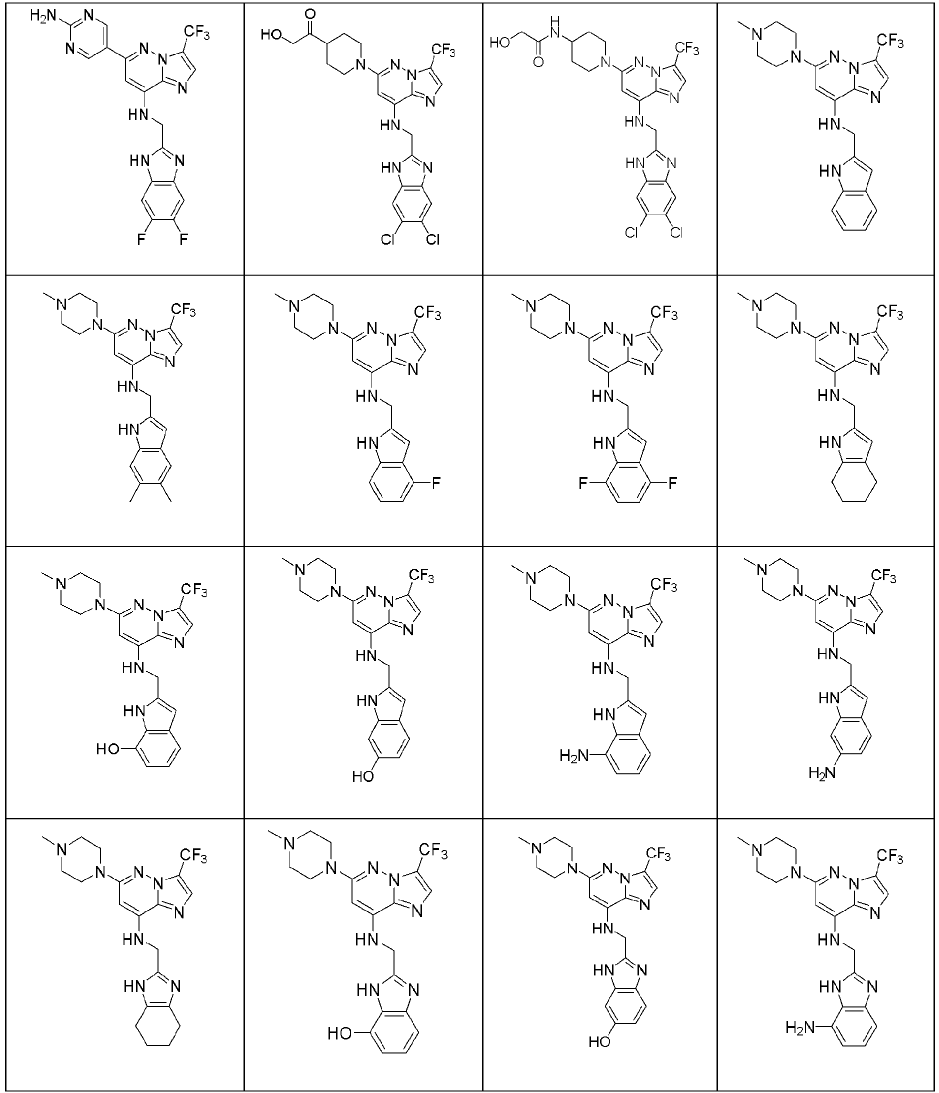

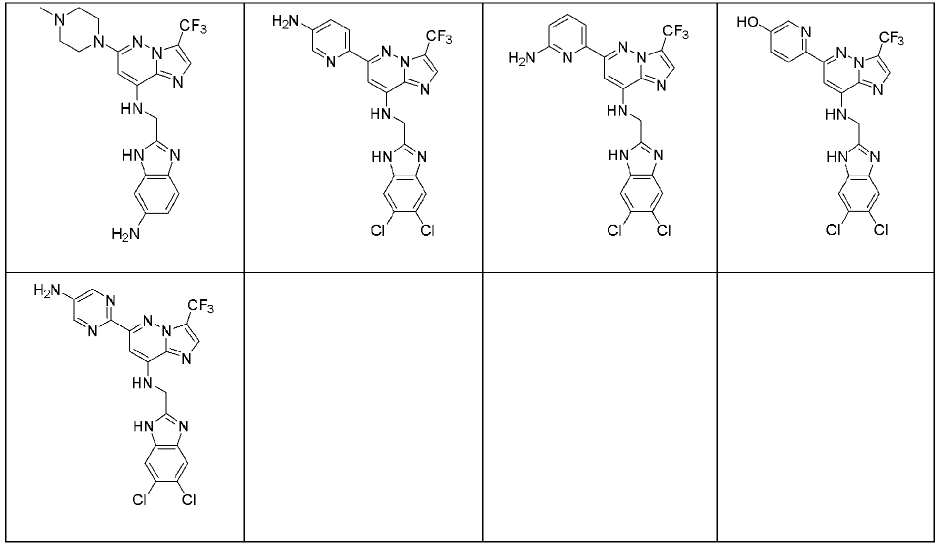

20. A pharmaceutical composition comprising the compound, stereoisomer, hydrate, solvate, or pharmaceutically acceptable salt thereof according to any one of claims 1 to 19 as an active ingredient.

21. The pharmaceutical composition according to claim 20, wherein the pharmaceutical composition is used for the treatment of a disease caused by overexpression, excessive activity, mutation of cyclin dependent protein kinase (CDK) 12 and/or CDK13, and/or activation of a signaling pathway associated with cyclin K.

22. The pharmaceutical composition according to claim 21, wherein the disease is cancer, a proliferative disease, a neurodegenerative disease, an autoimmune disease, or a disease caused by an abnormality in intracellular protein translation function.

23. The pharmaceutical composition according to claim 22, wherein the disease is cancer.

24. The pharmaceutical composition according to claim 23, wherein the cancer is selected from the group consisting of breast cancer, ovarian cancer, colorectal cancer, lung cancer, prostate cancer, gastric cancer, liver cancer, kidney cancer, bladder cancer, pancreatic cancer, head and neck cancer, thyroid cancer, skin cancer, bile duct cancer, esophageal cancer, hematopoietic tumors of the lymphoid and myeloid systems, tumors of the central and peripheral nervous systems, and sarcoma.

25. The pharmaceutical composition according to claim 23, wherein the pharmaceutical composition is used for the treatment of high-grade cancers or cancers that have become resistant to treatment with previously administered therapeutic agents.

26. The pharmaceutical composition according to claim 23, wherein the pharmaceutical composition is administered in combination with one or more therapeutic agents selected from the group consisting of radiation therapy, a taxane derivative, a platinum compound, an antimetabolite, anti-CTLA4 therapy, anti-PD1 therapy, anti-PD-L1 therapy, anti-VEGF therapy, anti-EGFR therapy, a topoisomerase inhibitor, anti-HER2 therapy, anti-hormone therapy, an estrogen receptor inhibitor, an ERK inhibitor, a PARP inhibitor, an mTOR inhibitor, a CDK4/6 inhibitor, an EGFR inhibitor, a HER2 inhibitor, an ALK inhibitor, a tyrosine kinase inhibitor, a MEK inhibitor, a BCR-ABL inhibitor, a PI3K inhibitor, an FGFR inhibitor, a ROS1 inhibitor, an androgen biosynthesis inhibitor, an androgen receptor inhibitor, a Hedgehog inhibitor, a MET inhibitor, an AXL inhibitor, an NTRK1 inhibitor, a RET inhibitor, a KRAS inhibitor, and a RAF inhibitor.

27. The pharmaceutical composition according to claim 22, wherein
the neurodegenerative disease is Alzheimer's disease, Parkinson's disease, or Lou Gehrig's disease; and
the autoimmune disease is rheumatoid arthritis, systemic lupus erythematosus, psoriasis, or Sjögren's syndrome.

28. A method for treating a disease caused by overexpression, excessive activity, mutation of CDK12 and/or CDK13, and/or activation of a signaling pathway associated with cyclin K, comprising administering the compound, stereoisomer, hydrate, solvate, or pharmaceutically acceptable salt thereof according to any one of claims 1 to 19 to a subject.

29. A method for inhibiting CDK12 and/or CDK13 or degrading cyclin K, comprising contacting an effective amount of the compound, stereoisomer, hydrate, solvate, or pharmaceutically acceptable salt thereof according to any one of claims 1 to 19, with a cyclin dependent kinase.
